(19) Europäisches Patentamt
European Patent Office
Office européen des brevets

(11) EP 4 574 175 A1

(12) EUROPEAN PATENT APPLICATION
published in accordance with Art. 153(4) EPC

(43) Date of publication:
**25.06.2025 Bulletin 2025/26**

(21) Application number: **23853874.8**

(22) Date of filing: **24.03.2023**

(51) International Patent Classification (IPC):
***A61K 47/68*** *(2017.01)* ***A61K 31/4725*** *(2006.01)*
***A61P 43/00*** *(2006.01)* ***A61P 11/00*** *(2006.01)*
***A61P 35/00*** *(2006.01)*

(52) Cooperative Patent Classification (CPC):
**A61K 31/4725; A61K 47/68; A61P 11/00; A61P 35/00; A61P 43/00**

(86) International application number:
**PCT/CN2023/083593**

(87) International publication number:
**WO 2024/036961 (22.02.2024 Gazette 2024/08)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **19.08.2022 CN 202210997622**

(71) Applicant: **BEIJING SUNCELL BIO-MEDICAL CO., LTD.**
**Beijing 100085 (CN)**

(72) Inventors:
- **DENG, Hongkui**
  **Beijing 100085 (CN)**
- **DONG, Jiebin**
  **Beijing 100085 (CN)**
- **LI, Honggang**
  **Beijing 100085 (CN)**

(74) Representative: **Hoffmann Eitle**
**Patent- und Rechtsanwälte PartmbB**
**Arabellastraße 30**
**81925 München (DE)**

# (54) PRODRUG FOR ANTI-AGING RELATED DISEASES AND USE METHOD THEREFOR

(57) Provided is an anti-senescence and anti-inflammatory prodrug, which is designed from a cytotoxic agent by chemically modifying the cytotoxic agent to incorporate a site that can be cleaved by SA-β-gal after the delivery of the prodrug *in vivo* to release an active parent compound. The prodrug comprises a galactosyl moiety, a benzyloxycarboxyl and a cytotoxic moiety that can be preferably modified. The anti-senescence prodrug is used to selectively kill one or more senescent cells and/or reduce an acute or chronic inflammatory response in a subject in need thereof by administering a therapeutically effective amount of the anti-senescence prodrug to the subject. The disclosed compositions can be used to alleviate an aging-related disease or disorder or an inflammatory disorder such as idiopathic pulmonary fibrosis and an age-related ocular lesion in a subject.

EP 4 574 175 A1

## Description

### TECHNICAL FIELD

The present invention relates to a compound capable of selectively killing senescent cells rather than non-senescent cells, *i.e.,* an anti-senescence compound, and a method of using the same.

### BACKGROUND

Aging is a major risk factor for a physiological degeneration, an increased incidence of chronic diseases and an age-related mortality (Lopez-Otin, et al., Cell 153, 1194-1217 (2013)). During aging, the senescent cells accumulate in multiple tissues and cause a tissue dysfunction (van Deursen, et al., Nature 509, 439-446 (2014); and McHugh, et al., J Cell Biol 217, 65-77 (2018)). The senescent cells can also secrete a variety of pro-inflammatory factors called the senescence-associated secretory phenotypes (SASPs), which lead to an age-related physical decline (Coppe, et al., PLoS Biol 6, 2853-2868 (2008); and Coppe, et al., Annu Rev Pathol 5, 99-118 (2010)). Cellular senescence can also impair the ability of tissues to regenerate and lead to a chronic low-grade inflammation, thereby exacerbating the aging process. Removal of the senescent cells has emerged as an attractive potential approach to ameliorate the age-related diseases and improve health (Xu et al., Nat Med 24, 1246-1256 (2018); Baker et al., Nature 479, 232-236 (2011); and Baker et al., Nature 530, 184-189 (2016)). There is still a need to develop a new strategy that allows for the selective removal of the senescent cells in a broad spectrum of cell types or tissues for anti-aging interventions.

However, specifically and effectively removing multiple types of the senescent cells remains challenging due to the complexity and heterogeneity of the senescent cells (Kirkland, et al., J Am Geriatr Soc 65, 2297-2301 (2017); and Lozano-Torres, et al., Nature Reviews Chemistry 3, 426- 441 (2019)). The compound that selectively kills the senescent cells, called 'senolytics', has attracted considerable interest and revealed the anti-apoptotic pathway (Zhu, et al., Aging Cell 14, 644-658 (2015), and Chang, et al., Nat Med 22, 78-83 (2016)). The BCL-2 family inhibitors (Zhu, et al., Aging Cell 15, 428-435 (2016)), the HSP90 inhibitors (Fuhrmann-Stroissnigg, et al., Nat Commun 8, 422 (2017)) and the FOXO4-p53 complex (Baar, et al., Cell 169, 132-147 e116 (2017)), and the like can be targeted to achieve this goal. The BCL-2 family, particularly the BCL-XL gene, is specifically highly expressed in a variety of senescent cells (He, et al., Nat Commun 11, 1996 (2020)) and plays a central role in the regulation of programmed cell death through the control of intracellular pro-apoptotic and anti-apoptotic signal (Czabotar, P. E, et al., Nat Rev Mol Cell Biol 15, 49-63 (2014)). Despite the successful preclinical proof-of-concept for 'senolytics', its potential drug translatability is limited by its associated toxicity, for example, the anti-senescence effects of the BCL-2 family inhibitors have been validated in a variety of preclinical models, and it also has a significant on-target hematologic toxicity, including thrombocytopenia (Cang, et al., Journal of Hematology & Oncology 8, 129 (2015)). Therefore, there is a need to develop more refined and novel strategies to improve the efficacy and specificity against the senescent cells and to reduce the toxicity.

Another strategy for targeting senescence is to capitalize on the distinction between the senescent and normal cells. A conserved and well-defined feature of the senescent cells is that the lysosomes and the lysosomal proteins are enriched in them, including the senescence-associated β-galactosidase (SA-β-gal), which is widely used as an senescence marker (Hernandez-Segura, et al., Trends in Cell Biology 28, 436-453 (2018)). Drugs and macromolecular agents can be preferentially released in the senescent cells after cleavage by β-galactosidase (Ana Guerrero, et al., Aging Cell 19:e13133 (2020)). Galactose conjugation is already a versatile therapeutic and detection strategy that facilitates the preferential targeting of the senescent cells through the use of a variety of available formulations, including the modular systems, nanocarriers, activatable prodrugs, probes and small molecules. Galactose conjugation strategy can therefore be utilized to design more specific and innovative anti-senescence drugs.

### SUMMARY OF THE INVENTION

One object of the present invention is to provide a composition for selectively targeting the senescent cells.

Another object of the present invention is to provide a composition for alleviating an inflammation in a subject in need thereof.

Another object of the present invention is to provide a method for selectively killing one or more senescent cells in a subject in need thereof.

A further object of the present invention is to provide a method for ameliorating one or more symptoms associated with an aging-related disorder in a subject in need thereof.

Another object of the present invention is to provide a method for ameliorating one or more symptoms associated with a virus-induced inflammation.

Provided is a prodrug with the anti-senescence and anti-inflammatory effects. The prodrug is designed from a cytotoxic agent (a parent cytotoxic agent) by chemically modifying the cytotoxic agent to incorporate a site that can be

cleaved by SA-β-gal (to release the active parent cytotoxic agent). The prodrug is then delivered *in vivo* so as to preferentially kill the senescent cells. The prodrug comprises a galactosyl moiety preferably modified by acetylation (referred to herein as a modified galactose moiety), a benzyloxycarbonyl, and a cytotoxic moiety (provided by the parent cytotoxic agent). In a preferred embodiment, the cytotoxic parent cytotoxic agent used to prepare the prodrug is:

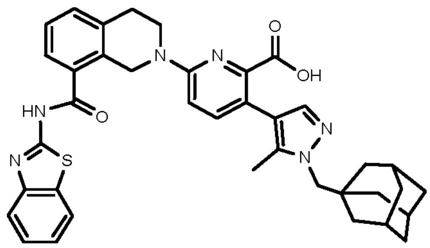

A1331852

In an embodiment, the prodrug may be in a crystalline form.

A preferred acetylated galactose moiety is D-galactose tetraacetate moiety, as shown below.

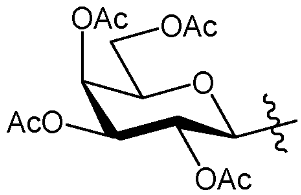

In another embodiment, one or more (*e.g.*, two, three, or four) acetyl (Ac) groups may be removed from the galactosyl moiety.

Particularly preferred benzyloxycarbonyl is shown below.

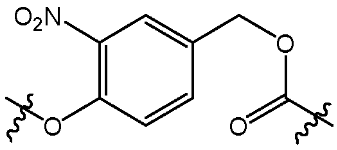

3-nitro-4-oxy-benzylcarboxyl

In another embodiment, $NO_2$ may be removed from the benzyloxycarbonyl.

Further disclosed is a method for selectively killing one or more senescent cells in a subject in need thereof. The method comprises administering to the subject a therapeutically effective amount of one or more of the disclosed anti-senescence prodrugs. In some embodiments, the active agent selectively kills the cells undergoing the oncogene-induced senescence, the drug-induced senescence, the age-induced senescence, the disease-associated senescence and/or the radiation-induced senescence.

Further provided is a method for alleviating an inflammation in a subject in need thereof. The disclosed composition may be used to ameliorate the symptoms associated with the pro-inflammatory conditions, such as the accumulation of the over-activated macrophages (and the reduction of the associated cytokines). In a particularly preferred embodiment, the disclosed composition may be administered to the subject in need thereof to reduce an inflammatory response associated with a viral infection, such as a coronavirus (CoV) infection, and more specifically, a SARS-CoV or SARS-CoV2 infection. In this embodiment, the composition is administered in an effective amount to reduce one or more macrophages, preferably SA-β-gal positive macrophages, in the subject.

The disclosed composition may be used to ameliorate one or more symptoms associated with an aging-related disease or discorder in a subject and/or one or more inflammation-related discorders in a subject involving a long list of other pathologies, including neuropathology (*e.g.*, cerebral aneurysm, Alzheimer's disease, and Parkinson's disease), pulmonary pathology (*e.g.*, idiopathic pulmonary fibrosis, chronic obstructive pulmonary disease, and cystic fibrosis), ophthalmic pathology (*e.g.*, cataract, glaucoma, macular degeneration), musculoskeletal pathology (*e.g.*, sarcopenia, intervertebral disc degeneration), cardiovascular pathology (*e.g.,* atherosclerosis, cardiac fibrosis, aortic aneurysm), renal pathology (*e.g.,* transplantation complications), and other diseases, such as mucositis, hypertension, hepatic

fibrosis, and myelofibrosis (OMF).

Specifically, the technical problem of the field is solved by the following embodiments.

1. An active agent for selectively killing one or more senescent cells or alleviating one or more symptoms associated with an inflammatory disorder, wherein the active agent comprises:

(i) a β-gal moiety as shown in Formula I

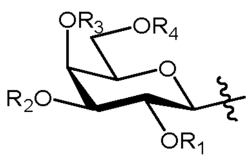

Formula I

(ii) a cytotoxic moiety as shown in Formula II,

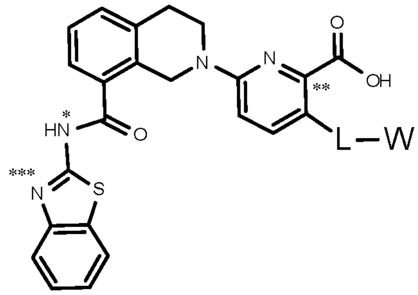

Formula II

wherein, one or more of the hydrogen bonded to the N atom at position *, the carboxyl of the C atom at position ** and the hydrogen on W are substituted with a linker X, thereby being linked to the β-gal moiety via the linker X; or the N atoms at position *, position *** are directly linked to the β-gal moiety;

(iii) the linker X as shown in any of Formulae (a)-(j):

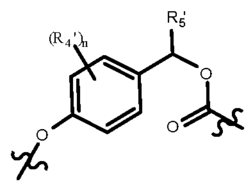

(a)

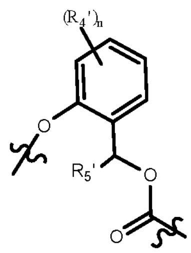

(b)

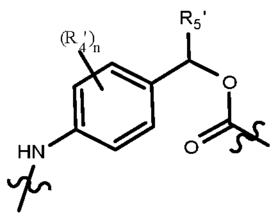

(c)

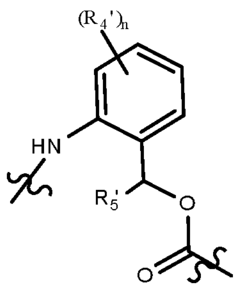

(d)

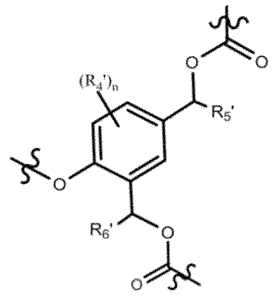

(e)

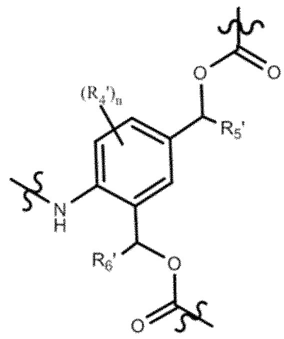

(f)

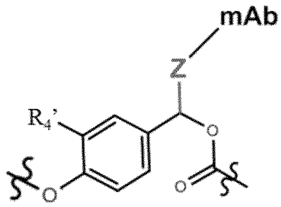 (g) 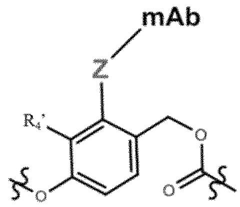 (h) 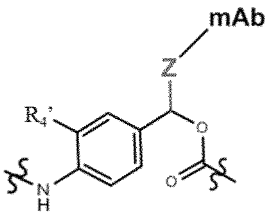 (i)

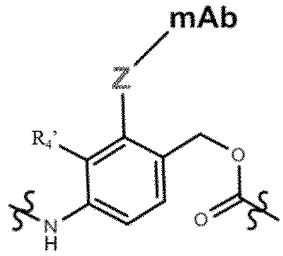 (j)

wherein, the linker X is linked to the β-gal moiety at the

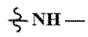

or

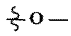

linking position, and to the cytotoxic moiety at the

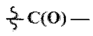

linking position,

wherein, the cytotoxic moieties linked at the two C(O)- linking positions are the same or different in the linker X of Formulae (e) and (f);

wherein

$R_1$, $R_2$, $R_3$, and $R_4$ are independently selected from H, -C(O)$R_5$, -C(O)O$R_6$, -C(O)N$R_7R_8$, and -P$O_3R_9$, wherein $R_5$, $R_6$, $R_7$, $R_8$, and $R_9$ are independently hydrogen, substituted or unsubstituted alkyl, substituted or unsubstituted aryl, substituted or unsubstituted heteroaryl, substituted or unsubstituted cyclic group, and substituted or unsubstituted heterocyclic group;

Each of $R_4$', $R_5$' and $R_6$' are independently selected from hydrogen, nitro, cyano, isocyano, amino, hydroxy, thiol, halogen, substituted or unsubstituted alkyl, substituted or unsubstituted alkoxyl, alkylamino, dialkylamino, carboxy, substituted or unsubstituted carbonyl, acylamino, sulfonyl, sulfo, phosphoryl, and phosphono;

n is 0, 1, 2, 3 or 4;

mAb is a monoclonal antibody and the linking group Z is a linking group for the antibody conjugation;

wherein

the linking group L is selected from substituted or unsubstituted alkyl, substituted or unsubstituted alkoxyl, substituted or unsubstituted aryl, substituted or unsubstituted heteroaryl, substituted or unsubstituted cyclic group and substituted or unsubstituted heterocyclic group;

L is preferably a linking group selected from the following structures:

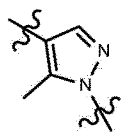 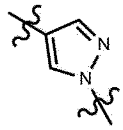 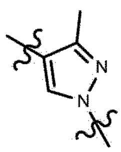

which is linked to the W group at the nitrogen atom in the ring; and

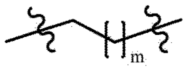 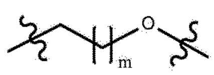 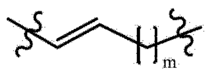 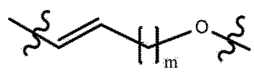 ;

m is 0, 1, 2, or 3;
L is most preferably a linking group selected from the following structures:

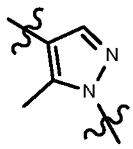 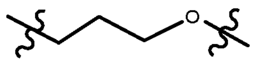 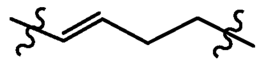 ;

W is selected from substituted or unsubstituted cyclic group, substituted or unsubstituted heterocyclic group, substituted or unsubstituted aryl, substituted or unsubstituted heteroaryl, substituted or unsubstituted bridging cycloalkyl, substituted or unsubstituted hetero-bridging cycloalkyl, substituted or unsubstituted spirocycloalkyl, substituted or unsubstituted hetero-spirocycloalkyl, substituted or unsubstituted fused cycloaryl, and substituted or unsubstituted fused heterocycloaryl;
On the premise that the hydrogen on W is substituted with the linker X, W has an amino group, a sec-amino group, a cyclic sec-amino group or a hydroxy, and the hydrogen bonded to the N atom or O atom in the amino group, the sec-amino group, the cyclic sec-amino group or the hydroxy is substituted with the linker X.
2. The active agent according to item 1, wherein $R_4$' is independently selected from hydrogen, nitro, cyano, isocyano, amino, hydroxy, thiol and halogen.
3. The active agent according to item 2, wherein $R_4$' is independently hydrogen or nitro.
4. The active agent according to item 1, wherein $R_5$' and $R_6$' are independently hydrogen.
5. The active agent according to item 1, wherein $R_5$' and $R_6$' are independently the lysosomal-directed units shown below:

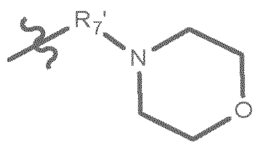

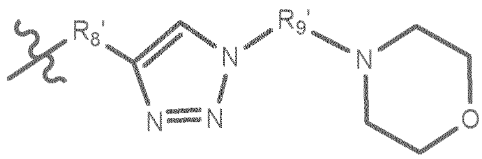

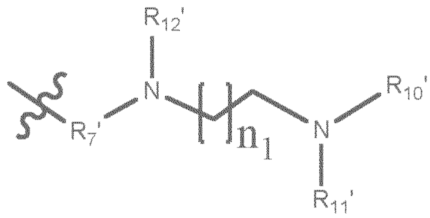

wherein, $R_7$', $R_8$' and $R_9$' are independently absent, alkylene, alkyleneoxyl, alkyleneamino or alkylenecarbonyl; wherein, $R_{10}$', $R_{11}$' and $R_{12}$' are independently selected from hydrogen, nitro, cyano, isocyano, amino, hydroxy, thiol, halogen, substituted or unsubstituted alkyl, substituted or unsubstituted alkoxyl, alkylamino, dialkylamino, carboxy, substituted or unsubstituted carbonyl, acylamino, sulfonyl, sulfo, phosphoryl, and phosphono;
$n_1$ is 0, 1, 2, 3 or 4.
6. The active agent according to item 5, wherein $R_5$' and $R_6$' are independently selected from the following structures:

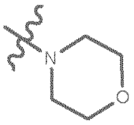 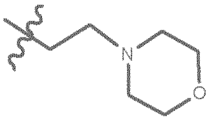 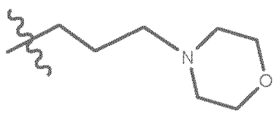 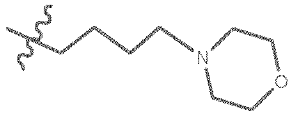

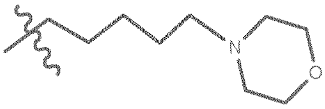 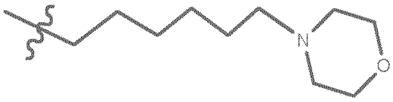

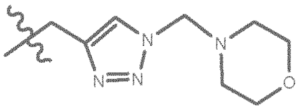 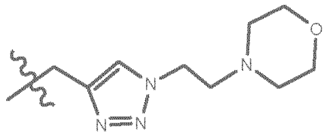 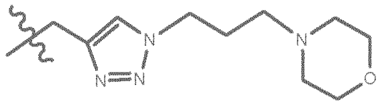

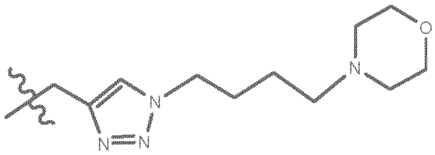

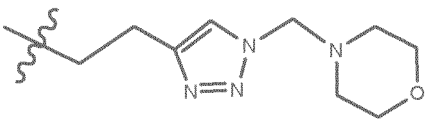 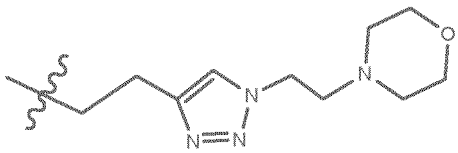

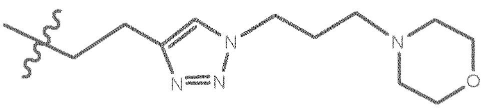 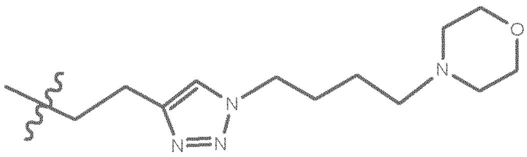

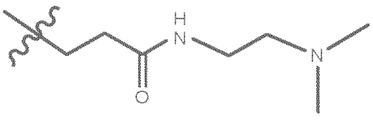 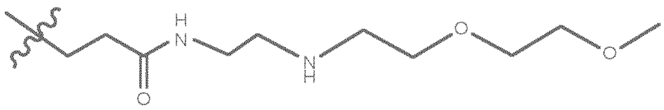

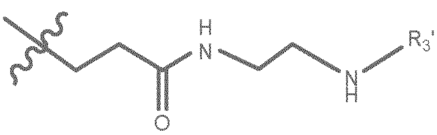 ,

wherein, $R_3$' is substituted or unsubstituted alkyl, substituted or unsubstituted alkylamino, or substituted or unsubstituted alkoxyl.

7. The active agent according to any one of items 1-6, wherein the linker X is independently selected from the following structures:

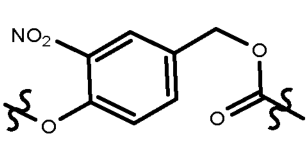 , 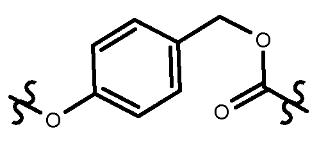 , 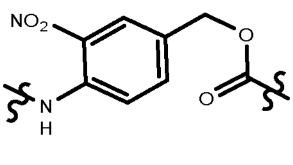 ,

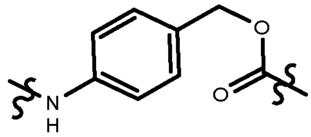, 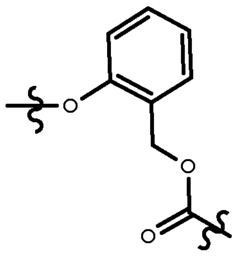, 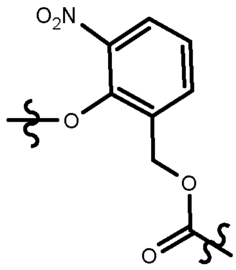, 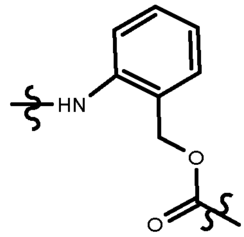,

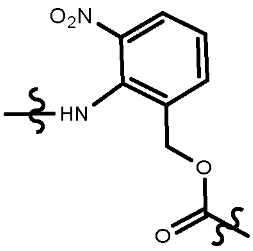, 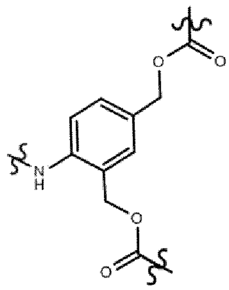;

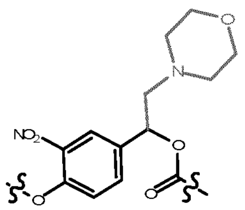, 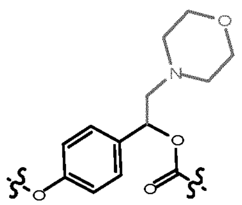, 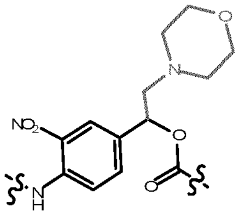, 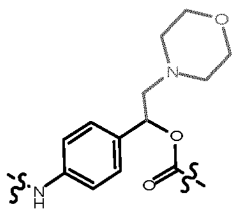,

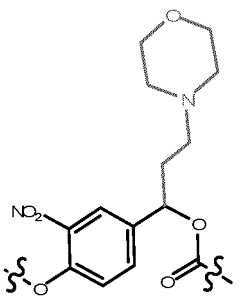, 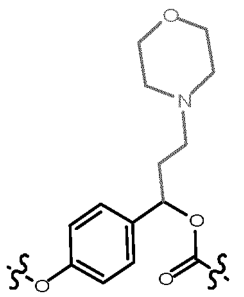, 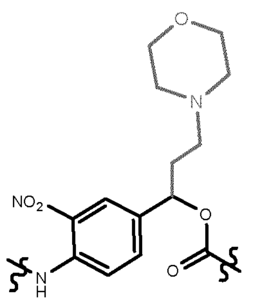, 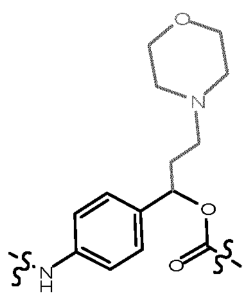,

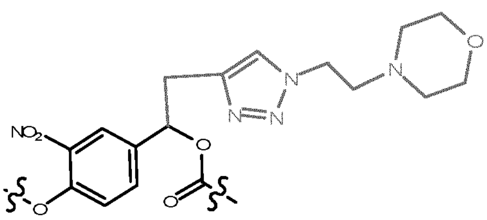, 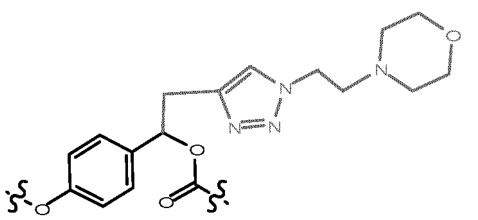,

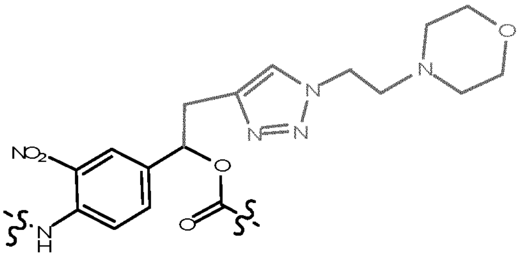,
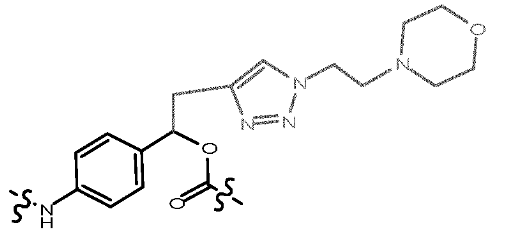,

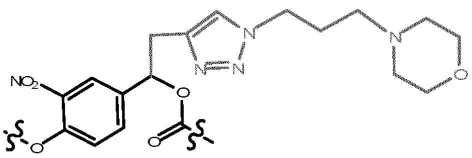,
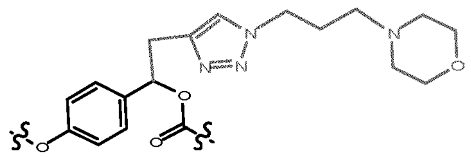,

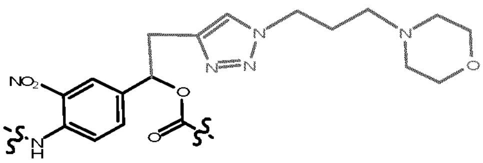, and
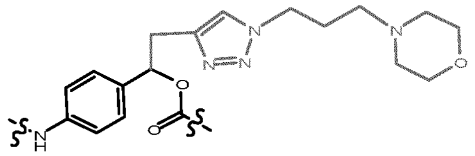.

8. The active agent according to claim 7, wherein the linker X is independently selected from the following structures:

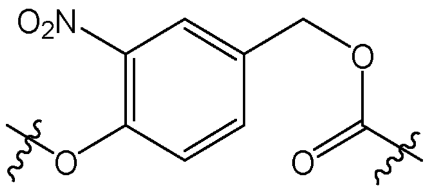
or
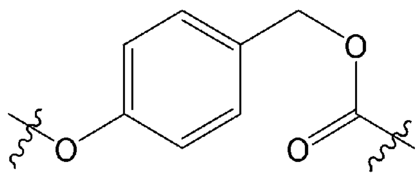.

9. The active agent according to item 1, wherein the Z group is selected from a group consisting of:

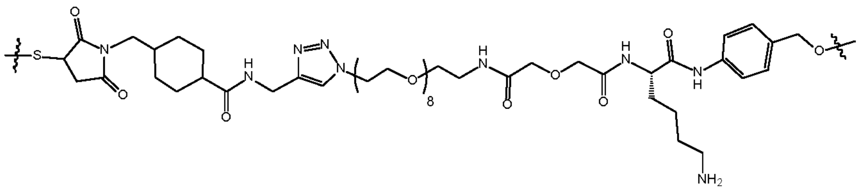
CL2A,

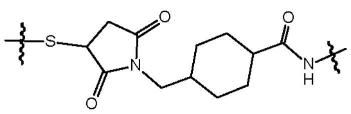
MCC,

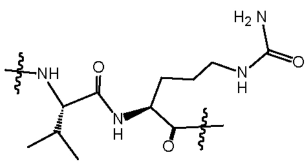
VC,

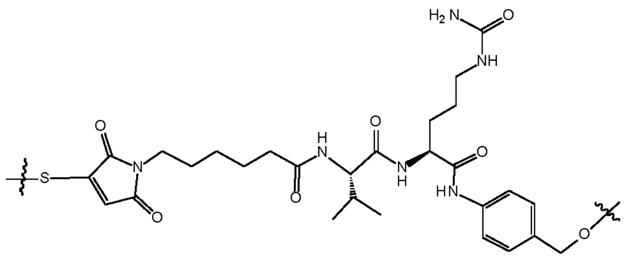

MC-VC-PAB,

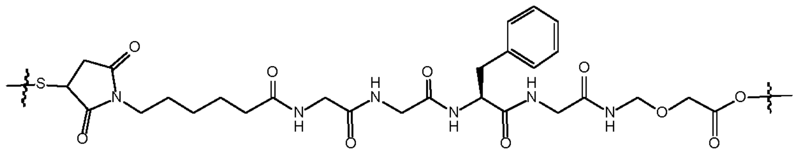

MC-GGFG,

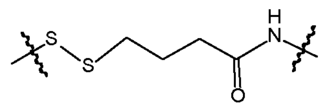

SPDB,

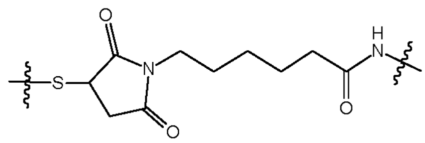

MHH.

10. The active agent according to item 1, wherein $R_1$, $R_2$, $R_3$ and $R_4$ are independently H or -C(O)Rs.
11. The active agent according to item 10, wherein $R_5$ is independently substituted or unsubstituted alkyl.
12. The active agent according to item 1, wherein the β-gal moiety is independently selected from the following structure:

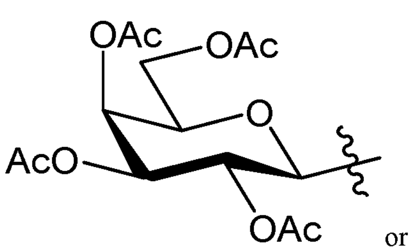

or

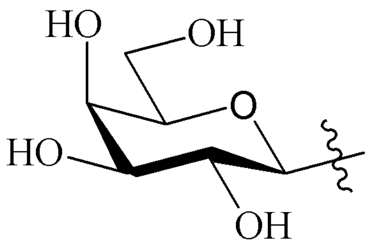

.

13. The active agent according to item 1, wherein W is selected from the following groups:

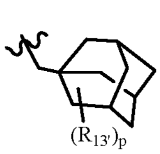

,

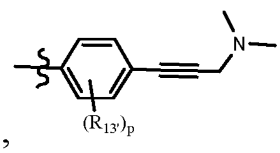

,

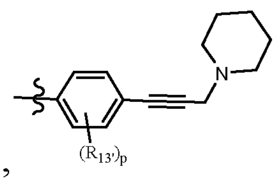

,

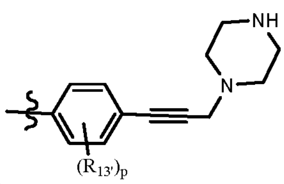

,

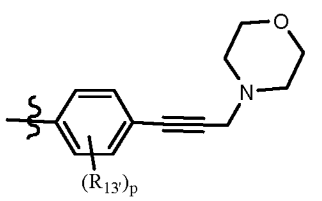

,

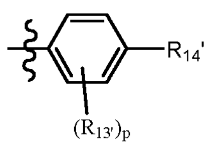

,

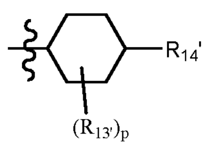

,

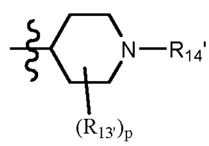

,

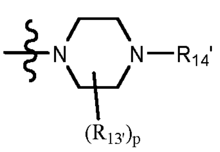 , 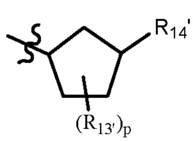 , 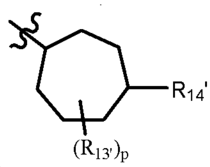 , 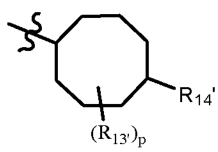

wherein, $R_{13'}$ is hydrogen, deuterium, halogen, amino, hydroxy, nitro, thiol, cyano, isocyano, substituted or unsubstituted alkyl, substituted or unsubstituted alkoxyl, alkylamino, dialkylamino, carboxyl, substituted or unsubstituted carbonyl, acylamino, sulfonyl, sulfo, phosphoryl and phosphono; wherein, $R_{14}'$ is hydrogen, deuterium, substituted or unsubstituted alkyl, substituted or unsubstituted aryl, substituted or unsubstituted heteroaryl, substituted or unsubstituted cyclic group, and substituted or unsubstituted heterocyclic group; wherein, p is 0, 1, 2, 3, or 4.

14. The active agent according to item 1, wherein the active agent has the following structure:

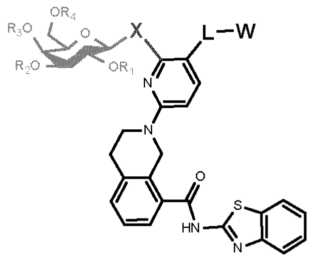 ,

a

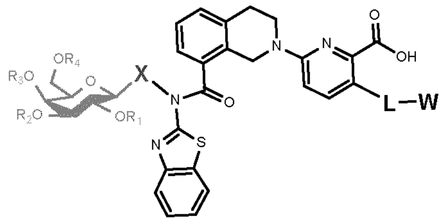 ,

b

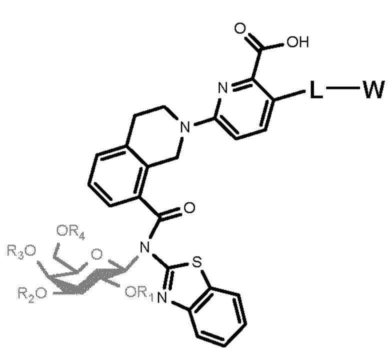 ,

c

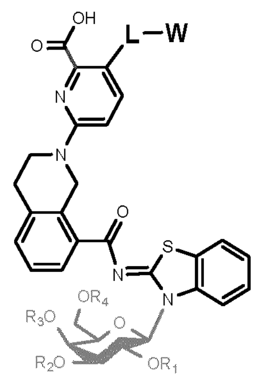 ,

d

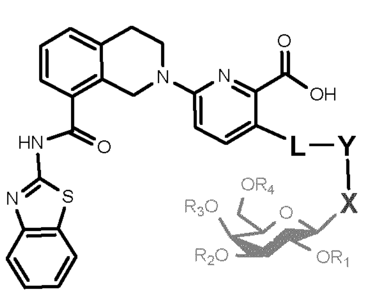 ,

e

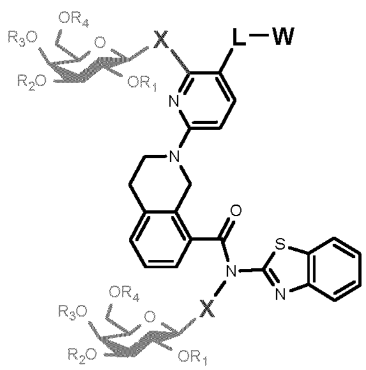 ,

f

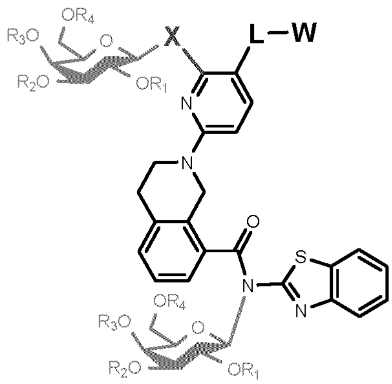

,

g

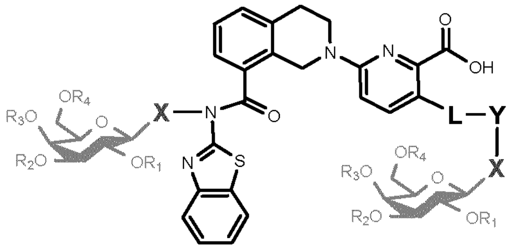

,

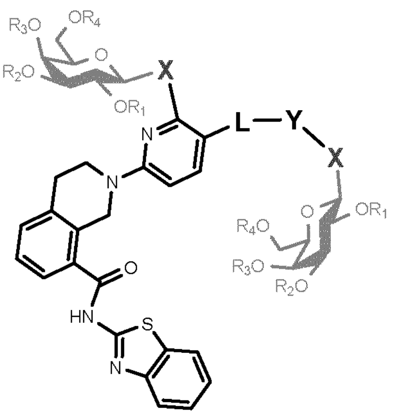

,

h

i

or

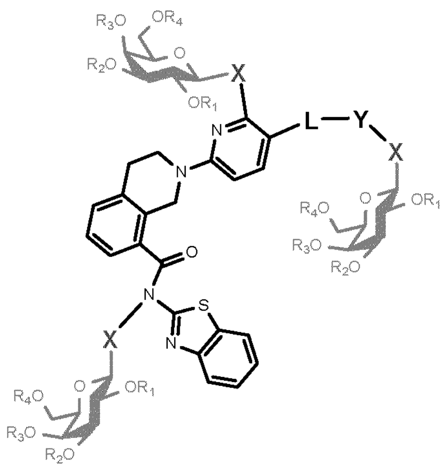

,

j

wherein, Y is W and has an amino group, a sec-amino group, a cyclic sec-amino group, or a hydroxy, and wherein, the hydrogen bonded to the N atom or O atom in the amino group, the sec-amino group, the cyclic sec-amino group or the hydroxy is substituted with the linker X.

15. The active agent according to item 1, the active agent has the following structure:

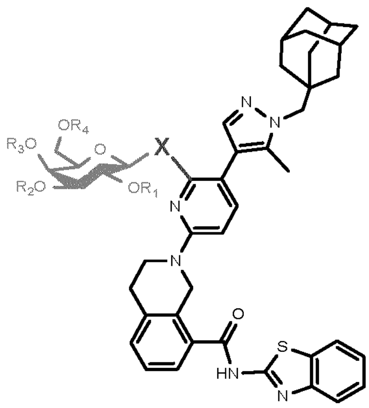

a1

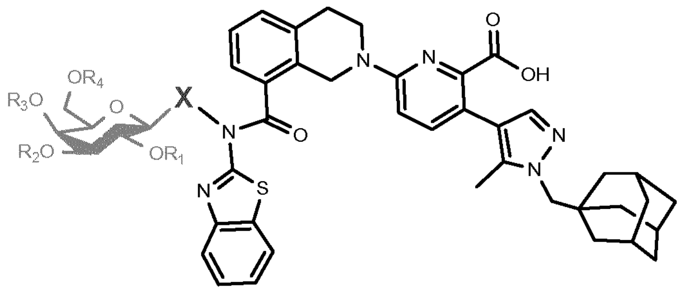

b1

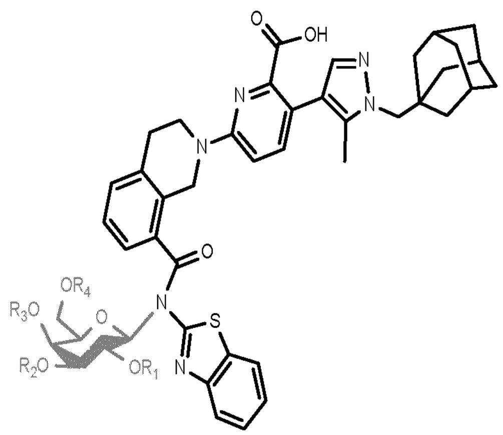

c1

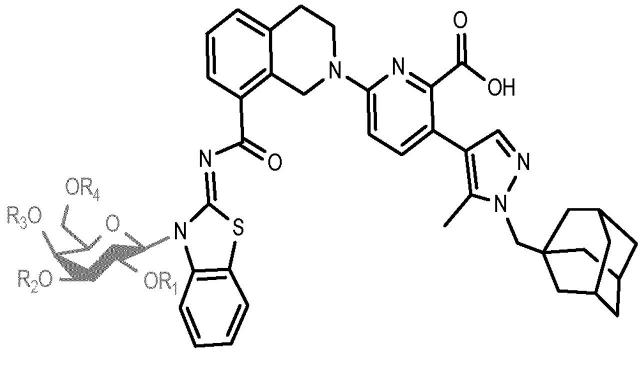

d1

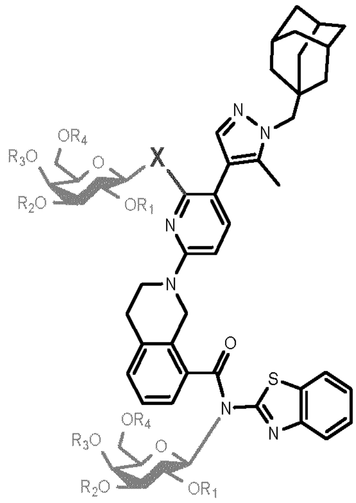

e1

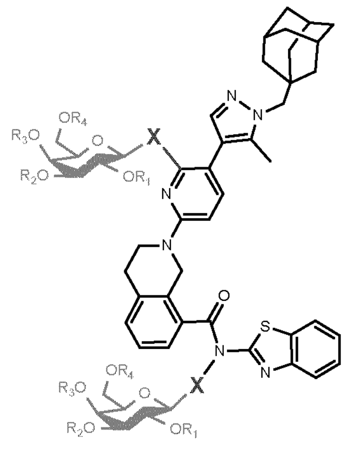

f1.

16. The active agent according to item 14, wherein Y is selected from the following structures:

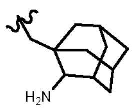 , 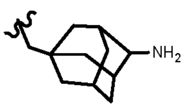 , 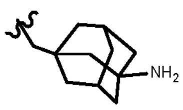 , 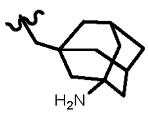 , 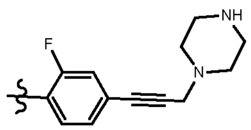 ,

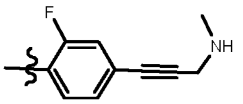 , 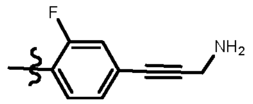 , 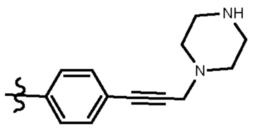 , 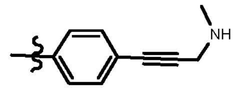 ,

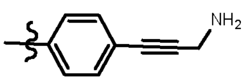 , 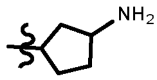 , 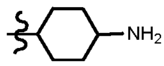 , 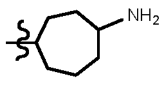 , 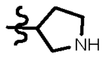 , 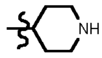 ,

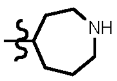 , 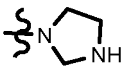 , 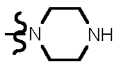 , 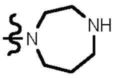 , 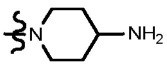 , 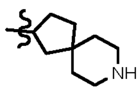 , 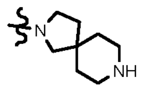 .

17. The active agent according to item 1, wherein the active agent is selected from a group consisting of:

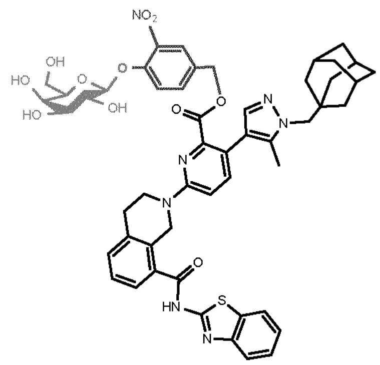 , 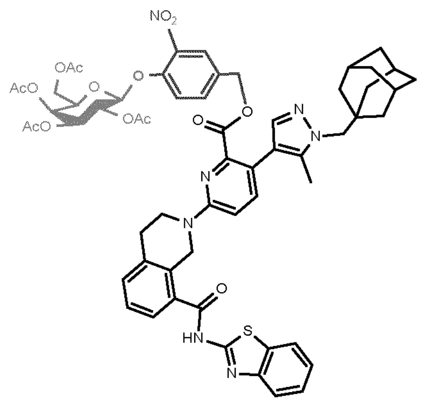 ,

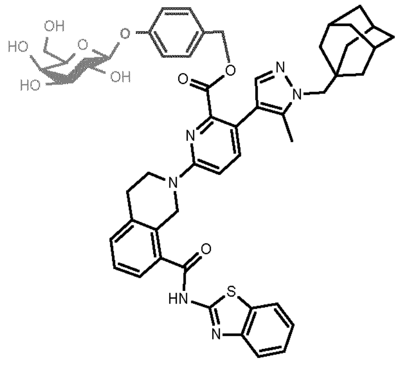 , 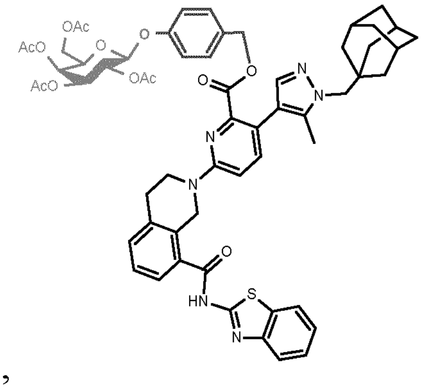 ,

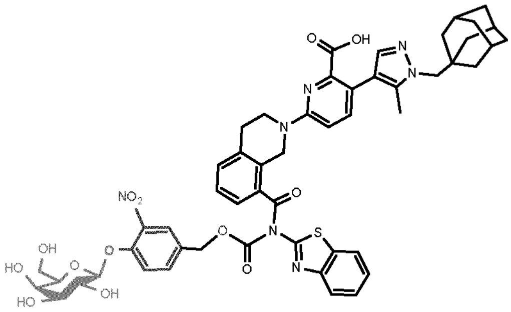

,

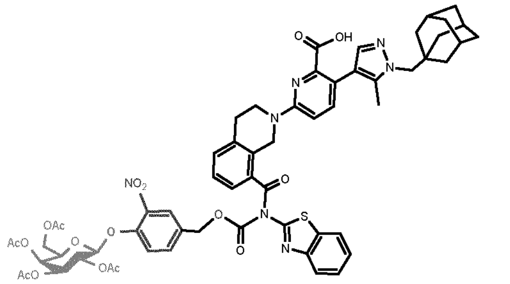

,

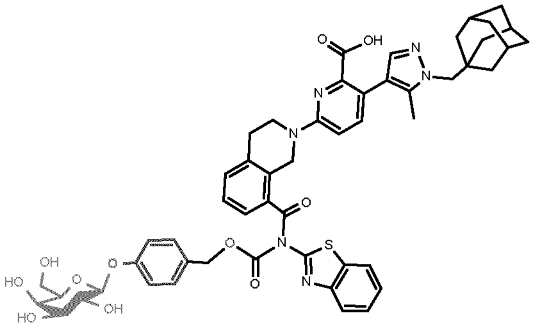

,

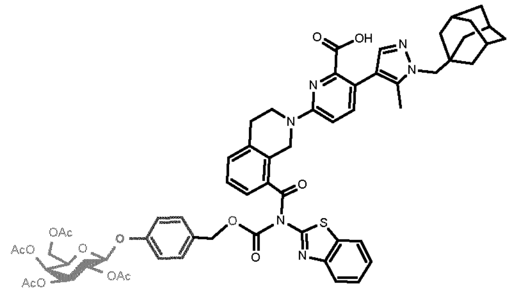

,

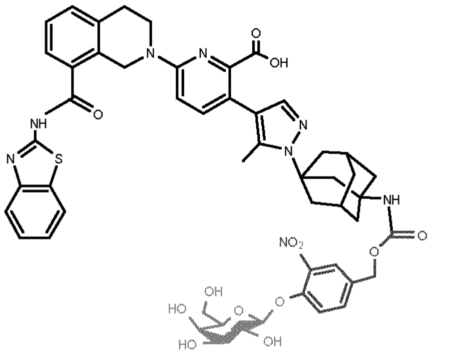

,

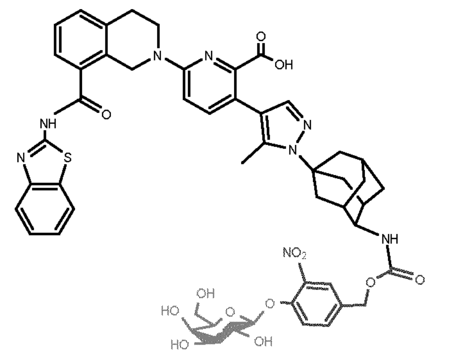

,

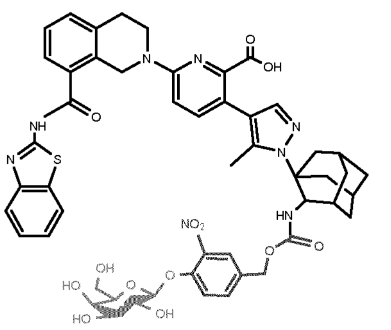

,

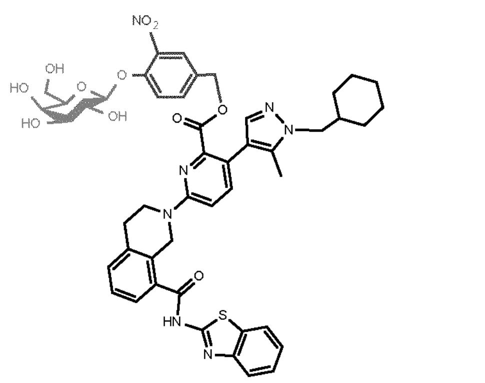

,

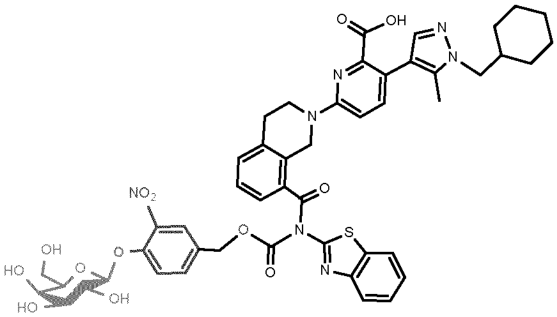
,
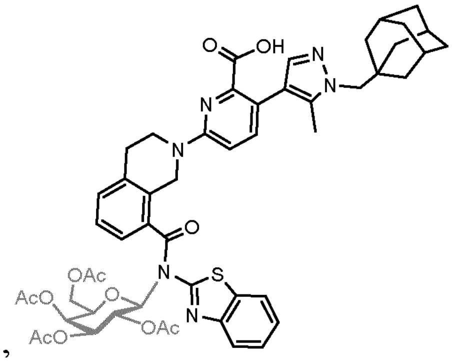
,

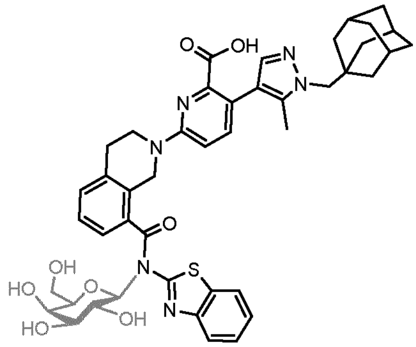
,
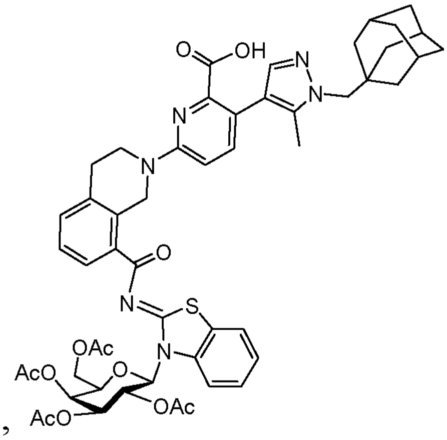
,

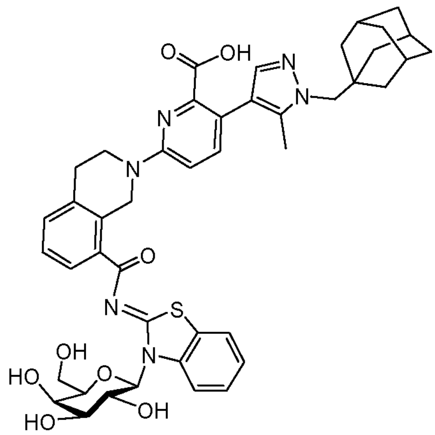
,

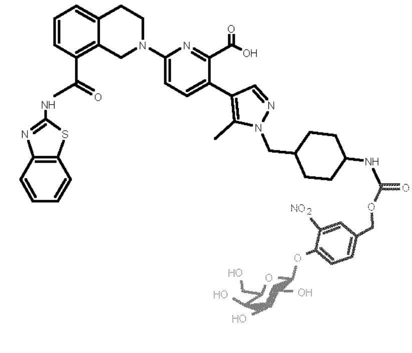
,
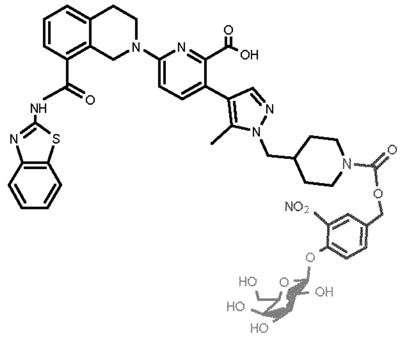
,

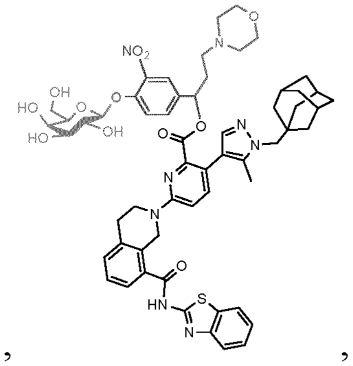

,  ,

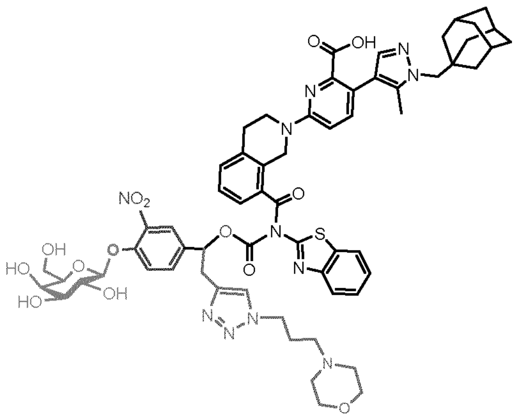

,

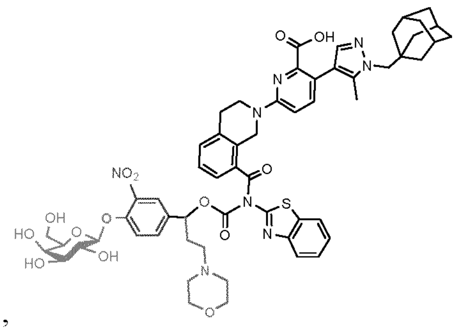

.

18. The active agent according to item 1, wherein, when W comprises a phenyl group substituted with an amino group or a hydroxy, the hydrogen bonded to the N atom or O atom in the amino group or hydroxy is substituted with the β-gal moiety such that the cytotoxic moiety is directly linked to the β-gal moiety without via the linker X.

19. The active agent according to item 18, wherein the active agent is:

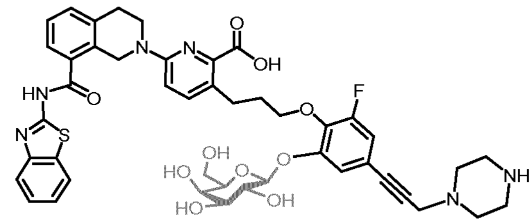

,

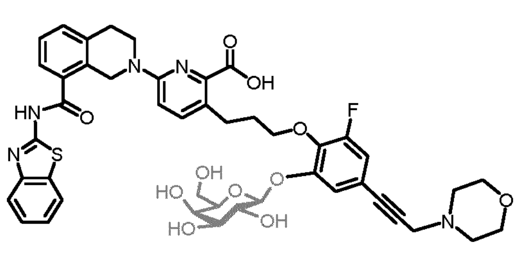

,

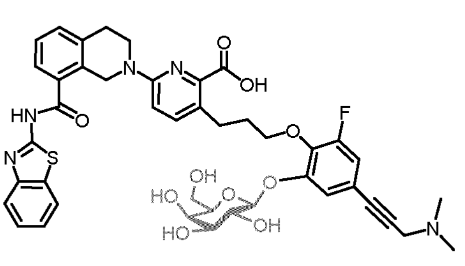,

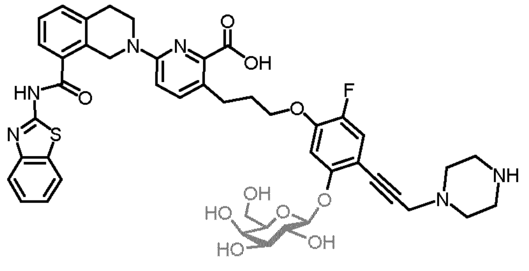,

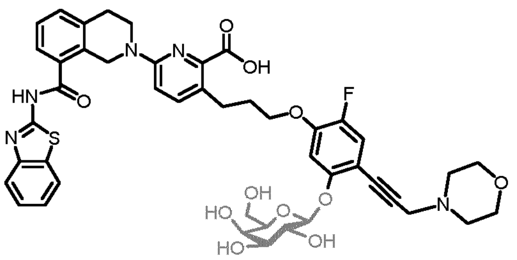,

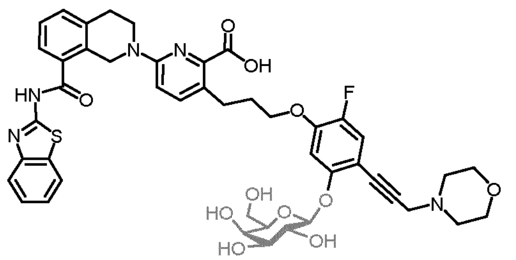,

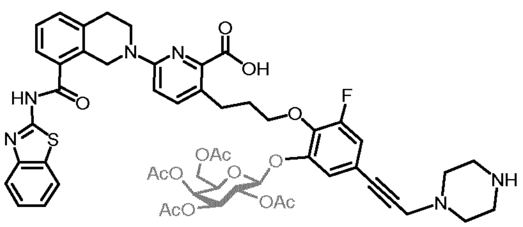,

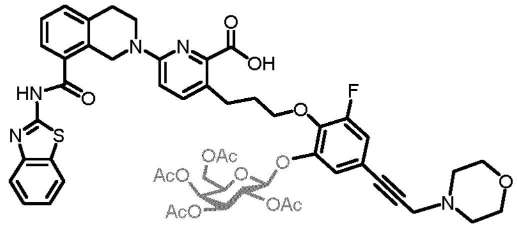,

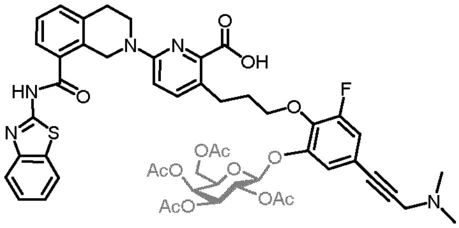,

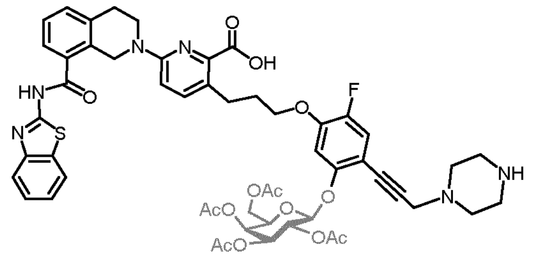,

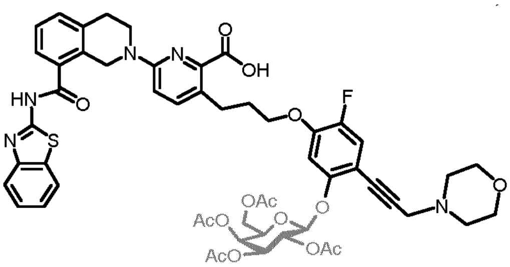,

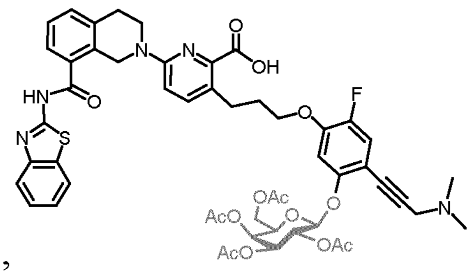,

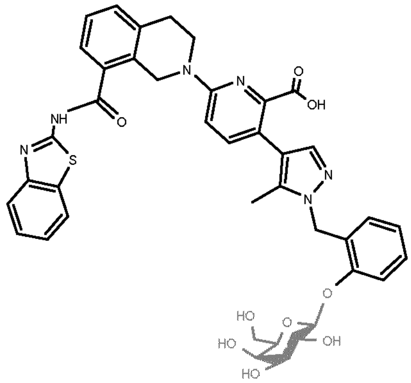,
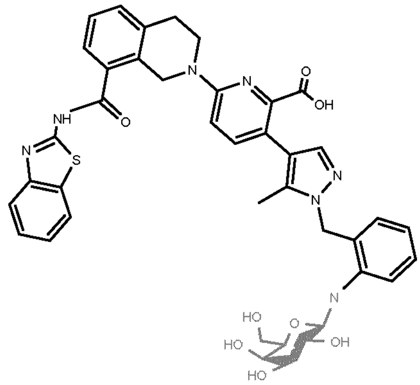.

20. A composition comprising an effective amount of the active agent according to any one of items 1-19 for killing one or more senescent cells or alleviating one or more symptoms associated with an inflammatory disorder.
21. The composition according to item 20, further comprising a pharmaceutically acceptable carrier.
22. A method of selectively killing one or more senescent cells or alleviating one or more symptoms associated with an inflammatory disorder in a subject, the method comprising administering to the subject a therapeutically effective amount of the composition according to item 20 or 21.
23. The method according to item 22, wherein the subject suffers from an aging-related disease or disorder, or an inflammatory disease or disorder.
24. The method according to item 22 or 23, wherein the aging-related disease or disorder is a metabolic disease, an inflammatory disease or disorder, a pulmonary disease or disorder, a neurological disease or disorder, a proliferative disorder, a renal disease or disorder, an ocular disease or disorder, or a dermatologic disorder or disease.
25. The method according to any one of items 22-24, wherein the aging-related disease or disorder is selected from a group consisting of:

(i) an inflammatory or autoimmune disease or disorder selected from oral mucositis, inflammatory bowel disease, kyphosis, and intervertebral disc herniation;
(ii) a neurological disease or disorder selected from Alzheimer's disease, Parkinson's disease, Huntington's disease, dementia, mild cognitive impairment, macular degeneration, and motor neuron dysfunction;
(iii) a metabolic disease selected from diabetic ulcer, metabolic syndrome and obesity;
(iv) a pulmonary disease selected from pulmonary fibrosis, chronic obstructive pulmonary disease, asthma, cystic fibrosis, emphysema, bronchiectasis and age-related loss of lung function;
(v) an ocular disease or disorder selected from macular degeneration, glaucoma, cataract, presbyopia and loss of vision;
(vi) an age-related disorder selected from renal failure, weakness, hearing loss, muscle fatigue, skin condition, skin wound healing, hepatic fibrosis, pancreatic fibrosis, oral submucous fibrosis, and sarcopenia; and
(vii) a skin disease or disorder selected from eczema, psoriasis, hyperpigmentation, nevus, rash, atopic dermatitis, urticaria, a disease and disorder associated with photosensitivity or photoaging, wrinkle, pruritus, dysesthesia, eczematous rash, eosinophilic dermatosis, reactive neutrophilic dermatosis, pemphigus, pemphigoid, immune bullous dermatosis, cutaneous fibrohistiocytic hyperplasia, cutaneous lymphomas, and cutaneous lupus.

26. The method according to item 25, wherein the subject is diagnosed with cancer and optionally is undergoing a cancer treatment selected from a chemotherapy and a radiotherapy.
27. The method according to item 26, wherein the active agent

(i) reduces the cells that have been determined to be senescent or cancer cells;
(ii) reduces one or more side effects produced by the senescent cells, wherein the side effects include an inflammation, promotions of cancer growth and metastasis;
(iii) reduces one or more side effects of a chemotherapy; or
(iv) reduces one or more side effects of a radiotherapy.

28. The method according to any one of items 22-27, wherein the cytotoxic moiety is cleaved after *in vivo* delivery.

29. The method according to any one of items 22-28, wherein the composition produces the following structure in an effective amount of killing one or more senescent cells after *in vivo* administration:

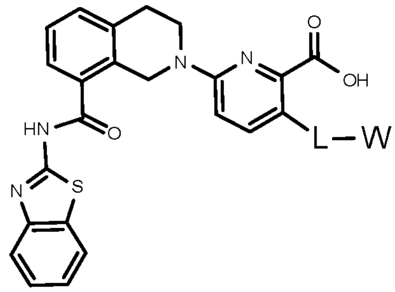

wherein, the linking group L is selected from substituted or unsubstituted alkyl, substituted or unsubstituted alkoxyl, substituted or unsubstituted aryl, substituted or unsubstituted heteroaryl, substituted or unsubstituted cyclic group, and substituted or unsubstituted heterocyclic group;
L is preferably a linking group selected from the following structures:

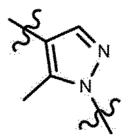 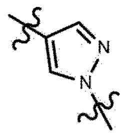 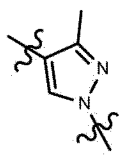

which is linked to the W group at the nitrogen atom in the ring; and

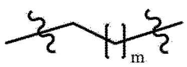 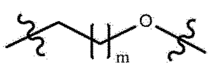 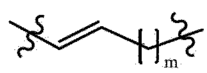 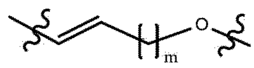 ;

m is 0, 1, 2, or 3;
L is most preferably a linking group selected from the following structures:

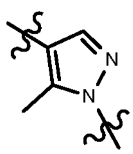 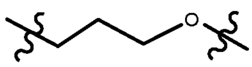 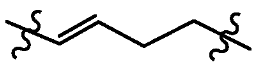

W is selected from substituted or unsubstituted cyclic group, substituted or unsubstituted heterocyclic group, substituted or unsubstituted aryl, substituted or unsubstituted heteroaryl, substituted or unsubstituted bridging cycloalkyl, substituted or unsubstituted hetero-bridging cycloalkyl, substituted or unsubstituted spirocycloalkyl, substituted or unsubstituted hetero-spirocycloalkyl, substituted or unsubstituted fused ring aryl, and substituted or unsubstituted hetero fused ring aryl;
30. The method according to item 22 or 23, wherein the subject has a viral infection, optionally, wherein the viral infection causes an overproduction of the activated macrophages in the subject.
31. The method according to item 30, wherein the viral infection is a coronavirus infection selected from a group consisting of coronaviruses, Severe Acute Respiratory Syndrome (SARS) coronavirus (CoV), SARS-CoV-2 (which causes COVID-19 (coronavirus disease) 2019)) and Middle East Respiratory Syndrome (MERS)-CoV.
32. The method according to item 31, wherein the subject has a SARS-CoV-2 infection and has been diagnosed with coronavirus disease 2019.

**DESCRIPTION OF THE DRAWINGS**

Figure 1. Screening to obtain A1331852 as the most promising β-gal-modified parent compound

A. The killing results of some selected compounds in the *in vitro* compound library screening on the induced senescent A549 cell model. Wherein, MZ-1, dBET6, ARV-825, I-BET726, I-BET151, and INCB057643 are all BET inhibitors; A1331852 and A1155463 are novel Bcl-xL inhibitors; Dasatinib, Quercetin, Fisetin, Curcumin are the familiar active drugs or natural products.
B. Comparison of the killing effects of three Bcl-xL inhibitors on the induced senescent A549 cell model. A549 represents the normal uninduced A549 cell group; and A549-CDDP represents the senescent A549 cell group induced with Cisplatin (CDDP) *in vitro.*
C. Comparison of the killing effects of three Bcl-xL inhibitors on the induced senescent HEF cell model. HEF represents the isolated early generation uninduced HEF cell group; and HEF-ETO represents the senescent HEF cell group induced with Etoposide (ETO) *in vitro.*

Figure 2. Synthesis and testing of the prodrug A13bGa1 obtained after the β-gal modification of A1331852

A. The synthesis route of the prodrug obtained based on the β-gal modification of the parent compound A1331852. Each step of the synthetic reaction was carried out under the described conditions according to the raw materials and equivalent ratios indicated by the arrows in the panel. eq.: raw material addition ratios; rt: room temperature reaction. All of the reactions were carried out under the anhydrous conditions with the dry solvent in nitrogen atmosphere. Compound 5 is the parent compound A1331852, abbreviated as A13, and the final product 7a is the prodrug obtained after the β-gal modification of of A13, named as A13bGa1.
B. The schematic diagram of the prodrug A13bGa1 releasing the parent compound A13 after the cleavage of the β-gal enzyme.
C. The high-performance liquid chromatogram showing the changes in the peak graphs of the prodrug before and after the cleavage of the β-gal enzyme. A13 represents the parent compound A1331852, A13bGa1 represents the prodrug obtained after the β-gal modification of A13, and A13bGa1 + β-gal represents the prodrug of A13 after being hydrolyzed by the β-gal enzyme *in vitro.*

Figure 3. Confirmation of the effect of the β-gal prodrug A13bGa1 by *in vitro* killing assays

A. Results of the SA-β-gal staining assay after *in vitro* induction of senescence in different cells. Senescence was induced by Etoposide in HUVEC cells, HEF cells and HELF cells, by Cisplatin in A549 cells and by Bleomycin in BJ cells and MRC5 cells.
B-G). Comparison of *in vitro* killing effects of the prodrug A13bGa1 and the prodrug A13 thereof on human HEF cells (B), HELF cells (C), BJ cells (D), A549 cells (E), HUVEC cells (F), and MRC5 cells (G).

Figure 4. *In vivo* testing of the platelet toxicity of the β-gal prodrug A13bGa1

A. Results of the platelet counts in peripheral blood on days 1, 3, 5, 8, and 10 after a single intraperitoneal injection of different doses of the given drug. Each dot in the panel represents the count of platelets detected in 1 mouse at that time point (n = 4 mice); A13 represents the parent compound A1331852 administration group, and A13bGa1 represents the prodrug obtained after the β-gal modification of A13, and mpk represents the milligram dose per kilogram of the body weight. There were three different dose groups of 2, 5, and 20 mg/kg for both A13 and A13bGa1 administration. The dashed line in the panel indicates the mean value of the platelet counts of the mice in the control single vehicle administration group.
B. The schematic diagram of the multiple dosing and the subsequent blood routine examinations in mice. The drug was administered on 3 consecutive days per week, followed by a 4-day interval, for 3 weeks continuous administration. The drug was administered by intraperitoneal injection in the afternoon on days 0, 1, 2, 7, 8, 9, 14, 15, and 16, and the blood was collected and tested in the morning on days 1, 3, 5, 8, 10, 12, 15, 17, and 19, respectively.
C. Results of the platelet counts in peripheral blood on days 1, 3, 5, 8, 10, 12, 15, 17, and 19 after multiple intraperitoneal injections of different doses of the given drug. Each dot in the panel represents the count of platelets detected in 1 mouse at that time point (n = 4 mice); mpk represents the milligram dose per kilogram of the body weight, A13 represents the parent compound A1331852 administration group, and A13bGa1 represents the prodrug obtained after the β-gal modification of A13. The dashed line in the panel indicates the mean value of platelet counts in mice in the single vehicle administration control group.
D. Mice were observed to be depilated and in poor state in A13 group after 3 consecutive rounds of administration. Daily observations revealed that mice in the A13 administration group were depilated, dispirited and in poor state after 3 consecutive rounds of administration of 5 mg/kg and 20 mg/kg, with 2 out of 4 (2/4) in A13-5 mg/kg dose group and 2 out of 4 (2/4) in A13-20 mg/kg dose group.

Figure 5. Demonstration of the therapeutic effect of the prodrug A13bGa1 using an *in vivo* Bleomycin mouse model

A. Modelling of a mouse model of Bleomycin-induced pulmonary fibrosis, and the schematic diagram of treatment. Mice were modelled with Bleomycin administered via tracheal instillation on day 0, and the treatment with intraperitoneal injection of the drug was started on day 4. The drug was administered for 3 consecutive days per week, followed by a 4-day withdrawal, for 4 weeks.
B. Body weight monitoring of mice during the treatment. A13 represents the A1331852 treatment group at a dose of 2 mg/kg, with a number of animals n = 6; A13bGa1 represents prodrug treatment group at a dose of 5 mg/kg; VEH represents the vehicle treatment control group, n = 6; and NC represents the saline tracheal instillation modelling control group, n = 5.
C. Comparison of lung specific gravity index calculated for different groups of mice. After completion of drug administration according to the dosing protocol, the mice were executed and the lungs of the mice were taken and weighed to calculate the lung specific gravity index, lung index = the lung weight (g) / the body weight (g) $\times$ 100 %. A13 represents the A1331852 treatment group at a dose of 2 mg/kg; A13bGa1 represents prodrug treatment group at a dose of 5 mg/kg; VEH represents the vehicle treatment control group; and NC represents the saline tracheal instillation modelling control group. Each dot in the panel represents the result of the lung specific gravity index of 1 mouse, n = 5 in the saline modelling control group, and n = 6 in other groups. The statistical analysis was performed using unpaired T-test, ns indicates no statistical difference; with *$p < 0.05$; and **$p < 0.01$.
D. Representative results of HE and Masson staining of the pathological sections of lungs taken from different groups of mice after execution. Normal represents the saline tracheal instillation modelling control group; Vehicle represents the vehicle administration control group; A13bGa1 represents the prodrug treatment group; A13 represents the A1331852 treatment group. Scale bar: 500 $\mu$m.

Figure 6. The prodrug A13bGa1 exerts a therapeutic effect in a mouse model of oxygen-induced retinopathy.

A. Modelling of a mouse model of oxygen-induced retinopathy, and the schematic diagram of treatment. C57BL6/J mice were cultured in 75% hyperoxia for 5 consecutive days starting from postnatal day 7, and treated by a single intravitreal administration of the drug on postnatal day 12, and then transferred to normal air atmosphere until day 17 for retinal sampling and analysis. P0, P7, P12, and P17 in the panel represent mice on postnatal days 0, 7, 12, and 17, respectively.
B. Representative diagram of the reduction of pathological avascular areas in the mouse retina after the treatment by intravitreal injection of A13bGa1. Mouse retinal microvessels were labelled by IB4 staining for the central avascular area analysis, wherein the green fluorescence represents the IB4-labelled microvascular areas, and the avascular areas are circled with white markers in the panel. The left panel shows the VEH control group (the single vehicle administration control); the middle panel shows the A13bGa1 administration group with a single dose of 50 ng; and the right panel shows the A13bGa1 administration group with a single dose of 100 ng.
C. Statistical results of the retina pathological avascular areas after treatment of different groups of mice. VEH represents the single vehicle administration control group, with the number of animals n=7; 50 ng, A13bGa1 treatment group, with a dose of 50 ng; and 100 ng, A13bGa1 treatment group, with a dose of 100 ng. The statistics in the panel are the quantitative results of the ischemic areas in the retinas of each sample compared with that of the VEH control group after the areas were taken as the means, and the error bar is a standard deviation. The statistical analysis was performed using unpaired T-test, with ***$p < 0.001$; and ****$p < 0.0001$.
D. Representative diagram of the reduction of the pathological abnormal neovascularization areas in the mouse retina after the treatment by intravitreal injection of A13bGa1. Mouse retinal microvessels were labelled by IB4 staining for the abnormal neovascularization analysis, wherein the green fluorescence represents the IB4-labelled microvascular areas, and the abnormal neovascular areas are circled with white markers in the panel. The left panel shows the VEH control group (the single vehicle administration control); the right panel shows the A13bGa1 administration group with a single dose of 100 ng.
E. Statistical results of the retinal pathological abnormal neovascularisation areas after treatment of different groups of mice. VEH represents the single vehicle administration control group, with the number of animals n=7; 50 ng, A13bGa1 treatment group, with a dose of 50 ng; and 100 ng, A13bGa1 treatment group, with a dose of 100 ng. The statistics in the panel are the quantitative results of the abnormal neovascularisation areas in the retinas of each sample compared with that of the VEH control group after the areas were taken as the means, and the error bar is a standard deviation. The statistical analysis was performed using unpaired T-test, with *$p < 0.05$; and ***$p < 0.001$.

Figure 7. In vitro testing of the selective killing effect of the $\beta$-gal prodrug on the senescent cells:

A-B). Killing effects of the β-gal prodrug A13bGa1-lyso (*i.e.,* d-12 in Example 4) on A549 cells, the Cisplatin-induced senescent A549 cells (Figure 7A), and on human HEF cells, and the Etoposide-induced senescent human HEF cells (Figure 7B).

C-D). Killing effects of the β-gal prodrug A13bGa2-lyso (*i.e.,* d-11 in Example 4) on A549 cells, the Cisplatin-induced senescent A549 cells (Figure 7C), and on human HEF cells, and the Etoposide-induced senescent human HEF cells (Figure 7D).

E-F). Killing effects of the β-gal prodrug A13bGb1 (*i.e.,* b-4 in Example 3) on A549 cells, the Cisplatin-induced senescent A549 cells (FIG. 7E), and on human HEF cells, and the Etoposide-induced senescent human HEF cells (FIG. 7F).

A549 represents the normal uninduced A549 cell group; A549-CDDP represents the *in vitro* Cisplatin (CDDP)-induced senescent A549 cell group. HEF represents the isolated early generation uninduced HEF cell group; HEF-ETO represents the *in vitro* Etoposide (ETO)-induced senescent HEF cell group. The statistical analysis was performed using unpaired T-test, ns indicates no statistical difference; with $*p < 0.05$; $**p < 0.01$, $***p < 0.001$; and $****p < 0.0001$.

## DETAILED DESCRIPTION OF THE INVENTION

In order to make the objects, technical solutions and advantages of the present invention clearer and more understandable, the present invention is further described in detail below in conjunction with the specific examples and with reference to the drawings.

SA-β-gal is a widely used marker of celluar senescence (Dimri, et al., PNAS, 92: 9363-9367 (1995); and Lee, et al., Aging Cell 5, 187-195 (2006)), and its activity is used herein to selectively metabolize the small molecules in the senescent cells (Lozano-Torres, et al., J Am Chem Soc 139, 8808-8811 (2017)). The compositions and methods disclosed herein are based on the development of SA-β-gal based prodrug strategy to release the active parent compound to preferentially kill the senescent cells. The disclosed compositions can be used to ameliorate a symptom associated with proinflammation, e.g., in response to an infection, such as an excessive accumulation of the activated macrophages in a viral infection.

In the field of pulmonary fibrosis, idiopathic pulmonary fibrosis (IPF) is the most common and severe form, which is defined as a specific form of chronic, progressive, and fibrotic unexplained interstitial pneumonia (Raghu G, et al., Am J Respir Crit Care Med 183, 788-824 (2011)). IPF occurs mostly in the middle-aged and elderly populations and is a fatal lung disease in which the patient's healthy tissue is replaced by the altered extracellular matrix and the alveolar structure is disrupted, leading to a decreased lung compliance, a disruption of the gas exchange, and ultimately a respiratory failure and death (Wolters PJ, et al., Annu Rev Pathol 9, 157-179 (2014)). Although the course of IPF is variable and somewhat unpredictable, the survival after diagnosis is low, with a median survival time of only 2-4 years, and it is considered to be a "tumor-like disease" more aggressive than cancer (Ley B, et al., Am J Respir Crit Care Med 183, 431-440 (2011); and Hutchinson JP, et al., Annals of the American Thoracic Society 11(8), 1176-1185 (2014). There is no cure for IPF, and the two drugs on the market, Pirfenidone and Nintedanib, only serve to slow the progression of the disease, but do not provide a cure, and are more toxic and costly (Richeldi L, et al., N Engl J Med 370, 2071-2082 (2014); King TE, et al., N Engl J Med 370, 2083-2092 (2014); and Noble PW, et al., Lancet 377, 1760-1769 (2011)). Thus, curing IPF is a significant unmet medical need in human health, and a better mechanistic understanding of pathogenesis is needed to develop new disease-modifying therapies.

There is growing evidence that aging plays a key accelerating role in the pathogenesis of IPF (Faner R, et al., Am J Respir Crit Care Med 186, 306-313 (2012); and Marissa J, et al., Nat Commun 8, 14532 (2017)). Established biomarkers of cellular senescence, including p16, p21, and the senescence-associated β-galactosidase activity (SA-β-gal), have been observed in the fibroblasts and the epithelial cells from the lung tissues of IPF patients (Kuwano K, et al., Am J Respir Crit Care Med 154, 477-483 (1996); and Lomas N. J, et al., Int. J. Clin. Exp. Pathol. 5, 58-71 (2012)), and there is a notable trend of accelerated cellular senescence observed in human IPF-derived cells in vitro (Yanai H, et al., Aging (Albany NY) 7, 664-672 (2015)). Therefore, the treatment of IPF with senolytics has become a very reasonable and attractive option, and has been developing rapidly in recent years (Jin Pan, et al., Int J Radiat Oncol Biol Phys 99(2), 353-361 (2017); and Jamie N, et al., EBioMedicine 40, 554-563 (2019)).

The major difficulty in developing an effective treatment for IPF is that the pathogenesis of this disease is very poorly defined. As a result, modelling of the fibrotic disease has become an established area of research. A common model used over the last 20 years is the Bleomycin (BLM) model, an experimental technique in which BLM is injected directly into the lungs of rodents. In rodents, BLM is administered by a variety of routes, including intravenous (IV), subcutaneous (SC), intranasal (IN), and intratracheal (IT) administration, but IT BLM administration is by far the most popular mode of administration. In mice, the intratracheal perfusion of the chemotherapeutic drug Bleomycin leads to a soluble pulmonary fibrosis, and such a model encapsulates the key features of human IPF (Izbicki G, et al., Int. J. Exp. Pathol 83, 111-119 (2002)).

Like many organs, impaired ocular function occurs with age and is becoming more common as the population ages. When vision loss and visual field loss become significant, they begin to affect the patient's ability to perform activities of daily living such as reading, writing, driving and walking. The Beaver Dam Ophthalmology study shows that poor visual acuity, poor contrast sensitivity, and binocular vision differences are biomarkers of aging and weakness that are positively correlated with the risk of tumble (Knudtson, Klein, et al., Arch Ophthalmol 124(2): 243-249 (2009)). More alarmingly, the same study identifis the visual impairment as an independent risk factor for death (Knudtson, Klein, et al., Arch Gerontol Geriatr 49(1): 22-26 (2006)). Now, a number of eye-related diseases have been shown to be age-related, including age-related macular degeneration (AMD), diabetic retinopathy (PDR), diabetic macular edema (DME), and Glaucoma, and the like. The current major treatment for age-related ocular diseases is vascular care of the retina, which has some therapeutic effects but also significant risks. Laser photocoagulation treatments ablate the ischaemic retinal tissues, which produce angiogenic and inflammatory factors in the centre of the disease, but when they burn and destroy the neural retina, they cause dark spots (Little, et al., Ophthalmology 92, 279-283 (1985)). Anti-vascular endothelial growth factor (VEGF) therapy requires regular injections directly into the vitreous body of the eye and exposes the patients to a potential infection and endophthalmitis (Fintak, et al., Retina 28, 1395-1399 (2008)). Furthermore, in some patients, VEGF neutralization can accelerate photoreceptor atrophy (Grunwald, et al., Ophthalmology 121, 150-161 (2014)). In addition to the antiangiogenic therapies, the drugs currently in clinical trials for age-related ocular diseases primarily target the complement regulation or inflammation. However, most of these attempts have failed (Holz FG, et al., JAMA Ophthalmol. 136(6): 666-77 (2018)), which is not surprising as such diseases are caused by a variety of factors that are not fully understood.

Although the exact mechanisms of age-related ocular diseases are not fully understood, as the same as in other adult tissues, the ageing programme of the eye over time may be caused by a variety of exogenous and endogenous factors such as oxidative stress, metabolic disorders, nutrient and growth factor signals, inflammation and immune system disorders, telomere dysfunction and DNA damage, and the like (Marazita MC, et al., Redox Biol. 7:78-87 (2016); Sreekumar PG, et al., Investigative Ophthalmology & Visual Science. 57(3):1238-53 (2016); and Supanji, et al., Exp Eye Res. 112:79-92 (2013)). The senescent retinal pigment epithelium (RPE) cells, retinal vascular endothelial cells, and retinal ganglion cells have been found in a variety of age-related ocular diseases (e.g., AMD, DR, DME, Glaucoma), and the removal of these senescent cells with relevant senolytics can significantly ameliorate the disease symptoms in the relevant animal models (Chae JB, et al., Geroscience. 43(6):2809-2833 (2021); Crespo- Garcia S, et al., Cell Metab. 33, 818-832 (2021); and Rocha LR, et al., Aging Cell. 19(2):e13089. (2020)). Therefore, the development of new drugs with a broader spectrum and specific elimination of the senescent cells in the eye is an approach that can fundamentally target the complex pathogenesis of the age-related ocular diseases and effectively treat them.

The oxygen induced retinopathy (OIR) model is the gold standard preclinical model for the study of the age-related ocular disease pathology and is one of the most widely cited disease models in ophthalmology and vascular biology researches (Smith, LE, et al., Invest Ophthalmol Vis Sci 35, 101 -111 (1994); and Connor, KM, et al., Nat Protoc 4, 1565-1573 (2009)). Modelled on retinopathy of the premature infants, it is now also widely used to understand several retinal diseases characterised by hypoxia- or inflammation-driven angiogenesis, such as neovascular age-related macular degeneration and proliferative diabetic retinopathy (Grisanti, S, et al., Prog Retin Eye Res 27, 372-390 ( 2008); and Simo, R, et al., Curr Diabetes Rev 2, 71-98 (2006)), and it is these diseases that together constitute the main cause of blindness (Stahl, A, et al., Invest Ophthalmol Vis Sci 51, 2813- 2826 (2010)). In the OIR model, the cellular senescence, initially observable in the retinal ganglion neurons, and subsequently propagating to other cell populations of the retina (the microglia, the vascular endothelial cells, and the retinal pigment epithelial cells), further contributes to the retinopathy (Oubaha M, et al., Sci Transl Med. 8(362):362ra144 (2016)). Based on the above features, the oxygen-induced retinopathy (OIR) model used in this experiment is well suited for use in the development of anti-senescence drugs for age-related ocular diseases.

I. Definitions

As used herein, "a cytotoxic moiety" refers to the moiety of the anti-senescence prodrug provided by the parent cytotoxic agent.

As used herein, "a cytotoxic agent" refers to a chemical or drug used to kill cells.

As used herein, "the parenteral administration" indicates an administration by any method other than via the digestive tract or non-invasive local or regional routes.

As used herein, "a patient" or "a subject" to be treated is a human or non-human animal.

As used herein, "the parenteral administration" refers to an administration by any method other than by the digestive tract or non-invasive local or regional routes.

As used herein, "a patient" or "a subject" to be treated is a human or non-human animal.

As used herein, "an anti-senescence drug" selectively kills one or more senescent cells compared with the non-senescent cells.

As used herein, "therapeutically effective" or "an effective amount" indicates that the amount of the composition

used is an amount sufficient to ameliorate one or more causes or symptoms of a disease or disorder. Such amelioration requires only reduction or changes, not necessarily elimination. As used herein, the terms "a therapeutically effective amount", "a therapeutic amount" and "a pharmaceutical effective amount" are synonymous. A person skilled in the art can readily determine the appropriate therapeutic amount.

As used herein, the term "treat" includes eliminating, substantially inhibiting, slowing or reversing the progression of a disease or disorder, substantially ameliorating the clinical symptoms of a disease or disorder, or substantially preventing the occurrence of the clinical symptoms of a disease or disorder.

As used herein, "substituted" refers to all permissible substituents of a compound or a functional group described herein. In the broadest sense, the permissible substituents include an acyclic and a cyclic, a branched and an unbranched, a carbocyclic and a heterocyclic, an aromatic and a non-aromatic substituents of the organic compounds. Exemplary substituents include, but are not limited to, halogen, hydroxy, or any other organic group containing any number of carbon atoms, preferably 1-14 carbon atoms, and optionally include one or more heteroatoms in the form of the linear, branched, or the cyclic structures, such as oxygen, sulfur, or nitrogen groups. Representative substituents include alkyl, substituted alkyl, alkenyl, substituted alkenyl, alkynyl, substituted alkynyl, phenyl, substituted phenyl, aryl, substituted aryl, heteroaryl, substituted heteroaryl, halogen, hydroxy, alkoxyl, substituted alkoxyl, phenoxy, substituted phenoxy, aryloxy, substituted aryloxy, alkylthio group, substituted alkylthio group, thiophenyl, substituted thiophenyl, arylthio group, substituted arylthio group, cyano, isocyano, substituted isocyano, carbonyl, substituted carbonyl, carboxy, substituted carboxy, amino, substituted amino, acylamino, substituted acylamino, sulfonyl, substituted sulfonyl, sulfo, phosphoryl, substituted phosphoryl, phosphono, substituted phosphono, polyaryl, substituted polyaryl, $C_3$-$C_{20}$ cyclic group, substituted $C_3$-$C_{20}$ cyclic group, heterocyclic ring, substituted heterocyclic ring, amino acid, poly(lactic-co-ethanolic acid), peptide, and polypeptide group. Such alkyl, substituted alkyl, alkenyl, substituted alkenyl, alkynyl, substituted alkynyl, phenyl, substituted phenyl, aryl, substituted aryl, heteroaryl, substituted heteroaryl, halogen, hydroxy, alkoxyl, substituted alkoxyl, phenoxy, substituted phenoxy, aryloxy, substituted aryloxy, alkylthio group, substituted alkylthio group, thiophenyl, substituted thiophenyl, arylthio group, substituted arylthio group, cyano, isocyano, substituted isocyano, carbonyl, substituted carbonyl, carboxy, substituted carboxy, amino, substituted amino, acylamino, substituted acylamino, sulfonyl, substituted sulfonyl, sulfo, phosphoryl, substituted phosphoryl, phosphono, substituted phosphono, polyaryl, substituted polyaryl, $C_3$-$C_{20}$ cyclic group, substituted $C_3$-$C_{20}$ cyclic group, heterocyclic ring, substituted heterocyclic ring, amino acid, poly(lactic-co-ethanolic acid), peptide, and polypeptide group may be further substituted.

Heteroatom, such as nitrogen, may have a hydrogen substituent and/or any permissible substituent of the organic compounds described herein that satisfy the valency of the heteroatom. It is to be understood that "substitution" or "substituted" includes the implied condition that such substitution meets the allowable valency of the substituted atom and the substituent, and that the substitution produces a stable compound, *i.e.,* a compound that does not spontaneously undergo transformation such as by rearrangement, cyclisation, elimination, *etc.*

Unless specifically and explicitly stated otherwise, the term "substituted" refers to a structure, for example, a compound or a moiety of a larger compound, regardless of how the structure is formed. The structure is not limited to a structure made by any particular method.

As used herein, "aryl" refers to a $C_5$-$C_{26}$-membered aromatic, a fused aromatic, a fused heterocyclic ring, or a bis-aromatic ring system. As used herein, "aryl" is broadly defined to include 5-, 6-, 7-, 8-, 9-, 10-, 14-, 18-, and 24-membered monocyclic aromatic groups, which may include from zero to four heteroatoms, such as benzene, naphthalene, anthracene, phenanthrene, chrysene, pyrene, corannulene, coronene, and the like.

"Aryl" also includes polycyclic systems having two or more rings, wherein the two or more carbons are shared to by adjacent rings (*i.e.,* "a fused ring"), wherein at least one of the rings is an aromatic ring, *e.g.*, the other cyclic ring or rings may be cycloalkyl, cycloalkenyl, cycloalkynyl, aryl and/or heterocyclic ring.

The term "substituted aryl" refers to an aryl group in which one or more hydrogen atoms on one or more aryl rings are substituted with one or more substituents including, but not limited to, halogen, azide, alkyl, aralkyl, alkenyl, alkynyl, cycloalkyl, hydroxy, alkoxyl, carbonyl (*e.g.*, ketone, aldehyde, carboxyl, alkoxycarbonyl, formoxyl, or acyl), silyl, ether, ester, thiocarbonyl (*e.g.*, thioester, thioacetate or thioformate), alkoxyl, phosphoryl, phosphate, phosphonate, phosphinate, amino (or quaternized amino), acylamino, amidine, imine, cyano, nitro, azido, sulfydryl, imino group, alkylthio group, sulphate, sulfonate, sulfamine, sulfoxide, sulfamino, sulfonyl, heterocyclic group, alkaryl, haloalkyl (*e.g.*, $CF_3$, $-CH_2-CF_3$, $-CCL_3$), -CN, aryl, heteroaryl, and a combination thereof.

"Heterocyclic ring", "heterocycle" and "heterocyclic group" are used interchangeably and refer to a cyclic group connected by a single or double ring carbon or nitrogen atom containing 3 to 10 ring atoms, preferably 5 to 6 ring atoms, consisting of carbon and one to four heteroatoms, each heteroatom is selected from non-peroxide oxygen, sulfur, and N(Y), wherein Y is absent or H, O, $C_1$-$C_{10}$ alkyl, phenyl, or benzyl, and optionally contains 1-3 double bonds and is optionally substituted with one or more substituents. A heterocyclic group is different from a heteroaryl group according to the definitions. Examples of the heterocyclic rings include, but are not limited to, piperazinyl, piperidinyl, piperidinonyl, 4-piperidinonyl, dihydrofuryl[2,3-b]tetrahydrofuran, morpholinyl, piperazinyl, piperidinyl, piperidinonyl, 4-piperidinonyl, piperonyl, pyranyl, 2H-pyrrolyl, 4H- quinolyl, quinuclidinyl, tetrahydrofuryl, and 6H-1,2,5-thiadiazinyl. The heterocyclic

group may optionally be substituted with one or more substituents as defined above for alkyl and aryl.

The term "heteroaryl" refers to a $C_5$-$C_{26}$-membered aromatic, a fused aromatic, a bis-aromatic ring system, or a combination thereof, in which one or more carbon atoms on one or more aryl ring structures have been substituted with heteroatoms. Suitable heteroatoms include, but are not limited to, oxygen, sulfur and nitrogen. As used herein, "heteroaryl" is broadly defined to include 5-, 6-, 7-, 8-, 9-, 10-, 14-, 18-, and 24-membered monocyclic aryl groups, which may include one to four heteroatoms, such as pyrrole, furan, thiophene, imidazole, oxazole, thiazole, triazole, tetrazole, pyrazole, pyridine, pyrazine, pyridazine and pyrimidine, etc. Heteroaryl group can also be referred to as "aryl heterocycle" or "heteroaromatic compound". "Heteroaryl" further includes polycyclic systems having two or more rings, wherein the two or more carbons are shared by two adjacent rings (*i.e.,* "a fused ring"), wherein at least one of the rings is heteroaromatic, for example, the other ring or rings can be cycloalkyl, cycloalkenyl, cycloalkynyl, aryl, heterocycle, or a combination thereof. Examples of heteroaromatic rings include, but are not limited to, benzimidazolyl, benzofuryl, benzothiofuryl, benzothiophenyl, benzoxazolyl, benzoxazolinyl, benzothiazolyl, benzotriazolyl, benzotetrazolyl, benzisoxazolyl, benzisothiazolyl, benzimidazolinyl, carbazolyl, 4aH-carbazolyl, carbolinyl, benzodihydropyranyl, benzopyranyl, cinnolinyl, decahydroquinolyl, 2H,6H-1,5,2-dithiazinyl, furyl, furazanyl, imidazolidinyl, imidazolinyl, imidazolyl, 1H-indazolyl, indolenyl, indolinyl, indolizinyl, indolyl, 3H-indolyl, isatinoyl, iso benzofuryl, isochromanyl, isoindazolyl, isoindolinyl, isoindolyl, isoquinolyl, isothiazolyl, isoxazolyl, methylenedioxyphenyl, naphthyridinyl, octahydroisoquinolyl, 1,2,3-oxadiazolyl, 1,2,4-oxadiazolyl, 1,2,5-oxadiazolyl, 1,3,4-oxadiazolyl, oxazolidinyl, oxazolyl, hydroxyindolyl, pyrimidinyl, phenanthridinyl, phenanthrolinyl, phenazinyl, phenothiazinyl, phenoxathinyl, phenoxazinyl, phthalazinyl, pteridyl, purinyl, pyrazinyl, pyrazolidinyl, pyrazolinyl, pyrazolyl, pyridazinyl, pyrido-oxazole, pyrido-imidazole, pyrido-thiazole, pyridinyl, pyridyl, pyrimidinyl, pyrrolidinyl, pyrrolinyl, pyrrolyl, quinazolinyl, quinolyl, quinoxalinyl, tetrahydroisoquinolyl, tetrahydroquinolyl, tetrazolyl, 1,2,3-thiadiazolyl, 1,2,4-thiadiazolyl, 1,2,5-thiadiazolyl, 1,3,4-thiadiazolyl, thianthrenyl, thiazolyl, thienyl, thienothiazolyl, thienooxazolyl, thienoimidazolyl, thiophenyl and xanthenyl. One or more rings may be substituted as defined below for "substituted heteroaryl".

The term "substituted heteroaryl" refers to a heteroaryl in which one or more hydrogen atoms on one or more heteroaryl rings are substituted with one or more substituents including, but not limited to, halogen, azide, alkyl, aralkyl, alkenyl, alkynyl, cycloalkyl, hydroxy, alkoxyl, carbonyl (*e.g.*, ketone, aldehyde, carboxyl, alkoxycarbonyl, formoxyl or acyl), silyl, ether, ester, thiocarbonyl (*e.g.*, thioester, thioacetate or thioformate), alkoxyl, phosphoryl, phosphate, phosphonate, phosphinate, amino (or quaternized amino), acylamino, amidine, imine, cyano, nitro, azido, sulfydryl, imino group, alkylthio group, sulphate, sulfonate, sulfamine, sulfoxide, sulfamino, sulfonyl, heterocyclic group, alkaryl, haloalkyl (*e.g.*, $CF_3$, $-CH_2-CF_3$, $-CCL_3$), -CN, aryl, heteroaryl, and a combination thereof.

As used herein, "alkyl" refers to a group of saturated aliphatic groups, including linear alkyl, branched alkyl, cycloalkyl (alicyclic), alkyl-substituted cycloalkyl and cycloalkyl-substituted alkyl. In the preferred embodiments, the linear or branched alkyl has 30 or less carbon atoms in its backbone (for example, $C_1$-$C_{30}$ for a linear chain and $C_3$-$C_{30}$ for a branched chain), preferably 20 or less, more preferably 15 or less, and most preferably 10 or less. Similarly, the preferred cycloalkyl groups have 3 to 10 carbon atoms in their ring structure, and more preferably have 5, 6 or 7 carbon atoms in their ring structures. The term "alkyl" (or "lower alkyl") as used throughout the specification, examples and claims is intended to include "unsubstituted alkyl" and "substituted alkyl", and the latter refers to an alkyl group having one or more substituents that partially replaces the hydrogen on one or more carbons of the hydrocarbon backbone. Such substituents include, but are not limited to, halogen, hydroxy, carbonyl (such as carboxyl, alkoxycarbonyl, formoxyl, or acyl), thiocarbonyl (*e.g.*, thioester, thioacetate or thioformate), alkoxyl, phosphoryl, phosphate, phosphonate, phosphinate, amino, acylamino, amidine, imine, cyano, nitro, azido, sulfydryl, alkylthio group, sulphate, sulfonate, sulfamine, sulfoxide, sulfamino, sulfonyl, heterocyclic group, aralkyl, aromatic or heteroaromatic moiety.

Unless the number of carbon atoms is otherwise specified, "lower alkyl" as used herein refers to an alkyl group as defined above, but having 1 to 10 carbon atoms, preferably 1 to 6 carbon atoms, in its backbone structure. Similarly, "lower alkenyl" and "lower alkynyl" have similar chain lengths. Throughout the application, the preferred alkyl group is a lower alkyl. In the preferred embodiments, the substituent designated herein as alkyl is a lower alkyl.

"Alkyl" includes one or more substitutions at one or more carbon atoms of a hydrocarbyl and a heteroalkyl. Suitable substituents include, but are not limited to, halogen, such as fluorine, chlorine, bromine, or iodine; hydroxy; -NRR', wherein R and R' are independently hydrogen, alkyl, or aryl, and wherein the nitrogen atom is optionally quaternized; -SR, wherein R is hydrogen, alkyl, or aryl; -CN; $-NO_2$; -COOH; carboxylate; -COR , -COOR or $CON(R)_2$, wherein R is hydrogen, alkyl, or aryl; azide, aralkyl, alkoxyl, imino group, phosphonate, phosphinate, silyl, ether, sulfonyl, sulfamino, heterocyclic group, aromatic or heteroaromatic moiety, haloalkyl (*e.g.*, $-CF_3$, $-CH_2-CF_3$, $-CCl_3$); CN; $-NCOCOCH_2CH_2$; NCOCOCH; -NCS; and a combination thereof.

It will be appreciated by those skilled in the art that the substituted moiety of the hydrocarbon chain may itself be substituted if appropriate. For example, the substituents for alkyl may include halogen, hydroxy, nitro, thiol, amino, azido, imino group, acylamino, phosphoryl (including phosphonate and phosphinate), sulfonyl (including sulfate, sulfamino, sulfamine, sulfoxide, and sulfonate), and silyl, as well as ether, alkylthio group, carbonyl (including ketone, aldehyde, carboxylate, and ester), haloalkyl, -CN, and the like. Cycloalkyl may be substituted in the same manner.

The terms "alkenyl" and "alkynyl" refer to the unsaturated aliphatic groups similar in length and possible substituents to the alkyl described above, but containing at least one double or triple bond, respectively.

The term "substituted alkenyl" refers to an alkenyl moiety having one or more substituents substituting one or more hydrogen atoms on one or more carbons of the hydrocarbon backbone. Such substituents include, but are not limited to, halogen, azide, alkyl, aralkyl, alkenyl, alkynyl, cycloalkyl, hydroxy, carbonyl (*e.g.*, carboxyl, alkoxycarbonyl, formoxyl, or acyl), silyl, ether, ester, thiocarbonyl (*e.g.*, thioester, thioacetate or thioformate), alkoxyl, phosphoryl, phosphate, phosphonate, phosphinate, amino (or quaternized amino), acylamino, amidine, imine, cyano, nitro, azido, sulfydryl, alkylthio group, sulphate, sulfonate, sulfamine, sulfoxide, sulfamino, sulfonyl, heterocyclic group, alkaryl, haloalkyl, -CN, aryl, heteroaryl, and a combination thereof.

The term "substituted alkynyl" refers to an alkynyl moiety having one or more substituents substituting one or more hydrogen atoms on one or more carbons of the hydrocarbon backbone. Such substituents include, but are not limited to, halogen, azide, alkyl, aralkyl, alkenyl, alkynyl, cycloalkyl, hydroxy, carbonyl (*e.g.*, carboxyl, alkoxycarbonyl, formoxyl, or acyl), silyl, ether, ester, thiocarbonyl (*e.g.*, thioester, thioacetate or thioformate), alkoxyl, phosphoryl, phosphate, phosphonate, phosphinate, amino (or quaternized amino), acylamino, amidine, imine, cyano, nitro, azido, sulfydryl, alkylthio group, sulphate, sulfonate, sulfamine, sulfoxide, sulfamino, sulfonyl, heterocyclic group, alkaryl, haloalkyl, -CN, aryl, heteroaryl, and a combination thereof.

As used herein, "amino" and "amine" are recognized in the art and refer to a substituted and unsubstituted amine, *e.g.,* a moiety that can be represented by the following general formula:

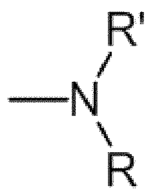

or

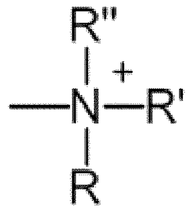

wherein, R, R', and R" each independently represents hydrogen, substituted or unsubstituted alkyl, substituted or unsubstituted alkenyl, substituted or unsubstituted alkynyl, substituted or unsubstituted carbonyl, $-(CH_2)_m-R'''$, or R and R' together with the N atoms to which they are attached form a heterocyclic ring having 3 to 14 atoms in the ring structure; R''' represents hydroxy, substituted or unsubstituted carbonyl, aryl, cycloalkyl ring, cycloalkenyl ring, heterocyclic ring or polycyclic ring; m is 0 or an integer selected from 1 to 8. In the preferred embodiments, only one of R and R' can be carbonyl, for example, R and R' together with nitrogen do not form a diimide. In the preferred embodiments, R and R' (and optionally R") each independently represents a hydrogen atom, substituted or unsubstituted alkyl, substituted or unsubstituted alkenyl or $-(CH_2)_m-R'''$. Accordingly, the term "alkylamine" as used herein refers to an amine group as defined above having a substituted or unsubstituted alkyl attached thereto (*i.e.,* at least one of R, R' or R" is alkyl).

As used herein, "carbonyl" is recognized in the art and includes the moiety which may be represented by the following general formula:

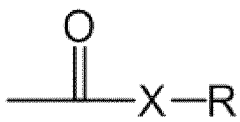

or

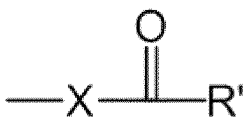

wherein, X is a bond, or represents oxygen or sulfur; R represents hydrogen, substituted or unsubstituted alkyl, substituted or unsubstituted alkenyl, substituted or unsubstituted alkynyl, substituted or unsubstituted cycloalkyl, substituted or unsubstituted heterocyclic group, substituted or unsubstituted alkaryl, substituted or unsubstituted aralkyl, substituted or unsubstituted aryl, or substituted or unsubstituted heteroaryl, $-(CH_2)_m-R''$ or a pharmaceutically acceptable salt; R' represents hydrogen, substituted or unsubstituted alkyl, substituted or unsubstituted alkenyl, substituted or unsubstituted alkynyl, substituted or unsubstituted cycloalkyl, substituted or unsubstituted heterocyclic group, substituted or unsubstituted alkaryl, substituted or unsubstituted aralkyl, substituted or unsubstituted aryl, or substituted or unsubstituted heteroaryl or $(CH_2)_m-R''$; R" represents hydroxy, substituted or unsubstituted carbonyl, aryl, cycloalkyl ring, cycloalkenyl ring, heterocyclic ring, or polycyclic ring; and m is 0 or an integer selected from 1 to 8. Wherein, X is oxygen and R is as defined above, the moiety is also referred to carboxyl. When X is oxygen and R is hydrogen, the formula represents "carboxylic acid". Wherein, X is oxygen and R' is hydrogen, the formula represents "formic acid". Wherein, X is oxygen and R or R' is not hydrogen, the formula represents "ester". Typically, where the oxygen atom of the above formula is replaced by a sulphur atom, the formula represents 'thiocarbonyl' group. Wherein, X is sulphur and R or R' is not hydrogen, the formula represents 'thioester'. Wherein, X is sulfur and R is hydrogen, the formula represents 'thiocarboxylic acid'.

Wherein, X is sulfur and R' is hydrogen, the formula represents "thioformate". Wherein, X is a bond and R is not hydrogen, the above formula represents 'ketone'. Wherein, X is a bond and R is hydrogen, the above formula represents 'aldehyde'.

The term "substituted carbonyl" refers to carbonyl as defined above,

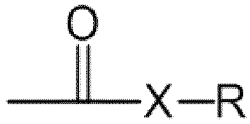 or 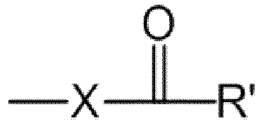

wherein one or more hydrogen atoms in the group to which R, R' or portion thereof is attached are independently substituted. Such substituents include, but are not limited to, halogen, azide, alkyl, aralkyl, alkenyl, alkynyl, cycloalkyl, hydroxy, carbonyl (*e.g.*, carboxyl, alkoxycarbonyl, formoxyl, or acyl), silyl, ether, ester, thiocarbonyl (*e.g.*, thioester, thioacetate or thioformate), alkoxyl, phosphoryl, phosphate, phosphonate, phosphinate, amino (or quaternized amino), acylamino, amidine, imine, cyano, nitro, azido, sulfydryl, alkylthio group, sulphate, sulfonate, sulfamine, sulfoxide, sulfamino, sulfonyl, heterocyclic group, alkaryl, haloalkyl, -CN, aryl, heteroaryl, and a combination thereof.

The term "carboxyl" is defined above for the following formula:

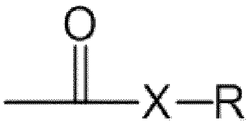 or 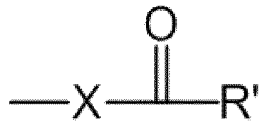

and more specifically defined by the formula -$R^{iv}$COOH, wherein $R^{iv}$ is alkyl, alkenyl, alkynyl, cycloalkyl, heterocyclic group, alkaryl, aralkyl, aryl or heteroaryl. In the preferred embodiments, the linear or branched alkyl, alkenyl and alkynyl groups have 30 or less carbon atoms (for example, $C_1$-$C_{30}$ for a linear alkyl, $C_3$-$C_{30}$ for a branched alkyl, $C_2$-$C_{30}$ for a linear alkenyl and alkynyl, $C_3$-$C_{30}$ for a branched alkenyl and alkynyl), preferably 20 or less, more preferably 15 or less, and most preferably 10 or less in their backbone. Similarly, the preferred cycloalkyl, heterocyclic group, aryl and heteroaryl have 3 to 10 carbon atoms in their ring structures, and more preferably have 5, 6 or 7 carbon atoms in the ring structures.

The term "substituted carboxyl" refers to the carboxyl as defined above in which one or more hydrogen atoms in $R^{iv}$ are substituted. Such substituents include, but are not limited to, halogen, azide, alkyl, aralkyl, alkenyl, alkynyl, cycloalkyl, hydroxy, carbonyl (*e.g.*, carboxyl, alkoxycarbonyl, formoxyl, or acyl), silyl, ether, ester, thiocarbonyl (*e.g.*, thioester, thioacetate or thioformate), alkoxyl, phosphoryl, phosphate, phosphonate, phosphinate, amino (or quaternized amino), acylamino, amidine, imine, cyano, nitro, azido, sulfydryl, alkylthio group, sulphate, sulfonate, sulfamine, sulfoxide, sulfamino, sulfonyl, heterocyclic group, alkaryl, haloalkyl, -CN, aryl, heteroaryl, and a combination thereof.

As used herein, "heteroalkyl" refers to a linear or branched chain or cyclic carbon-containing group containing at least one heteroatom, or a combination thereof. Suitable heteroatoms include, but are not limited to, O, N, Si, P, and S, wherein the nitrogen, phosphorus, and sulfur atoms are optionally oxidized and the nitrogen heteroatom is optionally quaternized.

Examples of the saturated hydrocarbyl include, but are not limited to, methyl, ethyl, n-propyl, isopropyl, n-butyl, tert-butyl, isobutyl, sec-butyl, cyclohexyl, (cyclohexyl)methyl, cyclopropylmethyl, as well as the homologues and isomers thereof, *e.g.*, n-pentyl, n-hexyl, n-heptyl, n-octyl. Examples of the unsaturated alkyl include, but are not limited to, vinyl, 2-propenyl, crotyl, 2-isopentenyl, 2-(butadienyl), 2,4-pentadienyl, 3-(1,4-pentadienyl), ethynyl, 1- and 3-propinyl, and 3-butynyl.

The terms "alkoxyl" or "alkoxy", "aroxy" or "aryloxy" generally describe the compounds represented by the formula-$OR^{v}$, wherein $R^{v}$ includes, but are not limited to, substituted or unsubstituted alkyl, alkenyl, alkynyl, cycloalkyl, heterocyclic group, cycloalkenyl, heterocycloalkenyl, aryl, heteroaryl, aralkyl, heteroalkyl, alkaryl, alkylheteroaryl.

As used herein, the term "alkoxyl" or "alkoxy" refers to the alkyl as defined above to which an oxygroup is attached. Representative alkoxyl includes methoxy, ethoxy, propoxy, tert-butoxy, and the like. "Ether" comprises two hydrocarbons covalently linked by oxygen. Thus, the substituents of the alkyl that make the alkyl into an ether are or are analogous to alkoxyl, and may be represented, for example, by one of -O-alkyl, -O-alkenyl, and -O-alkynyl. If the valency permits, the term alkoxyl also includes cycloalkyl, heterocyclic group, cycloalkenyl, heterocycloalkenyl and aralkyl, which has an oxygroup attached to at least one carbon atom.

The term "substituted alkoxyl" refers to an alkoxyl having one or more substituents that replace one or more hydrogen atoms on one or more carbons of the alkoxyl backbone. Such substituents include, but are not limited to, halogen, azide, alkyl, aralkyl, alkenyl, alkynyl, cycloalkyl, hydroxy, carbonyl (*e.g.*, carboxyl, alkoxycarbonyl, formoxyl, or acyl), silyl, ether, ester, thiocarbonyl (*e.g.*, thioester, thioacetate or thioformate), alkoxyl, phosphoryl, phosphate, phosphonate, phosphinate, amino (or quaternized amino), acylamino, amidine, imine, cyano, nitro, azido, sulfydryl, alkylthio group, sulphate, sulfonate, sulfamine, sulfoxide, sulfamino, sulfonyl, heterocyclic group, alkaryl, haloalkyl, -CN,

aryl, heteroaryl, and a combination thereof.

The term "alkylthio group" refers to an alkyl as defined above to which a sulfur group is attached. The "alkylthio" moiety is represented by -S-alkyl. Representative alkylthio group includes methylthio, ethylthio, and the like. The term "alkylthio group" also includes a cycloalkyl to which a sulfur group is attached.

The term "substituted alkylthio group" refers to an alkylthio group having one or more substituents that replace one or more hydrogen atoms on one or more carbon atoms of the alkylthio backbone. Such substituents include, but are not limited to, halogen, azide, alkyl, aralkyl, alkenyl, alkynyl, cycloalkyl, hydroxy, carbonyl (*e.g.*, carboxyl, alkoxycarbonyl, formoxyl, or acyl), silyl, ether, ester, thiocarbonyl (*e.g.*, thioester, thioacetate or thioformate), alkoxyl, phosphoryl, phosphate, phosphonate, phosphinate, amino (or quaternized amino), acylamino, amidine, imine, cyano, nitro, azido, sulfydryl, alkylthio group, sulphate, sulfonate, sulfamine, sulfoxide, sulfamino, sulfonyl, heterocyclic group, alkaryl, haloalkyl, -CN, aryl, heteroaryl, and a combination thereof.

"Arylthio" refers to -S-aryl or -S-heteroaryl, wherein the aryl and heteroaryl are as defined herein.

The term "substituted arylthio" represents the -S-aryl or -S-heteroaryl having one or more substituents that replace the hydrogen atoms on one or more ring atoms of the aryl and heteroaryl rings as defined herein. Such substituents include, but are not limited to, halogen, azide, alkyl, aralkyl, alkenyl, alkynyl, cycloalkyl, hydroxy, carbonyl (*e.g.*, carboxyl, alkoxycarbonyl, formoxyl, or acyl), silyl, ether, ester, thiocarbonyl (*e.g.*, thioester, thioacetate or thioformate), alkoxyl, phosphoryl, phosphate, phosphonate, phosphinate, amino (or quaternized amino), acylamino, amidine, imine, cyano, nitro, azido, sulfydryl, alkylthio group, sulphate, sulfonate, sulfamine, sulfoxide, sulfamino, sulfonyl, heterocyclic group, alkaryl, haloalkyl, -CN, aryl, heteroaryl, and a combination thereof.

As used herein, "aralkyl" refers to an alkyl substituted with a substituted or unsubstituted aryl or heteroaryl.

As used herein, "alkaryl" refers to an aryl (*e.g.*, an aromatic group or heteroaromatic group) substituted with a substituted or unsubstituted alkyl.

The term "amide" or "acylamino" can be used interchangeably and refers to "an unsubstituted acylamino group" and "an substituted acylamino group", and is represented by the generic formula:

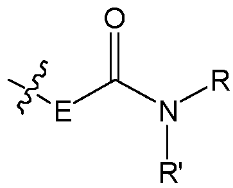

wherein, E is absent, or E is substituted or unsubstituted alkyl, substituted or unsubstituted alkenyl, substituted or unsubstituted alkynyl, substituted or unsubstituted aralkyl, substituted or unsubstituted cycloalkyl, substituted or unsubstituted aryl, substituted or unsubstituted heteroaryl, substituted or unsubstituted heterocyclic group, wherein independently of E, R and R' each independently represents hydrogen, substituted or unsubstituted alkyl, substituted or unsubstituted alkenyl, substituted or unsubstituted alkynyl, substituted or unsubstituted carbonyl, substituted or unsubstituted cycloalkyl, substituted or unsubstituted heterocyclic group, substituted or unsubstituted alkaryl, substituted or unsubstituted aralkyl, substituted or unsubstituted aryl, or substituted or unsubstituted heteroaryl, $-(CH_2)_m-R'''$, or R and R' together with the N atoms to which they are attached constitute a heterocycle having 3 to 14 atoms in the ring structure; R''' represents hydroxy, substituted or unsubstituted carbonyl, aryl, cycloalkyl ring, cycloalkenyl ring, heterocyclic ring or polycyclic ring; and m is 0 or an integer selected from 1 to 8. In the preferred embodiments, only one of R and R' can be carbonyl, for example, R and R' together with nitrogen do not form a diimide. In the preferred embodiments, R and R' each independently represent a hydrogen atom, substituted or unsubstituted alkyl, substituted or unsubstituted alkenyl or $-(CH_2)_m-R'''$. When E is oxygen, a carbamate is formed. As understood by one of ordinary skill in the art, the carbamate cannot be linked to another chemical substance, for example to form an oxygen-oxygen bond or other unstable bond.

The term "sulfonyl" is represented by the following formula:

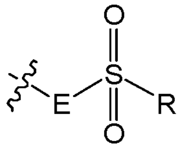

wherein, E is absent, or E is alkyl, alkenyl, alkynyl, aralkyl, alkaryl, cycloalkyl, aryl, heteroaryl, heterocyclic group, wherein independently of E, R represents hydrogen, substituted or unsubstituted alkyl, substituted or unsubstituted alkenyl, substituted or unsubstituted alkynyl, substituted or unsubstituted amine, substituted or unsubstituted cycloalkyl, substituted or unsubstituted heterocyclic group, substituted or unsubstituted alkaryl, substituted or unsubstituted aralkyl,

substituted or unsubstituted aryl, or substituted or unsubstituted heteroaryl, $-(CH_2)_m-R'''$, or E and R together with the S atoms to which they are attached constitute a heterocycle having 3 to 14 atoms in the ring structure; R‴ represents hydroxy, substituted or unsubstituted carbonyl, aryl, cycloalkyl ring, cycloalkenyl ring, heterocyclic ring or polycyclic ring; and m is 0 or an integer selected from 1 to 8. In the preferred embodiments, only one of E and R can be substituted or unsubstituted amine to form "sulfonamide" or "sulfamino". The substituted or unsubstituted amine is as defined above.

The term "substituted sulfonyl" represents a sulfonyl in which E and R are independently substituted. Such substituents include, but are not limited to, halogen, azide, alkyl, aralkyl, alkenyl, alkynyl, cycloalkyl, hydroxy, carbonyl (*e.g.*, carboxyl, alkoxycarbonyl, formoxyl, or acyl), silyl, ether, ester, thiocarbonyl (*e.g.*, thioester, thioacetate or thioformate), alkoxyl, phosphoryl, phosphate, phosphonate, phosphinate, amino (or quaternized amino), acylamino, amidine, imine, cyano, nitro, azido, sulfydryl, alkylthio group, sulphate, sulfonate, sulfamine, sulfoxide, sulfamino, sulfonyl, heterocyclic group, alkaryl, haloalkyl, -CN, aryl, heteroaryl, and a combination thereof.

The term "sulfo" refers to the sulfonyl as defined above, wherein R is hydroxy and E is absent, or E is substituted or unsubstituted cycloalkyl, substituted or unsubstituted heterocyclic group, substituted or unsubstituted alkaryl, substituted or unsubstituted aralkyl, substituted or unsubstituted aryl, or substituted or unsubstituted heteroaryl.

The term "sulphate" refers to the sulfonyl as defined above, wherein E is absent, oxygen, alkoxyl, aryloxy, substituted alkoxyl or substituted aryloxy, as defined as above; and R is independently hydroxy, alkoxyl, aryloxy, substituted alkoxyl or substituted aryloxy, as defined above. As understood by one of ordinary skill in the art, when E is oxygen, the sulphate cannot be linked to another chemical species, for example to form an oxygen-oxygen bond or other unstable bond.

The term "sulfonate" refers to the sulfonyl as defined above, wherein E is oxygen, alkoxyl, aryloxy, substituted alkoxyl, or substituted aryloxy, as defined above, and R is independently hydrogen, substituted or unsubstituted alkyl, substituted or unsubstituted alkenyl, substituted or unsubstituted alkynyl, substituted or unsubstituted amine, substituted or unsubstituted cycloalkyl, substituted or unsubstituted heterocyclic group, substituted or unsubstituted alkaryl, substituted or unsubstituted aralkyl, substituted or unsubstituted aryl, or substituted or unsubstituted heteroaryl, $-(CH_2)_m-R'''$, R‴ represents hydroxy, substituted or unsubstituted carbonyl, aryl, cycloalkyl ring, cycloalkenyl ring, heterocyclic ring or polycyclic ring; and m is 0 or an integer selected from 1 to 8. As understood by one of ordinary skill in the art, when E is oxygen, the sulfonate cannot be linked to another chemical species, for example to form an oxygen-oxygen bond or other unstable bond.

The term "sulfamine" refers to a sulfonamide or a sulfonamide represented by the following formula:

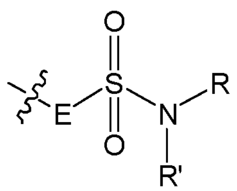

wherein, E is absent, or E is substituted or unsubstituted alkyl, substituted or unsubstituted alkenyl, substituted or unsubstituted alkynyl, substituted or unsubstituted aralkyl, substituted or unsubstituted alkaryl, substituted or unsubstituted cycloalkyl, substituted or unsubstituted aryl, substituted or unsubstituted heteroaryl, substituted or unsubstituted heterocyclic group, wherein E, R and R' each independently represents hydrogen, substituted or unsubstituted alkyl, substituted or unsubstituted alkenyl, substituted or unsubstituted alkynyl, substituted or unsubstituted carbonyl, substituted or unsubstituted cycloalkyl, substituted or unsubstituted heterocyclic group, substituted or unsubstituted alkaryl, substituted or unsubstituted aralkyl, substituted or unsubstituted aryl, or substituted or unsubstituted heteroaryl, $-(CH_2)_m-R'''$, or R and R' together with the N atoms to which they are attached constitute a heterocycle having 3 to 14 atoms in the ring structure; R‴ represents hydroxy, substituted or unsubstituted carbonyl, aryl, cycloalkyl ring, cycloalkenyl ring, heterocyclic ring or polycyclic ring; and m is 0 or an integer selected from 1 to 8. In the preferred embodiments, only one of R and R' can be carbonyl, for example, R and R' together with nitrogen do not form a diimide.

The term "sulfoxide" is represented by the following formula:

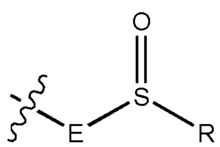

wherein, E is absent, or E is alkyl, alkenyl, alkynyl, aralkyl, alkaryl, cycloalkyl, aryl, heteroaryl, heterocyclic group, wherein independently of E, R represents hydrogen, substituted or unsubstituted alkyl, substituted or unsubstituted alkenyl,

substituted or unsubstituted alkynyl, substituted or unsubstituted amine, substituted or unsubstituted cycloalkyl, substituted or unsubstituted heterocyclic group, substituted or unsubstituted alkaryl, substituted or unsubstituted aralkyl, substituted or unsubstituted aryl, or substituted or unsubstituted heteroaryl, $-(CH_2)_m-R'''$, or E and R together with the S atoms to which they are attached constitute a heterocycle having 3 to 14 atoms in the ring structure; R‴ represents hydroxy, substituted or unsubstituted carbonyl, aryl, cycloalkyl ring, cycloalkenyl ring, heterocyclic ring or polycyclic ring; and m is 0 or an integer selected from 1 to 8.

The term "phosphono" is represented by the following formula:

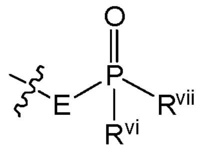

wherein, E is absent, or E is substituted or unsubstituted alkyl, substituted or unsubstituted alkenyl, substituted or unsubstituted alkynyl, substituted or unsubstituted aralkyl, substituted or unsubstituted alkaryl, substituted or unsubstituted cycloalkyl, substituted or unsubstituted aryl, substituted or unsubstituted heteroaryl, substituted or unsubstituted heterocyclic group, wherein independently of E, $R^{vi}$ and $R^{vii}$ each independently is hydrogen, substituted or unsubstituted alkyl, substituted or unsubstituted alkenyl, substituted or unsubstituted alkynyl, substituted or unsubstituted carbonyl, substituted or unsubstituted cycloalkyl, substituted or unsubstituted heterocycle, substituted or unsubstituted alkaryl, substituted or unsubstituted aralkyl, substituted or unsubstituted aryl, or substituted or unsubstituted heteroaryl, $-(CH_2)_m-R'''$, or R and R' together with the P atoms to which they are attached constitute a heterocycle having 3 to 14 atoms in the ring structure; R‴ represents hydroxy, substituted or unsubstituted carbonyl, aryl, cycloalkyl ring, cycloalkenyl ring, heterocyclic ring or polycyclic ring; and m is 0 or an integer selected from 1 to 8.

The term "substituted phosphono" represents the phosphono in which E, $R^{vi}$ and $R^{vii}$ are independently substituted. Such substituents include, but are not limited to, halogen, azide, alkyl, aralkyl, alkenyl, alkynyl, cycloalkyl, hydroxy, carbonyl (e.g., carboxyl, alkoxycarbonyl, formoxyl, or acyl), silyl, ether, ester, thiocarbonyl (*e.g.*, thioester, thioacetate or thioformate), alkoxyl, phosphoryl, phosphate, phosphonate, phosphinate, amino (or quaternized amino), acylamino, amidine, imine, cyano, nitro, azido, sulfydryl, alkylthio group, sulphate, sulfonate, sulfamine, sulfoxide, sulfamino, sulfonyl, heterocyclic group, alkaryl, haloalkyl, -CN, aryl, heteroaryl, and a combination thereof.

The term "phosphoryl" defines a phosphoryl in which E is absent, is an oxygen, alkoxyl, aryloxy, substituted alkoxyl or substituted aryloxy, as defined above, and independently of E, $R^{vi}$ and $R^{vii}$ are independently hydroxy, alkoxyl, aryloxy, substituted alkoxyl or substituted aryloxy, as defined above. When E is oxygen, the phosphoryl cannot be linked to another chemical species, for example to form an oxygen-oxygen bond or other unstable bond, as understood by one of ordinary skill in the art. When E, $R^{vi}$ and $R^{vii}$ are substituted, the substituents include, but are not limited to halogen, azide, alkyl, aralkyl, alkenyl, alkynyl, cycloalkyl, hydroxy, carbonyl (e.g., carboxyl, alkoxycarbonyl, formoxyl, or acyl), silyl, ether, ester, thiocarbonyl (*e.g.*, thioester, thioacetate or thioformate), alkoxyl, phosphoryl, phosphate, phosphonate, phosphinate, amino (or quaternized amino), acylamino, amidine, imine, cyano, nitro, azido, sulfydryl, alkylthio group, sulphate, sulfonate, sulfamine, sulfoxide, sulfamino, sulfonyl, heterocyclic group, alkaryl, haloalkyl, -CN, aryl, heteroaryl, and a combination thereof.

The term "$C_3$-$C_{20}$ cyclic group" refers to a substituted or unsubstituted cycloalkyl, a substituted or unsubstituted cycloalkenyl, a substituted or unsubstituted cycloalkynyl, a substituted or unsubstituted heterocyclic group having 3 to 20 carbon atoms, where geometrical constraints permit. The ring structure is formed by a monocyclic or fused ring system. The substituted cycloalkyl, cycloalkenyl, cycloalkynyl and heterocyclic group are substituted as defined above for alkyl, alkenyl, alkynyl and heterocyclic group, respectively.

As used herein, the term "a lysosomal-directed unit" refers to a lysosomal-directed unit (*e.g.,* $R_5$' or $R_6$' as described herein) contained in the active agent of the present invention, which usually has an amine-containing, weakly basic group, such as a morpholine ring, or dimethylethylenediamine. The active agent of the present invention containing the lysosomal-directed unit is readily accessible to lysosomes.

The linker Z described herein is selected from CL2A, MCC, VC, MC-VC-PAB, MC-GGFG, SPDB, MHH, which are well known and commonly used linkers in the art, and the structures thereof are shown below:

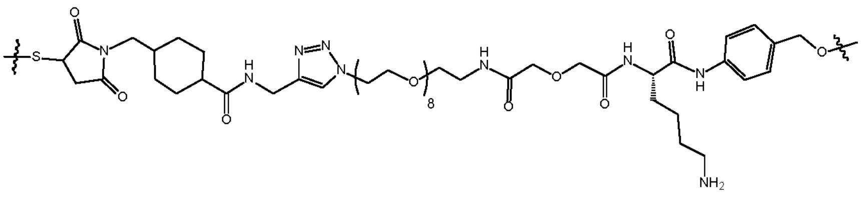

CL2A,

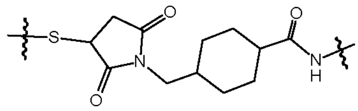

MCC,

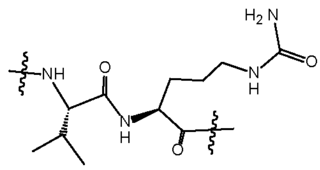

VC,

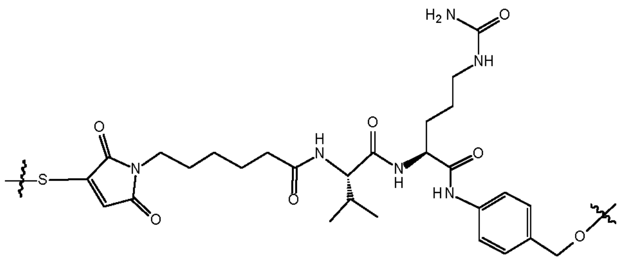

MC-VC-PAB,

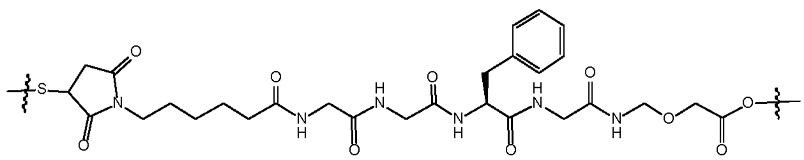

MC-GGFG,

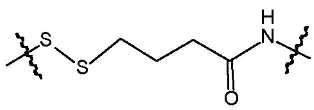

SPDB,

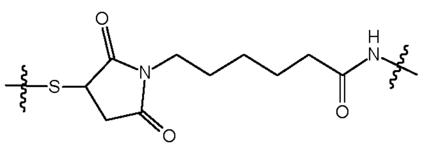

MHH.

II. Compositions

A. Anti-senescence prodrug compounds

The anti-senescence prodrugs disclosed herein are selectively designed based on the cytotoxic agents to introduce the sites that can be cleaved by SA-β-gal after *in vivo* delivery of the prodrug to release the active parent cytotoxic agent for preferential killing of the senescent cells.

In the preferred embodiments, the cytotoxic agent is A1331852.

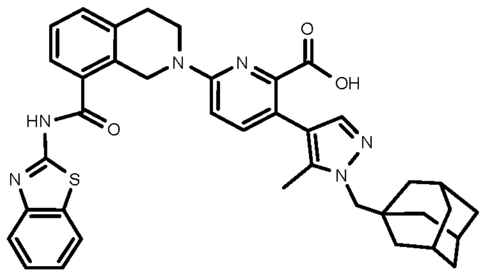

A1331852

The cytotoxic agent may also be a derivative of A1331852. For example, the adamantyl of A1331852 is substituted with a group. For example, the cytotoxic moiety is

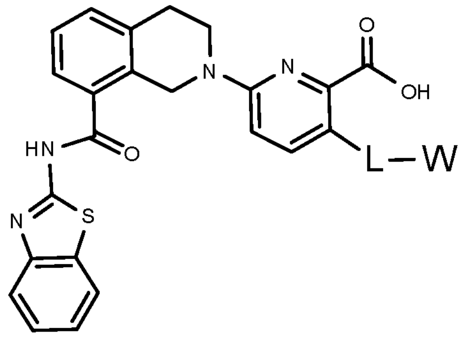

an A1331852 derivative,

wherein the linking group L is selected from substituted or unsubstituted alkyl, substituted or unsubstituted alkoxyl, substituted or unsubstituted aryl, substituted or unsubstituted heteroaryl, substituted or unsubstituted cyclic group, and substituted or unsubstituted heterocyclic group.

L is preferably a linking group selected from the following structures:

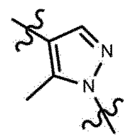 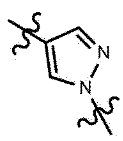 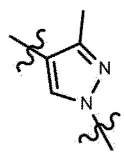

which is linked to the W group at the nitrogen atom in the ring; and

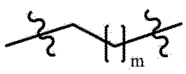 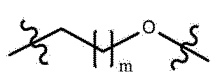 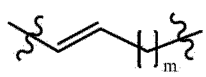 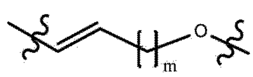;

m is 0, 1, 2, or 3;

L is most preferably a linking group selected from the following structures:

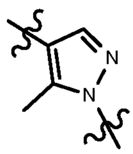 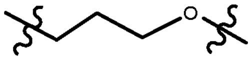 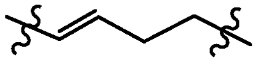

W is selected from the substituted or unsubstituted cyclic group, substituted or unsubstituted heterocyclic alkyl, substituted or unsubstituted aryl, substituted or unsubstituted heteroaryl, substituted or unsubstituted bridging cycloalkyl, substituted or unsubstituted hetero-bridging cycloalkyl, substituted or unsubstituted spirocycloalkyl, substituted or unsubstituted hetero-spirocycloalkyl, substituted or unsubstituted fused cycloaryl, and substituted or unsubstituted fused heterocycloaryl.

In some embodiments, wherein W is the substituted $C_3$-$C_{10}$ cyclic group, unsubstituted $C_3$-$C_{10}$ cyclic group or substituted $C_3$-$C_{10}$ heterocyclic group, unsubstituted $C_3$-$C_{10}$ heterocyclic group, substituted $C_6$-$C_{10}$ aryl, unsubstituted $C_6$-$C_{10}$ aryl, unsubstituted $C_6$-$C_{10}$ heteroaryl, unsubstituted $C_6$-$C_{10}$ heteroaryl, unsubstituted $C_6$-$C_{12}$ bridging cyclic group, substituted $C_6$-$C_{12}$ bridging cyclic group, unsubstituted $C_6$-$C_{12}$ hetero-bridging cyclic group, substituted $C_6$-$C_{12}$ hetero-bridging cyclic group, unsubstituted $C_6$-$C_{12}$ fused cyclic group, substituted $C_6$-$C_{12}$ fused cyclic group, unsubstituted $C_6$-$C_{12}$ hetero fused cyclic group, substituted $C_6$-$C_{12}$ hetero fused cyclic group, unsubstituted $C_6$-$C_{12}$ fused cycloaryl, substituted $C_6$-$C_{12}$ fused cycloaryl, unsubstituted $C_6$-$C_{12}$ fused heterocycloaryl, substituted $C_6$-$C_{12}$ fused heterocycloaryl.

In the preferred embodiments, W is selected from the following groups:

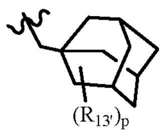,
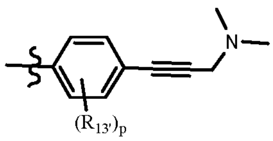,
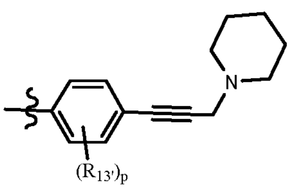,
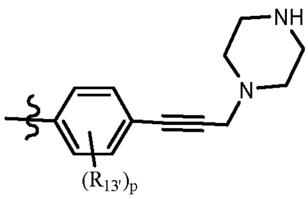,
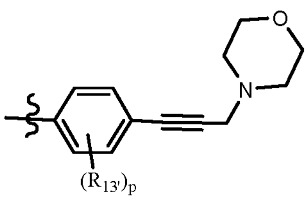,
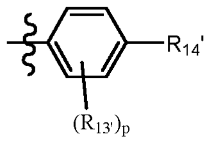,
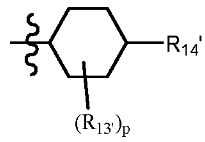,
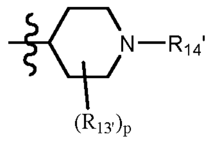,
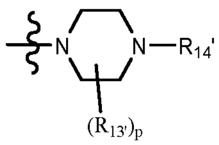,
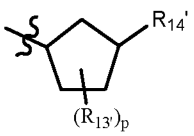,
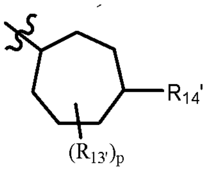,
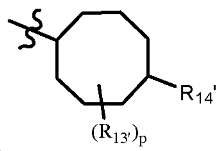

wherein, $R_{13'}$ is hydrogen, deuterium, halogen, amino, hydroxy, nitro, thiol, cyano, isocyano, substituted or unsubstituted alkyl, substituted or unsubstituted alkoxyl, alkylamino, dialkylamino, carboxyl, substituted or unsubstituted carbonyl, acylamino, sulfonyl, sulfo, phosphoryl and phosphono; wherein, $R_{14}$' is hydrogen, deuterium, substituted or unsubstituted alkyl, substituted or unsubstituted aryl, substituted or unsubstituted heteroaryl, substituted or unsubstituted cyclic group, and substituted or unsubstituted heterocyclic group; wherein, p is 0, 1, 2, 3, or 4.

In some embodiments, the cytotoxic agent is:

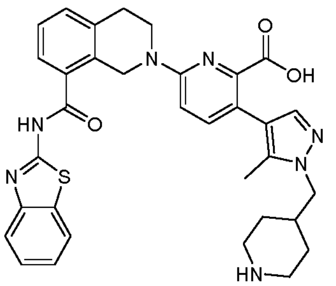,
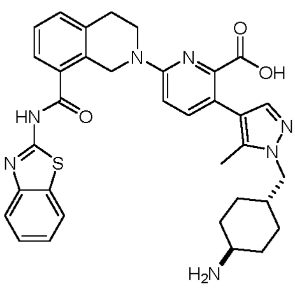,

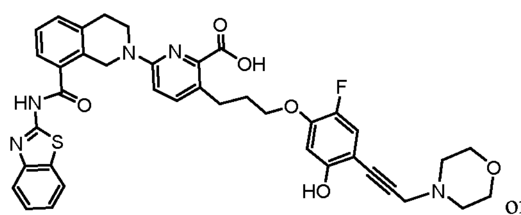 or 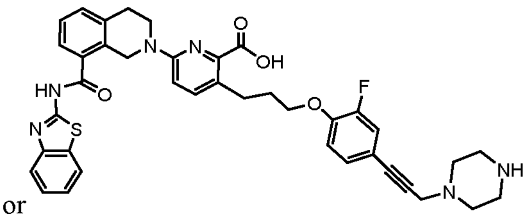.

Therefore, the prodrug compounds disclosed herein include the cytotoxic agent, exemplified herein by A1331852 modified to be cleaved by SA-β-gal. The cytotoxic agent is modified to include a preferred acetylated galactose-based moiety, and a benzyloxycarbonyl. Therefore, in the preferred embodiments, the prodrug does not include a free hydroxy (-OH) on the galactose-based moiety. In some embodiments, the prodrug may comprise a modification (*e.g.*, removal of the nitro (*e.g.,* $-NO_2$) on the aryl or heteroaryl (*e.g.,* phenyl) of the benzyloxycarbonyl moiety or addition of other functional groups thereto) to reduce its potential immunogenicity. In some embodiments, the prodrug may comprise the nitro (*e.g.,* $-NO_2$) on the aryl or heteroaryl (*e.g.,* phenyl) in the benzyloxycarbonyl moiety of the prodrug, which may, *e.g.*, affect the efficiency of the prodrug to produce a cytotoxic agent upon the activation of β-galactosidase. In some embodiments, the prodrug may comprise further modifications (*e.g.*, removal of one or more protecting groups (*e.g.*, acetyl (Ac) groups) from the galactose-based moiety) to increase its water solubility, which may, *e.g.,* facilitate the formation of stable crystalline forms, simplify the *in vivo* metabolic pathway, and/or obtain the better drug-like properties.

In some forms, the benzyloxycarbonyl moiety has the structure as shown below:

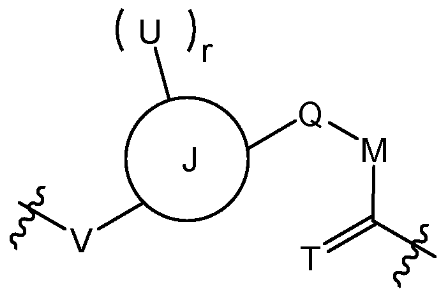

Formula 1

wherein:

J is aryl or heteroaryl, preferably $C_6$ aryl, such as phenyl,
V is O, S or $NR_1$', wherein $R_1$' is hydrogen, substituted $C_1$-$C_5$ alkyl, unsubstituted $C_1$-$C_5$ alkyl, substituted $C_6$-$C_{10}$ aryl, or unsubstituted $C_6$-$C_{10}$ aryl, wherein preferably, V is O,
Q is substituted $C_1$-$C_5$ alkylene or unsubstituted $C_1$-$C_5$ alkylene, *e.g.*, unsubstituted $C_1$ alkylene, substituted $C_1$ alkylene, unsubstituted $C_2$ alkylene, substituted $C_2$ alkylene, unsubstituted $C_3$ alkylene, substituted $C_3$ alkylene, unsubstituted $C_4$ alkylene, substituted $C_4$ alkylene, unsubstituted $C_1$ alkylene, substituted $C_5$ alkylene, wherein preferably Q is methylene,
M is O, S or $NR_2$', wherein $R_2$' is hydrogen, substituted $C_1$-$C_5$ alkyl, unsubstituted $C_1$-$C_5$ alkyl, substituted $C_6$-$C_{10}$ aryl or unsubstituted $C_6$-$C_{10}$ aryl, wherein preferably M is O,
T is O, S or $NR_3$', wherein $R_3$' is hydrogen, substituted $C_1$-$C_5$ alkyl, unsubstituted $C_1$-$C_5$ alkyl, substituted $C_6$-$C_{10}$ aryl or unsubstituted $C_6$-$C_{10}$ aryl, wherein preferably, T is O,
Each U is independently nitro (*e.g.,* $-NO_2$), cyano *(e.g.,* -CN), amino (*e.g.,* $-NH_2$), hydroxy (-OH), thiol (-SH), halogen (*e.g.*, F, Cl, I, Br), alkoxyl, alkylamino, dialkylamino, substituted alkoxyl, carboxyl, carbonyl, substituted carbonyl, acylamino, sulfonyl, sulfonic acid, phosphoryl, phosphono, hydroxy, alkyl, substituted alkyl, wherein preferably U is $-NO_2$ or hydrogen, and
r is an integer between 0 and 10, including, for example, 0, 1, 2, 3, 4, 5, 6, 7, 8, 9, and 10, if the valence permits.

In some forms of Formula 1, J is $C_6$ aryl. In some forms, U is $-NO_2$. In some forms, U is hydrogen. In some forms, V is O. In some forms of Formula 1, T is O. In some forms, Q is substituted $C_1$ alkylene. In some forms of Formula 1, Q is substituted $C_1$ alkylene. In some forms, M is O.

In some forms of Formula 1, J is $C_6$ aryl and Q is substituted $C_1$ alkylene or substituted $C_1$ alkylene. In some forms of Formula 1, J is $C_6$ aryl, Q is substituted $C_1$ alkylene or substituted $C_1$ alkylene, and V and T are O.

In some forms of Formula 1, J is $C_6$ aryl, Q is substituted $C_1$ alkylene or substituted $C_1$ alkylene, and V, M and T are O.

In some forms of Formula 1, the benzyloxycarbonyl is the linker X as shown in any one of (a)-(j) of Formula II:

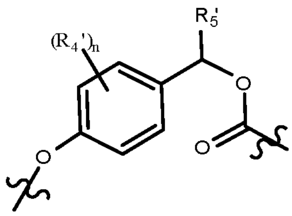 (a), 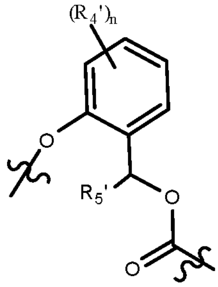 (b), 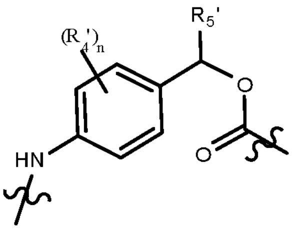 (c),

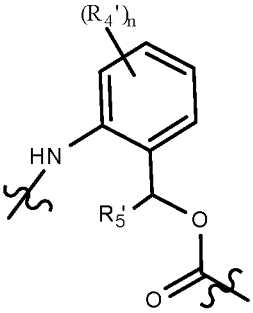 (d), 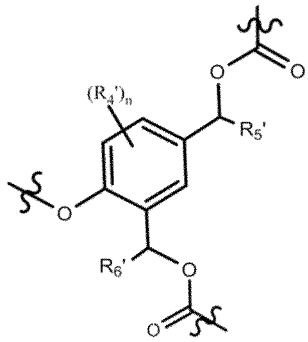 (e), 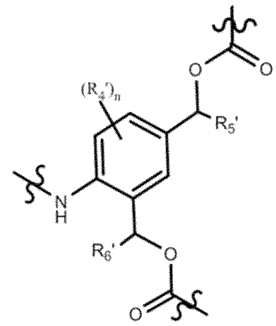 (f),

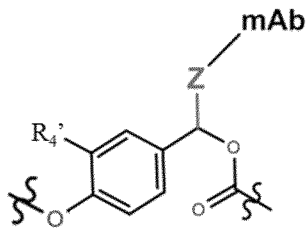 (g) , 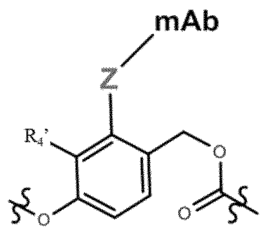 (h), 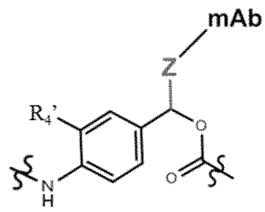 (i),

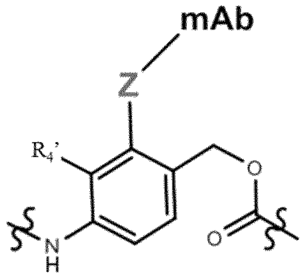 (j)

wherein, each of $R_4$', $R_5$' and $R_6$' is independently selected from hydrogen, nitro (e.g., $-NO_2$), cyano (*e.g.,* -CN), isocyano, amino (*e.g.,* $-NH_2$), hydroxy (-OH), thiol (-SH), halogen (*e.g.,* F, Cl, I, Br), substituted or unsubstituted alkyl, substituted or unsubstituted alkoxyl, alkylamino, dialkylamino, carboxy, substituted or unsubstituted carbonyl, acylamino, sulfonyl, sulfo, phosphoryl, and phosphono.

mAb is a monoclonal antibody, and the linking group Z is a linking group for the antibody conjugation,

n is an integer between 0 and 4, including 0 and 4,

wherein the linker X is linked to the β-gal moiety at the

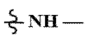

or

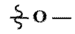

linking position, and to the cytotoxic moiety at the

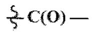

linking position,

wherein, the cytotoxic moieties linked at the two

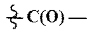

linking positions are the same or different in the linker X of Formulae (e) and (f).

In some embodiments, at least one $R_4$' is hydrogen, nitro (*e.g.,* $-NO_2$), cyano (*e.g.,* -CN), amino (*e.g.,* $-NH_2$), hydroxy (-OH), thiol (-SH), halogen (*e.g.,* F, Cl, I, Br), substituted or unsubstituted alkyl, substituted or unsubstituted alkoxyl, alkylamino, dialkylamino, carboxyl, substituted or unsubstituted carbonyl, acylamino, sulfonyl, sulfo, phosphoryl, or phosphono.
In some embodiments, at least one $R_4$' is hydrogen, nitro (*e.g.,* $-NO_2$), cyano (*e.g.,* -CN), amino (*e.g.,* $-NH_2$), hydroxy (-OH), thiol (-SH), or halogen (*e.g.,* F, Cl, I, Br).
In some embodiments, at least one $R_4$' is hydrogen or nitro.
In some embodiments, at least one $R_4$' is nitro, which may, for example, increase the efficiency to produce a cytotoxic agent upon activation.
In some embodiments, at least one $R_4$' is hydrogen (rather than nitro (*e.g.*, $-NO_2$)), which may, for example, reduce the potential immunogenicity.
In some embodiments, $R_5$' is hydrogen, alkyl, or substituted alkyl.
In some embodiments, $R_5$' is hydrogen.
In some embodiments, $R_6$' is hydrogen.
In some embodiments, the linking group Z is selected from CL2A, MCC, VC, MC-VC-PAB, MC-GGFG, SPDB, MHH, and has the following structure:

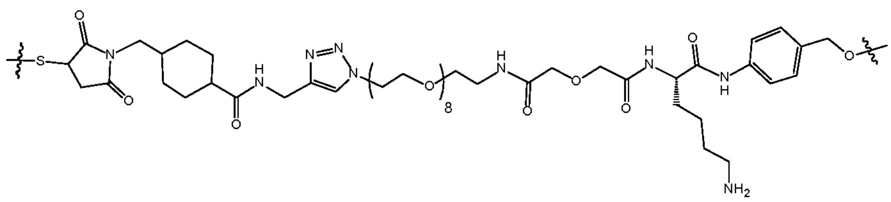

CL2A,

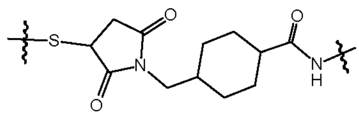

MCC,

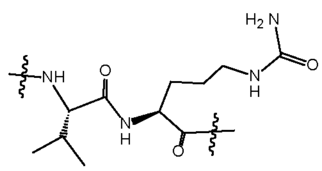

VC,

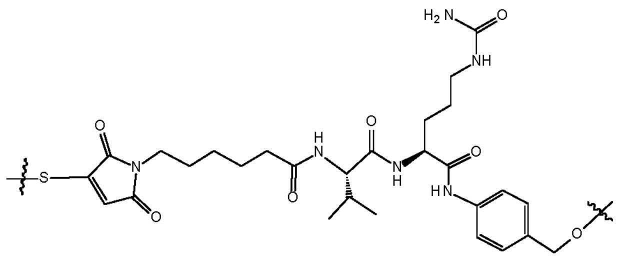
MC-VC-PAB,

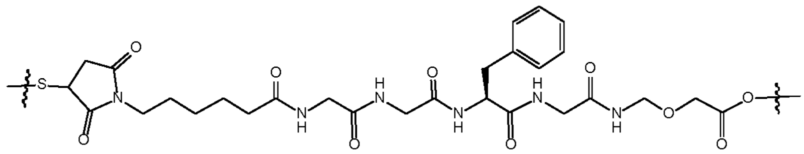
MC-GGFG,

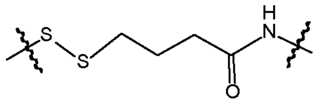
SPDB,

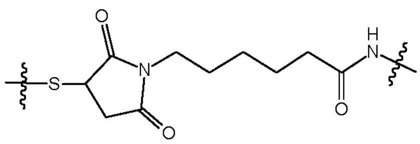
MHH.

In some embodiments, $R_5$' and $R_6$' are independently lysosome guidance units as shown below:

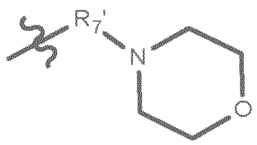

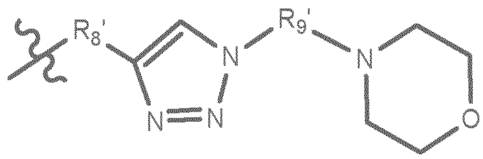

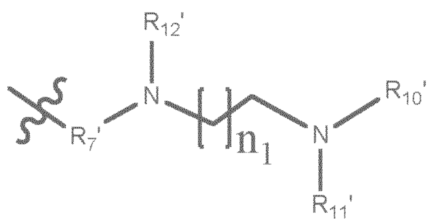

wherein, $R_7$', $R_8$' and $R_9$' are independently absent, alkylene, alkyleneoxyl, alkylene amino or alkylene carbonyl; wherein, $R_{10}$', $R_{11}$' and $R_{12}$' are independently selected from hydrogen, nitro, cyano, isocyano, amino, hydroxy, thiol, halogen, substituted or unsubstituted alkyl, substituted or unsubstituted alkoxyl, alkylamino, dialkylamino, carboxy, substituted or unsubstituted carbonyl, acylamino, sulfonyl, sulfo, phosphoryl, and phosphono;

$n_1$ is 0, 1, 2, 3 or 4.

$R_5$' and $R_6$' are independently selected from the following structures:

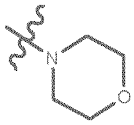 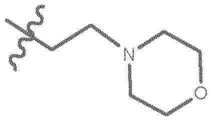 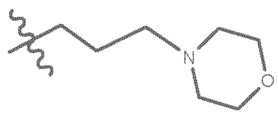 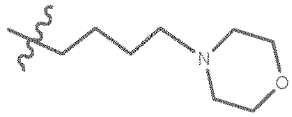

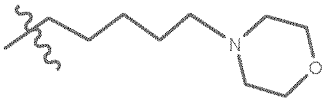 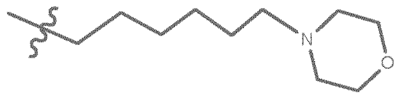

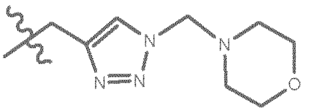 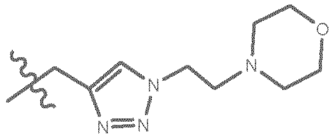 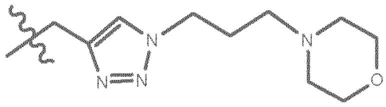

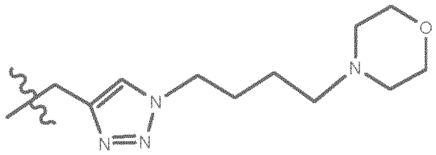

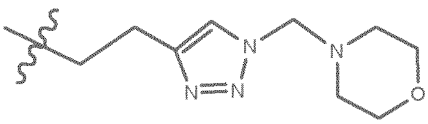 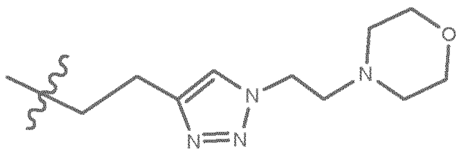

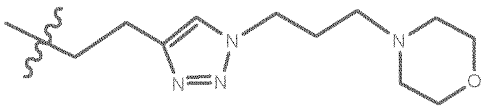 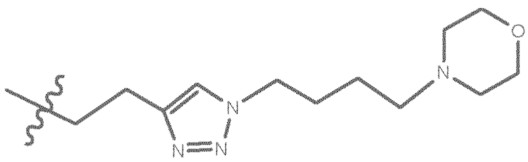

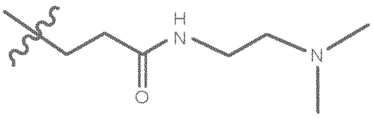 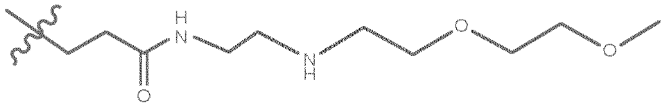

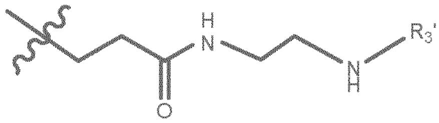 ,

wherein, $R_3$' is substituted or unsubstituted alkyl, substituted or unsubstituted alkylamino, or substituted or unsubstituted alkoxyl.

In some embodiments, the linker X is independently selected from the following structures:

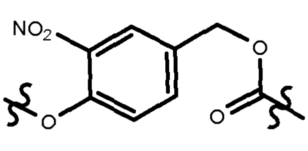 , 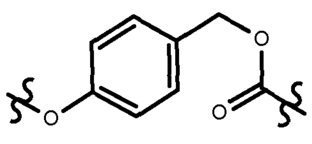 , 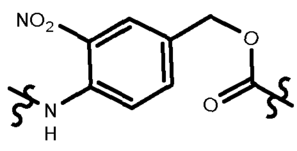 ,

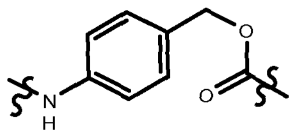 , 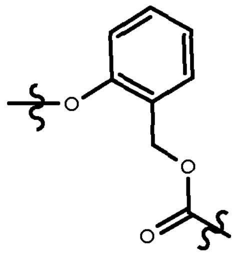 , 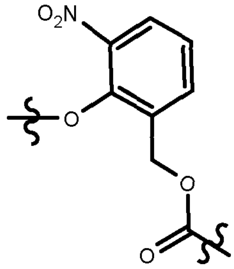 , 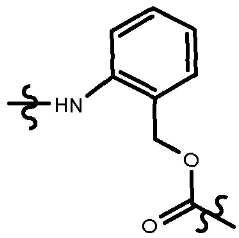 ,

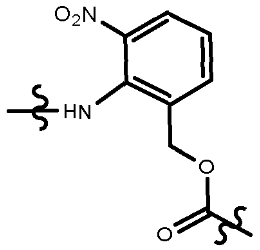 , 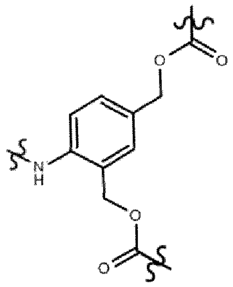 ;

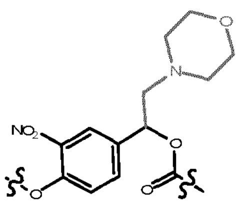 , 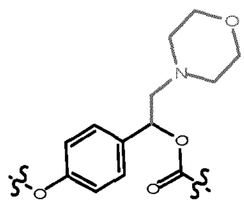 , 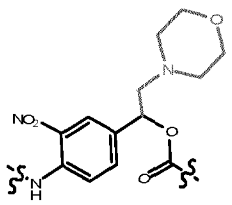 , 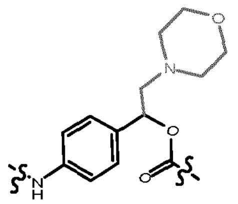 ,

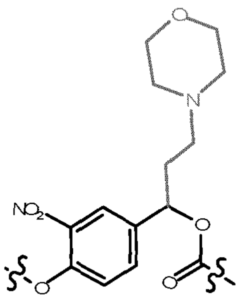 , 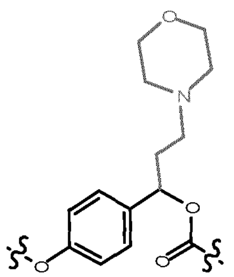 , 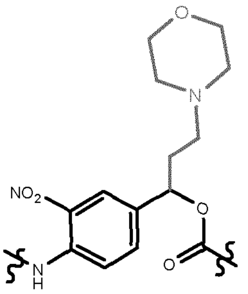 , 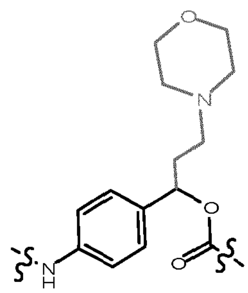 ,

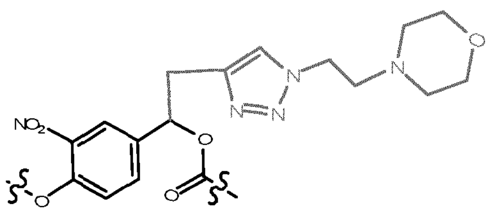 , 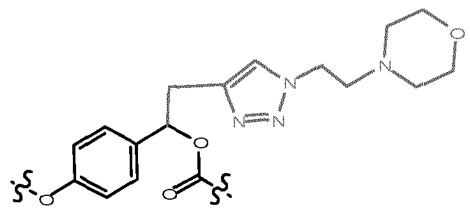 ,

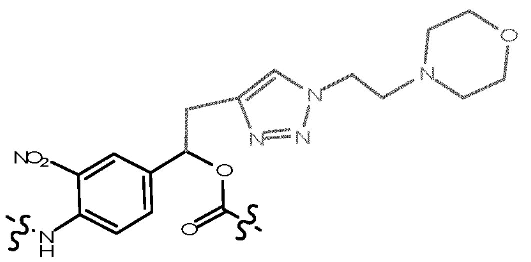,
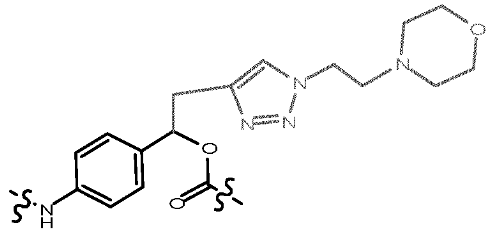,
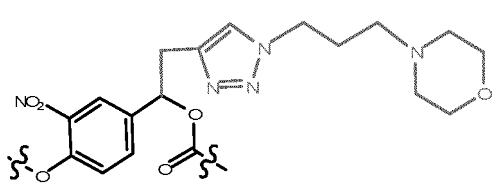,
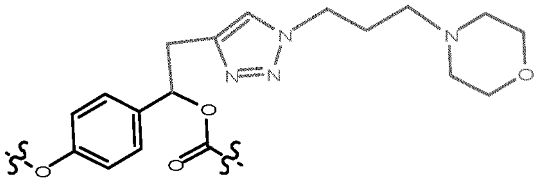,
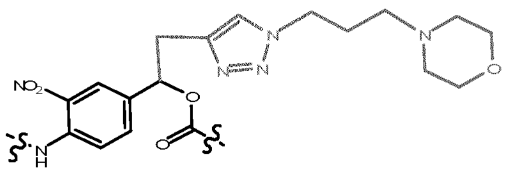, and
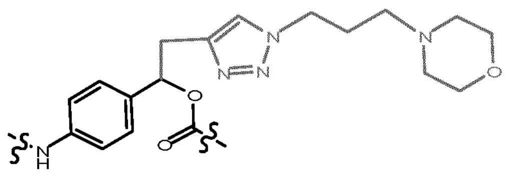.

In some embodiments, the linker X is independently selected from the following structure:

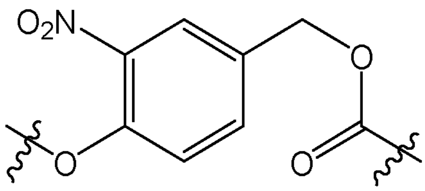
or
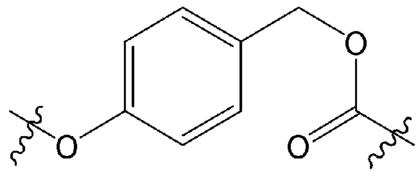.

The compounds of the present invention comprise a galactose-based moiety (β-gal moiety) of the structure as shown below.

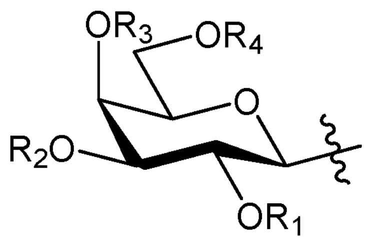

Formula I

$R_1$, $R_2$, $R_3$, and $R_4$ may each/independently be H or any substituent that can be hydrolysed intracellularly.

In some embodiments, $R_1$, $R_2$, $R_3$, and $R_4$ are each hydrogen or a substituent such that $-OR_1$, $-OR_2$, $-OR_3$, and/or $-OR_4$ may be hydrolyzed intracellularly.

In some embodiments, $R_1$, $R_2$, $R_3$, and $R_4$ are each independently H, $-C(O)R_5$, $-C(O)OR_6$, $-C(O)NR_7R_8$, and $-PO_3R_9$, such that $-OR_x$ is each independently an ester, carbonate, carbamate, or phosphodiester group, wherein x is 1, 2, 3, or 4.

In some embodiments, $R_1$, $R_2$, $R_3$, and $R_4$ are independently $-C(O)R_5$, $-C(O)OR_6$, and $-C(O)NR_7R_8$. In these forms, $R_5$, $R_6$, $R_7$, $R_8$, and $R_9$ are independently hydrogen, substituted alkyl, unsubstituted alkyl, substituted aryl, unsubstituted aryl, substituted heteroaryl, unsubstituted heteroaryl, substituted $C_3$-$C_{10}$ cyclic group, unsubstituted $C_3$-$C_{10}$ cyclic group, substituted $C_3$-$C_{10}$ heterocyclic group, unsubstituted $C_3$-$C_{10}$ heterocyclic group. In these forms, $R_5$, $R_6$, $R_7$, $R_8$ and $R_9$ are independently hydrogen, substituted $C_1$-$C_5$ alkyl, unsubstituted $C_1$-$C_5$ alkyl, substituted $C_6$-$C_{10}$

aryl, unsubstituted $C_6$-$C_{10}$ aryl, substituted $C_6$-$C_{10}$ heteroaryl, unsubstituted $C_6$-$C_{10}$ heteroaryl, substituted $C_3$-$C_{10}$ cyclic group, unsubstituted $C_3$-$C_{10}$ cyclic group, or substituted $C_3$-$C_{10}$ heterocyclic group, unsubstituted $C_3$-$C_{10}$ heterocyclic group. In these forms, $R_5$, $R_6$, $R_7$, $R_8$ and $R_9$ are independently hydrogen, substituted $C_1$-$C_3$ alkyl, unsubstituted $C_1$-$C_3$ alkyl, substituted $C_6$ aryl, unsubstituted $C_6$ aryl, substituted $C_6$ heteroaryl, unsubstituted $C_6$ heteroaryl, substituted $C_6$ cyclic group, unsubstituted $C_6$ cyclic group, or substituted $C_6$ heterocyclic group, unsubstituted $C_6$ heterocyclic group.

In some embodiments, one or more (or all) of $R_1$, $R_2$, $R_3$, and $R_4$ may be H, which may, for example, increase water solubility, promote the formation of a stable crystalline form, simplify an *in vivo* metabolic pathway, and/or obtain a better drug-like property.

In some embodiments, one or more (or all) of $R_1$, $R_2$, $R_3$, and $R_4$ is H when the prodrug is in crystalline form.

In some embodiments, $R_1$, $R_2$, $R_3$, and $R_4$ are -C(O)Rs. In these forms, $R_5$ is independently substituted $C_1$-$C_3$ alkyl or unsubstituted $C_1$-$C_3$ alkyl. In these forms, $R_5$ is preferably methyl.

A preferred galactose-based moiety is the D-galactose tetracetate moiety, as shown below.

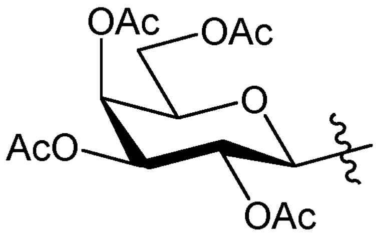

In some forms, one or more (*e.g.,* two, three, or four) protecting groups (*e.g.,* acetyl (Ac) groups) can be removed from the galactose-based moiety. In some embodiments, removal of one or more (*e.g.*, two, three, or four) protecting groups may increase water solubility, promote formation of a stable crystalline form, simplify an *in vivo* metabolic pathway, and/or obtain a better drug-like property. In some embodiments, the prodrug may be in a crystalline form, which may, for example, contain no protecting groups (*e.g.*, acetyl (Ac) groups) or contain a reduced number of protecting groups (*e.g*, acetyl (Ac) groups) on the galactose-based moiety. In some forms, *e.g.*, one or more (or all) of $R_1$, $R_2$, $R_3$, and $R_4$ is H when the prodrug is in the crystalline form.

In some embodiments, the β-gal moiety has the following structure:

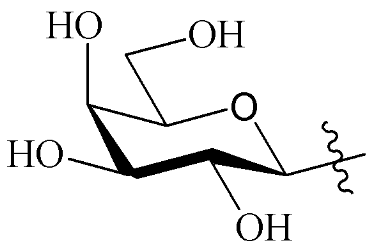

In some forms, the active agent

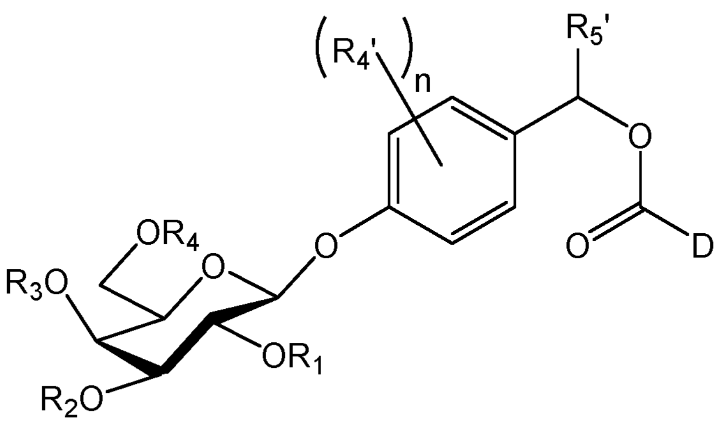

is represented by Formula IV;
wherein, $R_1$, $R_2$, $R_3$, $R_4$, $R_4$' and $R_5$' are as described above.

D comprises a cytotoxic agent.

In some forms, one or more (*e.g.,* two, three, or four) protecting groups (*e.g.,* acetyl (Ac) groups) can be removed from the galactose-based moiety. In some forms, for example, one or more (or all) of $R_1$, $R_2$, $R_3$, and $R_4$ is H when the prodrug is in a crystalline form. In some forms, $R_4$' may be nitro (*e.g.,* $-NO_2$). In some forms, $R_4$' may be hydrogen (rather than, *e.g.,* nitro (*e.g.,* $-NO_2$)). The preferred cytotoxic agents are the chemotherapeutic agents/drugs known in the art. Examples of the chemotherapeutic agents include, but are not limited to, A1331852.

In some embodiments, the active agent has the following structure:

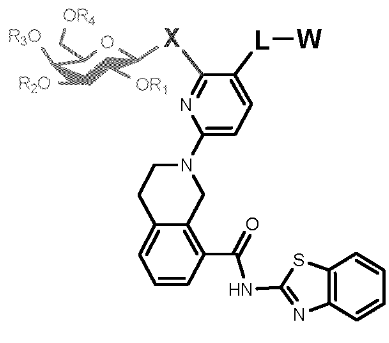
,
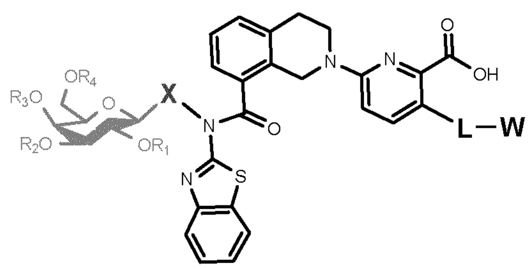
,

a b

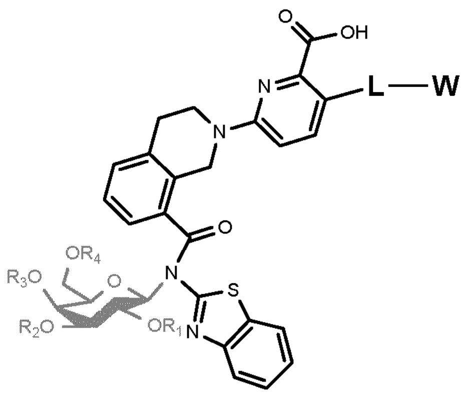
,
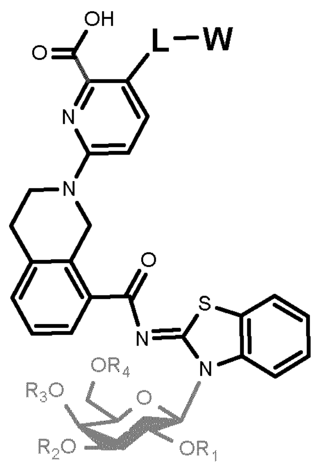
,

c d

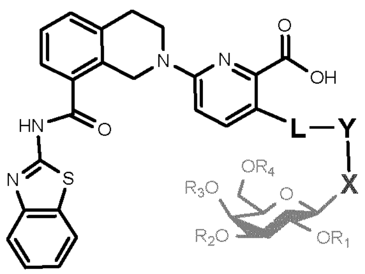
,
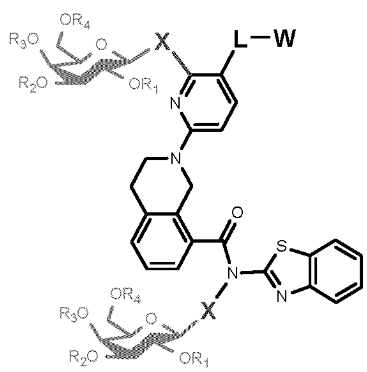
,

e f

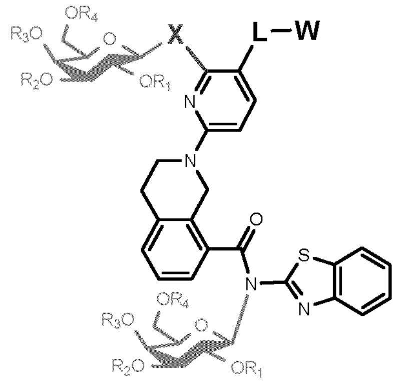

,

g

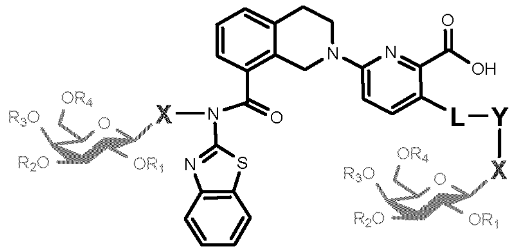

,

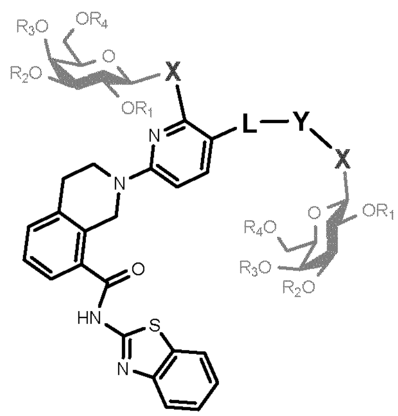

,

h i

or

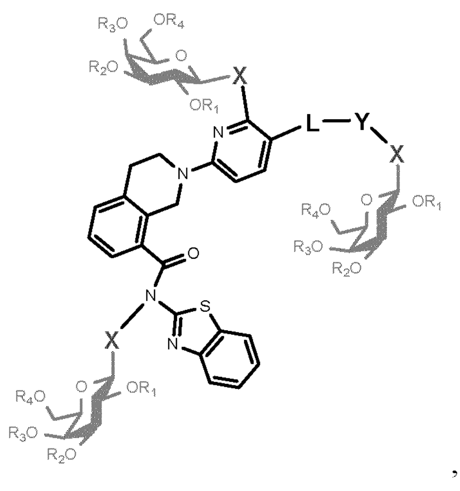

,

j.

wherein, Y is W and has an amino group, a sec-amino group, a cyclic sec-amino group or a hydroxy group, and wherein, the hydrogen bonded to the N atom or the O atom in the amino group, cyclic sec-amine group or hydroxy group is substituted with the linker X.

In the preferred embodiments, Y is selected from the following structures:

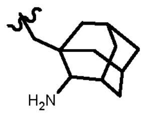, 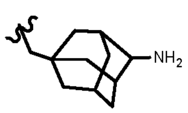, 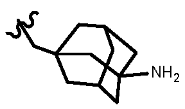, 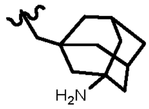, 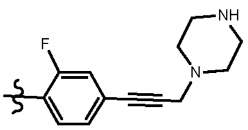,

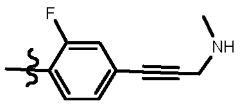, 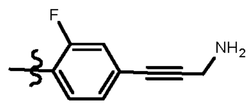, 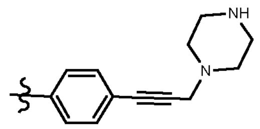, 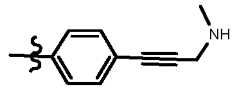,

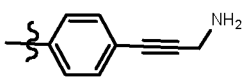, 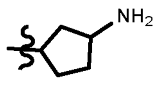, 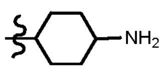, 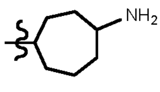, 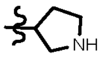, 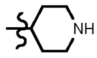,

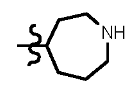, 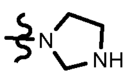, 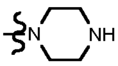, 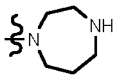, 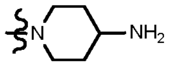, 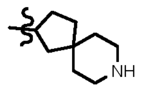, 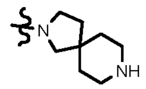.

In some embodiments, the active agent has the following structures:

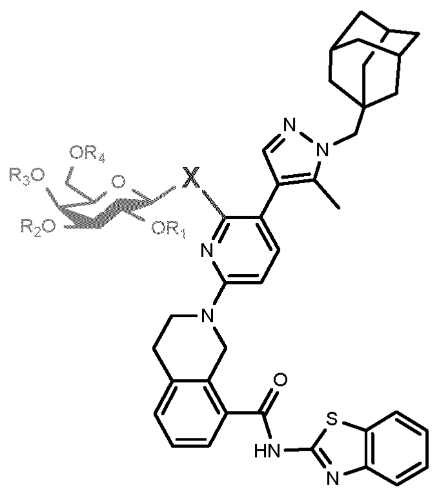

a1

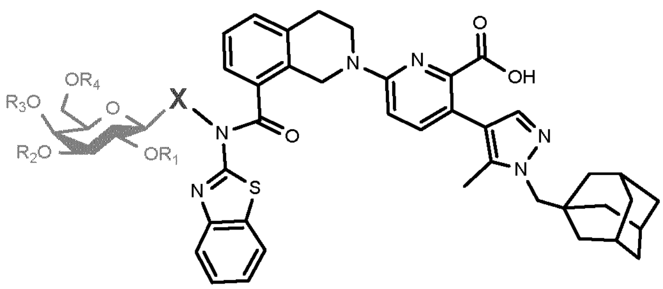

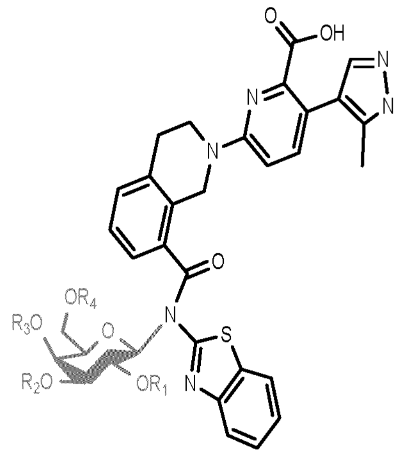

c1

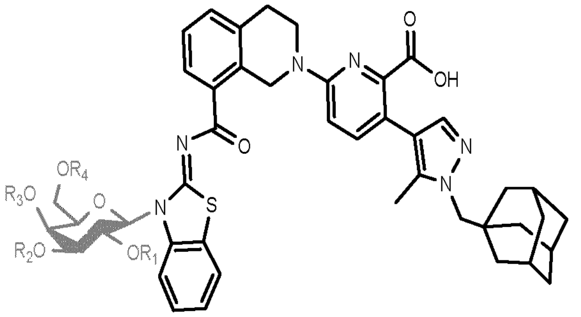

d1

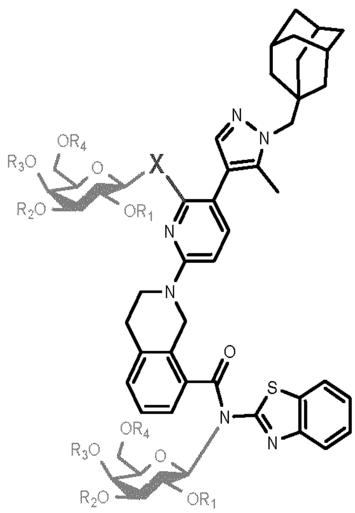

e1

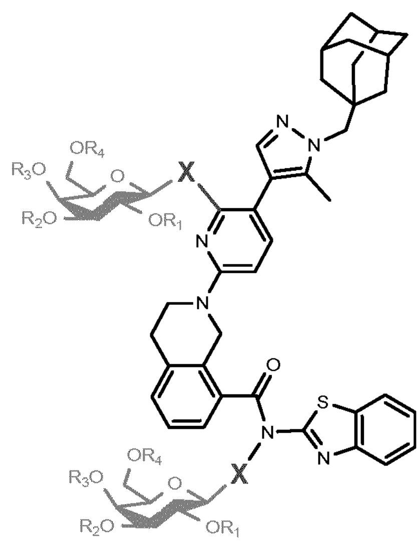

f1.

wherein, X is the linker as defined herein, and $R_1$, $R_2$, $R_3$, and $R_4$ are as defined herein.

In some forms, one or more (*e.g.,* two, three, or four) protecting groups (*e.g.,* the acetyl (Ac) groups) can be removed from the galactose-based moiety.

In some forms, $NO_2$ may be removed. In some forms, all of $NO_2$ and one or more (*e.g.,* two, three, or four) protecting groups (*e.g.,* the acetyl (Ac) groups) may be removed.

In the preferred embodiments, the active agent may be selected from the following structures:

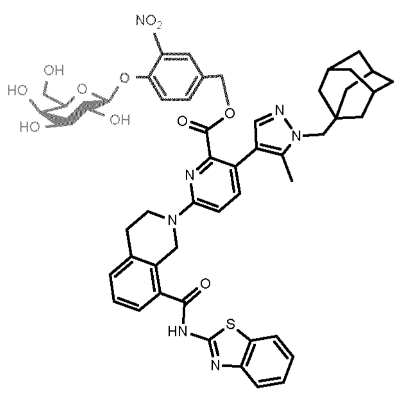

,

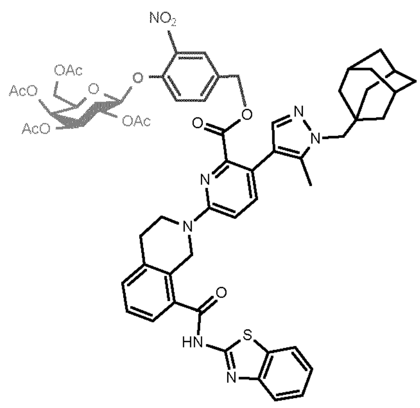

,

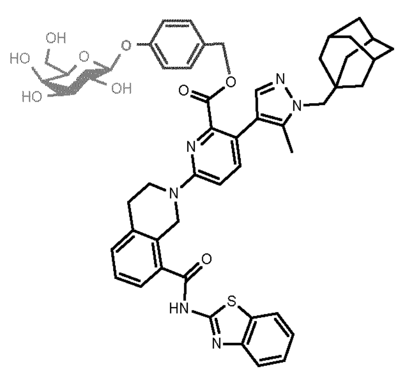

,

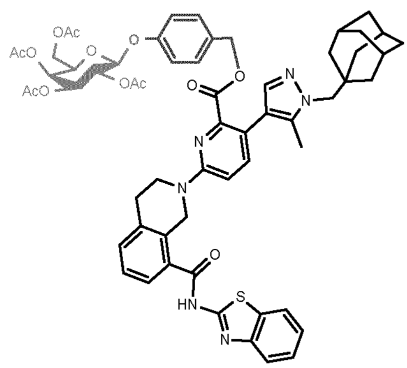

,

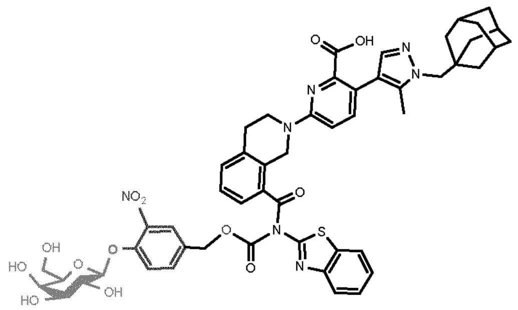

,

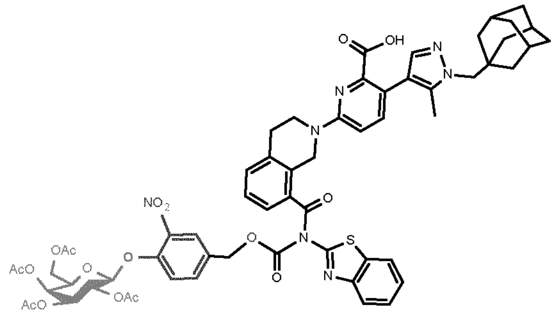

,

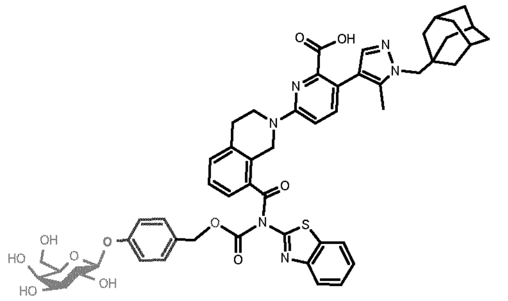

,

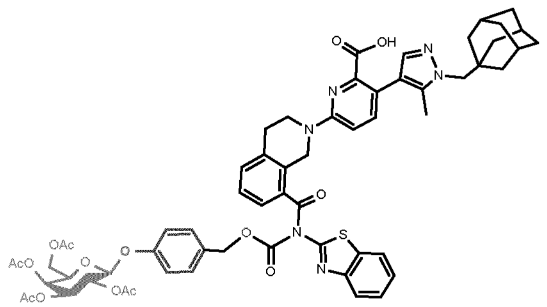

,

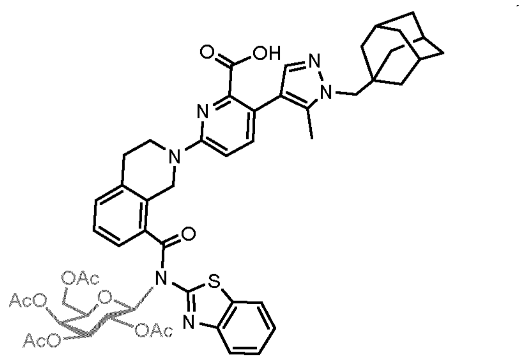

,

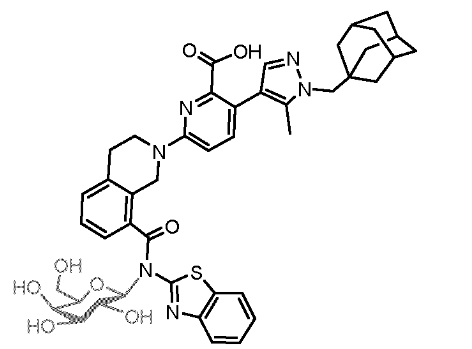

,

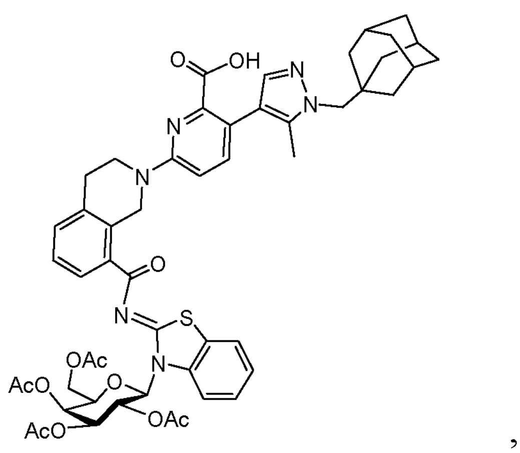

,

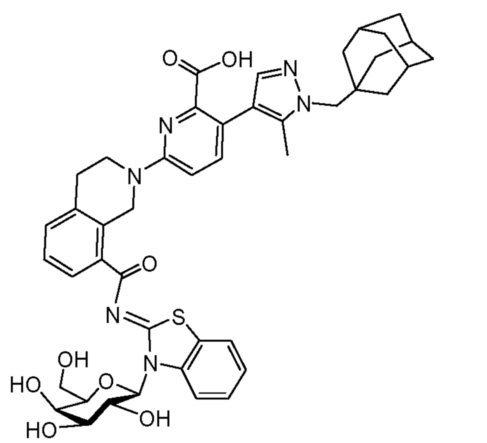

,

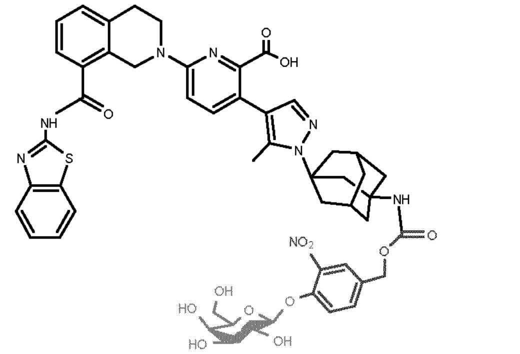,

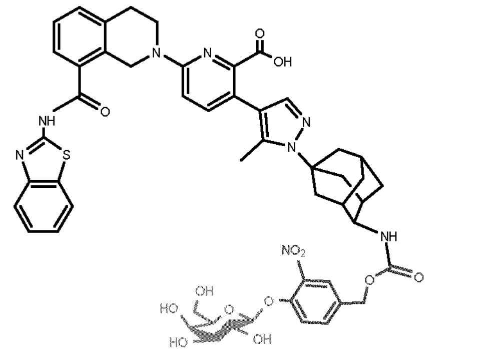,

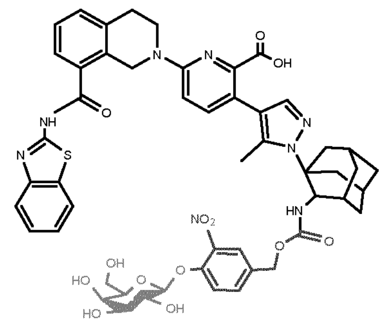,

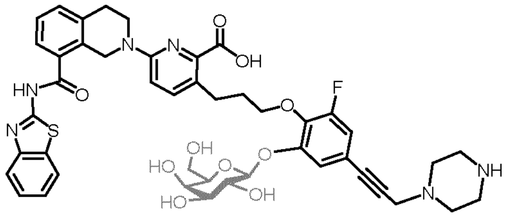,

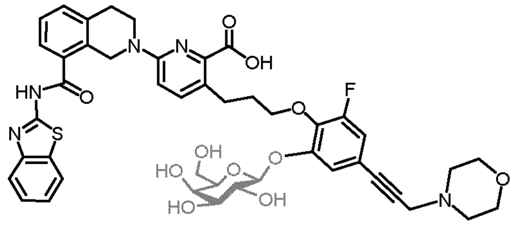,

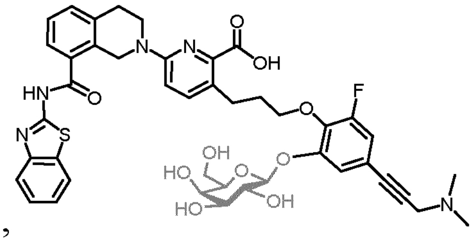,

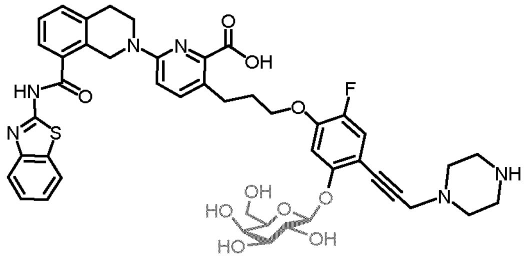,

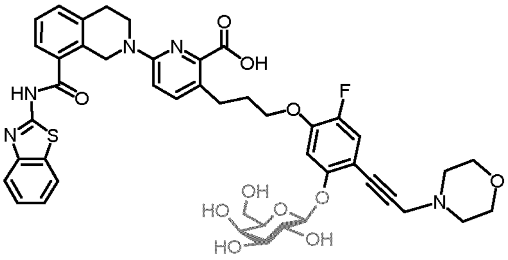,

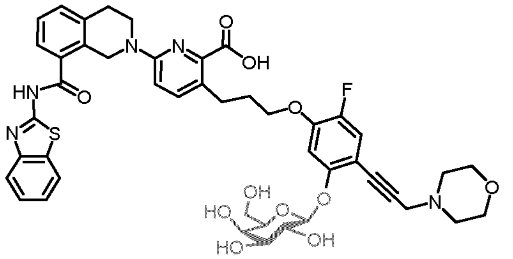,

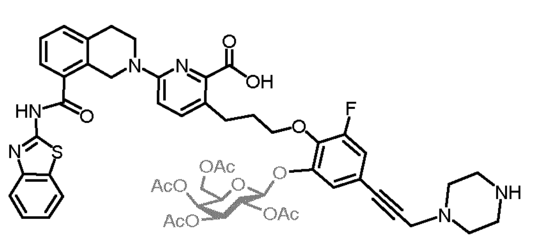,

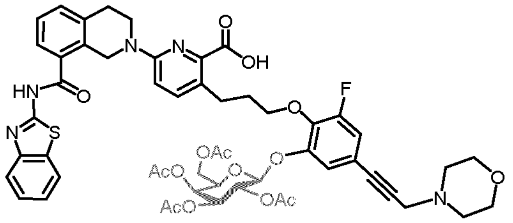
,
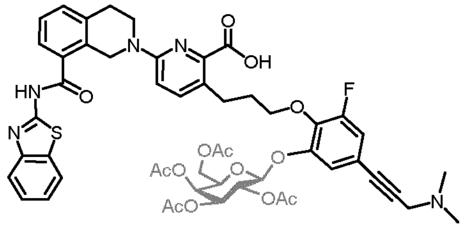
,

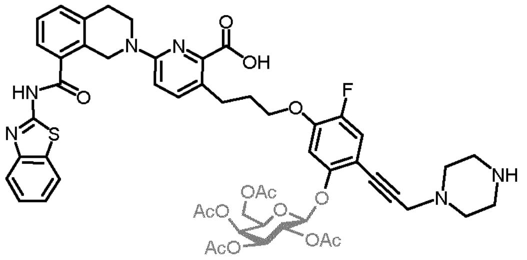
,
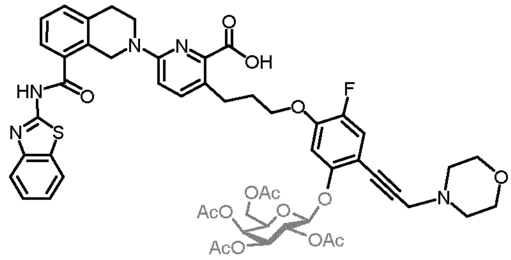
,

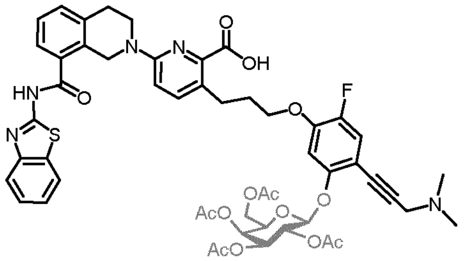
,
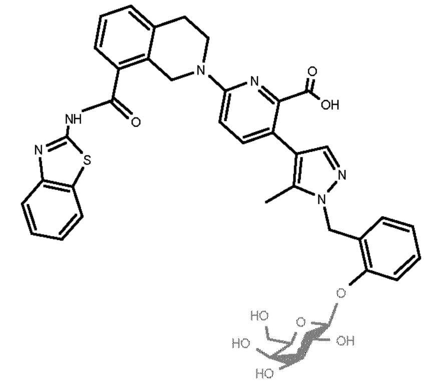
,

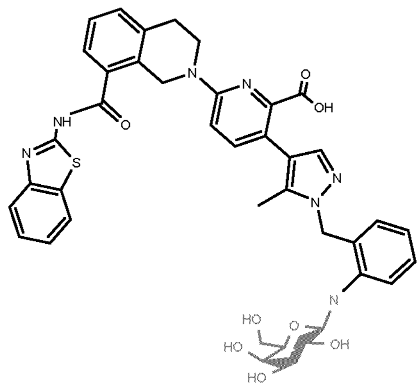
,
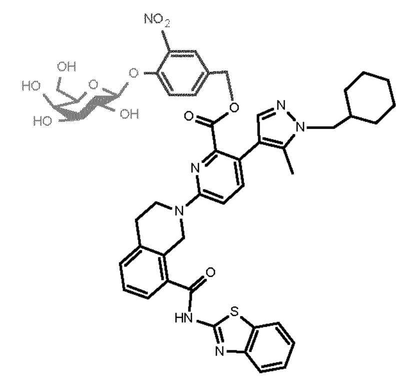
,

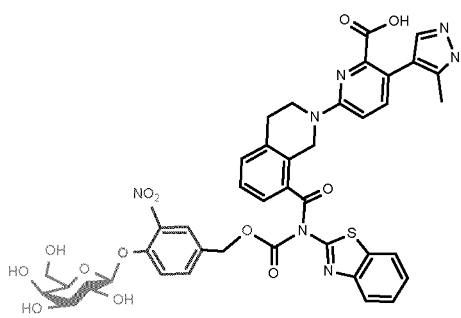
,
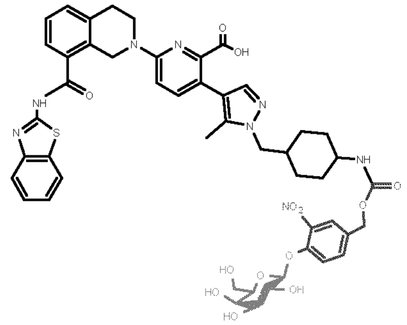
,

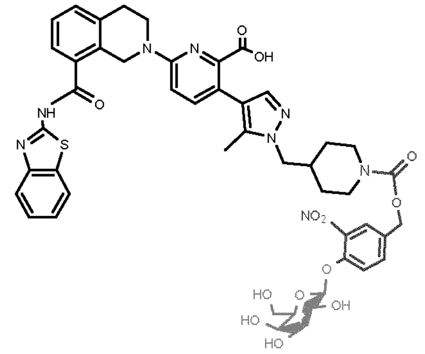

, ,

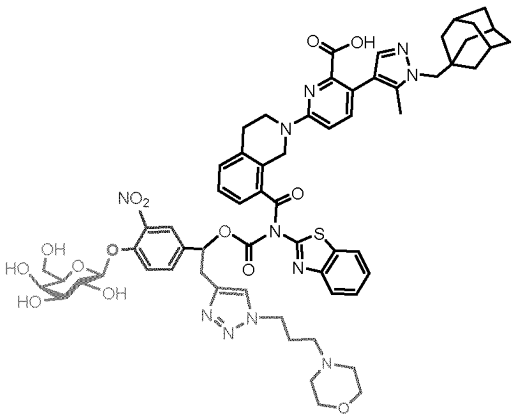

,

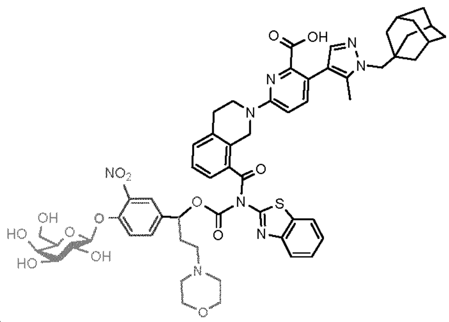

.

B. Formulations

The compounds described herein may be formulated for enteral, parenteral, topical or pulmonary administration. The compounds may be combined with one or more pharmaceutically acceptable carriers and/or excipients, wherein the carriers and/or excipients are considered safe and effective and can be administered to an individual without causing undesired biological side effects or unwanted interactions. Carriers are all components of a pharmaceutical formulation other than one or more active ingredients.

The compound is included in a formulation in a therapeutically effective amount. The therapeutically effective amount can be initially estimated from a cell culture assay. For example, a dose can be formulated in an animal model to achieve a circulating concentration range that includes an IC50 or IC100 determined in cell culture. Such information can be used to more accurately determine a useful dose for the human body. Initial dose can also be estimated from *in vivo* data. Using these initial guidelines, one of ordinary skill in the art can determine the useful dose for the human body.

1. Parenteral Formulations

The compounds described herein can be formulated for the parenteral administration.

For example, the parenteral administration may include an administration to the patient via intravenous, intradermal, intraarterial, intraperitoneal, intralesional, intracranial, intraarticular, intraprostatic, intrapleural, intratracheal, intravitreal, intratumoral, intramuscular, subcutaneous, subconjunctival, intracapsular, intrapericardial, intraumbilical, and intracoronal injection and infusion.

Parenteral formulation can be prepared as an aqueous composition using the techniques known in the art. Typically, such composition can be prepared as an injectable formulation, such as a solution or suspension; a solid form suitable for use in the preparation of a solution or suspension after the addition of a reconstitution medium prior to injection; an emulsion, such as a water-in-oil (w/o) emulsion, an oil-in-water (o/w) emulsion, and a microemulsion, a liposomes or an emulsion thereof.

The carrier may be a solvent or dispersion medium comprising, for example, water; ethanol; one or more polyols (*e.g.*, glycerol, propylene glycol, and liquid polyethylene glycol); oils, such as vegetable oils (*e.g.,* peanut oil, corn oil, sesame oil, *etc.*); and a combination thereof. For example, the proper flowability may be maintained by the use of a coating such as lecithin, by maintaining a desired particle size in the case of dispersion, and/or by the use of a surfactant. In many cases, it is preferred to include an isotonic agent, such as sugar or sodium chloride.

A solution and dispersion of the active compound as a free acid or base or a pharmaceutically acceptable salt thereof may be prepared in water or another solvent or in a dispersion medium suitably mixed with one or more pharmaceutically acceptable excipients, wherein said excipients include, but are not limited to, a surfactant, a dispersing agents, an emulsifier, a pH regulator, a viscosity modifier and a combination thereof.

Suitable surfactants may be the anionic, cationic, amphoteric or non-ionic surfactants. Suitable anionic surfactants include, but are not limited to, those containing carboxylate, sulfonate and sulfate ions. Examples of the anionic surfactants include the sodium, potassium, ammonium salts of long chain alkyl sulfonic acids and alkylaryl sulfonate, such as sodium dodecylbenzene sulfonate; sodium dialkylsulfosuccinate, such as sodium dodecylbenzene sulfonate; sodium dialkylsulfosuccinate, such as sodium bis(2-ethylhexyl) sulfosuccinate; and alkyl sulfates, such as sodium dodecyl sulfate. Cationic surfactants include, but are not limited to, the quaternary ammonium compounds, such as benzalkonium chloride, benzethonium chloride, cetrimonium bromide, stearyldimethylbenzylammonium chloride, polyoxyethylene, and cocamine. Examples of the non-ionic surfactants include ethylene glycol monostearate, propylene glycol myristate, glyceryl monostearate, glyceryl stearate, polyglyceryl-4-oleate, dehydrated sorbitan acylates, sucrose acylates, PEG-150 laurate, PEG-400 monolaurate, polyoxyethylene monolaurate, polysorbate, polyoxyethylene octylphenylether, PEG-1000 cetyl ether, polyoxyethylene tridecyl ether, polypropyleneglycol butyl ether, Poloxamer® 401, stearoyl monoisopropanolamide, and polyoxyethylene hydrogenated tallow amide. Examples of the amphoteric surfactants include sodium N-dodecyl-β-alanine, sodium N-lauryl-β-iminodipropionate, myristoamphoacetate, lauryl betaine, and lauryl sulfobetaine.

The formulation may contain a preservative to prevent the growth of microorganisms. Suitable preservatives include, but are not limited to, parabens, chlorobutanol, phenol, sorbic acid and thiomersal. The formulation may also contain an antioxidant to prevent the degradation of an active agent.

The formulation is typically buffered to a pH of 3-8 for the parenteral administration after reconstitution. Suitable buffers include, but are not limited to, a phosphate buffer, an acetate buffer, and a citrate buffer.

Water-soluble polymers are commonly used in the formulations for parenteral administration. Suitable water-soluble polymers include, but are not limited to, polyvinylpyrrolidone, glucan, carboxymethylcellulose and polyethylene glycol.

Sterile injectable solutions can be prepared by incorporating the desired amount of the active compound with one or more excipients listed above (as needed) into a suitable solvent or dispersion medium, and then filtering to remove bacteria. Typically, the dispersant is prepared by incorporating a plurality of sterilized active ingredients into a sterile carrier containing the basic dispersion medium and other ingredients from those listed above as required. In the case of a sterile powder for the preparation of a sterile injectable solution, the preferred methods of preparation are the vacuum drying and freeze-drying techniques which produce a powder of the active ingredient plus any additional desired ingredient from its previously sterile filtered solution. Powders may be prepared in such a way that the particles are inherently porous, which may increase the dissolution of the particles. Methods of preparing the porous particles are well known in the art.

(a). Formulations for Controlled Release

The parenteral formulations described herein may be formulated for a controlled release, including an immediate release, a delayed release, an extended release, a pulsed release, and a combination thereof.

1. Nano- and Microparticles

For the parenteral administration, one or more compounds and optionally one or more additional active agents may be incorporated into the microparticles, nanoparticles, or a combination thereof that provide the controlled release of the compounds and/or one or more additional active agents. In the embodiments in which the formulation contains two or more drugs, the drugs can be formulated for the same type of controlled release (*e.g.,* a delayed, extended, immediate, or pulsed release), or the drugs can be independently formulated for different types of release (*e.g.,* an immediate and delayed, an immediate and extended, a delayed and extended, a delayed and pulsed release, *etc.*).

For example, the compound and/or one or more additional active agents may be incorporated into the polymeric microparticles, which provide the controlled release of the drug. The controlled release of the drug is achieved by diffusion of the drug from the microparticles and/or the degradation of the polymer particles by hydrolysis and/or the enzymatic degradation. Suitable polymers include ethyl cellulose and other natural or synthetic cellulose derivatives.

Polymers that dissolve slowly in an aqueous environment and form gels, such as hydroxypropylmethylcellulose or poly(ethylene oxide), may also be suitable for use as the drug-containing microparticles. Other polymers include, but are not limited to, polyanhydrides, poly(ester anhydrides), polyhydroxy acids, such as polylactide (PLA), polyglycollide (PGA), poly(lactide-co-glycollide) (PLGA), poly(3-hydroxybutyrate) (PHB) and a copolymer thereof, poly(4-hydroxybutyrate) (P4HB) and a copolymer thereof, polycaprolactone and a copolymer thereof, and a combination thereof.

Alternatively, the drug may be incorporated into the microparticles prepared from the materials that are insoluble

in an aqueous solution or slowly soluble in an aqueous solution, but are capable of being degraded in the gastrointestinal tract through the enzymatic degradation, the surface-active action of bile acid, and/or the mechanical erosion. As used herein, the term "slowly soluble in an aqueous solution" refers to the materials that are insoluble in water within 30 minutes. Preferred examples include fats, fatty substances, waxes, waxy materials and a mixture thereof. Suitable fats and fatty substances include fatty alcohols (*e.g.*, lauryl alcohol, myristyl alcohol, stearyl alcohol, cetyl alcohol, or cetostearyl alcohol), fatty acids and the derivatives including, but not limited to, fatty acid esters, fatty acid glycerides (monoglyceride, diglyceride and triglyceride ), and hydrogenated fats. Specific examples include, but are not limited to, hydrogenated vegetable oils, hydrogenated cottonseed oils, hydrogenated castor oils, hydrogenated oils available under the trade name Sterotex®, stearic acid, cocoa butter, and stearyl alcohol. Suitable waxes and waxy materials include natural or synthetic waxes, hydrocarbons and common waxes. Specific examples of waxes include beeswax, sugar wax, castor wax, carnauba wax, paraffin wax and candelilla wax. As used herein, a waxy material is defined as any material that is generally solid at room temperature and has a melting point of about 30 to 300°C.

In some cases, it may be necessary to change the rate at which water penetrates into the microparticles. For this purpose, a rate control (wicking) agent may be formulated with the fats or waxes as listed above. Examples of the rate control materials include certain starch derivatives (*e.g.,* the waxy maltodextrin and roller-dried corn starch), the cellulose derivatives (*e.g.,* hydroxypropylmethylcellulose, hydroxypropylcellulose, methylcellulose, and carboxymethylcellulose), alginic acid, lactose, and talc. In addition, the pharmaceutically acceptable surfactants (*e.g.*, lecithin) may be added to facilitate the degradation of such microparticles.

Water-insoluble proteins, such as zein, may also be used as the materials for the formation of the drug-containing microparticles. In addition, the water-soluble proteins, polysaccharides, and a combination thereof can be formulated into the microparticles with a drug and then crosslinked to form an insoluble network. For example, cyclodextrins can be complexed with the individual drug molecules and subsequently crosslinked.

2. Methods of Preparing Nano- and Microparticles

Encapsulation or incorporation of a drug into a carrier material to produce the drug-containing microparticles can be achieved by the known pharmaceutical formulation techniques. In the case of preparation using a fatty, wax or waxy material, the carrier material is typically heated above its melting temperature and the drug is added to form a mixture comprising the drug particles suspended in the carrier material, the drug dissolved in the carrier material, or a mixture thereof. The microparticles may subsequently be formulated by several methods, including, but not limited to, a method of coagulation, extrusion, spray cooling, or water dispersion. In a preferred method, the wax is heated above its melting temperature, the drug is added, and the melted wax-drug mixture is allowed to coagulate under a continuous stirring as the mixture cools. Alternatively, the melted wax-drug mixture may be extruded and rolled to form pills or perle. These methods are known in the art.

For some carrier materials, it may be necessary to use the solvent evaporation techniques to produce the drug-containing microparticles. In this case, the drug and the carrier material are co-dissolved in a mutual solvent, and the microparticles may subsequently be produced by several techniques, including, but not limited to, forming an emulsion in water or other suitable medium, spray drying, or by evaporating the solvent from the bulk solution and grinding the resulting material.

In some embodiments, the drug in particulate form is uniformly dispersed in a water-insoluble or slowly water-soluble material. In order to minimize the size of the drug particles in the composition, the drug powder itself may be ground prior to the preparation to produce the fine particles. The Jet grinding method known in the pharmaceutical field can be used for this purpose. In some embodiments, the drug in particulate form is uniformly dispersed in the wax or wax-like substance by heating the wax or wax-like substance above its melting point and adding the drug particles while stirring the mixture. In this case, a pharmaceutically acceptable surfactant may be added to the mixture to facilitate dispersion of the drug particles.

The particles may also be coated with one or more release coatings. Solid fatty acid esters hydrolyzed by lipases can be sprayed onto the microparticles or drug particles. Zein is an example of a natural water insoluble protein. It can be coated onto the drug-containing microparticles or drug particles by spraying or the wet granulation techniques. In addition to the naturally water-insoluble materials, some substrates of the digestive enzymes can be processed using a crosslinking procedure, which leading to the formation of the insoluble networks. Many methods of crosslinking proteins by the chemical and physical initiation have been reported. One of the most common methods of obtaining crosslinks is the use of a chemical crosslinking agent. Examples of the chemical crosslinking agents include aldehydes (glutaraldehyde and formaldehyde), epoxides, carbodiimide and genipin. In addition to these crosslinking agents, the oxidized and natural sugars have been used to crosslink gelatin. Crosslinking can also be accomplished using an enzymatic method; for example, transglutaminase has been approved as a GRAS substance for crosslinking the seafood. Finally, crosslinking can be initiated by the physical methods such as the heat treatment, UV radiation and gamma radiation.

To produce a coating of crosslinked protein that surrounds the drug-containing microparticles or drug particles,

the water-soluble proteins can be sprayed onto the microparticles and then crosslinked by one of the methods described above. Alternatively, the drug-containing microparticles may be microencapsulated within the protein by the condensed phase separation (e.g., by the addition of salt) and then crosslinked. Some suitable proteins for this purpose include gelatin, albumin, casein, and gluten.

Polysaccharides can also be crosslinked to form a water-insoluble network. For many polysaccharides, this can be achieved by reacting with the calcium salts or polyvalent cations which crosslink the polymer backbones. Pectin, alginate, glucan, the straight chain starch and guar gum are crosslinked in the presence of the multivalent cations. Complexes between the oppositely charged polysaccharides can also be formed; for example, pectin and chitosan can be complexed by an electrostatic interaction.

(b). Injectable/Implantable Formulations

The compounds described herein may be incorporated into an injectable/implantable solid or semi-solid implant, such as a polymer implant. In one embodiment, the compounds are incorporated into a polymer being a liquid or paste at room temperature, but exhibiting an increase in viscosity upon contacting with an aqueous medium, such as a physiological fluid, to form a semi-solid or solid material. Exemplary polymers include, but are not limited to, the hydroxyalkanoic acid polyesters derived from a copolymerisation reaction with at least one unsaturated hydroxy fatty acid copolymerised with a hydroxyalkanoic acid. The polymers may be melted, mixed with an active substance and cast or injection moulded into the device. Such melt manufacturing requires the polymer to have such a melting point that is below the temperature at which the substance is to be delivered and the polymer degrades or becomes reactive. Said device may also be prepared by solvent casting, wherein the polymer is dissolved in a solvent, the drug is dissolved or dispersed in the polymer solution, and the solvent is then evaporated. The solvent method requires that the polymer can be soluble in an organic solvent. Another method involves compression moulding of a mixed powder of the polymer and the active agent-containing drug or the polymer particles.

Alternatively, the compounds may be incorporated into a polymer matrix and moulded, compressed or extruded into a device that is solid at room temperature. For example, the compounds may be incorporated into the biodegradable polymers, such as polyanhydrides, polyhydroxyalkanoic acid (PHA), PLA, PGA, PLGA, polycaprolactone, polyesters, polyamides, polyorthoesters, polyphosphazenes, proteins and polysaccharides such as collagen, hyaluronic acid, albumin, and gelatin, as well as a combination thereof, and compressed into a solid device, such as a disc, or extruded into a device, such as a rod.

The release of one or more compounds from the implant can be altered to increase degradation by a polymer selection, a molecular weight of the polymer, and/or a polymer modification, such as a pore formation and/or an incorporation of a hydrolysable bond. Methods for modifying the properties of the biodegradable polymers to alter the release profile of a compound from an implant are well known in the art.

2. Enteral Formulations

Suitable oral dosage forms include a tablet, a capsule, a solution, a suspension, a syrup and a lozenge. Tablets can be prepared using the compression or moulding techniques well known in the art. Gelatin or non-gelatin capsules may be prepared as hard or soft capsule shells which may encapsulate the liquid, solid and semi-solid filling materials using the techniques well known in the art.

Formulations may be prepared using the pharmaceutically acceptable carriers. As commonly used herein, "a carrier" includes, but is not limited to, a diluent, a preservative, a binder, a lubricant, a disintegrant, a swelling agent, a filler, a stabilizer and a combination thereof.

The carrier also includes all components of the coating composition, which may include a plasticizer, a pigment, a colorant, a stabilizer and a flow aid.

Examples of suitable coating materials include, but are not limited to, the cellulosic polymers, such as cellulose acetate phthalate, hydroxypropyl cellulose, hydroxypropylmethyl cellulose, hydroxypropylmethyl cellulose phthalate and hydroxypropylmethyl cellulose acetate succinate; poly(vinyl acetate) phthalate, the acrylic polymers and copolymers, and the methacrylic acid resins purchased under the trade name EUDRAGIT® (Roth Pharma, Westerstadt, Germany), zein, shellac and polysaccharides.

In addition, the coating materials may comprise the conventional carriers such as a plasticizer, a pigment, a colorant, a flow aid, a stabilizer, a pore-forming agent and a surfactant.

A "diluent", also referred to as "a filler", is typically necessary to increase the volume of a solid dosage form in order to provide the actual size for the compression of tablets or the formation of beads and granules. Suitable diluents include, but are not limited to, dicalcium phosphate dihydrate, calcium sulphate, lactose, sucrose, mannitol, sorbitol, cellulose, microcrystalline cellulose, kaolin, sodium chloride, dry starch, hydrolyzed starch, pre-gelatinized starch, silicon dioxide, titanium oxide, aluminium magnesium silicate and icing sugar.

A "binder" is used to impart a cohesive property to the solid dosage forms, thereby ensuring that the tablets or beads or particles remain intact after the formation of the dosage form. Suitable binder materials include, but are not limited to, starch, pre-gelatinized starch, gelatin, sugars (including sucrose, glucose, dextrose, lactose, and sorbitol), polyethylene glycol, wax, the natural and synthetic gums, such as arabic gum, tragacanth gum, sodium alginate, cellulose including hydroxypropylmethyl cellulose, hydroxypropyl cellulose, ethylcellulose, and aluminium magnesium silicate, as well as the synthetic polymers, such as an acrylic acid and methacrylic acid copolymer, a methacrylic acid copolymer, a methyl methacrylate copolymer, an amino alkyl methacrylate copolymer, polyacrylic acid/polymethacrylic acid and polyvinylpyrrolidone.

A "lubricant" is used to facilitate the manufacture of tablets. Examples of suitable lubricants include, but are not limited to, magnesium stearate, calcium stearate, stearic acid, glyceryl behenate, polyethylene glycol, talc, and mineral oil.

A "disintegrant" is used to promote the disintegration or "breakage" of the dosage form after administration and typically includes, but is not limited to, starch, sodium starch hydroxyacetic acid, carboxymethyl starch sodium, sodium carboxymethylcellulose, hydroxypropyl cellulose, pre-gelatinized starch, clay, cellulose, and algin, gum or the crosslinked polymers such as the crosslinked PVP (Polyplasdone® XL from GAF Chemical Corp).

A "stabilizer" is used to inhibit or delay a drug breakdown reaction, which include, for example, an oxidation reaction. Suitable stabilizers include, but are not limited to, an antioxidant, butylated hydroxytoluene (BHT); ascorbic acid, the salts and esters thereof; vitamin E, tocopherol, and the salts threrof; sulphites such as sodium pyrosulfite; cysteine and the derivatives thereof; citric acid; propyl gallate and butylated hydroxyanisole (BHA).

(a) Enteral Formulations for Controlled Release

An oral dosage form, such as a capsule, a tablet, a solution and a suspension, may be formulated for a controlled release. For example, one or more compounds and optionally one or more additional active agents may be formulated as the nanoparticles, microparticles and a combination thereof, and encapsulated in the soft or hard gelatin or non-gelatin capsules or dispersed in a dispersion medium to form an oral suspension or syrup. The granules may be formed from the drug and a controlled release polymer or matrix. Alternatively, the drug particles may be coated with one or more controlled release coatings prior to the incorporation into the final dosage form.

In another embodiment, one or more compounds and optionally one or more additional active agents are dispersed in a matrix material gelling or emulsifying upon contacting with an aqueous medium, such as a physiological fluid. In the case of gelation, the matrix swells and entrains the active agent(s) being slowly released over time by the diffusion and/or degradation of the matrix material. Such matrix can be formulated as a filler material for the tablets or hard and soft capsules.

In yet another embodiment, one or more compounds and optionally one or more additional active agents are formulated into an oral dosage form for sale, such as a tablet or a capsule, and the solid dosage form is coated with one or more controlled release coatings, such as a delayed release coating or an extended release coating. One or more coatings may also contain the compounds and/or the additional active agents.

(i) Extended Release Dosage Forms

Extended release formulations are typically prepared as the diffusion or osmotic systems known in the art. Diffusion systems typically consist of two types of devices, a reservoir and a matrix, and are well known and described in the art. Matrix devices are typically prepared by compressing the drug into a tablet form with a slowly dissolving polymeric carrier. Three main types of materials used to prepare the matrix devices are an insoluble plastic, a hydrophilic polymer, and a fatty compound. Plastic matrices include, but are not limited to, methyl acrylate-methyl methacrylate, polyvinyl chloride, and polyethylene. Hydrophilic polymers include, but are not limited to, the cellulose polymers such as methyl and ethyl cellulose, hydroxyalkyl cellulose such as hydroxypropyl cellulose, hydroxypropylmethyl cellulose, sodium carboxymethyl cellulose, and Carbopol® 934, poly(ethylene oxide) and a mixture thereof. Fatty compounds include, but are not limited to, a wide range of waxes such as carnauba wax and tristearin and wax-like substances including hydrogenated castor oil or hydrogenated vegetable oil, or a mixture thereof.

In certain preferred embodiments, the plastic material is a pharmaceutically acceptable acrylic polymer including, but not limited to, an acrylic and methacrylic acid copolymer, methyl methacrylate, a methyl methacrylate copolymer, ethoxyethyl methacrylate, cyanoethyl methacrylate, an amino alkyl methacrylate copolymer, poly(acrylic acid), poly(methacrylic acid), an alkyl amine methacrylate copolymer, poly(methyl methacrylate), poly(methacrylic acid) (anhydride), poly(methacrylate), polyacrylamide, poly(methacrylic anhydride), and a glycidyl methacrylate copolymer.

In certain preferred embodiments, the acrylic polymers comprise one or more ammonium methacrylate copolymers. Ammonium methacrylate copolymers are well known in the art and are described in NF XVII as the fully polymerized copolymers of acrylate and methacrylate having a low content of quaternary ammonium groups.

In one preferred embodiment, the acrylic polymer is an acrylic resin paint, such as those commercially available

from Rohm Pharma under the trade name EUDRAGIT t®. In a further preferred embodiment, the acrylic polymer comprises a mixture of two acrylic resin paints commercially available from Rohm Pharma under the trade names EUDRAGIT® RL30D and EUDRAGIT® RS30D, respectively. EUDRAGIT® RL30D and EUDRAGIT ® RS30D are the copolymers of acrylate and methacrylate having a low content of quaternary ammonium groups, and a molar ratio of the ammonium groups to the remaining neutral (meth)acrylate is 1:20 in EUDRAGIT ® RL30D and 1:40 in EUDRAGIT® RS30D. The average molecular weight is about 150,000. EUDRAGIT® S-100 and EUDRAGIT® L-100 are also preferred. The code names RL (high permeability) and RS (low permeability) refer to the permeability properties of these active agents. EUDRAGIT® RL/RS mixtures are insoluble in water and the digestive fluid. However, the multiparticle systems formed to contain them are swellable and permeable in an aqueous solution and the digestive fluid.

The above polymers, e.g. EUDRAGIT® RL/RS can be mixed together in any desired ratio to end up with a sustained release formulation having a desired dissolution profile. Desired sustained release multiparticle systems can be obtained from, for example, 100% EUDRAGIT® RL, 50% EUDRAGIT® RL and 50% EUDRAGIT t® RS, and 10% EUDRAGIT® RL and 90% EUDRAGIT® RS. Those skilled in the art will recognize that other acrylic polymers, such as EUDRAGIT® L, may also be used.

Alternatively, the extended release formulations can be prepared using the osmotic systems or by applying a semi-permeable coating to the dosage form. In the latter case, the desired drug release profile can be achieved by combining the low-permeability and high-permeability coating materials in suitable proportions.

Devices having the different drug release mechanisms as described above may be combined in a final dosage form comprising a single or multiple units. Examples of the multiple units include, but are not limited to, a multilayer tablet and a capsule containing tablets, beads, or particles. An immediate release portion may be added to an extended release system by using a coating or compression process or by applying an immediate release layer to the top of the extended release core in a multi-unit system such as a capsule containing the extended and immediate release beads.

Extended release tablets containing the hydrophilic polymers are prepared by the techniques well known in the art, such as the direct compression, the wet granulation or the dry granulation. Their formulations are typically incorporated with the polymers, diluents, binders and lubricants as well as active pharmaceutical ingredients. Typical diluents include inert powdered substances such as starch, the powdered cellulose, especially the crystalline and microcrystalline cellulose, sugars such as fructose, mannitol and sucrose, cereal powders and similar edible powders. Typical diluents include, for example, many types of starch, lactose, mannitol, kaolin, calcium phosphate or calcium sulphate, the inorganic salts such as sodium chloride and icing sugar. Powdered cellulose derivatives are also important. Typical tablet binders include the substances such as starch, gelatin and sugars such as lactose, fructose and glucose; the natural and synthetic gums can also be used, including arabic gum, alginate, methyl cellulose and polyvinylpyrrolidone. Polyethylene glycol, the hydrophilic polymers, ethyl cellulose and waxes may also be used as the binders. Lubricants are required in the tablet formulations to prevent the tablet and the punche from sticking in the mould. The lubricant is selected from such smooth solids as talc, magnesium stearate and calcium stearate, stearic acid and the hydrogenated vegetable oil.

Extended release tablets containing the wax materials are typically prepared using the methods known in the art, such as direct mixing, solidification and water dispersion. In the solidification method, the drug is mixed with the wax material and spray solidified or solidified and screened and processed.

(ii) Delayed Release Dosage Forms

Delayed release dosage forms can be produced by coating the solid dosage forms with the polymeric films being insoluble in the acidic environment of the stomach and soluble in the neutral environment of the small intestine.

Delayed-release dosage units can be prepared, for example, by coating a drug or drug-containing composition with a selected coating material. The drug-containing composition may be, for example, a tablet for incorporation into a capsule, a tablet for use as a core in a "core-coated" dosage form, or a plurality of drug-containing beads, particles, or granules for incorporation into a tablet or capsule. Preferred coating materials include the bioerosible, progressively hydrolysable, progressively water-soluble and/or enzymatically degradable polymers, and may be the conventional "enteric" polymers. As will be appreciated by those skilled in the art, the enteric polymers become soluble in the higher pH environment of the lower gastrointestinal tract or are slowly eroded as the dosage form passes through the gastrointestinal tract, while the enzymatically degraded polymers are degraded by the bacterial enzymes present in the lower gastrointestinal tract, particularly the colon. Suitable coating materials for achieving a delayed release include, but are not limited to, the cellulosic polymers such as hydroxypropyl cellulose, hydroxyethyl cellulose, hydroxymethyl cellulose, hydroxypropylmethyl cellulose, hydroxypropylmethyl cellulose acetate succinate, hydroxypropylmethyl cellulose phthalate, cellulose acetate trimellite ester and sodium carboxymethyl cellulose; the acrylic polymers and copolymers, preferably from acrylic acid, methacrylic acid, methyl acrylate, ethyl acrylate, methyl methacrylate and/or ethyl methacrylate, and other methacrylic resins commercially available under the trade name Eudragit® (Rohm Pharma; Westerstadt, Germany), including EUDRAGIT® L30D-55 and L100-55 (soluble at pH 5.5 and above), EUDRAGIT® L-100 (soluble at pH 6.0 and above), EUDRAGIT® S (soluble at pH 7.0 and above due to the higher degree of esterification), and EUDRAGITS® NE, RL

and RS (the water-insoluble polymers with different degrees of permeability and swellability); the ethylene polymers and copolymers, such as polyvinylpyrrolidone, vinyl acetate, polyvinyl acetate phthalate, a vinyl acetate crotonic acid copolymer, and an ethylene-vinyl acetate copolymer; the enzymatically degradable polymers, such as azo-polymers, pectin, chitosan, the straight-chain starch, and guar gum; and zein and shellac. Combinations of different coating materials can also be used. Multilayer coatings using different polymers can also be applied.

The preferred coating weight for a particular coating material can be readily determined by those skilled in the art via evaluating the individual release profiles of the tablets, beads, and granules prepared with varying amounts of the multiple coating materials. Combinations of materials, methods and application forms that produce the desired release characteristics can only be determined from the clinical studies.

The coating composition may include the conventional additives such as a plasticizer, a pigment, a colorant, a stabilizer, a flow aid and the like. A plasticizer is typically present to reduce the brittleness of the coating and typically account for about 10 wt. % to 50 wt. % relative to the dry weight of the polymer. Examples of the typical plasticizers include polyethylene glycol, propylene glycol, triacetin, dimethyl phthalate, diethyl phthalate, dibutyl phthalate, dibutyl sebacate, triethyl citrate, tributyl citrate, triethyl acetyl citrate, castor oil, and acetylated glycerol monoester. A stabilizer is preferably used to stabilize the particles in the dispersion. Typical stabilizers are non-ionic emulsifiers such as sorbitan ester, polysorbitan ester and polyvinylpyrrolidone. A flow aid is recommended to reduce the adhesion effect during the film formation and drying, and typically account for about 25 wt. % to 100 wt. % of the weight of the polymer in the coating solution. One effective flow aid is talc. Other flow aids such as magnesium stearate and glycerol monostearate may also be used. A pigment such as titanium dioxide may also be used. A small amount of an antifoam agent, such as silicone (e.g., dimethicone), may also be added to the coating composition.

3. Topical Formulations

Suitable dosage forms for the topical application include a cream, an unguentum, an ointment, a spray, a gel, a lotion, an emulsion and a transdermal patch. Said formulations may be prepared for a transmucosal, transepithelial, transendothelial or transdermal administration. The compounds may also be formulated for an intranasal delivery, a pulmonary delivery or an inhalation. The compositions may further comprise one or more chemical penetration enhancers, membrane permeants, membrane transporters, emollients, surfactants, stabilizers, buffers and a combination thereof.

In certain embodiments, it may be desired to provide a continuous delivery of one or more compounds to a patient in need thereof. For a topical application, a repeat application may be made, or a patch may be used to provide continuous administration of a compound over an extended period of time.

A "buffer" is used to control the pH of the composition. Preferably, the buffer buffers the pH of the composition from about 4 to about 7.5, more preferably from about 4 to about 7, and most preferably from about 5 to about 7. In preferred embodiments, the buffer is triethanolamine.

An "emollient" is a topical agent that soften or soothe the skin, and is generally known in the art and listed in the pharmacopoeia such as the "Handbook of Pharmaceutical Excipients", 4th edition, Pharmaceutical Press, 2003. These include, but are not limited to, almond oil, castor oil, a longhorn bean extract, cetostearoyl alcohol, cetanol, cetyl ester wax, cholesterol, cottonseed oil, cyclomethicone oil, glycol cetearate, glycerin, glycerol monostearate, glycerol monooleate, isopropyl myristate, isopropyl palmitate, lanolin, lecithin, light mineral oil, medium chain triglyceride, mineral oil and lanolin alcohol, vaseline oil, vaseline oil and lanolin alcohol, soybean oil, starch, stearyl alcohol, sunflower oil, xylitol, and a combination thereof. In one embodiment, the emollient is ethylhexyl stearate and ethylhexyl palmitate.

An "emulsifier" is a surface-active substance that promotes the suspension of one liquid in another and the formation of a stable mixture or emulsion of oil and water. Common emulsifiers are: a metal soap, certain animal and vegetable oils, and a variety of polar compounds. Suitable emulsifiers include arabic gum, an anionic emulsifying wax, calcium stearate, carbomer, cetostearoyl alcohol, cetanol, cholesterol, diethanolamine, glycol cetearate, glycerol monostearate, glycerol monooleate, hydroxypropyl cellulose, hydroxypropylmethyl cellulose, lanolin, hydration, lanolin alcohol, lecithin, medium chain triglyceride, methylcellulose, mineral oil and lanolin alcohol, sodium dihydrogen phosphate, monoethanolamine, a non-ionic emulsifying wax, oleic acid, poloxamer, poloxamer, polyoxyethylene alkyl ether, the polyoxyethylene castor oil derivatives, polyoxyethylene dehydrated sorbitan fatty acid ester, polyoxyethylene stearate, propylene glycol alginate, self-emulsifying glycerol monostearate, dehydrated sodium citrate, sodium dodecyl sulphate, the dehydrated sorbitan esters, stearic acid, sunflower seed oil, tragacanth gum, triethanolamine, xanthan gum, and a combination thereof. In one embodiment, the emulsifier is glyceryl stearate.

A "penetration enhancer" is known in the art and includes, but is not limited to, fatty alcohol, fatty acid ester, fatty acid, fatty alcohol ether, amino acid, phospholipid, lecithin, cholate, enzyme, amine and amide, a complexing agent (liposome, cyclodextrin, a modified cellulose, and diimide), a macrolide compound, such as macrolide, ketone, and anhydride, and cyclic urea, surfactant, N-methylpyrrolidone and derivatives thereof, DMSO and the related compounds, an ionic compound, azone and the related compounds, and solvent, such as alcohol, ketone, amide, and polyol (e.g., diol). Examples of these classes are known in the art.

A "preservative" may be used to prevent the growth of fungi and microorganisms. Suitable antifungal and antimicrobial agents include, but are not limited to, benzoic acid, butylparaben, ethylparaben, methylparaben, propylparaben, sodium benzoate, sodium propionate, benzalkonium chloride, benzethonium chloride, benzyl alcohol, cetylpyridinium chloride, chlorobutanol, phenol, phenylethanol, and thimerosal.

A "surfactant" is a surfactant which reduces the surface tension, thereby increasing the emulsifying, foaming, dispersing, spreading and wetting properties of the product. Suitable non-ionic surfactants include an emulsifying wax, glyceryl monooleate, polyoxyethylene alkyl ether, polyoxyethylene castor oil derivatives, polysorbate, dehydrated sorbitan ester, benzyl alcohol, benzyl benzoate, cyclodextrin, glyceryl monostearate, poloxamer, povidone, and a combination thereof. In one embodiment, the non-ionic surfactant is stearyl alcohol.

(a) Emulsions

An emulsion is a formulation of a liquid distributed in the form of small spheres throughout the body of a second liquid. In particular embodiments, the immiscible components of the emulsion comprises a lipophilic component and an aqueous component. The dispersion liquid is a discontinuous phase and the dispersion medium is a continuous phase. When the oil is a dispersed liquid and the aqueous solution is a continuous phase, it is referred to as an oil-in-water emulsion, while when the water or aqueous solution is a dispersion phase and the oil or oily substance is a continuous phase, it is referred to as a water-in-oil emulsion. One or both of the oil and water phases may contain one or more surfactants, emulsifiers, emulsion stabilizers, buffers and other excipients. Preferred excipients include the surfactants, especially the non-ionic surfactants; the emulsifiers, especially the emulsifying waxes; and the liquid non-volatile non-aqueous materials, especially diols, such as propylene glycol. The oil phase may comprise other oily pharmaceutically approved excipients. For example, the materials such as the hydroxylated castor oil or sesame oil may be used as the surfactants or emulsifiers in the oil phase.

The oil phase may consist of, at least in part, a propellant, such as an HFA propellant. One or both of the oil phase and the aqueous phase may contain one or more surfactants, emulsifiers, emulsion stabilizers, buffers and other excipients. Preferred excipients include the surfactants, especially the non-ionic surfactants; the emulsifiers, especially the emulsifying waxes; and the liquid non-volatile non-aqueous materials, especially diols, such as propylene glycol. The oil phase may comprise other oily pharmaceutically approved excipients. For example, the materials such as the hydroxylated castor oil or sesame oil may be used as the surfactants or emulsifiers in the oil phase.

A subset of the emulsions is a self-emulsifying system. These drug delivery systems are typically capsules (hard or soft shell) consisting of a drug dispersed or dissolved in a mixture of a surfactant and a lipophilic liquid (e.g., an oil or other liquid immiscible with water). When the capsule is exposed to an aqueous environment and the outer gelatin shell is dissolved, the contact between the aqueous medium and the capsule contents can immediately produce very small emulsion droplets. These are typically in the size range of micelles or nanoparticles. No mixing forces are required to produce the emulsion, which is typical in the emulsion preparation process.

(b). Lotions

By using the suspending and dispersing agents, the lotion may comprise a fine powdery substance insoluble in the dispersing medium. Alternatively, the lotion may have a liquid substance as a dispersed phase, said liquid substance is immiscible with the carrier and typically dispersed by an emulsifier or other suitable stabiliser. In one embodiment, the lotion is in the form of an emulsion with a viscosity between 100 and 1000 centistokes. The fluidity of the lotion permits rapid and uniform application over a wide range of surface areas. The lotion is typically intended to dry on the skin, leaving a thin layer of its pharmaceutical component on the surface of the skin.

(c). Creams

The creams may contain an emulsifier and/or other stabilizer. In one embodiment, the formulation is in the form of a cream having a viscosity greater than 1,000 centistokes, typically in the range of 20,000-50,000 centistokes. The creams are often preferred over the unguentums because they are generally easier to apply and remove.

The difference between a cream and a lotion is the viscosity, which depends on the amount/use of the multiple oils and the percentage of water used to prepare the formulation. The cream is usually thicker than the lotion, may have multiple uses, and often one uses more different oils/butter depending on the desired effect on the skin. In cream formulation, the water-based percentage is about 60-75% of the total, the oil-based percentage is about 20-30%, and the other percentage is the percentage of an emulsifier, a preservative, and an additive, totalling 100%.

(d). Unguentums

Examples of suitable unguentum matrices include the hydrocarbon matrices (e.g., vaseline, white vaseline, yellow unguentum, and mineral oil); the absorbent matrices (hydrophilic vaseline, anhydrous lanolin, lanolin, and cold cream); the water-soluble matrices (e.g., the hydrophilic unguentums) and the water-soluble matrices (e.g., polyethylene glycol unguentum). The pastes typically differ from the unguentums in that they contain a larger percentage of solids. The pastes are typically more absorbent and less greasy than the unguentums prepared using the same components.

(e). Gels

Gel is a semi-solid system containing the dispersions of small or large molecules in a liquid carrier becoming a semi-solid by the action of a thickener or a polymeric material dissolved or suspended in the liquid carrier. The liquid may comprise a lipophilic component, an aqueous component, or both. Some emulsions may be gels or otherwise include a gel component. However, some gels are not the emulsions because they do not include a homogeneous mixture of immiscible components. Suitable gels include, but are not limited to, a modified cellulose, such as hydroxypropyl cellulose and hydroxyethyl cellulose; Carbopol homopolymers and copolymers; and a combination thereof. Suitable solvents in the liquid carriers include, but are not limited to, diethylene glycol monoethyl ether; alkylene glycol, such as propylene glycol; dimethyl isosorbide; alcohols, such as isopropanol and ethanol. Solvents are usually selected based on their ability to dissolve a drug. Other additives that improve the skin feel and/or emollient character of the formulation may also be incorporated. Examples of such additives include, but are not limited to, isopropyl myristate, ethyl acetate, $C_{12}$-$C_{15}$ alkyl benzoate, mineral oil, squalane, cyclomethicone, caprylic/capric triglycerides and a combination thereof.

(f). Foaming Agent

The foaming agent consists of a combination of an emulsion and a gaseous propellant. The gaseous propellant consists mainly of hydrofluoroalkane (HFA). Suitable propellants comprise HF , such as 1, 1, 1, 2-tetrafluoroethane (HFA 134a) and 1, 1, 1, 2, 3, 3, 3-heptafluoropropane (HFA 227). The propellant is preferably not a hydrocarbon propellant gas, which generates the flammable or explosive vapours during spraying. Furthermore, the composition preferably does not contain a volatile alcohol, which generates the flammable or explosive vapours during use.

II. Methods of preparation and use

Accordingly, the disclosed compositions may be used in a subject in need thereof for the treatment of the aging-related disorders and the disorders associated with an abnormal inflammation. Preferably, the subject is a mammal, more preferably a human. As used herein, the aging-associated disorders or diseases include the disorders or diseases associated with or caused by the cellular senescence, including the age-related diseases and disorders. Aging-associated diseases or disorders may also be referred to as the senescent cell-associated diseases or disorders.

Celluar senescence is a cellular response characterized by a stable growth arrest and other phenotypic changes, including a pro-inflammatory secretome. It plays a role in the normal development, maintaining the tissue homeostasis and limiting the tumor development. However, Celluar senescence has also been shown to be a major cause of the age-related diseases. Senescent cells accumulate with age and have been involved in a number of diseases, including cancer, fibrosis and many age-related pathologies. Recent evidence suggests that the senescent cells are harmful in multiple pathologies and that their elimination has many advantages, ameliorating multiple pathologies and prolonging a health span and a lifespan.

Senescent cells are present in many preneoplastic lesions, fibrotic tissues (e.g., liver, kidney, heart, pancreas) and old tissues.

While SA-β-gal is widely used as a marker of cellular senescence (Childs, et al., Nat Med, 21(12), 1424-1435 (2015); Hernandez-Segura et al., Trends Cell Biol, 28(6), 436-453 (2018); Sharpless & Sherr, Nat Rev Cancer, 15(7), 397-408 (2015)), and an elevated activity has been found in some non-senescent cells, such as the activated macrophages (Bursuker, Rhodes, & Goldman, J Cell Physiol 112, 385-390 (1982); Frescas, et al., Cell Cycle 16, 1526-1533 (2017)). These SA-β-gal-positive macrophages can be harmful and have been found to accumulate in the injured and aged tissues, leading to a chronic inflammation (Hall, et al., Aging (Albany NY) 8, 1294-1315 (2016); Oishi & Manabe, NPJ Aging Mech Dis 2, 16018 (2016)).

Viral Disorders.

The disclosed compositions can be used to alleviate the symptoms associated with an abnormal cytokine production caused by a virus-induced inflammation. Exemplary viral infections during which a subject may benefit from the

administration of an effective amount of a formulation disclosed herein include coronaviruses, for example coronaviruses, such as Severe Acute Respiratory Syndrome (SARS) Coronavirus (CoV), SARS-CoV-2 (which causes COVID-19 (Coronavirus Disease 2019)), and Middle East Respiratory Syndrome (MERS)-CoV. SARS-CoV-2 virus is a beta coronavirus, like MERS-CoV and SARS-CoV. These highly pathogenic human respiratory coronaviruses cause an acute lethal illness characterised by an inflammatory response and a lung injury. Robust replication of SARS-CoV accompanied by a delayed type I interferon (IFN-I) signalling can coordinate the inflammatory response and pulmonary immunopathology with a reduced viability. IFN-I remains detectable until the virus titer reaches its peak, but early IFN-I administration alleviates immunopathology. This delayed IFN-I signalling promotes the accumulation of the pathogenic inflammatory monocyte-macrophages (IMMs), leading to an elevated lung cytokine/chemokine level, a vascular leakage and an impaired virus-specific T-cell response. Increasing evidences suggest that a subset of the patients with severe coronavirus disease 2019 (COVID-19) may suffer from a cytokine storm syndrome (Mehta, *et al.,* DOI: https://doi.org/10.1016/50140-6736 (20)30628 -0).

The composition is administered in an effective amount to reduce the level of one or more macrophages in the subject. In the particularly preferred embodiments, the disclosed compositions are administered to a subject infected with a coronavirus, preferably, SARS-CoV or SARS coronavirus 2 (Sars-CoV-2).

Cancer and Cancer Treatment Applications

Studies have shown that through SASP (the senescence-associated secretory phenotype), the aged tissues provide a supportive microenvironment for cancer. Senescent cells can promote the tumor development by enhancing the proliferative potential of cancer cells or by promoting an epithelial-to-mesenchymal transition. Thus, an increase in the number of senescent cells in aged tissues may lead to an increased incidence of cancer with age. Supporting this, a delayed onset of tumor formation is observed when the senescent cells are eliminated. Anti-senescence drug therapy also reduces the incidence of metastasis, the leading cause of a cancer-related death. (Summaried in McHugh, et al., J. Cell Biol., 217 (1):65-77 (2018).

In some embodiments, the compositions disclosed herein may be used in combination with a cancer therapeutic agent. Therefore, in some preferred embodiments, the treated subject has been diagnosed with cancer. Non-limiting examples of cancers that may be treated according to this aspect of the present invention include: adenocarcinomas, adrenal tumor, ameloblast tumor, anaplastic carcinoma, anaplastic thyroid carcinoma, angiofibromas, hemangiomas, hemangiosarcomas, abscesses, argyria tumor, nephroblastomas, ascites tumor cells, ascites tumor, astroblastomas, astrocytomas, ataxia telangiectasia, atrial myxoma, basal cell carcinoma cells, bone cancer, brain stem glioma, brain tumor, breast cancer, Burkitt's lymphoma, cerebellar astrocytoma, cervical cancer, cherry angioma, cholangiocarcinoma, cholangioma, chondroblastoma, chondroma, chondrosarcoma, chorioblastoma, choriocarcinoma, colon cancer, common acute lymphocytic leukemia, craniopharyngioma, cystic carcinoma, cystofibroma, cystoma, ductal carcinoma in situ, ductal papilloma, disgerminoma, brain tumor, endometrial carcinoma, endothelioma, ependymoma, epithelioma, erythroleukaemia, Ewing's sarcoma, extranodal lymphoma, feline sarcoma, adenoma fibrosum, fibrosarcoma, follicular thyroid carcinoma, ganglioglioma, gastrinoma cell, glioblastoma multiforme, neuroglioma, gonadoblastoma, hemangioblastoma, hemangioendothelioblastoma, hemangioendothelioma, hemangiopericytoma, hemocytoblastoma, hemocytoma, hairy cell leukemia, hamartoma, liver cancer, hepatocellular carcinoma, histioma, Hodgkin's disease, adrenal tumor, infiltrating cancer, infiltrating ductal cell cancer, insulinoma, angioforoma, Kaposi's sarcoma, renal tumor, large cell lymphoma, leukaemia, leukaemia, acute leukaemia, lipoma, liver cancer, liver metastases, Lucké carcinoma, lymphadenoma, lymphangioma, lymphocytic leukemia, lymphocytic lymphoma, lymphocytoma, lymphoedema, lymphoma, lung cancer, malignant mesothelioma, malignant teratoma, mast cell tumor, medulloblastoma, melanoma, meningioma, mesothelioma, Morton's neuroma, multiple myeloma, myeloblastoma, myeloid leukaemia, myelolipoma, myeloma, myoblastoma, myxoma, nasopharyngeal carcinoma, tumor, nephroblastoma, neuroblastoma, neurofibroma, neurofibromatosis, neuroglioma, neuroma, non-Hodgkin's lymphoma, oligodendroglioma, optic glioma, osteochondroma, osteogenic sarcoma, osteosarcoma, ovarian carcinoma, Paget's disease of the nipple, superior pulmonary sulcus tumor, pancreatic cancer, phaeochromocytoma, pheochromocytoma, plasma cell tumor, primary brain tumor, progonoma, prolactinoma, renal cell carcinoma, retinoblastoma, rhabdomyosarcoma, rhabdo sarcoma, solid tumor, sarcoma, secondary tumor, seminoma, skin cancer, small cell carcinoma, squamous cell carcinoma, strawberry hemangioma, T-cell lymphoma, teratoma, testicular carcinoma, thymoma, trophoblastic neoplasia, Wilm's tumor.

In one preferred embodiment, the disclosed anti-senescence prodrugs are used in combination with the treatments for other cancers that induce senescence, such as a radiotherapy or a chemotherapy (*e.g.*, treatments with Palbociclib, ribociclib or abemaciclib or other chemotherapeutic agents). Thus, by treating at the same time or after other treatments, said active agent may: (a) eliminate the cancer cells that have been pushed to senescence; and/or eliminate or reduce certain side effects produced by the senescent cells, such as an inflammation, a promotion of cancer growth, a promotion of metastasis, and other side effects of a chemotherapy or a radiotherapy; and/or (b) reduce or eliminate the pre-cancerous lesions; and/or (c) eliminate or reduce the cancer cells that undergo senescence by treating with a CDK4/ 6

inhibitor. The disclosed active agents may reduce or eliminate pre-cancerous (or pre-cancerous) lesions. Senescent cells are present in a pre-cancerous tumor. In this regard, it should be understood that a large number of cells in the pre-cancerous tumor undergo oncogene-induced senescence. Therefore, the disclosed active agents may be used to reduce or eliminate the pre-cancerous (or pre-tumor) lesions.

In another preferred embodiment, the disclosed anti-senescence prodrugs are administered, for example, before, during or after the treatment with a chemotherapeutic agent to eliminate or reduce the chemotherapy-induced senescence. Therefore, in one preferred embodiment, said therapeutic agent may be used for a treatment in combination with a chemotherapeutic agent, wherein the therapeutic agent is administered separately from, sequentially to, or concurrently with the chemotherapeutic agent. Exemplary chemotherapeutic agents include, but are not limited to, anthracyclines, doxorubicin, etoposide, daunorubicin, taxol, paclitaxel, gemcitabine, pomalidomide, and lenalidomide. The disclosed anti-senescence prodrugs may eliminate or reduce the senescence induced by treatment with a CDK inhibitor, such as a CDK4 or CDK6 inhibitor. Therefore, in one preferred embodiment, the disclosed anti-senescence prodrug may be used in combination for a treatment with a CDK4 or CDK6 inhibitor, wherein said therapeutic agent is administered separately from, sequentially to, or concurrently with a CDK4 or CDK6 inhibitor. The disclosed anti-senescence prodrugs can eliminate or reduce a Palbociclib-induced senescence. In the context of eliminating or reducing the Palbociclib-induced senescence, the administration of said therapeutic agent may potentially prevent cancer remission when the cells re-enter the cell cycle. In another preferred embodiment, said therapeutic agent may eliminate the cancer cells that have been pushed to senescence. In one preferred embodiment, the therapeutic agent delays tumorigenesis.

In another preferred embodiment, the disclosed anti-senescence prodrugs reduce certain side effects produced by the senescent cells, such as an inflammation, a promotion of cancer growth, a promotion of metastasis, and other side effects of chemotherapy or radiotherapy.

Chemotherapy-induced side effects or radiotherapy-induced side effects include, but are not limited to, weight loss, an endocrine change, a hormonal imbalance, a hormonal signalling change, a cardiotoxic change, the body composition, a reduced physical mobility, a gastrointestinal toxicity, nausea, vomiting, constipation, anorexia, diarrhoea, peripheral neuropathy, fatigue, malaise, a physical inactivity, a haematological toxicity, anaemia, hepatotoxicity, hair loss, pain, infection, mucositis, fluid retention, a dermatological toxicity, rash, dermatitis, hyperpigmentation, urticaria, photosensitivity, a nail change, the problems with the mouth, gum, or throat, and any toxic side effect caused by a chemotherapy or a radiotherapy. Therefore, in one preferred embodiment, said therapeutic agent may be used for a combination therapy with a chemotherapeutic agent, wherein the therapeutic agent is administered separately from, sequentially to, or concurrently with the chemotherapeutic agent. Similarly, said therapeutic agent may be used in a combination therapy with a radiotherapy, wherein the therapeutic agent is administered before, during or after the radiotherapy. Another embodiment of the present invention relates to a therapeutic agent as described herein, which is used to reduce or alleviate one or more chemotherapy-induced or radiotherapy-induced side effects.

In some embodiments, provided is a method for treating or reducing the likelihood of metastasis comprising administering a therapeutic agent as described herein during a non-chemotherapy or non-radiotherapy interval or after a chemotherapy or radiotherapy treatment regimen has been completed. Another embodiment of the present invention relates to a therapeutic agent as described herein for treating metastasis or reducing the likelihood of metastasis.

In one preferred embodiment, the disclosed anti-senescence prodrugs may be used to treat the chronic or long-term chemotherapy-induced or radiotherapy-induced side effects. Certain toxic effects may appear for a long time after the treatment and may be due to the damage to the organs or systems caused by the treatment. Organ dysfunctions, such as neurological, pulmonary, cardiovascular and endocrine dysfunctions, can be observed in the subjects treated for cancer during the childhood. The chronic or late toxic and side effects occurring in the subjects receiving a chemotherapy or a radiotherapy include, for example, cardiomyopathy, congestive heart disease, inflammation, premature climacteric, infertility, an impaired cognitive function, peripheral neuropathy, secondary cancer, cataracts and other vision problems, hearing loss, chronic fatigue, diminished lung capacity, and pulmonary disease.

Non-Cancer/Cancer Therapeutic Applications

In other preferred embodiments, the subject is not diagnosed with cancer and does not receive a cancer treatment. In these embodiments, the disclosed compositions are administered to reduce a population of senescent cells in a subject in need thereof.

Senescent cells are associated with a long list of other pathologies, including neuropathology (*e.g.*, cerebral aneurysm, Alzheimer's disease, and Parkinson's disease), pulmonary pathology (*e.g.*, idiopathic pulmonary fibrosis, chronic obstructive pulmonary disease, and cystic fibrosis), ophthalmic pathology (*e.g.*, cataract, glaucoma, and macular degeneration), musculoskeletal pathology (*e.g.*, sarcopenia, and intervertebral disc degeneration), cardiovascular pathology (*e.g.*, atherosclerosis, cardiac fibrosis, aortic aneurysm), renal pathology (*e.g.*, transplantation complications), and others, such as mucositis, hypertension, hepatic fibrosis, and myelofibrosis (OMF).

A distinctive feature of aging is a progressive loss of function, or a degeneration that occurs at the molecular, cellular, tissue and organismal levels. Age-related degeneration causes recognized pathologies, such as sarcopenia, atherosclerosis and heart failure, pulmonary insufficiency, renal failure, neurodegeneration (including macular degeneration, Alzheimer's disease and Parkinson's disease), among others. Aging-related diseases and disorders include, but are not limited to, the cardiovascular diseases and disorders, the inflammatory diseases and disorders, the autoimmune diseases and disorders, the pulmonary diseases and disorders, the ocular diseases and disorders, the metabolic diseases and disorders, the nervous system diseases and disorders (*e.g.*, the neurodegenerative diseases and disorders); the age-related diseases and disorders caused by aging; the skin conditions; the age-related diseases; the skin diseases and disorders; and the transplantation-related diseases and disorders.

Diseases Associated with an Elevated β-galactosidase Activity

In another embodiment, said therapeutic agents may be used to treat a disease or disorder associated with or related to an elevated β-galactosidase activity. In preferred embodiments, the elevated β-galactosidase activity is the result of elevated β-galactosidase expression or overexpression of β-galactosidase activity relative to a baseline level, which can be determined by a standard method. The expression of β-galactosidase can be detected in cells by a histochemical or immunohistochemical method. For example, the senescence marker SA-β galactosidase (SA β-Gal) can be detected by a known method (Dimri, et al., Proc. Natl. Acad. Sci. USA 92: 9363-9367 (1995)).

In a more preferred embodiment of the present invention, said disease is selected from Wiedemann-Rautenstrauch syndrome of the neonatal progeria, Werner syndrome of the adult progeria, Hutchinson-Gilford syndrome, Rothmund Thompson syndrome, Mulviii-Smith syndrome, Cockayne syndrome, congenital dyskeratosis, idiopathic pulmonary fibrosis, aplastic anaemia, emphysema, and cartilage degeneration.

Hutchinson-Gilford progeria syndrome ("progeria" or 'HGPS') is a rare and fatal genetic disorder characterized by an accelerated ageing in children. Although they appear healthy at birth, the children with progeria begin to show many signs of an accelerated aging within the first two years of life. Signs of progeria include growth disorders, loss of body fat and hair, aging skin, stiff joints, hip dislocation, systemic atherosclerosis, cardiovascular (heart) disease and stroke. Other progeria syndromes include WerneR's syndrome, also known as "adult progeria", which does not develop until late adolescence. There is no cure for progeria, but if a child has stiff joints, an occupational and physical therapy can help them stay active. The disclosed compositions and methods can alleviate the accelerated aging symptoms associated with progeria syndrome. The following examples show that the elimination of L1 RNA from the cells obtained from the HGPS mouse model (LAKI) using the antisense oligonucleotides (AON) restores the levels of epigenetic markers and reduces the expression of aging-related genes, extending lifespan.

Cardiovascular Diseases

In a preferred embodiment of the present invention, the aging-related disease or disorder is cardiovascular disease. Examples include, but are not limited to, atherosclerosis, angina pectoris, arrhythmia, cardiomyopathy, congestive heart failure, coronary artery disease, carotid artery disease, endocarditis, coronary thrombosis, myocardial infarction, hypertension, aortic aneurysm, cardiac diastolic dysfunction, hypercholesterolemia, hyperlipidemia, mitral valve prolapse, peripheral vascular disease, cardiac stress resistance, cardiac fibrosis, cerebral aneurysm and stroke. In one preferred embodiment, the aging-related disease or disorder is related to or caused by atherosclerosis (*i.e.*, the sclerosis of the arteria).

Atherosclerosis is characterised by the patchy intimal plaques (the atheromatous plaques) which invade the lumen of medium and large arteries. Administration of a therapeutic agent according to the present invention reduces the lipid content of the atherosclerotic plaques in the blood vessels of the subject and/or increases the thickness of the fibrous cap.

Inflammatory and Autoimmune Diseases and Disorders

In one preferred embodiment of the present invention, the aging-related disease or disorder is an inflammatory or autoimmune disease or disorder. Non-limiting examples of the autoimmune diseases include psoriasis, oral mucositis, rheumatoid arthritis, inflammatory bowel disease, eczema, kyphosis (spinal curvature), intervertebral disc herniation, and a pulmonary disease, COPD and idiopathic pulmonary fibrosis. In one preferred embodiment, the aging-related disease or disorder is a chronic inflammation.

Therefore, in some embodiments, the aging-related disease or disorder may be rheumatoid arthritis. Rheumatoid arthritis is a chronic inflammatory disorder which typically affects the joints of the hands and feet.

Nervous System Diseases or Disorders

In one preferred embodiment of the present invention, the aging-related diseases or disorders are a neurological disease or disorder selected from Alzheimer's disease (and other dementias), Parkinson's disease, Huntington's disease, dementia, mild cognitive impairment (MCI), macular degeneration, and motor neuron dysfunction (MND), as well as a disease and disorder of the eye, such as an age-related macular degeneration.

Parkinson's disease (PD) is a disabling condition of the brain characterized by slow movement (bradykinesia), tremor, stiffness, postural instability and loss of balance. Many of these symptoms are due to the loss of certain nerves in the brain, which results in a dopamine deficiency. Aging of the dopamine-producing neurons is thought to result in the cell death observed in PD through the production of reactive oxygen species.

Alzheimer's disease (AD) is a neurodegenerative disease that manifests itself as a slowly progressive mental decline with memory loss, disorientation and confusion, leading to severe dementia. As the disease progresses, the impaired judgement, confusion, behavioral changes, disorientation, and difficulty walking and swallowing occur. Age is the single greatest predisposing risk factor for the development of AD, and is the leading cause of dementia in the elderly (Hebert, et al., Arch. Neurol. 60:1 19-1122 (2003)). Early clinical symptoms show significant similarity to mild cognitive impairment.

Mild cognitive impairment (MCI) is a syndrome of brain function that involves the onset and progression of cognitive impairment beyond what would be expected based on an individual's age and education, but not enough to interfere with an individual's daily activities. MCI is an aspect of cognitive aging, which is considered to be a transitional state between the normal aging and its possible transformation into dementia (Pepeu, Dialogues in Clinical Neuroscience, 6:369-377, 2004). MCI that primarily affects memory is referred to as "amnestic MCI" and are often regarded as a prodromal stage of Alzheimer's disease. MCI that affects thinking skills other than memory is referred to as "non-amnesic MCI".

MND is a group of progressive nervous system disorders that destroy the motor neurons being the cells that control a voluntary muscle activity, such as speaking, walking, breathing, and swallowing. Examples of MND include amyotrophic lateral sclerosis (ALS), also known as Lou Gehrig's disease, progressive bulbar paralysis, pseudo-bulbar paralysis, primary lateral sclerosis, progressive muscular dystrophy, lower motor neurone disease and spinal muscular atrophy (SMA) (*e.g.*, SMA1 (also known as Werdnig-Hoffmann disease), Kugelberg-Welander syndrome and Kennedy disease, post-polio syndrome, and hereditary spastic paraplegia.

Ophthalmic Diseases and Disorders

In one preferred embodiment of the present invention, the aging-related disease or disorder is an ocular disease, disorder or condition. Examples include, but are not limited to, presbyopia, macular degeneration, cataracts, and glaucoma. Macular degeneration is a neurodegenerative disease that causes the loss of photoreceptor cells in the central portion of the retina (known as the macula).

Metabolic Diseases

In one preferred embodiment of the present invention, the aging-related disease or disorder is a metabolic disease selected from diabetic ulcer, metabolic syndrome and obesity.

Senescent cells are understood to play a role in the metabolic diseases, such as obesity. Studies have shown that the adipose tissue from the obese mouse show an induction of the senescence markers SA-p-Gal, p53 and p21 (Tchkonia, et al., Aging Cell 9: 667-684 (2010); Minamino, et al., Nat. Med. 15: 1082-087 (2009)). The therapeutic agents described herein have potential applications in the management or prevention of obesity and the metabolic syndrome.

Pulmonary Diseases or Disorders

In one preferred embodiment of the present invention, the aging-related disease or condition is a lung disease. Examples include, but are not limited to, pulmonary fibrosis, chronic obstructive pulmonary disease (COPD), asthma, cystic fibrosis, emphysema, bronchiectasis and an age-related loss of lung function.

COPD is a lung disease defined as the persistent poor airflow caused by rupture of the lung tissue (emphysema) and the small airway dysfunction (obstructive bronchiolitis). Pulmonary fibrosis is a chronic progressive lung disease characterised by sclerosis and scarring of the lungs which may lead to respiratory failure, lung cancer and heart failure. The therapeutic agents of the present invention may also be used to treat the subjects who are aging and have loss (or degeneration) of lung function (*i.e.*, decreased or impaired lung function as compared with the younger subjects) and/or degeneration of the lung tissue.

Other Age-Related Disorders

In some embodiments, the aging-related disease or disorder treated is an age-related disorder selected from renal failure, frailty, hearing loss, muscle fatigue, skin conditions, skin wound healing, hepatic fibrosis, pancreatic fibrosis, oral submucous fibrosis, and sarcopenia.

Dermatological Diseases or Disorders

In one preferred embodiment of the present invention, the aging-related disease or disorder is a dermatological disease or disorder, examples include, but are not limited to, eczema, psoriasis, hyperpigmentation, nevus, rash, atopic dermatitis (a form of eczema and inflammation related to it), urticaria, a disease and disorder related to photosensitivity or photo-aging, wrinkle (wrinkle due to aging); pruritis (related to aging); sensory dullness (side effect of chemotherapy associated with multiple sclerosis); eczematous drug rash (often observed in the older patients and associated with side effects of certain drugs); eosinophilic dermatosis (associated with certain types of haematological cancers); reactive neutrophilic dermatosis (associated with the underlying diseases such as inflammatory bowel syndrome); pemphigus; pemphigoid; immune bullous dermatosis (skin autoimmune blister); skin fibrohistiocytic hyperplasia; cutaneous lymphoma; and cutaneous lupus. Late-onset lupus may be associated with the reduced (*i.e.*, decreased) function of age-related T and B cells and cytokines (immunesenescence).

Lifespan and Age-Related Diseases or Disorders

In one preferred embodiment, the therapeutic agents described herein may be used to extend the lifespan of a subject by selectively killing the senescent cells rather than the non-senescent cells. In some embodiments, extending the lifespan of the subject comprises delaying the onset or progression of an age-related disease or disorder. In some embodiments, the age-related disease or disorder is selected from atherosclerosis, cardiovascular disease, cancer, arthritis, dementia, cataract, hypertension, age-related fat loss, and lipodystrophy. In some embodiments, the age-related disease or disorder is an elderly anxiety disorder. In some embodiments, the age-related disease or disorder is an age-related inactivity. In some embodiments, the age-related disease or disorder is a decrease in spontaneous activity. In some embodiments, the age-related disease or disorder is a decrease in exploratory behaviour.

## Examples

The experimental methods in the following examples are conventional unless otherwise stated.

Unless otherwise stated, all chemical reactions were carried out with the dry solvents under the anhydrous conditions in a nitrogen atmosphere. Unless otherwise stated, the reagents were purchased at the highest commercial quality without further purification. The yield was measured by chromatography.

Reactions were monitored by thin layer chromatography on plates (GF254) supplied by Yantai Chemicals (China) using UV light as the color developer, the ethanol solution of phosphomolybdic acid and cerium sulphate and heat as the developer. Unless otherwise stated, the silica gel (200-300 mesh) supplied by Tsingtao Haiyang Chemicals (China) was used for the flash column chromatography.

Unless otherwise stated, NMR spectra were recorded on a Brüker Advance 600 ($^{13}C$ 150 MHz, $^{19}F$ 565 MHz) and a Brüker Advance 400 ($^{1}H$ 400 MHz) spectrometers being calibrated with a residual undeuterated solvent ($CD_3OD$ at 3.31 ppm $^{1}H$ NMR, 49.0 ppm $^{13}C$ NMR). The following abbreviations are used to interpret the multiplicity: s = single peak, d = double peak, t = triple peak, dd = double peak of double peak, m = multiple peak. Mass spectrometry data were obtained using the Brüker Apex IV FTMS using ESI (electrospray ionisation).

Example 1

Synthesis of the prodrug A13bGa1 by the β-gal modification of A13

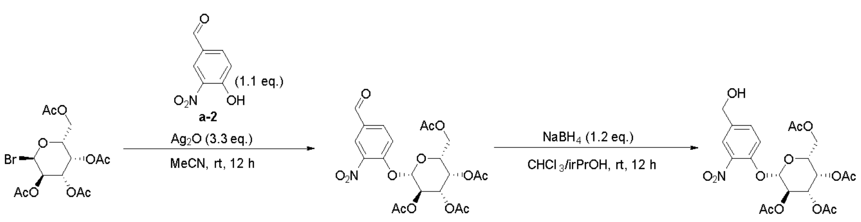


a-1 a-3 a-4

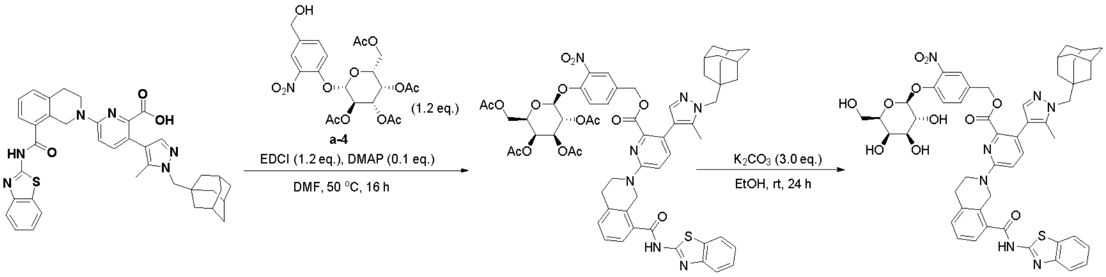


a-5 (A13) a-6 a-7 (A13bGa1)

Synthesis of the compound a-3

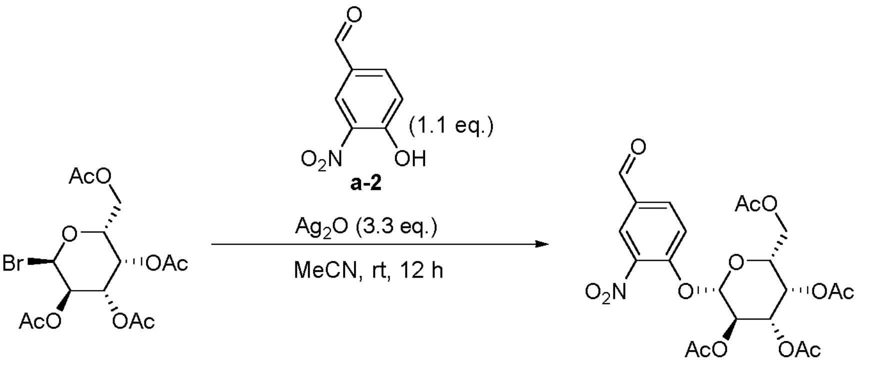


a-1 a-3

**[0244]** , 4-formyl-2-nitrophenol (2.24 g, 13.376 mmol, 1.1 eq) and $Ag_2O$ (9.30 g, 40.128 mmol, 3.3 eq) were added into a stirred solution of [(2R, 3S, 4S, 5R, 6R)-3, 4, 5-tris (acetyloxy) -6-bromotetrahydropyran-2-yl] methylacetate (5.0g, 12.160 mmol, 1 eq) and MeCN (100 mL) solution at room temperature. The obtained mixture was stirred at room temperature for 12 hours, and the reaction was detected by the thin layer chromatography (TLC) and the liquid chromatography (LCMS). The obtained mixture was concentrated under a reduced pressure, the residue was purified by the silica gel column chromatography, and the compound a-3 (5.1g, 84%) obtained by eluting with PE/EA (1/1) was a white solid.

LCMS15-compound a-3: (ESI, m/z): $[M+NH4]^+$ = 515.2.

H-NMR16-compound a-3: $^1$H NMR (300 MHz, chloroform-d) δ 9.98 (s, 1H), 8.30 (d, J = 2.1 Hz, 1H), 8.07 (dd, J = 8.7, 2.1 Hz, 1H), 7.48 (d, J = 8.6 Hz, 1H), 5.59 (dd, J = 10.4, 7.9 Hz, 1H), 5.49 (d, J = 3.4 Hz, 1H), 5.21 (d, J = 7.9 Hz, 1H), 5.13 (dd, J = 10.4, 3.4 Hz, 1H), 4.32 - 4.05 (m, 3H), 2.19 (s, 3H), 2.13 (s, 3H), 2.08 (s, 3H), 2.02 (s, 3H).

Synthesis of the compound a-4

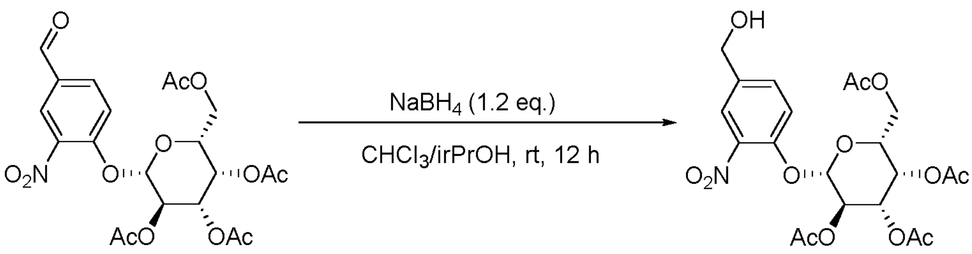


a-3 a-4

$NaBH_4$ (0.47 g, 12.304 mmol, 1.2 eq) was added to a stirred solution of chloroform (100 mL), propan-2-ol (20 mL), and [(2R, 3S, 4S, 5R, 6S)-3, 4, 5-tris (acetyloxy)-6-(4-formyl-2-nitrophenoxy) tetrahydropyran-2-yl] methylacetate (5.1 g, 10.253 mmol, 1 eq) at room temperature. The obtained mixture was stirred at room temperature for 12 hours, and the reaction was detected by the thin layer chromatography (TLC) and the liquid chromatography (LCMS). The obtained mixture was concentrated under a reduced pressure, the residue was purified by the silica gel column chromatography eluting, and the compound a-4 (4.8 g, 93%) obtained by eluting with PE/EA (1:2) was a white solid.

LCMS15-compound a-4: (ESI, m/z): $[M+NH_4]^+$ = 517.2.

I-NMR30-compound a-4: $^1$H NMR (400 MHz, DMSO-$d_6$) δ 7.79 (d, J = 2.1 Hz, 1H), 7.62 (dd, J = 8.6, 2.1 Hz, 1H),

7.37 (d, J = 8.6 Hz, 1H), 5.54 (d, J = 7.5 Hz, 1H), 5.41 (d, J = 5.7 Hz, 1H), 5.36 (dd, J = 3.3, 1.1 Hz, 1H), 5.29 - 5.18 (m, 2H), 4.51 (d, J = 5.7 Hz, 2H), 4.47 (m, 1H), 4.13 (t, J = 6.1 Hz, 2H), 2.15 (s, 3H), 2.03 (d, J = 0.8 Hz, 6H), 1.94 (s, 3H).

Synthesis of the compound a-6

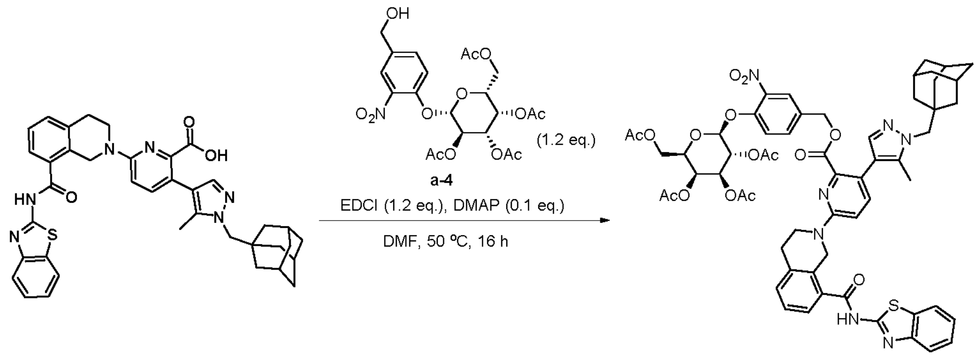


a-5 (A13) a-6

EDC. HCl (69.83 mg, 0.365 mmol, 1.2 eq) was added to a stirred solution of 3-[1-(adamantan-1-ylmethyl)-5-methylpyrazol-4-yl]-6-{8-[(1,3-benzothiazol-2-yl)carbamoyl]-3,4-dihydro-1H-isoquinolin-2-yl}pyridine-2-carboxylic acid (200 mg, 0.304 mmol, 1 eq), compound 4a (181.93 mg, 0.365 mmol, 1.2 eq) and DMF (50 mL) at room temperature. The obtained mixture was stirred at 50°C for 16 hours, and the reaction was monitored by liquid chromatography (LCMS). The obtained mixture was concentrated under vacuum, and the residue was purified by the reverse flash chromatography under the following conditions to obtain the product: the column: C18, 330 g; mobile phase A: water/0.05% FA, mobile phase B: ACN; the flow rate: 100 mL/min; the gradient: from 5% B to 10% B in 3 minutes; from 10% B to 25% B in 0 minute; and from 25% B to 80% B in 35 minutes; from 80% B to 95% B in 5 minutes; detection: 254/220 nm. 24 batches were reacted in parallel to finally obtain the compound a-6 (4.5 g, 52%) as a light yellow solid.

LCMS24-compound a-6: (ESI, m/z): $[M+H]^+$ =1140.1.

H-NMR10- compound a-6: $^1$H NMR (300 MHz, DMSO-$d_6$) δ 12.86 (s, 1H), 8.04 - 7.97 (m, 1H), 7.76 (d, J = 8.2 Hz, 1H), 7.68 - 7.52 (m, 3H), 7.51 - 7.41 (m, 3H), 7.40 - 7.29 (m, 3H), 7.22 (s, 1H), 7.01 (d, J = 8.9 Hz, 1H), 5.54 (d, J = 7.3 Hz, 1H), 5.37 (dd, J = 3.1, 1.1 Hz, 1H), 5.28 - 5.19 (m, 2H), 5.08 (s, 2H), 4.97 (s, 2H), 4.57 - 4.45 (m, 1H), 4.12 (d, J = 6.2 Hz, 2H), 3.87 (d, J = 5.9 Hz, 2H), 3.62 (s, 2H), 3.02 (d, J = 5.9 Hz, 2H), 2.14 (s, 3H), 2.07 (s, 1H), 2.02 (d, J = 2.2 Hz, 8H), 1.95 (s, 3H), 1.86 (s, 3H), 1.58 (s, 3H), 1.46 (s, 9H).

Synthesis of the compound a-7 (A13bGa1)

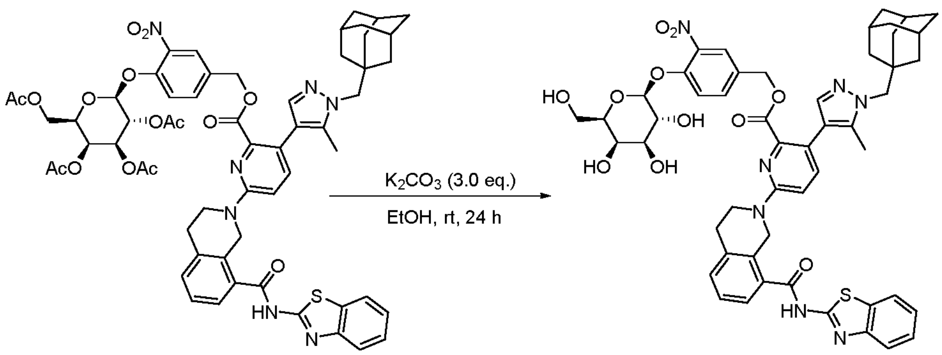


a-6 a-7 (A13bGa1)

Potassium carbonate (36.36 mg, 0.264 mmol, 3 eq) was added to a stirred solution of the compound a-6 (100 mg, 0.088 mmol, 1 eq), MeCN (1 mL) and EtOH (1 mL) at room temperature. The obtained mixture was stirred at room temperature for 24 hours, and the reaction was monitored by the liquid chromatography (LCMS). The obtained mixture was concentrated under a reduced pressure, and the residue was purified by the reverse flash chromatography under the following conditions to obtain the product: the column: C18, 120 g; mobile phase A: water/0.05% FA, mobile phase B: ACN; the flow rate: 100 mL/min; the gradient: from 5% B to 10% B in 3 minutes; from 10% B to 25% B in 0 minute; from 25% B to 80% B in 35 minutes; from 80% B to 95% B in 5 minutes; Detection: 254/220 nm. 44 batches were reacted in parallel to finally obtain the compound a-7 (1.68 g, 45%) as a light yellow solid.

LCMS24-compound a-7: (ESI, m/z): $[M+H]^+$ =972.20.

H-NMR30-compound a-7: $^1$H NMR (400 MHz, DMSO-$d_6$) δ 8.33 (t, J = 4.9 Hz, 1H), 7.98 (d, J = 7.9 Hz, 1H), 7.74

(d, J = 8.1 Hz, 1H), 7.65 - 7.59 (m, 2H), 7.54 (d, J = 8.7 Hz, 1H), 7.48 - 7.27 (m, 6H), 7.22 (s, 1H), 6.99 (d, J = 8.8 Hz, 1H), 5.06 - 4.99 (m, 3H), 4.98 (s, 2H), 3.87 (m, 3H), 3.71 (d, J = 3.1 Hz, 1H), 3.65 (m, 4H), 3.60 - 3.45 (m, 4H), 3.40 (m, 1H), 3.01 (t, J = 6.1 Hz, 3H), 2.03 (s, 3H), 1.89 (s, 3H), 1.61 (d, J = 12.2 Hz, 3H), 1.54 - 1.41 (m, 9H).

Example 2

a. Synthesis of the A13 derivative A13-c1 as a parent compound for the β-gal prodrug

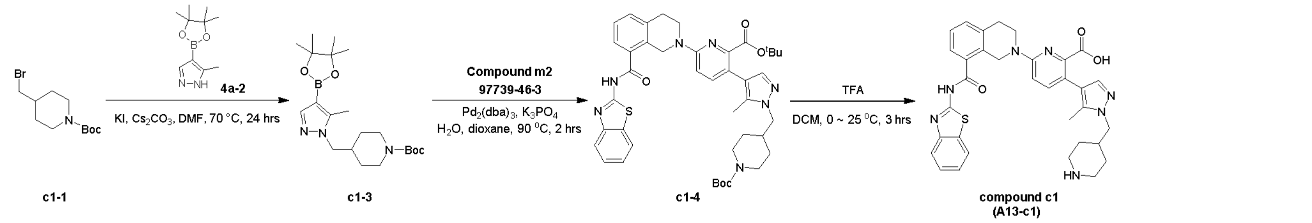


c1-1 c1-3 c1-4 compound c1 (A13-c1)

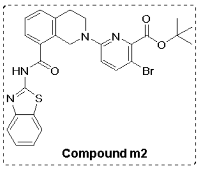

Compound m2

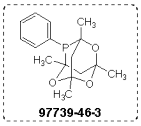

97739-46-3

1. Synthesis of c1-3

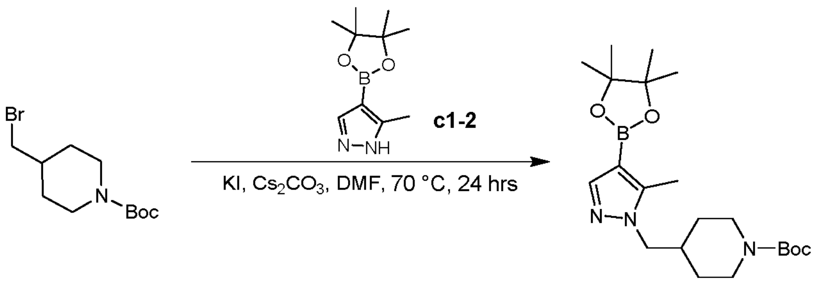


c1-1 c1-3

A solution of the compound c1-1 (300 mg, 1.08 mmol, 1.00 eq, dissolved in DMF solvent) was added to a solution of the compound c1-2 (145 mg, 700 umol, 0.65 eq), followed by KI (44.7 mg, 269 umol, 0.25 eq) and $Cs_2CO_3$ (446 mg, 1.37 mmol, 1.27 eq), and the mixture was stirred and reacted at 70°C for 24 hours. The c1-2 peak was detected by LC-MS at 0.366 minute and the c1-3 peak at 0.553 minute, a reaction recovery of the generated c1-3 was about 62%. The reaction was terminated by the addition of water (20 ml) at 20 °C, diluted using EA (10.0 ml), and extracted with EA (10 mL * 3). The resulting organic layer was washed with saline (20.0 mL * 1), dried with [$Na_2SO_4$], and the residual product was obtained by filtration and concentration under a low-pressure condition, and further purified using a pre-TLC chromatographic column ($SiO_2$, PE: EA = 0: 1, Rf [P1] = 0.79) to obtain the compound c1-3 as a colorless oil.

2. Synthesis of the compound c1-4

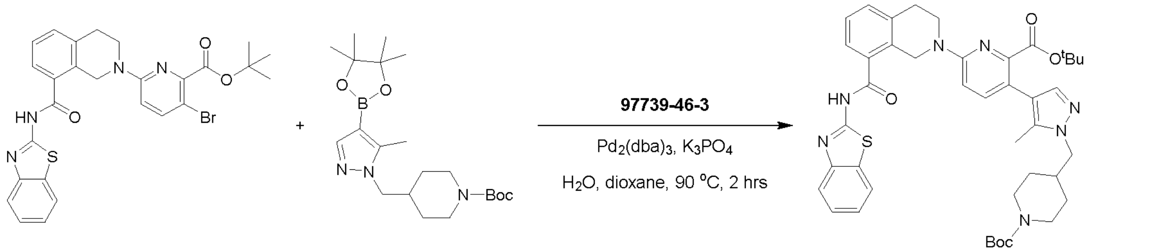


compound m2 c1-3 c1-4

The compound m2 (330 mg, 583 umol, 1.00 eq) and the compound c1-3 (591 mg, 1.46 mmol, 2.50 eq) were mixed, followed by the addition of $Pd_2(dba)_3$ (53.4 mg, 58.3 umol, 0.10 eq), 97739-46-3 (68.2 mg, 233 umol, 0.40 eq) and K3PO4 (433 mg, 2.04 mmol, 3.50 eq), plus dioxane (0.50 ml) and water (0.50 ml). ) and $K_3PO_4$ (433 mg, 2.04 mmol, 3.50 eq) using dioxane (0.50 ml) and water (0.50 ml) as the reaction solvent, and the reaction was stirred under a $N_2$ condition at 90°C for 2 hours. LC-MS detection showed that 71.6% of the final product was obtained (RT = 0.651 min, m/ z = 765.0 $[M+H]^+$). After termination of the reaction, the product was filtered, washed with methanol (20.0 mL), concentrated under a reduced pressure and further purified using the pre-HPLC (the chromatographic column: DAICEL CHIRALPAK IC (250 mm * 30 mm, 10 um); the mobile phase: [ACN/IPA (0.1% $NH_3H_2O$)]; B%: 40%-40%, A3.5. 35 min) to obtain the compound c1-4 (70.0 mg, 89.0 umol, 15.2% yield, 97.2% purity).

The compound c1-4 LCMS: RT = 0.651 min, $M+H^+$ = 765.0. HNMR: (400 MHz, CHLOROFORM-d) $\delta$ = 7.82 (d, J = 7.9 Hz, 1H), 7.52 (d, J = 7.6 Hz, 1H), 7.39 - 7.33 (m, 2H), 7.24 - 7.18 (m, 1H), 7.18 - 7.09 (m, 2H), 6.83 (d, J = 8.8 Hz, 1H), 5.02 (s, 2H), 4.11 (br d, J = 4.1 Hz, 1H), 4.07 - 3.97 (m, 4H), 3.91 (d, J = 7.1 Hz, 2H), 3.03 (br t, J = 5.7 Hz, 2H), 2.66 (br t, J = 11.7 Hz, 2H), 2.11 (s, 3H), 1.60 (br d, J = 11.8 Hz, 2H), 1.45 (s, 9H), 1.34 (s, 9H), 1.26 (br s, 2H), 0.91 - 0.80 (m, 1H).

3. Synthesis of the compound c1 (A13-c1)

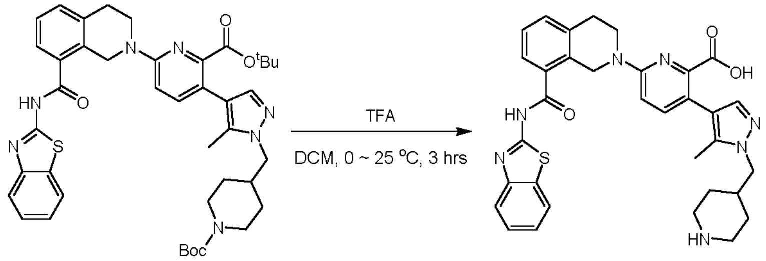


c1-4 **compound c1 (A13-c1)**

The compound c1-4 (70.0 mg, 91.6 umol, 1.00 eq) was dissolved in DCM (5.00 ml), TFA (1.00 ml) was added at 0°C, and the mixture was stirred and reacted at 25°C for 3 hours. LC-MS detected that the compound c1-4 fully reacted, and 95.1% of the product, c1, was generated (RT = 0.389 min, m/z = 608.5 $[M+H]^+$). The product was concentrated under a reduced pressure and purified by prep-HPLC (the TFA conditioned chromatographic column: Welch Xtimate C18 150*25mm*5um; the mobility: [water ($NH_4HCO_3$)-ACN]; B%: 15%-45%, 2 min) to obtain the product c1 (20.0 mg, 32.4 umol, 35.4% yield, 2 min). 35.4% yield, 98.6% purity, a light yellow solid).

The compound c1 LCMS: RT = 0.389 min, $M+H^+$ = 608.5. HNMR: (400 MHz, DMSO-$d_6$) $\delta$ = 8.01 (d, J = 7.8 Hz, 1H), 7.77 (d, J = 7.9 Hz, 1H), 7.60 (d, J = 6.6 Hz, 1H), 7.49 - 7.38 (m, 3H), 7.36 - 7.31 (m, 3H), 6.67 (d, J = 8.6 Hz, 1H), 4.90 (s, 2H), 3.89 - 3.83 (m, 4H), 2.98 (br d, J = 5.5 Hz, 4H), 2.16 (s, 3H), 2.00 (br d, J = 7.9 Hz, 2H), 1.49 (br d, J = 11.9 Hz, 2H), 1.30 (br d, J = 9.9 Hz, 2H), 0.85 (br t, J = 6.8 Hz, 1H).

b. Synthesis of the A13 derivative A13-c2 as a parent compound for the β-gal prodrug

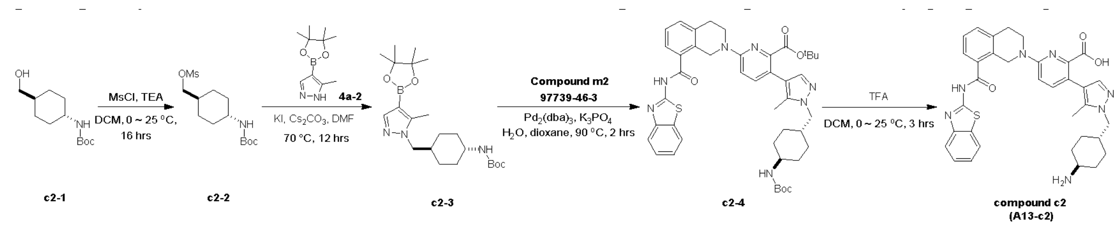


c2-1 c2-2 c2-3 c2-4 compound c2 (A13-c2)

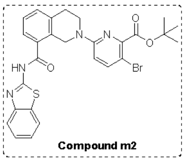

Compound m2

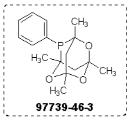

97739-46-3

1. Synthesis of the compound c2-2

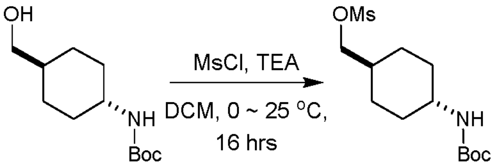


c2-1 c2-2

The compound c2-1 (800 mg, 3.49 mmol, 1.00 eq) was dissolved in DCM (8.00 mL) and MsCl (599 mg, 5.23 mmol, 405 uL, 1.50 eq) and TEA (564 mg, 5.58 mmol, 776 uL, 1.60 eq) were added at 0°C. The mixture was stirred and reacted at 25 °C for 16 hours. The TLC (PE: EA = 0: 1, Rf [c2-1] = 0.38, Rf [c2-2] = 0.81) method detected that the compound c2-1 fully reacted, and the product was generated. The reaction mixture was distributed in a 5.00% sodium bicarbonate solution (15.0 ml) and extracted using 45 ml dichloromethane (15.0 mL * 3) to obtain the organic phase, which was washed with saline (25.0 ml), dried with [$Na_2SO_4$] and concentrated by filtration to obtain the product c2-2 (1.5 g, a crude product), which was used directly in the next step of the reaction.

2. Synthesis of the compound c2-3

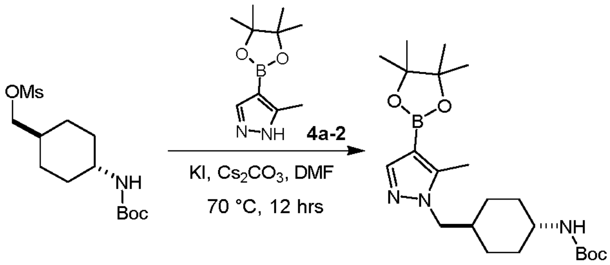


c2-2 c2-3

The compound c2-2 (1.00 g, 3.25 mmol, 1.00 eq) and the compound c1-2 (812 mg, 3.90 mmol, 1.20 eq) were mixed, followed by the addition of KI (135 mg, 813 umol, 0.25 eq), $Cs_2CO_3$ (2.65 g, 8.13 mmol, 2.50 eq), and dissolved in DMF ( 10.0 mL). The mixture was stirred and reacted under a $N_2$ condition at 70°C for 12 hours. LCMS detected that c2-2 fully reacted and 34.2% of the product c2-3 was generated (RT = 0.647 min, m/ z = 420.9 [M+H]$^+$). The reaction mixture was diluted with water (20.0 mL), extracted with ethyl acetate (20.0 mL * 3) to obtain the organic layer, which was washed with saline (20 mL * 1), dried with [$Na_2SO_4$], concentrated by filtration, and purified by the inverse HPLC (0.1% FA condition) to obtain the product c2-3 (300 mg, 370 umol, 11.3% yield, 51.8% purity).

3. Synthesis of the compound c2-4

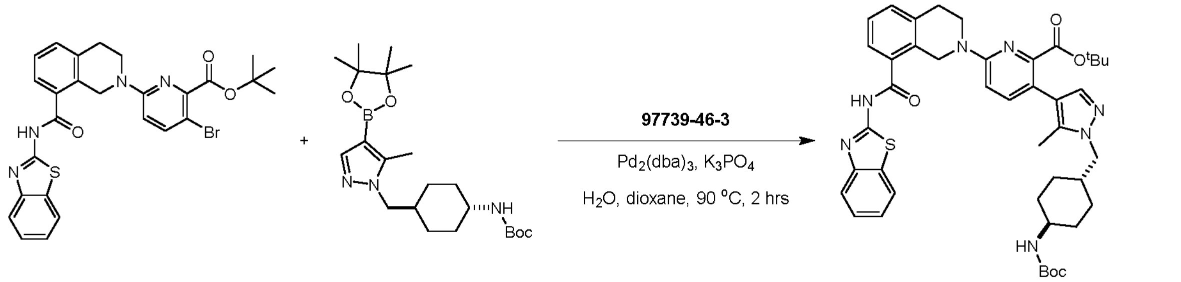


Compound m2 c2-3 c2-4

The compound c2-3 (278 mg, 663 umol, 2.50 eq) and the compound m2 (150 mg, 265 umol, 1.00 eq) were dissolved in dioxane (5.00 ml) and water (5.00 ml), followed by the addition of 97739-46-3 (31.0 mg, 106 umol, 0.40 eq), $Pd_2(dba)_3$ ( 24.2 mg, 26.5 umol, 0.10 eq) and $K_3PO_4$ (225 mg, 1.06 mmol, 4.00 eq). The resulting mixture was stirred and reacted at 90°C for 2 hours. LCMS detected that the compound m2 fully reacted and 48.9% of the product c2-4 was generated (RT = 0.588 min, m/z = 778.5 [M+H]$^+$) . The reaction mixture was filtered, washed with methanol (20.0 ml), concentrated under a reduced pressure and purified using the inverse HPLC (0.1% FA condition) to obtain the yellow solid product c2-4 (130 mg, 165 umol, 62.3% yield, 99.0% purity).

4. Synthetic of the compound c2 (A13-c2)

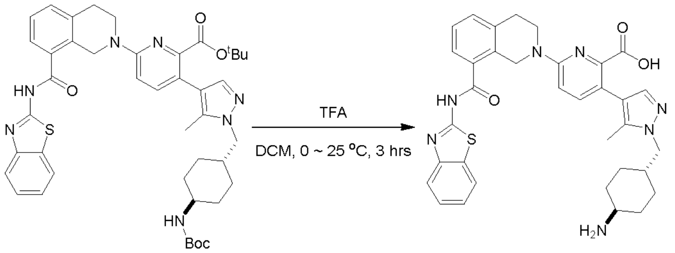


c2-4 compound c2 (A13-c2)

The compound c2-4 (130 mg, 167 umol, 1.00 eq) was dissolved in DCM (5.00 ml), TFA (1.00 ml) was added at 0°C, and the mixture reacted at 25°C for 10 hours. LCMS detected that the compound c2-4 fully reacted, and about 96.7% of the product c2 was generated (RT = 0.389 min, m/z = 622.4 [M+H]$^+$). The product was concentrated under a reduced pressure and purified by the SFC method (the condition: the chromatographic column: DAICEL CHIRALCEL OX (250 mm*30 mm, 10 um); the mobile phase: [ACN/MeOH (0.1% $NH_3H_2O$)]; B%: 65%-65%, A5; 50 min) to obtain the product c2 (24.0 mg, 35.4 umol. 21.2% yield, 91.8% purity) in a yellow-green solid form.

The compound c2 LCMS: RT = 0.389 min, M+H$^+$ = 622.4; SFC: EC7305-41-P1A2, RT = 1.950 min; HNMR: (400 MHz, METHANOL-$d_4$) $\delta$ = 7.91 (d, J = 7.9 Hz, 1H), 7.77 (d, J = 7.9 Hz, 1H), 7.60 (d, J = 7.4 Hz, 1H), 7.52 (d, J = 8.8 Hz, 1H), 7.44 (d, J = 8.0 Hz, 2H), 7.40 - 7.32 (m, 3H), 7.05 (d, J = 8.9 Hz, 1H), 5.06 (s, 2H), 4.00 - 3.93 (m, 4H), 3.09 (s, 1H), 3.16 - 3.01 (m, 1H), 3.02 (s, 1H), 3.14 - 2.98 (m, 1H), 2.21 (s, 1H), 2.13 (s, 3H), 2.05 (d, J = 11.1 Hz, 2H), 1.75 (d, J = 11.6 Hz, 2H), 1.25 - 1.10 (m, 3H), 0.94 - 0.83 (m, 2H)

c. Synthesis of the compound m1 as a β-gal modified intermediate compound

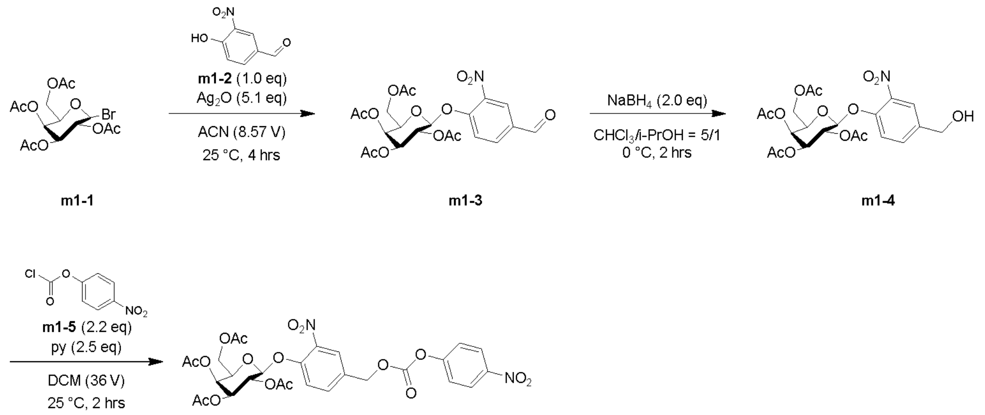


m1-1 m1-3 m1-4

Compound m1

1. Synthesis of the compound m1-3

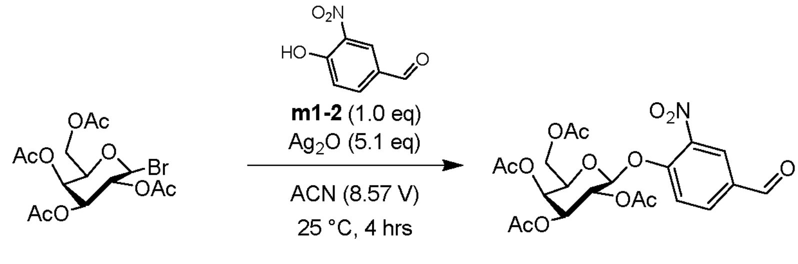


m1-1 m1-3

The compound m1-1 (20.0 g, 48.6 mmol) and $Ag_2O$ (57.4 g, 248 mmol) were dissolved in ACN (171 mL), to which the compound m1-2 (8.13 g, 48.6 mmol) dissolved in ACN (162 mL) was added. The mixture was stirred and reacted at 25°Cfor 4 hours. TLC (petroleum ether/ethyl acetate = 1/1, $R_f$ of the compound m1-3 = 0.20) detected that the compound m1-1 ($R_f$ = 0.80) was completely consumed and the compound m1-3 was generated. The reaction mixture was concentrated by filtration to obtain the crude product, which was purified by the column chromatography (petroleum ether/ethyl acetate = 1000/1 to 1/1) to obtain the compound m1-3 (16.0 g, 63.9% yield, 96.3% purity) as a white solid.

The compound m1-3 $^1$H NMR: (400 MHz, $CDCl_3$) δ 9.96 (s, 1H), 8.29-8.07 (m, 1H), 8.06-8.05 (m, 1H), 7.49-7.47 (m, 1H), 5.57-5.47 (m, 2H), 5.22-5.11 (m, 2H), 4.24-4.14 (m, 3H), 2.18-1.98 (m, 12H)

2. Synthesis of the compound m1-4

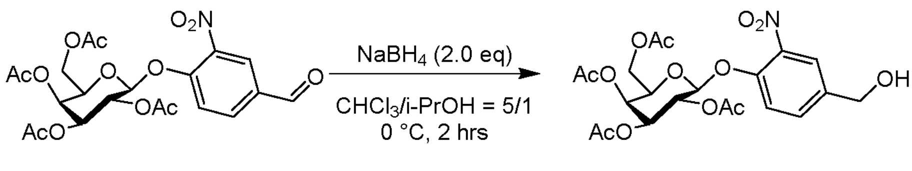


m1-3 m1-4

$NaBH_4$ (750 mg, 19.8 mmol) was added to a solution of the compound m1-3 (11.0 g, 22.1 mmol) dissolved in $CHCl_3$ (77.0 mL) and i-PrOH (25.6 mL) at 0°C, and the mixture was stirred and reacted at 0°C for 2 hours. TLC (petroleum ether/ethyl acetate = 1/1, $R_f$ of the compound m1-4 = 0.10) detected the compound m1-3 was completely consumed and the compound m1-4 was generated. The reaction was terminated by the addition of the saturated $NHCl_4$ solution (30.0 mL) at 0°C. The organic phase was washed using the saturated saline (30.0 ml), extracted using the ethyl acetate solution (90.0 mL), dried with $Na_2SO_4$ and concentrated by filtration to obtain the crude product. The crude product was purified by the

column chromatography ($SiO_2$, petroleum ether/ethyl acetate = 1000/1 to 1/1) to obtain the compound m1-4 (7.00 g, 54.3% yield, 85.3% purity) as a white solid.

The compound m1-4 $^1H$ NMR: (400 MHz, $CDCl_3$) $\delta$ 7.79-7.52 (m, 1H), 7.51-7.50 (m, 1H), 7.49-4.32 (m, 1H), 5.52-5.50 (m, 2H), 5.46-5.08 (m, 2H), 5.07-4.71 (m, 2H), 4.24-4.00 (m, 3H), 2.18-2.00 (m, 12H)

3. Synthesis of the intermediate compound m1

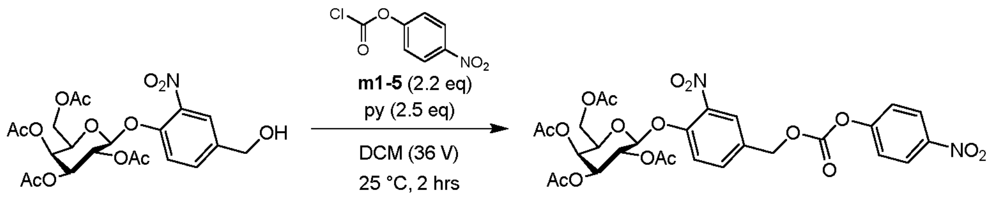


m1-4

Compound m1

The compound m1-5 (3.55 g, 17.6 mmol) and Py (1.58 g, 20.0 mmol, 1.62 mL) were added to a solution of the compound m1-4 (4.00 g, 8.01 mmol) dissolved in DCM (144 mL), and the mixture was stirred and reacted at 25°C for 2 hours. TLC (petroleum ether/ethyl acetate = 1/1, $R_f$ of the compound m1 = 0.20) detected that the compound m1-4 ($R_f$ = 0.30) was completely consumed and the compound m1 was generated. The reaction was terminated by the addition of $H_2O$ (20.0 ml). The resulting organic phase was washed with the saturated saline (20.0 ml), extracted with DCM (90.0 ml), dried with $Na_2SO_4$ and concentrated by filtration to obtain the crude product, which was purified by the column chromatography ($SiO_2$, petroleum ether/ethyl acetate = 1000/1 to 1/1) to obtain the compound m1 (3.68 g, 55.7% yield, 80.6% purity) as a white solid.

The compound m1 $^1H$ NMR: (400 MHz, $CDCl_3$) $\delta$ 8.28-8.26 (m, 2H), 7.89-7.61 (m, 1H), 7.59-7.58 (m, 1H), 7.40-7.36 (m, 3H), 5.54-5.52 (m, 2H), 5.47-5.09 (m, 2H), 4.25-4.13 (m, 3H), 2.18-2.00 (m, 12H)

Example 3

Synthesis of the prodrug A13bG-b1 obtained by the β-gal modification of A13

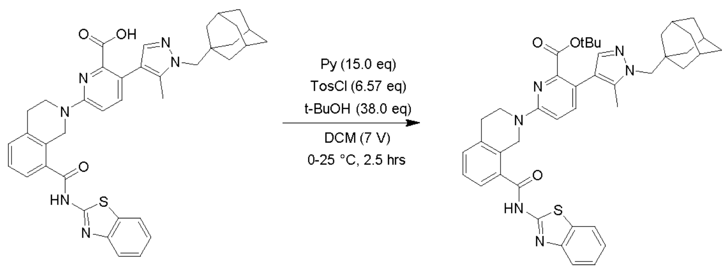


A-1331852

b-1

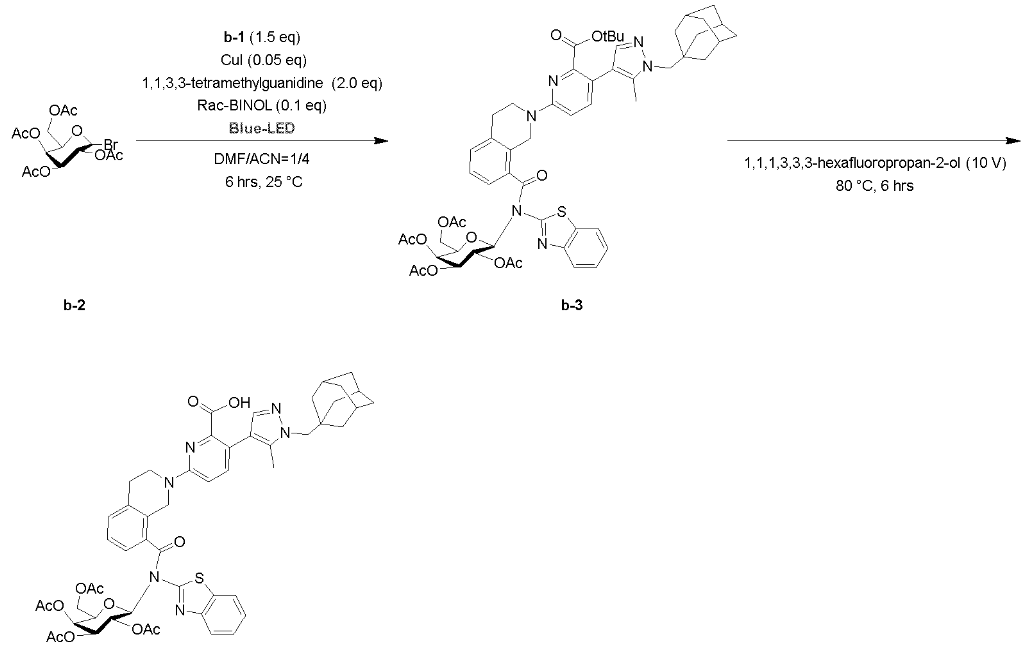


b-2

b-3

b-4 (A13bGb1)

1. Synthesis of the compound b-1

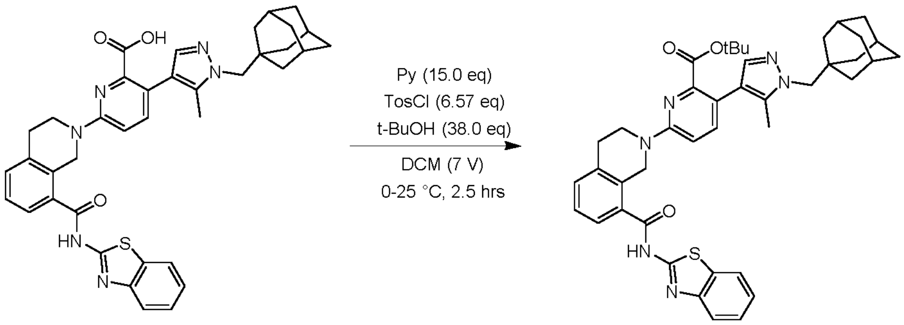


A-1331852

b-1

TosCl (95.0 mg, 498 μmol) was added to a solution of the compound A-1331852 (50 mg, 75.8 μmol), Py (90.0 mg, 1.14 mmol) dissolved in DCM (0.35 ml) and BuOH (213 mg, 2.88 mmol) at 0°C. The mixture was stirred at 0°Cfor 30 minutes, followed by stirring at 25°C for 2 hours. The TLC (petroleum ether/ethyl acetate = 1/1, the compound b-1, $R_f$ = 0.60) detection showed that the compound A-1331852 ($R_f$ = 0.20) fully reacted, and a new compound was generated. The reaction mixture was terminated by the addition of the saturated $NaHCO_3$ (5.00 mL) at 0°C. The resulting organic layer was washed with the saturated saline (5.00 mL), extracted using ethyl acetate (15.0 ml), dried with $Na_2SO_4$, filtered and concentrated under a low pressure to obtain the residual product, which was further purified using the pre-TLC chromatographic column ($SiO_2$, petroleum ether/ethyl acetate = 1:1) to obtain the compound b-1 (6.00 mg, 9.33% yield, 84.4% purity) as a yellow oil.

2. Synthesis of the compound b-3

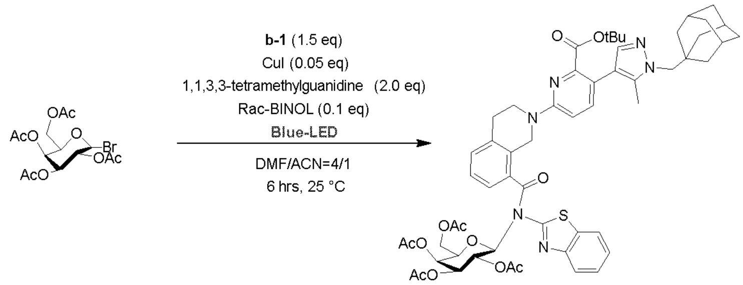


b-2 b-3

1, 1, 3, 3-tetramethylguanidine (966 μg, 8.39 μmol, 1.06 μL), Rac-BINOL (240 μg, 0.839 μmol) and CuI (79.9 μg, 0.420 μmol) were added to a solution of the compounds b-2 (17.2 mg, 41.9 μmol), b-1 (6.00 mg, 8.39 μmol) dissolved in DMF (0.144 ml) and MeCN (0.036 mL) was added. The mixture reacted at 25 °Cunder the irradiation environment of a 450 nm blue-LED lamp for 6 hours. LCMS showed that about 11.4% of the compound b-2 remained, multiple new peaks were generated, and about 7.0% of the compound b-3 was detected. The above reaction was carried out in 6 parallels and combined together for the subsequent isolation and purification after the completion of the reaction. The post-reaction suspension was filtered through the silica gel and washed using ethyl acetate (10.0 ml, 3 times). The residue was purified by the prep-HPLC (the chromatographic column: Welch Ultimate XB CN 10u100 x 30 mm; the mobile phase: [Heptane-EtOH]; the gradient elution: 5%-95% B, 10 minutes) to obtain the compound b-3 (1.00 mg, 5.00% yield) as a white solid.

$^1$H NMR: ET67304-79-P1A1 (400 MHz, $CDCl_3$) δ 8.27-8.25 (m, 1H), 7.99 (d, J = 8.4 Hz, 1H),7.68 (d, J = 8.0 Hz, 1H), 7.44-7.40 (m, 1H), 7.38-7.36 (m, 1H), 6.93-6.89 (m, 2H), 5.97-5.92 (m, 1H), 5.66 (d, J = 3.2 Hz, 1H), 5.43-5.39 (m, 1H), 5.26-5.10 (m, 1H), 4.32-4.30 (m, 2H), 4.21-4.17 (m, 1H), 4.13-4.10 (m, 2H), 3.73 (s, 2H), 3.09-3.05 (m, 2H), 2.35 (s, 3H), 2.14 (s, 3H), 2.05 (s, 3H), 2.03 (s, 3H), 1.77 (s, 3H), 1.72-1.69 (m, 4H), 1.63-1.57 (m, 12H), 1.40 (s, 9H).

3. Synthesis of the compound b-4 (*i.e.*, A13bG-b1)

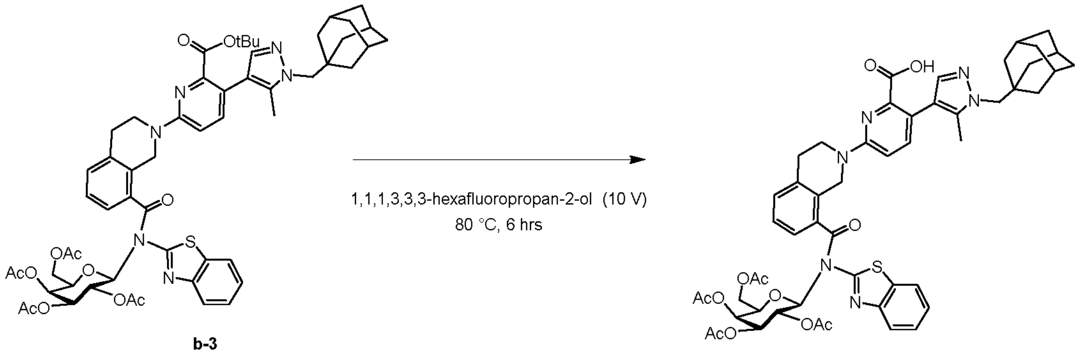


b-3 b-4 (A13bG-b1)

The compound b-3 (800 μg, 0.765 μmol) was added to 1,1,1,3,3,3-hexafluoride-2-propanol (0.800 mL), and the reaction was carried out at 80°C for 6 hours. LCMS (the product RT = 0.772 minutes) showed that the reaction was complete. The reaction product was concentrated under vacuum to obtain the compound b-4 (about 1.00 mg) as a yellow solid.

$^1$H NMR: ET55368-152-P1A1 (400 MHz, $CDCl_3$) δ 8.27-8.25 (m, 1H), 7.99 (d, J = 8.4 Hz, 1H), 7.68 (d, J = 8.0 Hz, 1H), 7.44-7.40 (m, 1H), 7.38-7.36 (m, 1H), 6.93-6.89 (m, 2H), 5.97-5.92 (m, 1H), 5.66 (d, J = 3.2 Hz, 1H), 5.43-5.39 (m, 1H), 5.26-5.10 (m, 1H), 4.32-4.30 (m, 2H), 4.21-4.17 (m, 1H), 4.13-4.10 (m, 2H), 3.73 (s, 2H), 3.09-3.05 (m, 2H), 2.35 (s, 3H), 2.14 (s, 3H), 2.05 (s, 3H), 2.03 (s, 3H), 1.77 (s, 3H), 1.72-1.69 (m, 4H), 1.63-1.57 (m, 12H).

Example 4

Synthesis of the prodrugs A13bG-c1 and A13bG-c2 obtained by the β-gal modification of A13

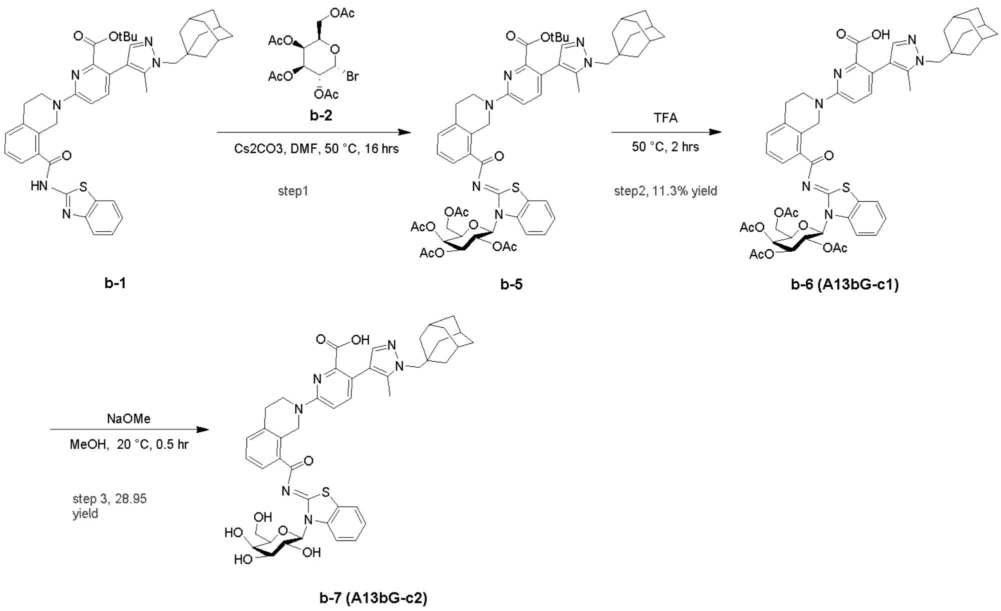


b-1 b-5 b-6 (A13bG-c1)

b-7 (A13bG-c2)

1. Synthesis of the compound b-5

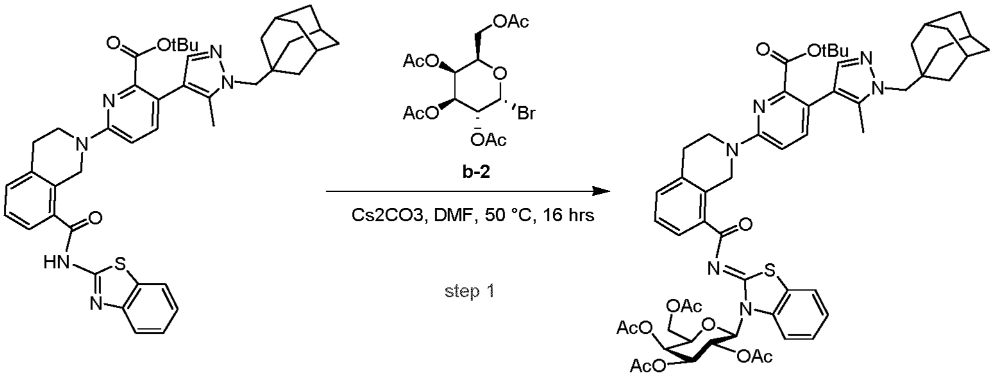


b-1 b-5

The compounds b-1 (200 mg, 279μmol, 1.00 eq), b-2 (460 mg, 1.12 mmol, 4.00 eq) and $Cs_2CO_3$ (319 mg, 979μmol, 3.50 eq) were mixed in DMF (4.00 mL), and stirred and reacted at 50°C for 16 hours. The LCMS (EC10054-196-p1, p1, RT = 2.182 min) detection showed that about 95.9% of the target product was generated. After the reaction, the mixture was filtrated and concentrated under a reduced pressure to obtain the crude product, which was purified by the flash column chromatography and detected by TLC (petroleum ether: ethyl acetate = 1:1, $R_f$ = 0.65) to obtain the compound b-5 (550 mg) in the form of a yellow gel.

LCMS: EC10054-196-p1, P1RT = 2.182 min, MS (ESI) m/z = 1045.7 $[M+H]^+$

2. Synthesis of the compound b-6, *i.e.*, A13bG-c1

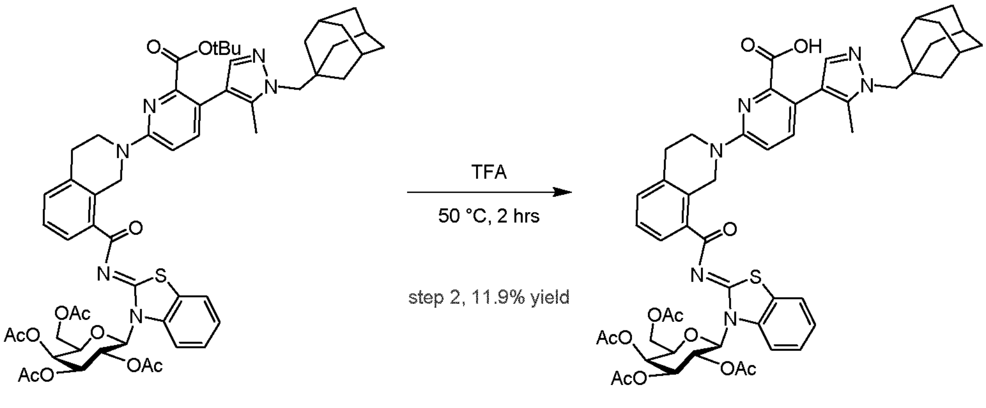


b-5 b-6 (A13bG-c1)

The compound b-5 (380 mg, 363μmol, 1.00 eq) was added to TFA (4.00 mL), and the mixture was stirred and reacted at 50°C for 2 hours. The LC-MS (EC10054-209-p1, P1, RT = 0.600 min) detection showed that about 87.2% of the target product was generated. The mixture was filtrated and concentrated under a reduced pressure to obtain the crude product b-6 (50.0 mg, 43.5μmol, 11.9% yield, 86.0%) as a white solid.

LCMS: EC10054-209-p1, P1 RT = 0.600 min, MS (ESI) m/z = 989.3 $[M+H]^+$

3. Synthesize of the compound b-7, *i.e.*, A13bG-c2

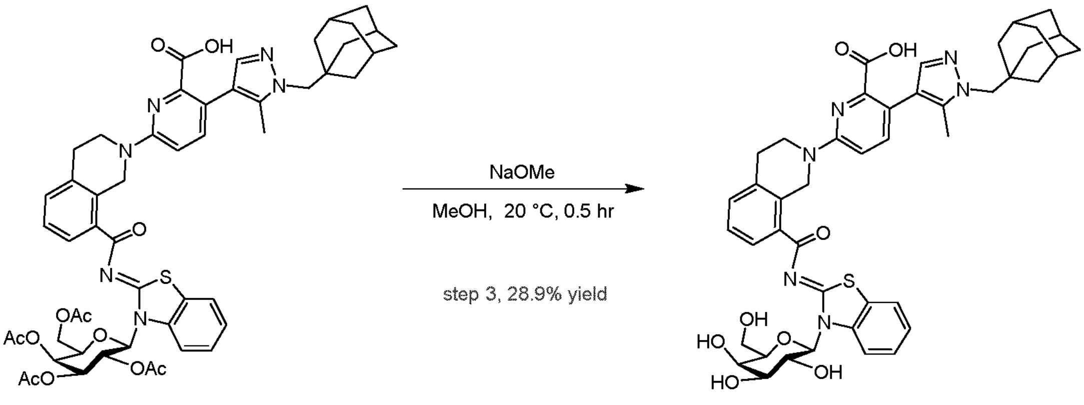


b-6 b-7 (A13bG-c2)

NaOMe (9.39 mg, 174 μmol, 4.00 eq) was added to a mixed solution of the compound b-6 (50.0 mg, 43.5 μmol, 1.00 eq) dissolved in methanol (3.00 mL), and the mixture was stirred and reacted at 25°C for 0.5 hour. The LC-MS (EC10054-215-p1, P1, RT = 0.488 min) detection showed that about 86.3% of the target product was generated. After the reaction, the mixture was filtrated and concentrated under a reduced pressure to obtain the crude product, which was further purified by the prep-HPLC (a neutral condition) to obtain the compound b-7 (11.1 mg, 12.6μmol, 28.9% yield, 94.0% purity) as yellow solid.

LCMS: EC10054-215-p1, P1RT = 0.488 min, MS (ESI) m/z = 821.5 $[M+H]^+$

$^1$HNMR: EC10054-215--p1, 400 MHz, DMSO δ ppm 8.08 - 8.22 (m, 2H), 7.93 (d, J = 8.0 Hz, 1H), 7.48 - 7.57 (m, 2H),. 7.41 (m, 3 H), 7.30 (s, 1H), 6.95 (d, J = 8.4 Hz, 1H), 6.47 (d, J = 9.2 Hz, 1H), 5.31 - 5.39 (m, 1H), 5.17 (s, 2H), 5.12 (d, J = 3.2 Hz, 1H), 4.64 - 4.82 (m, 1H), 4.34 (t, J = 8.8 Hz, 1H), 3.91 (s, 1H), 3.86 (s, 1H), 3.70 (s, 2H), 3.59 (s, 3H), 3.00 (t, J = 5.6 Hz, 2H), 2.12 (s, 3H), 1.93 (s, 3H), 1.63 (s, 3 H), 1.57 (s, 3H), 1.53 (s, 9H)

Example 5

Synthesis of the β-gal modified prodrugs A13bGa1-lyso and A13bGa2-lyso with teh lysosome directed unit

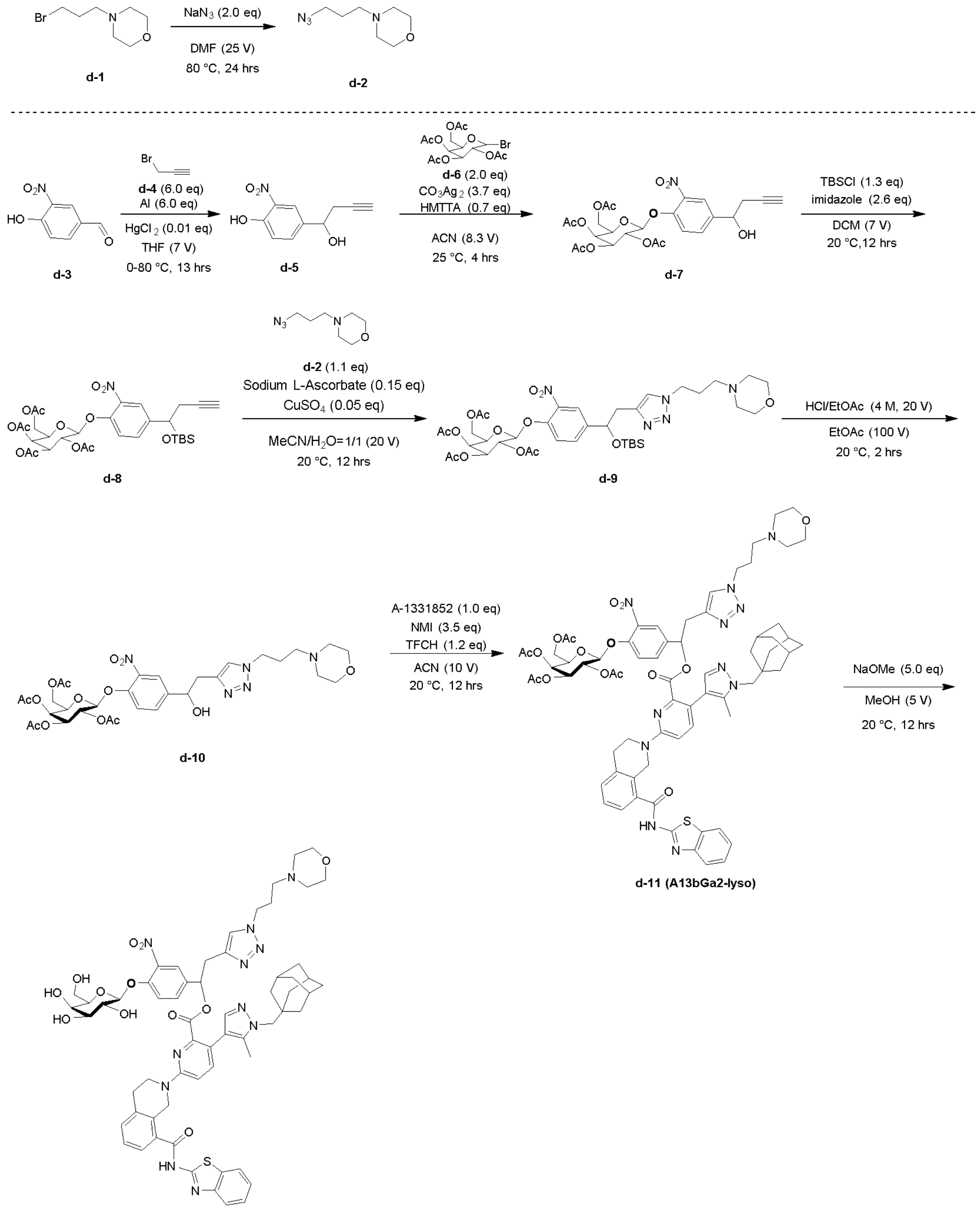


d-1 d-2 d-3 d-5 d-7 d-8 d-9 d-10 d-11 (A13bGa2-lyso) d-12 (A13bGa1-lyso)

1. Synthesis of the compound d-2

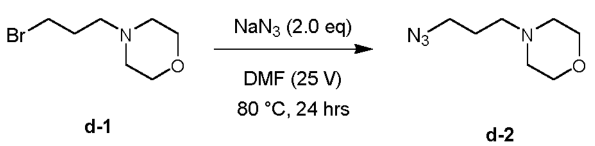


d-1 d-2

$NaN_3$ (4.47 g, 68.7 mmol) was added to a solution of the compound d-1 (7.50 g, 36.0 mmol) in DMF, and the mixture was stirred and reacted at 80°C for 24 hours. The TLC (Petroleum ether/Ethyl acetate = 0/1, $R_f$ of the product = 0.30) detection showed that the reaction of the compound d-1 ($R_f$ = 0.20) was complete, and the new product was formed. The reaction mixture was terminated by the addition of water (100 ml) at 0°C, extracted with ethyl acetate (100 ml, 3 times),

the resulting organic layer was washed with the saturated saline (100 ml, 3 times), dried with $Na_2SO_4$, and the residual solid was obtained by filtration and concentration under a reduced pressure. The crude product was further purified to obtain the compound d-2 (2.50 g, 40.7% yield) as a yellow oil.

$^1H$ NMR: ET65547-12-P1A1 (400 MHz, $CDCl_3$) δ 3.71-3.69 (m, 4H), 3.36-3.33 (m, 2H), 2.43-2.39 (m, 6H), 1.79-1.72 (m, 2H).

2. Synthesis of the compound d-5

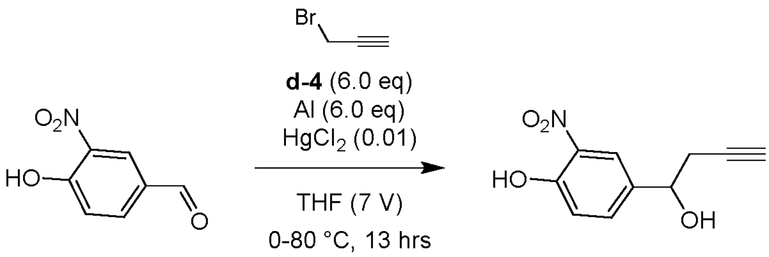


d-3 d-5

Al (10.0 g, 373 mmol, 3.74 mL) and $HgCl_2$ (162 mg, 598 μmol) were added dropwise to a solution of the compound d-4 (55.6 g, 373 mmol) dissolved in THF solvent (70.0 mL) at 0°C, and then the mixture was stirred at 80°C for 13 hours. After cooling to 0°C, the compound d-3 (10.0 g, 59.8 mmol) was added dropwise to the above mixture at 0°C, and the resulting mixture was stirred and reacted at 0°C for 5 hours. The TLC monitoring (petroleum ether/ethyl acetate = 5/1) showed that the reaction of the compound d-3 was complete, and the new product with a major peak ($R_f$ = 0.5) was generated. The reaction mixture was terminated by the addition of 1 N HCl (50.0 ml) at 0°C, and extracted using ethyl acetate (50.0 ml, 3 times). The resulting organic layer was washed with the saturated saline (50.0 ml, 3 times), dried with $Na_2SO_4$, filtered and concentrated under a reduced pressure to obtain the residue. The resulting crude product was purified by the column chromatography ($SiO_2$, petroleum ether/ethyl acetate = 50/1 to 0/1) to obtain the compound d-5 as a black oil.

3. Synthesis of the compound d-7

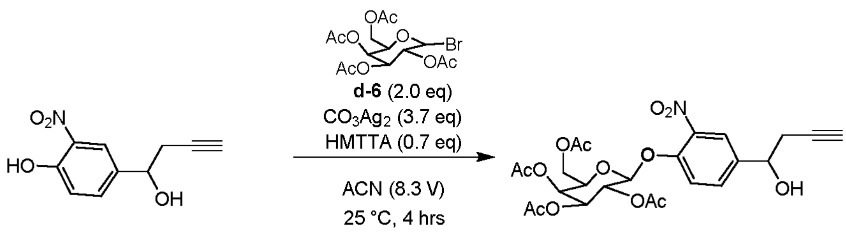


d-5 d-7

$CO_3Ag_2$ (14.8 g, 53.6 mmol) and HMTTA (2.34 g, 10.1 mmol) were added to a solution of the compound d-5 dissolved in ACN (25.0 mL), and then the compound d-6 (11.9 g, 28.9 mmol) was added to the mixture, stirred and reacted at 25°C for 4 hours. The reaction mixture was terminated by the addition of 1N HCl (5.00 ml) at 0°C, and extracted using ethyl acetate (10.0 ml, 3 times). The resulting organic layer was washed with the saturated saline (10.0 ml, 3 times), dried with $Na_2SO_4$, filtered and concentrated under vacuum. The resulting crude product was purified by the column chromatography ($SiO_2$, petroleum ether/ethyl acetate=20/1 to 1/1) to obtain the compound d-7 (1.10 g, 14.3% yield) as a yellow solid.

4. Synthesis of the compound d-8

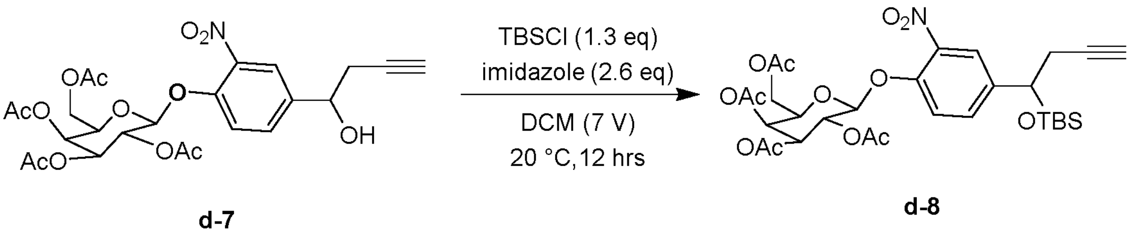


d-7

d-8

TBSCl (182 mg, 1.21 mmol) and IMIDAZOLE (165 mg, 2.42 mmol) were added to a solution of the compound d-7 (500 mg, 930 μmol) dissolved in DCM (3.50 ml), and the mixture was stirred and reacted at 20°C for 12 hours. The TLC monitoring (petroleum ether/ethyl acetate = 3/1, $R_f$ of the compound d-8 = 0.5) showed that the reaction was complete. The product was extracted using DCM (20.0 ml, 3 times), and the resulting organic layer was washed with the saturated saline (10.0 ml), dried with $Na_2SO_4$, filtered and concentrated under vacuum. The resulting crude product was purified by the column chromatography ($SiO_2$, petroleum ether/ethyl acetate = 50/1 to 2/1) to obtain the compound d-8 (500 mg, 77.5% yield) as a yellow oil.

$^1H$ NMR: ET67304-34-P1A1 (400 MHz, $CDCl_3$) δ 7.83 (dd, J = 15.6, 2.0 Hz, 1 H), 7.51-7.58 (m, 1 H), 7.32 (dd, J = 8.4, 6.6 Hz, 1 H), 5.53-5.58 (m, 1 H), 5.48 (d, J = 2.8 Hz, 1 H), 5.08-5.13 (m, 2 H), 4.83 (t, J=6.2 Hz, 1 H), 4.23-4.32 (m, 1 H), 4.05 - 4.21 (m, 3 H), 2.56-2.65 (m, 1 H), 2.41 - 2.52 (m, 1 H), 2.20 (s, 3 H), 2.15 (s, 3 H), 2.08 (d, J = 2.0 Hz, 3 H), 2.02 (s, 3 H), m0.90 (d, J = 2.0 Hz, 11 H), 0.11 (d, J = 1.6 Hz, 3 H), -0.03 (d, J = 2.4 Hz, 3 H).

5. Synthesis of the compound d-9

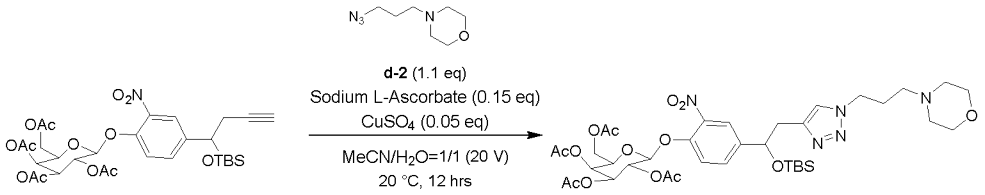


d-8

d-9

The compound d-2 (144 mg, 844 μmol), $CuSO_4$ (6.12 mg, 38.4 μmol), and sodium L-ascorbate (22.8 mg, 115 μmol) were added to a solution of the compound d-8 (500 mg, 767 μmol) dissolved in water (5.00 mL) and ACN (5.00 mL) solvent, and the mixture was stirred and reacted at 20°C for 12 hours. The reaction mixture was extracted using ethyl acetate (20.0 ml, 3 times), and the resulting organic layer was washed with the saturated saline (5.00 ml, 3 times), dried with $Na_2SO_4$, filtered and concentrated under vacuum. The obtained crude product was purified by the column chromatography ($SiO_2$, petroleum ether/ethyl acetate = 20/1 to 0/1, petroleum ether/ethyl acetate = 0/1, $R_f$ of the compound d-9 = 0.20) to obtain the compound d-9 (550 mg, 65.4% yield, 75% purity) as a white solid.

6. Synthesis of the compound d-10

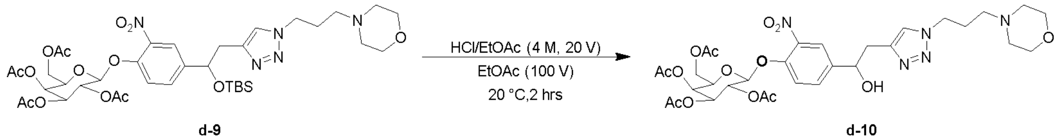


d-9

d-10

HCl/EtOAc (4 M, 11.0 ml) was added to a solution of the compound d-9 (550 mg, 669 μmol) dissolved in ethyl acetate (55.0 ml) at 20°C, stirred and reacted at 20°C for 2 hours. The reaction mixture was concentrated under vacuum to obtain the crude product, which was further purified by prep-TLC ($SiO_2$, DCM/MeOH = 10/1, $R_f$ of the compound d-10 = 0.45) to obtain the compound d-10 (110 mg, 23.9% yield) as a yellow oil.

$^1H$ NMR: ET67304-60-P1A1 (400 MHz, $CDCl_3$) δ 7.61-7.78 (m, 1 H), 7.47-7.53 (m, 1 H), 7.23-7.38 (m, 2 H), 5.35-5.52 (m, 2 H), 4.95-5.12 (m, 3 H), 4.30-4.45 (m, 2 H), 3.90-4.28 (m, 5 H), 3.69 (s, 4 H), 3.02-3.12 (m, 1 H), 2.89-3.01 (m, 1 H), 2.24-2.62 (m, 6 H), 2.12 (s, 3 H), 2.06 (s, 4 H), 2.01 (s, 3 H), 1.95 (s, 3 H).

7. Synthesis of the compound d-11 (*i.e.*, A13bGa2-lyso)

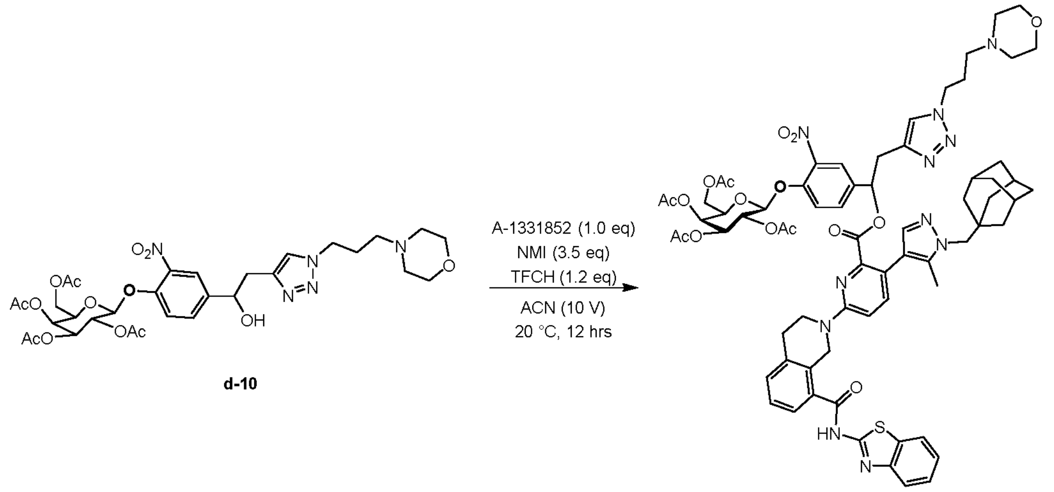


d-10

d-11 (A13bGa2-lyso)

A-1331852 (2.00 mg, 3.04 μmol), NMI (872 μg, 10.6 μmol), and TCFH (1.02 mg, 3.64 μmol) were added to a solution of the compound d-10 (2.79 mg, 3.95 μmol) dissolved in ACN (0.28 ml) at 20°C, and the mixture reacted at 20°C for 12 hours. The reaction mixture was extracted using ethyl acetate (20.0 ml, 3 times). The organic layer was washed with the saturated saline (5.00 ml, 3 times), dried with $Na_2SO_4$, filtered and concentrated under vacuum. The crude product was purified by prep-HPLC (the chromatographic column: Waters Xbridge BEH C18 100 x 30 mm x 10 μm; the mobile phase: [$H_2O$ (10 mM $NH_4HCO_3$)-ACN]; the gradient elution: 35%-65% B, 8.0 minutes) to obtain the compound d-11 (2.2 mg, 52.1% yield, 97% purity) as a white solid.

$^1H$ NMR: ET67304-65-P1B1 (400 MHz, $CDCl_3$) δ 7.80 (s, 1 H), 7.54-7.68 (m, 3 H), 7.26-7.46 (m, 7 H), 7.15-7.22 (m, 2 H), 6.90 (d, J = 8.8 Hz, 1 H), 5.94-6.11 (m, 1 H), 5.41-5.53 (m, 2 H), 5.14-5.28 (m, 1 H), 4.89-5.10 (m, 2 H), 4.53 (d, J = 1.2 Hz, 1 H), 3.98-4.28 (m, 5 H), 3.83-3.97 (m, 3 H), 3.54-3.66 (m, 3 H), 3.04-3.23 (m, 4 H), 2.16 (s, 3 H), 2.10 (d, J = 3.6 Hz, 3 H), 2.04 (d, J = 4.4 Hz, 3 H), 1.99 (s, 4 H), 1.90-1.98 (m, 8 H), 1.62-1.70 (m, 5 H), 1.42-1.51 (m, 10 H), 1.36 (s, 2 H), 1.23 (s, 4 H).

8. Synthesis of the compound d-12 (*i.e.*, A13bGa1-lyso)

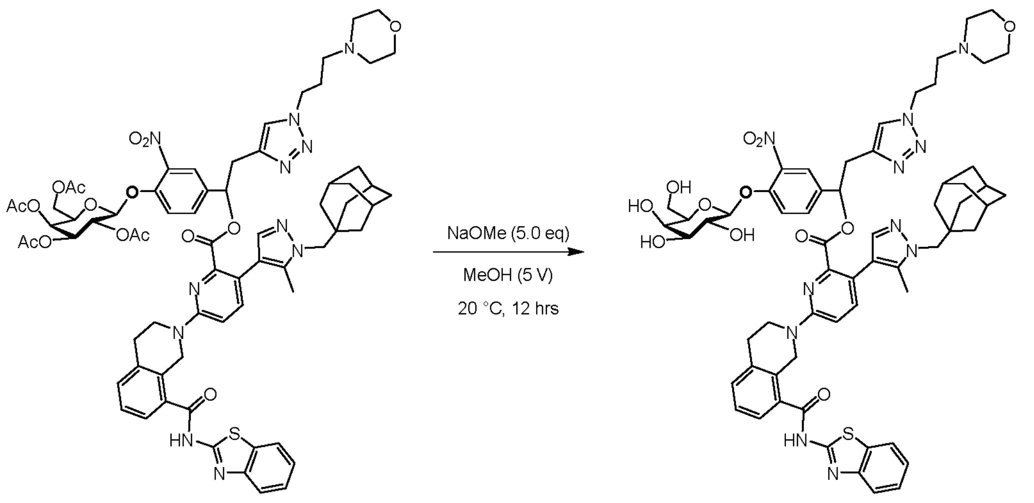


d-11 (A13bGa2-lyso)

d-12 (A13bGa1-lyso)

NaOMe (4.01 mg, 74.2 μmol) was added to a solution of the compound d-11 (20.0 mg, 14.8 μmol) dissolved in

methanol (0.10 ml) at 20°C, and the mixture was stirred and reacted at 20°C for 12 hours. The reaction mixture was concentrated under vacuum, and the resulting crude product was purified using prep-HPLC (the chromatographic column: Waters Xbridge BEH C18 100 x 30 mm x 10 μm; the mobile phase: [$H_2O$ (10 mM $NH_4HCO_3$)-ACN]; the gradient elution: 50%-80% B, 8.0 minutes) to obtain the compound d-12 (6.20 mg, 34.3% yield, 97% purity) as a white solid.

1H NMR: ET67304-73-P1B1 (400 MHz, $CDCl_3$) δ 7.72-7.82 (m, 1 H), 7.55-7.71 (m, 3 H), 7.29-7.45 (m, 5 H), 7.14-7.24 (m, 4 H), 6.91 (d, J = 8.8 Hz, 1 H), 5.97-6.08 (m, 1 H), 5.05-5.40 (m, 2 H), 4.74-4.93 (m, 1 H), 4.22-4.45 (m, 2 H), 3.80-4.16 (m, 7 H), 3.59-3.72 (m, 7 H), 3.16-3.47 (m, 4 H), 2.99-3.13 (m, 3 H), 2.31 (d, J = 3.6 Hz, 4 H), 2.21 (t, J = 6.4 Hz, 2 H), 1.92-1.98 (m, 6 H), 1.63-1.72 (m, 4 H), 1.43-1.54 (m, 8 H).

Example 6

Synthesis of the A13-derived compounds C3 and C4

a. Synthesis of the A13 derivative C3 as a parent compound for the β-gal prodrug

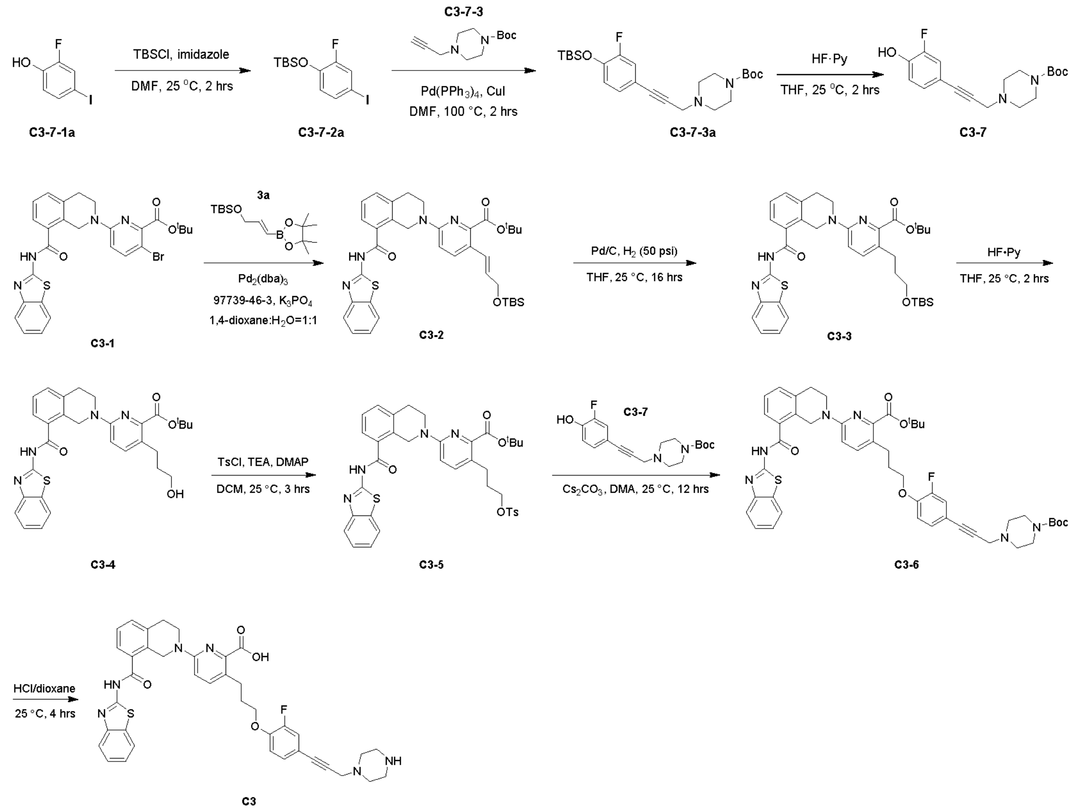


1. Synthesis of the compound C3-7-2a

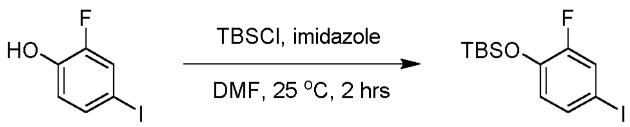


C3-7-1a C3-7-2a

The compound C3-7-1a (5.00 g, 21.0 mmol, 1.00 eq), TBSCl (4.12 g, 27.3 mmol, 3.35 mL, 1.30 eq) and IMIDAZOLE (2.86 g, 42.0 mmol, 2.00 eq) were mixed in DMF (30.0 ml), with expelling air and filling with $N_2$. The mixture was stirred and reacted at 25 °C in a $N_2$ atmosphere for 2 hours. The TLC (petroleum ether: ethyl acetate = 1: 0, P1: $R_f$=

0.86, R1: $R_f$ = 0.05) detection showed that the reaction of C3-7-1a was complete, and a new major peak with low polarity was generated. The reaction mixture was terminated by the addition of 300 ml water, and extracted using a total of 300 ml DCM (100 ml, 3 times). The resulting organic layer was washed with 300 ml of the saturated saline, dried with $Na_2SO_4$, filtered and concentrated under a reduced pressure to obtain a crude product, which was purified by the column chromatography ($SiO_2$, petroleum ether: ethyl acetate = 1: 0) to obtain the compound C3-7-2a (7.00 g, 19.9 mmol, 94.6% yield) as a colorless liquid.

HNMR: EC10607-21-P1A, $CDCl_3$, 400MHz

$^1$H NMR (400 MHz, $CDCl_3$) δ ppm 7.16 (dd, J = 10.0, 2.0 Hz, 1H), 7.07 (d, J=8.4 Hz, 1H), 6.44 (t, J=8.4 Hz, 1H), 0.78 (s, 10H), 0.03 (s, 6H).

2. Synthesis of the compound C3-7-3a

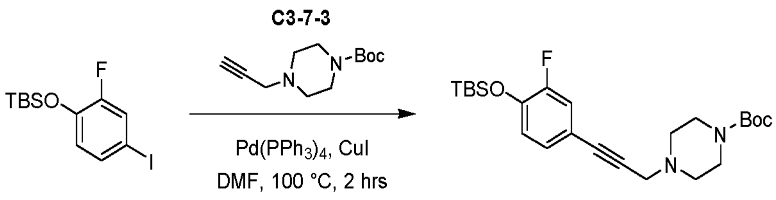


C3-7-2a C3-7-3a

The compounds C3-7-2a (2.00 g, 5.68 mmol, 1.00 eq), C3-7-3 (1.91 g, 8.52 mmol, 1.50 eq), CuI (54.1 mg, 284 μmol, 0.05 eq) and $Pd(PPh_3)_4$ (399 mg, 568 μmol, 0.10 eq) were mixed in DMF (30 ml), stirred and reacted in a $N_2$ atmosphere at 75°C for 2 hours. LCMS (EC10607-30-p1a1, P1: RT = 0.460 minute) detection showed that about 58.1% of the target product was generated. TLC (petroleum ether: ethyl acetate = 3: 1, P1: $R_f$ = 0.22, R1: $R_f$ = 0.98) showed that the reaction of the compound C3-7-2a was complete, and a new major peak with large polarity was generated. The reaction mixture was terminated by the addition of 200 ml water, extracted with 200 ml DCM (70.0 ml, 3 times). The organic layer was washed with 200 ml of the saturated saline, dried with $Na_2SO_4$, filtered and concentrated under a reduced pressure to obtain the crude product, which was purified by the column chromatography ($SiO_2$, petroleum ether: ethyl acetate = 3:1, $R_f$ = 0.22) to obtain the compound C3-7-3a (1.98 g, 4.41 mmol, 77.7% yield) as a yellow oil.

LCMS: EC EC10607-30-p1a1, RT = 0.460 min, M/Z (M+H+) = 449.9

3. Synthesis of the compound C3-7

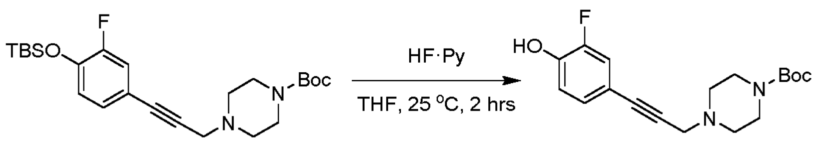


C3-7-3a C3-7

Pyridine • hydrofluoric acid (875 mg, 8.83 mmol, 795 uL, 2.00 eq) was added to a mixture of the compound C3-7-3a (1.98 g, 4.41 mmol, 1.00 eq) dissolved in THF (20.0 ml), and the mixture was stirred and reacted in a $N_2$ atmosphere at 25 °C for 2 hours. The LCMS (EC10607-35- p1a1, P1: RT = 0.241 minute) detection showed that about 89.2% of the target product was generated.TLC (petroleum ether: ethyl acetate = 2: 1, R1: $R_f$ = 0.22, P1: $R_f$ = 0.98) showed that the reaction of the compound C3-7-3a was complete, and a new major peak with large polarity was generated. The reaction mixture was terminated by the addition of 300 ml water, and extracted with 300 ml DCM (100 ml, 3 times). The organic layer was washed with 300 ml saline, dried with $Na_2SO_4$, filtered and concentrated under a reduced pressure to obtain the crude product, which was purified by the column chromatography ($SiO_2$, petroleum ether: ethyl acetate = 2:1, $R_f$ = 0.15) to obtain the compound C3-7 (1.16 g, 3.47 mmol, 78.6% yield) as a red solid.

LCMS: EC EC10607-35-p1a1, RT = 0.241 min, M/Z (M+H+) = 335.4

HNMR: EC10607-21-P1A, $CDCl_3$, 400MHz

$^1$H NMR (400 MHz, $CDCl_3$) δ ppm 7.01 - 7.08 (m, 2H), 6.90 (t, J=8.8 Hz, 1H), 3.49 - 3.57 (m, 6H), 2.62 (br t, J=4.5 Hz, 4H), 1.47 (s, 9H).

4. Synthesis of the compound C3-2

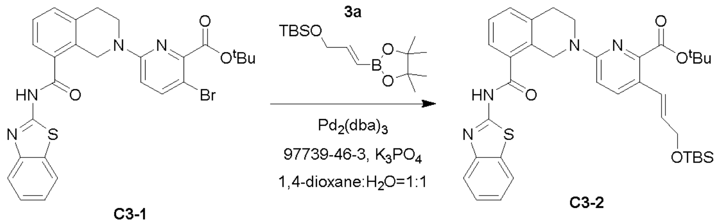


C3-1 C3-2

The compound C1-1 (1.50 g, 2.65 mmol, 1.00 eq), the compound 3a (2.37 g, 7.96 mmol, 3.00 eq), $Pd_2(dba)_3$ (243 mg, 265 μmol, 0.10 eq), $K_3PO_4$ (1.97 g, 9.28 mmol, 3.50 eq) and (1S,3R, 5R,7S)-1, 3, 5, 7-tetramethyl-8-phenyl-2,4,6-trioxa-8-phosphatricycline [3.3.1.13,7] decane (310 mg, 1.06 mmol, 0.40 eq) were mixed in 1,4-dioxane (10.0 ml) and water (10.0 ml), with expelling air and filling with $N_2$. The mixture was stirred and reacted in a $N_2$ atmosphere at 90°C for 2 hours. The LCMS (EC10607-26-pla2, P1: RT = 0.649 minute) detection showed that about 67.5% of the target product was generated. TLC (petroleum ether: ethyl acetate = 3: 1, P1: $R_f$ = 0.29, R1: $R_f$ = 0.25) showed that the reaction of the compound C3-1 was complete, and multiple new peaks were generated. The reaction mixture was terminated by the addition of 300 ml water, and extracted with 300 ml DCM (100 ml, 3 times). The organic layer was washed with 300 ml saline, dried with $Na_2SO_4$, filtered and concentrated under a reduced pressure to obtain the crude product, which was purified by the column chromatography ($SiO_2$, petroleum ether: ethyl acetate = 3: 1, $R_f$ = 0.29) to obtain the compound C3-2 (1.25 g, 1.90 mmol, 71.7% yield) as a yellow oil.

LCMS: EC10607-26-p1a2, RT = 0.650 min, M/Z (M+H+) = 657.8

5. Synthesis of the compound C3-3

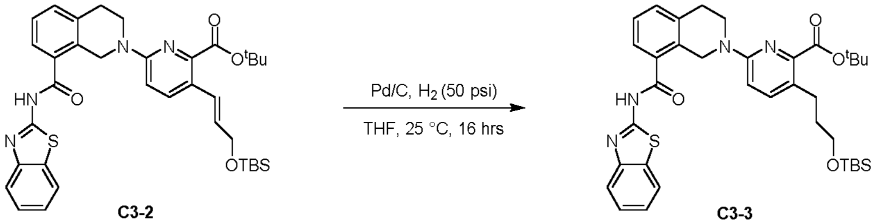


C3-2 C3-3

Pd/C (1.00 g, 1.83 mmol, 10% purity, 1.00 eq) was added to a solution of the compound C3-2 (1.20 g, 1.83 mmol, 1.00 eq) dissolved in THF (20.0 ml) and methanol (20.0 ml), and the reaction mixture was stirred and reacted in an atmosphere of 50 psi $H_2$ (1.23 mg, 609 μmol) at 25 °C for 24 hours. The TLC (petroleum ether: ethyl acetate = 3: 1, P1: $R_f$ = 0.36, R1: $R_f$ = 0.31) detection showed that the reaction of the compound C3-2 was complete, and a new peak was generated. The Pd/C was removed by filtratiing with diatomaceous earth, and the crude product was obtained by concentrating under a reduced pressure. The crude product was purified by the column chromatography ($SiO_2$, petroleum ether: ethyl acetate = 3: 1, $R_f$ = 0.30) to obtain the compound C3-3 (1.01 g, 1.53 mmol, 73.7% yield) as a yellow solid.

6. Synthesis of the compound C3-4

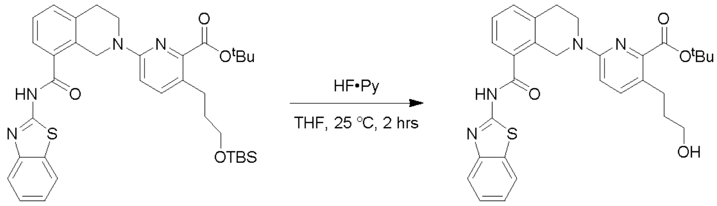


C3-3 C3-4

Pyridine • hydrofluoric acid (694 mg, 4.55 mmol, 631 uL, 65% purity, 3.00 eq) was added to a mixture of the compound C3-3 (1.00 g, 1.52 mmol, 1.00 eq) dissolved in THF (15.0 ml), and the mixture was stirred and reacted in a $N_2$ atmosphere at 25 °C for 2 hours. The LCMS (EC10607-37 LCMS (EC10607-37, P1: RT = 0.410 minute) detection showed that about 46.4% of the target product was generated. TLC (petroleum ether: ethyl acetate = 1: 1, R1: $R_f$ = 0.60, P1: $R_f$ = 0.16) showed that the reaction of C3-3 was complete, and a new major peak with large polarity was generated. The reaction mixture was terminated by the addition of 300 ml water, and extracted with 300 ml DCM (100 ml, 3 times). The organic layer was washed with 300 ml saline, dried with $Na_2SO_4$, filtered and concentrated under a reduced pressure to obtain the crude product, which was purified by prep-TLC ($SiO_2$, petroleum ether: ethyl acetate = 1:1, $R_f$ = 0.16) to obtain the compound C3-4 (700 mg, 1.29 mmol, 84.7% yield) as a white solid.

LCMS: EC EC10607-37-p1a1, RT = 0.410 min, M/Z (M+H+) = 545.4

7. Synthesis of the compound C3-5

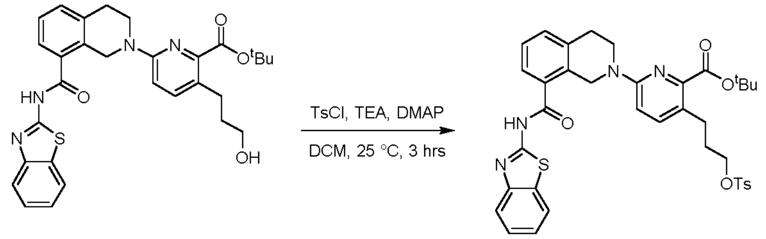


C3-4 C3-5

The compound C3-4 (680 mg, 1.25 mmol, 1.00 eq), TEA (133 mg, 1.31 mmol, 182 μL, 1.05 eq), DMAP (1.53 mg, 12.5 μmol, 0.01 eq) and 4-methylbenzenesulfonyl chloride (250 mg, 1.31 mmol, 1.05 eq) were mixed in DCM ( 5.00 ml), with expelling air and filling with $N_2$. The mixture was stirred and reacted in a $N_2$ atmosphere at 25°C for 3 hours. The LC-MS (EC10607-47-p1a1, P1: RT = 0.548 minute) detection showed that about 46.7% of the target product was generated. The TLC (petroleum ether: ethyl acetate = 2: 1, P1: $R_f$ = 0.36, R1: $R_f$ = 0.20) detection showed that the reaction of C3-4 was partially complete, and multiple new peaks were generated. The reaction mixture was terminated by the addition of 300 ml water, and extracted with 300 ml DCM (100 ml, 3 times). The organic layer was washed with 300 ml saline, dried with $Na_2SO_4$, filtered and concentrated under a reduced pressure to obtain the crude product, which was purified by the column chromatography ($SiO_2$, petroleum ether: ethyl acetate = 2: 1, $R_f$ = 0.36) to obtain the compound C3-5 (400 mg, 572 μmol, 45.8% yield) as a yellow solid.

LCMS: EC (EC10607-47-p1a1, RT = 0.548 min, M/Z (M+H+) = 699.1

8. Synthesis of the compound C3-6

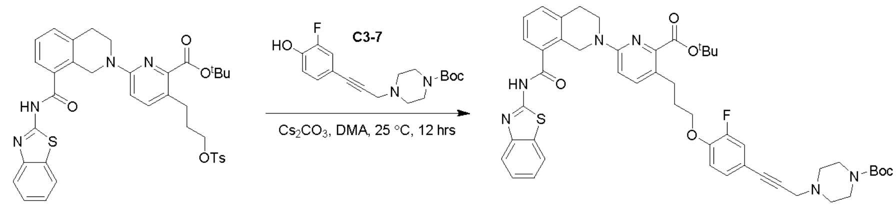


C3-5 C3-6

The compounds C3-5 (100 mg, 143 μmol, 1.00 eq), C3-7 (71.8 mg, 214 μmol, 1.50 eq), and $Cs_2CO_3$ (69.9 mg, 215 μmol, 1.50 eq) were mixed in DMA (5.00 ml), with expelling air and filling with $N_2$. The mixture was stirred and reacted in a $N_2$ atmosphere at 25 °C for 12 hours. The LC-MS (EC10607-49-p1a1, P1: RT = 0.493 minute) detection showed that about 36.9% of the target product was generated. The TLC (petroleum ether: ethyl acetate = 1: 1, P1: $R_f$ = 0.24, R1: $R_f$ = 0.42) detection showed that C3-5 was completely consumed, and multiple new peaks were generated. The reaction mixture was terminated by the addition of 30 ml water, and extracted with 30.0 ml DCM (10.0 ml, 3 times). The organic layer was washed with 30.0 ml saline, dried with $Na_2SO_4$, filtered and concentrated under a reduced pressure to obtain the crude product, which was purified by prep-TLC ($SiO_2$, petroleum ether: ethyl acetate = 1: 1, $R_f$ = 0.24) to obtain the compound C3-6 (106 mg, 123 μmol, 86.0% yield) as a yellow solid.

LCMS: EC10607-49-p1a1, RT = 0.493 min, M/Z (M+H+) = 861.2

9. Synthesis of the compound C3

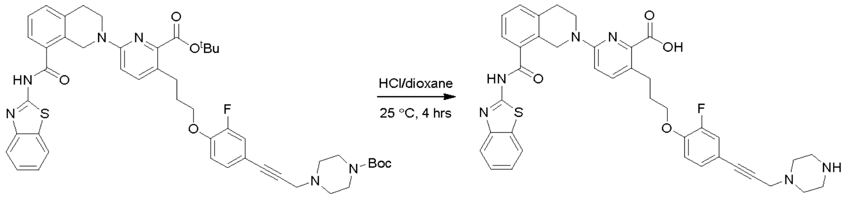


C3-6 C3

The compound C3-6 (100 mg, 116 μmol, 1.00 eq) was dissolved in HCl/dioxane (5.00 ml), with expelling air and filling with $N_2$. The mixture was stirred and reacted in a $N_2$ atmosphere at 25°C for 12 hours. The LC-MS (EC10607-51-p1a5, P1: RT = 0.412 minute) detection showed that about 86.5% of the target product was generated. The reaction mixture was concentrated under a reduced pressure to obtain the crude product, which was purified by prep-HPLC (under HCl condition, the chromatographic column: Welch Xtimate C18 150*25mm*5um; the mobile phase: [water (HCl)-ACN]; B%: 26%-46%, 10 minutes) to obtain the compound C3 (25.0 mg, 35.5 μmol, 89.3% yield) as a yellow solid. The LCMS (EC10607-57-p1a1, P1: RT = 0.42 minute) detection showed the purity of the product is about 100%. The HPLC (EC10607-57-pla2, P1: RT = 2.027 minutes) detection showed the purity of the product C3 is about 96.1%. 1HNMR (EC10607-57-P1D, 400MHz, DMSO+D2O) showed that the resulting product was consistent with the target compound.

LCMS: EC EC10607-51-p1a5, RT = 0.412, M/Z (M+H+) = 705.2

LCMS: EC10607-57-p1a1, RT = 0.402 min, M/Z (M+H+) = 705.2

$^1$H NMR (400 MHz, DMSO+$D_2O$) δ ppm 7.99 (d, J = 7.6 Hz, 1H), 7.77 (d, J = 8.0 Hz, 1H), 7.57 (d, J = 8.8 Hz, 1H), 7.54 (d, J = 7.2 Hz, 1H), 7.47 (t, J = 7.2 Hz, 1H), 7.39 - 7.42 (m, 1H), 7.32 - 7.37 (m, 3H), 7.27 (d, J = 8.9 Hz, 1H), 7.06 - 7.12 (m, 1H), 7.01 (d, J = 8.8 Hz, 1H), 4.91 (s, 2H), 4.22 (s, 2H), 4.08 (s, 2H), 3.75 - 3.81 (m, 2H), 3.33 - 3.43 (m, 8H), 2.94 - 2.99 (m, 2H), 2.77 - 2.83 (m, 2H), 1.88 - 1.96 (m, 2H).

b. Synthesis of the A13 derivative C4 as a parent compound for the β-gal prodrugs

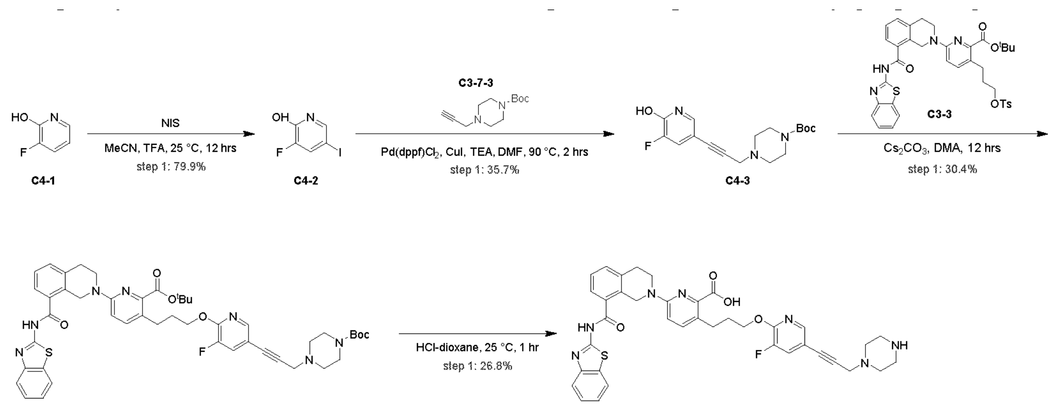


C4-1 C4-2 C4-3 C4-4 C4

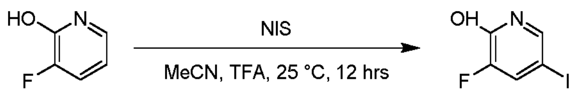


C4-1 C4-2

NIS (4.38 g, 19.5 mmol, 1.1 eq) was addd to a solution of the compound C4-1 (2.00 g, 17.7 mmol, 1.0 eq) dissolved in MeCN (20.0 mL) and TFA (3.34 mL), and the reaction mixture was stirred and reacted at 25 °C for 12 hours.

The TLC (petroleum ether: ethyl acetate = 0: 1, R1. Rf = 0.4; P1: Rf = 0.8) monitoring showed that C4-1 was completely consumed, and a new peak of the compound was generated. The reaction mixture was filtered to obtain the crude product C4-2 (3.00 g, a crude product) as a white solid.

2. Synthesis of the compound C4_3

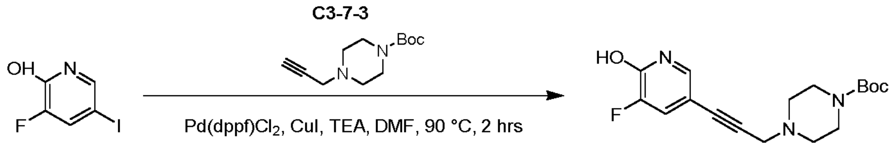


C4-2 C4-3

CuI (39.9 mg, 209 μmol, 0.05 eq), TEA (1.27 g, 12.6 mmol, 1.75 mL, 3.0 eq) and Pd(dppf)$Cl_2$ (306 mg, 418 μmol, 0.1 eq) were added to a solution of the compounds C4-2 (1.00 g, 4.18 mmol, 1.0 eq), and C3-7-3 (1.41 g, 6.28 mmol, 1.5 eq) dissolved in DMF (20.0 mL), and the reaction mixture was stirred and reacted at 90°C for 2 hours. The LCMS (EC13784-5-P1A, P1: RT = 0.242 minute) monitoring showed that the reaction of C4-2 was complete, and the main peak of the target product was generated. The reaction mixture was extracted by the addition of 20.0 mL water and 60.0 mL ethyl acetate. The resulting organic layer was washed with the saturated saline (20.0 mL, twice), dried with $Na_2SO_4$, filtered and concentrated under a reduced pressure to obtain the crude product C4-3 (500.0 mg) as a brown solid.

LC-MS: EC13784-5-P1A, the product: RT = 0.242 min, m/z = 336.3 $[M+H]^+$

3. Synthesis of the compound C4-4

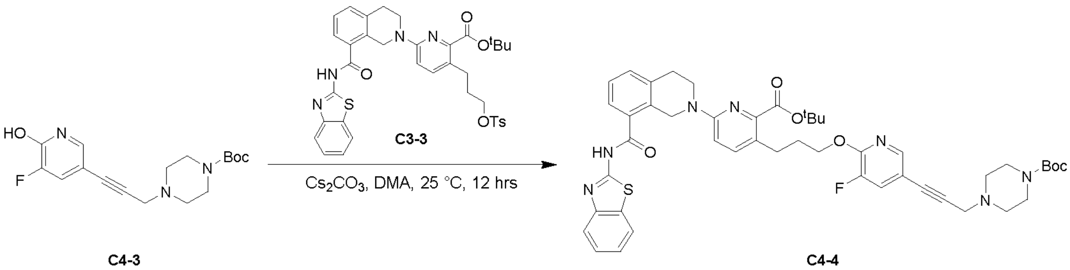


C4-3 C4-4

$Cs_2CO_3$ (175 mg, 537 μmol, 2.0 eq) and C3-3 (188 mg, 268 μmol, 1.0 eq) were added to a solution of the compound C4-3 (90.0 mg, 268 μmol, 1.0 eq) dissolved in DMA (3.00 mL), and the reaction mixture was stirred and reacted at 25°C for 12 hours. The LCMS (EC13784-6-P1A, P1: RT = 0.495 min) monitoring showed that the reaction of the compound C4-3 was complete, and the main peak of the target product was generated with an expected m/z. The TLC (petroleum ether: ethyl acetate = 1: 2, R1: $R_f$ = 0.1; R2: $R_f$ = 0.85; P1: $R_f$ = 0.55) monitoring showed that C4-3 was completely consumed, and two new compound peaks were generated. The reaction mixture was extracted by the addition of 10.0 mL water and 10.0 mL ethyl acetate. The resulting organic layer was washed with the saturated saline (10.0 mL, twice), dried with $Na_2SO_4$, filtered and concentrated under a reduced pressure to obtain the crude product, which was subjected to a TLC (petroleum ether: ethyl acetate = 1:2, P1: $R_f$ = 0.55; P2: $R_f$ = 0.30) detection, a purification by the column chromatography ($SiO_2$, petroleum ether: ethyl acetate = 1: 2) to obtain the compound C4-4 (55.0 mg) as a colorless oil.

LC-MS: EC13784-6-P1A, the product: RT = 0.495 min, m/z = 862.6 $[M+H]^+$

4. Synthesis of the compound C4

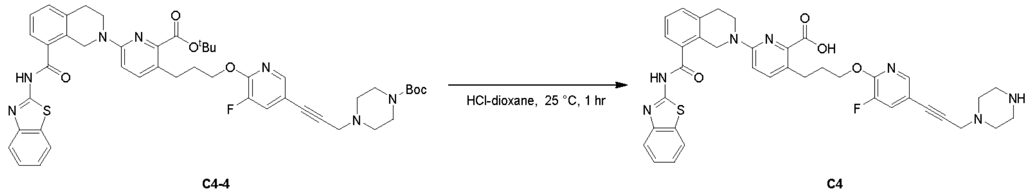


C4-4 C4

The compound C4-4 (50.0 mg, 58.0 μmol, 1.0 eq) was dissolved in HCl-dioxane (2.00 mL), and the mixture was stirred and reacted at 25°C for 1 hour. The LCMS (EC13784-11-P1A2, P1: RT = 0.418 minute) monitoring showed that the reaction of the compound C4-4 was complete, and the main peak of the target product was generated with an expected m/z. The reaction mixture was filtered and concentrated under a reduced pressure to obtain the crude product, which was purified by the prep-HPLC column chromatography (the chromatographic column: Welch Xtimate C18 150 * 25 mm * 5 μm; the mobile phase: [water (HCl)-ACN]; the gradient elution: 30%-60% B, 10 min) to obtain the compound C4 (11.0 mg, 24.46% yield, 91.03% purity) as a colorless oil.

LC-MS: EC13784-11-P1A2, the product: RT = 0.418 min, m/z = 706.3 $[M+H]^+$

HPLC: EC13784-11-P1C; the product: RT = 1.945 min

$^1H$ NMR: EC13784-11-P1C1 (400 MHz DMSO) δ ppm 8.17 (d, J = 1.6 Hz, 1H), 8.02 (d, J = 7.6 Hz, 1H), 7.90 (dd, J = 10.8, 1.8 Hz, 1H), 7.78 (d, J = 8.0 Hz, 1H), 7.59 (t, J = 8.8 Hz, 2H), 7.44 - 7.50 (m, 1H), 7.40 - 7.44 (m, 1H), 7.32 - 7.38 (m, 2H), 7.02 (d, J = 8.8 Hz, 1H), 4.92 (s, 2H), 4.37 - 4.42 (m, 2H), 4.29 - 4.37 (m, 2H), 3.83 (t, J = 6.0 Hz, 3H), 3.57 (d, J = 4.0 Hz, 5H), 3.42 - 3.49 (m, 4H), 2.98 (t, J = 5.6 Hz, 2H), 2.81 (t, J = 7.6 Hz, 2H), 1.92 - 2.01 (m, 2H).

Example 7 In vitro testing of the cell killing activity of β-gal precursors

Induction of the cellular senescence

To avoid the replication-induced celluar senescence, the low-generation proliferating HEF (<10 generations) and A549 cells were used as normal controls. For chemically induced senescence, the HEF cells were treated with 10 μM etoposide for 36-48 hours, and A549 cells were treated with 15 μM cisplatin (Selleck, S1166) for 72 hours. Three days after the treatment, the cells were passaged and cultured for 72 hours in fresh medium at a ratio of 1:3 before further analysis and use.

Cell killing activity assay

Preparation of the cell plates: The non-senescent, and the induced senescent HEF cells and A549 cells were taken respectively, digested with trypsin, and centrifuged at 1000 rmp for 5 minutes. The supernatants were removed, and the cells were resuspended in fresh DMEM medium containing 10% foetal bovine serum for counting. The cell density was adjusted so that the density of the non-senescent cells was at 4000 cells/100 μL and the density of the senescent cells was at 7000 cells/100 μL. The cell suspension was added to the 96-well transparent cell culture plates at 100 μL per well. Note that the edge wells of the 96-well plate were not used for the test wells, and 300 μL PBS is added separately to reduce the medium evaporation. The cell-spread 96-well plates were put back into the incubator at 37°C for the overnight incubation until the cells adhered to the wall.

Formulation of the drug concentration gradients: After the cells adhered to the wall on the second day, the small molecule compounds (d-12, d-11, and d-4) to be tested for activity are ready to be added. A series of small molecule concentration gradients, e.g., 0.01, 0.03, 1, 3, 10, 25 μM, were pre-set, and a 2-fold drug dilution of the above concentration was prepared in DMEM medium containing 10% fetal bovine serum, and the above DMEM medium containing the gradient concentration of the drug was added to the 96-well plate with cells plated in advance at 100 μL per well, so that the final concentration of the drug was the series of pre-set concentration in each well. 3 parallel replicate wells were set up for each test drug concentration. The 96-well plate was put back into the incubator at 37°C, and the cell survival was tested after three days.

Detection of the cell survival after the drug treatment using the CCK-8 method: Three days after the addition of the drug, the well plate was removed, the supernatant was discarded, and 100 μL of the pre-prepared CCK-8 working solution was added to each well. The CCK-8 working solution was obtained by diluting the CCK8 stock solution (C0041, Beyotime) with the serum-free DMEM medium in 10-fold volume. The 96-well plate added with the CCK-8 working solution was put back to the incubator at 37°C and incubated for 0.5-2 hours. The plate was taken out to determine the absorbance value (OD value) of each well at the wavelength of 450 nm. The cell viability in each experimental group was calculated. The following formula was used: the relative cell viability (%) = (the blank control group - the experimental group)/the blank control group x 100 %.

According to the calculated results of the cell viability, the killing curves of different drugs for the senescent and

non-senescent cells were made, as shown in Figure 7. As can be seen from Figure 7, the three tested prodrug molecules (d-12, d-11, and d-4) showed the selective killing of the senescent cells in both A549-induced senescence and HEF-induced senescence cell models, and none of the three molecules killed the non-senescent cells at the highest concentration (25 μM), which reflected their better senescent cell selectivity. Among the three molecules, d-12 and d-4 were relatively more active in killing the senescent cells. According to the analysis of the results, the $IC_{50}$ of d-12 and d-4 on the senescent A549 cells were about 6.9 μM and 10.4 μM, respectively. Based on the above results, it can be seen that he synthesized β-gal prodrug molecules were highly selective in targeting the senescent cells, and the higher concentration did not affect the non-senescent cells, wherein d-12 and d-4 have a higher killing activity on the senescent cells.

Example 8 Biochemical and pharmacological studies of the prodrug A13bGa1 (*i.e.*, a-7 in Example 1)

Study of the hydrolysis reaction of A13bG

The *in vitro* enzymatic hydrolysis reaction was carried out using *E. coli*-derived β-galactosidase (Sigma) in PBS solution (0.01 M, pH 7.3) at a reaction concentration of 10 μM for both A13 and A13bG, and for 6 hours at 37°C after the addition of β-galactosidase (10 U/μM). The hydrolysis reaction was monitored by the high performance liquid chromatography (HPLC). The compound A13 and the prodrug A13bG without enzyme reaction were analyzed by HPLC at the same conditions as the references. The chromatographic column model: Waters Acquity BEH C4, the conditions: 0.04 ml/min, (water (0.1% acetic acid)): the acetonitrile gradient elution: 98:2 1 min, 5:95 13 min, 5:95 18 min, 2:98 20 min.

Cell Culture and Processing

Isolation of Human Primary Cells

The study was approved by the Institute of Review Board in Peking University (IRB 00001052-15087) and was carried out according to the approved protocol. Samples were collected from the donors who gave the informed consent according to the ethical approval procedure of the China-Japanese Friendship Hospital.

The human embryonic skin fibroblasts (HEF) and the human fetal lung fibroblasts (HELF) were obtained as previously described (Xie, B., et al., Cell Res. 29, 696-710 (2019)). Briefly, the human embryonic skin tissues and the fetal lung tissues obtained from the aborted tissues with the patient's informed consent were minced with the forcep and incubated in 1 mg/ml collagenase IV at 37°C for 1-2 hours. After the enzyme treatment, the cells were collected by centrifugation and resuspended in HEF medium (Dulbecco's modified Eagle medium (DMEM, Gibco) containing 10% fetal bovine serum (FBS, Ausbian), 1% GlutaMAX (Gibco), 1% non-essential amino acids (NEAA, Gibco) and 1% penicillin/-streptomycin (Gibco). Gibco). Cells were plated on the 10 cm tissue culture dishes and grown in 10% FBS DMEM. HUVEC cells were purchased from Lonza (C2519A) and cultured in Endothelial Cell Medium (sciencell, 1001). They were passaged and cryopreserved according to Lonza's method. A549 cells, BJ cells and MRC5 cells were purchased from ATCC and cultured in DMEM medium containing 10% fetal bovine serum (FBS, Ausbian), and 1% penicillin/streptomycin (Gibco).

Induction of the Cellular Senescence

To avoid the replication-induced celluar senescence, the low-generation proliferating HEF/HELF (<10 generations) and HUVEC (<6 generations) were used as the normal controls.

For chemically induced celluar senescence, HEF/HELF cells were treated with 10 μM etoposide for 36-48 hours, and HUVEC cells were treated with 5 μM etoposide (Etoposide, S1225 ) for 36-48 hours. A549 cells were treated with 15 μM cisplatin (Selleck, S1166) for 72 hours, and BJ and MRC5 cells were treated with 20 μM bleomycin (Selleck, S1214) for 48-72 hours. Three days after the treatment, the cells were passaged and cultured inn fresh medium at a ratio of 1:3 for 72 hours before a further analysis.

The β-Galactosidase Staining of the Cultured Cells and the Frozen Sections

The cultured cells were washed once with PBS and fixed in the β-galactosidase staining fixative for 15 minutes at room temperature. Cells were then washed three times with PBS and incubated with the β-galactosidase staining solution (Beyotime Biotechnology) at 37°C for 16-20 hours. For 12-well plates, 1 ml of the β-galactosidase staining solution was added to each well. Plates were sealed with Parafilm to prevent an evaporation of the staining medium. After overnight incubation, the cells were washed with PBS and observed under a bright-field microscope. For the β-galactosidase staining of the frozen sections, the frozen sections were dried at 37°C for 20-30 minutes and then fixed in the β-galactosidase staining fixative for 15 minutes at room temperature. Frozen sections were washed three times with PBS and incubated in the β-galactosidase staining solution overnight at 37°C. After the completion of the β-galactosidase staining, the sections were stained with eosin for 1-2 minutes, rinsed under the running water for 1 minute, differentiated in 1% acidic alcohol for 10-20 seconds, and again rinsed under the running water for 1 minute. Sections were dehydrated in the alcohol with an increased concentration and made transparent in xylene. Excess xylene was drained and the coverslip was placed on the section. After drying, the samples were observed under a microscope.

Cell Viability Test

Cell Counting Kit-8 (CCK-8) assay was used to assess the cell viability. The senescent and non-senescent cells or chondrocytes were seeded into the 96-well plates and cultured overnight. The cells were then treated with control (0.05% DMSO) or different concentrations of the drug to be tested. After 48 hours or 72 hours, the CCK-8 solution (C0041, Beyotime) was added to each well at a ratio of 1:10 and incubated for 1-2 hours. Absorbance was measured at the wavelength of 450 nm.

Mice.

All animal experiments were carried out according to the Animal Protection Guidelines of Peking University, China. C57BL/6 (C57) mice were obtained from Beijing Vital River Laboratory Animal Technology Co, Ltd., and fed in a specific pathogen free facility (SPF) under 12 light/12 dark cycle with ad libitum access to food and water. Old male mice were fed in individual cages, and the female mice and the male pups from the same litter were fed no more than five in each cage.

*In vivo* drug treatment

All drugs were mixed in 85% physiological saline, 5% DMSO (Sigma, D8418) and 10% Solutol HS 15 (Sigma, 42966), and administered to mice by intraperitoneal (i.p.) injection. To treat the bleomycin-injured young mice, A13 (2 mg/kg), A13bGa1 (5 mg/kg), or an empty vehicle control was administered three consecutive days per week for four weeks. A1331852 (abbreviated as A13) was purchased from MCE.

*In vivo* platelet toxicity test

A single administration of A13 or A13bG was performed. Female 5-6 week old C57 mice were treated with a single intraperitoneal injection. A13 and A13bG were administered in three different dose groups of 2, 5, and 20 mg/kg. Approximately 50 μl of blood was collected from each mouse at different time points into the EDTA tubes (RAM Scientific) by means of inner canthal blood extraction, and analyzed using a haematology analyzer for a blood routine examination and a platelet counting. Multiple administrations of A13 or A13bG were performed. Female 5-6 week old C57 mice were treated with multiple intraperitoneal injections. A13 and A13bG were administered in three different dose groups of 2, 5 and 20 mg/kg, three consecutive days per week, followed by a 4-day withdrawal for 3 weeks. The time points of administration were in the afternoon of days 0, 1, 2, 7, 8, 9, 14, 15, and 16, and the time points of blood collection and testing were in the morning of days 1, 3, 5, 8, 10, 12, 15, 17, and 19, respectively.

Bleomycin-Induced Pulmonary Fibrosis

Bleomycin was purchased from Selleck. As previously reported (Bivas-Benita et al., Eur J Pharm Biopharm 61, 214-218 (2005)), a transtracheal injection of bleomycin (2 mg/kg) was performed to the young male C57 mice (8-10 weeks old) to induce lung fibrosis. Drug treatment was initiated four days after modelling, and the drug was administered three consecutive days per week for four consecutive weeks, after which the mice were executed for material collection.

Blood Analysis

For a blood routine examination, 50 μl of fresh blood was collected from each mouse and immediately mixed with EDTA. Blood samples were analyzed by Celltac Alpha MEK-6400 series haematology analyzer (Nihon Kohden, MEK-6400). Data were expressed as the number of different blood cells or platelets per μl of blood. For the serum biochemical analysis, the blood samples were collected, coagulated for 2 hours at room temperature or overnight at 4°C, and then centrifuged (1000 g, 10 minutes) to obtain serum. A 200 μl of serum was aliquoted and analyzed for alanine aminotransferase (ALT), aspartate aminotransferase (AST), uric acid (UA), and creatinine (CREA) by a chemistry analyzer (Mindray, BS-350E).

Histopathological Examination

All animals (including those found dead, dying, and euthanised) were autopsied, and the lung tissues were preserved. The lungs were separated, the fat and other tissues were removed, the surface fluid was absorbed using a filter paper, and the whole lungs were weighed (those found dead, dying, and euthanised) for calculation of the lung weight index. The right lung and bronchiole were fixed in 10% neutral buffered formalin solution, embedded in paraffin, sectioned, prepared into slices, and stained with HE for a histopathological and morphological observation of the lung. The degree of the lesions such as the inflammatory cell infiltration in the lung tissue were graded according to a 4-grade classification (slight, mild, moderate, severe) using a standard terminology for diagnosis and classification. The left lung tissue was clipped and stored temporarily below -60°C.

$$\text{Lung weight index (\%)} = \text{lung weight/body weight} \times 100\%.$$

Masson staining

Masson trichrome stain kit (Solarbio, G1340) was used to detect fibrosis in the lung tissue. The formalin-fixed, and paraffin-embedded lung sections were dewaxed with xylene, and then rehydrated with 100% alcohol, 95% alcohol and distilled water. The sections were then stained according to the manufacturer's instructions. Afterwards, the sections were rapidly dehydrated with 95% alcohol and 100% alcohol, cleaned with xylene and covered with the coverslips. Images were acquired using a bright-field optical microscope (10x eyepiece) (OLYMPUS, BX43), and the degree of fibrosis of the lung tissue was graded according to the intensity of staining (weakly positive, moderately positive, moderately strong positive, and very strong positive).

Modelling Oxygen-Induced Retinopathy in Mice

Neonatal C57BL6/J mice were exposed to a 75% hyperoxia chamber (Shanghai Tow-Int Tech Co., Ltd., Ox-100HE) from the 7th to the 12th day after birth, together with the surrogate ICR female mice, and the mice were removed and placed in a normal air environment for feeding after 12 days. Hypoxia-induced abnormal vasoproliferative lesions persisted in the retina of the neonatal mice after they were fed under a hyperoxic condition and then transferred to a normoxic condition. This mouse lesion model mimics the retinal lesion characteristic of some human vision-related diseases, such as proliferative diabetic retinopathy.

Administration by an Intravitreal Injection in mice

Mice on postnatal day 12 were treated with the intravitreal administration of A13bGa1 or vehicle control in each eye of each mouse. The drug vehicle was 5% Solutol HS 15 (Sigma, 42966), 95% physiological saline. Injection using the vehicle alone was used as a drug control group (VEH). A133bGa1 drug was dissolved using the vehicle (5% Solutol HS 15, 95% physiological saline) to prepare the different concentrations of injections for use. Intravitreal injections in mice were performed using a 2.5 μl microinjection needle (Hamilton, 7632-01) attached to a 34G bevelled injection needle (Hamilton), and the intravitreal dose volume is 1 μl per eyeball, with the drug injections pre-prepared according to the corresponding administered dose.

Retinal Microvessel Staining Analysis

The extracted eyeballs of mice were firstly fixed with 4% paraformaldehyde for 1 hour at room temperature. The retina was carefully detached using the micro ophthalmic scissors and the micro forceps, and then placed in the pre-prepared fluorescence-labelled phytohemagglutinin IB4 (Vector, FL-1201) staining solution, and stained overnight at 4 degrees Celsius away from light. Retinal sections were made after the completion of staining, and photographed using a confocal fluorescence microscope (Leica, TCS-SP8). Retinal microvascular stained sections were analyzed for the ischaemic areas and the abnormal neovascular areas using ImageJ (NIH, USA).

Plasma Metabolic Stability

A13bGa1 was co-incubated with the mouse, rat, canine, monkey and human plasma at a final drug concentration of 10 μM in a 200 μL incubation system for 120 minutes, and the samples were taken and analyzed at time points 0, 60 and 120 minutes during the incubation. Termination of the reaction was accomplished by the addition of 3x volume of acetonitrile and centrifugation at 16000 g for 20 minutes. The supernatant was taken for a LC-MS/MS analysis. The

chromatographic column: Waters XSelect HSS T3; the flow rate: 500 μL/min; the eluent: A, water (0.1% formic acid); B, acetonitrile (0.1% formic acid); the gradient elution: 0-1.5 min, 5% B, 1.5-9 min, 5%-80% B, 9-12 min, 80%-100% B, 12-14 min, 100% B, 14-14.3 min, 100%-5% B, 14.3-15 min, 5% B.

Hepatic microsomal metabolic stability

A13bGa1 was co-incubated with the mouse, rat, canine, monkey and human hepatocytes and the incubation medium at a final drug concentration of 1 μM in the incubation system for 60 minutes. Samples were taken and analyzed at time points 0, 15, 30, 45 and 60 minutes during the incubation. The reaction was terminated by adding an amount of acetonitrile solution containing the internal standard. Verapamil and 7-OH coumarin were used as the positive control drugs to quality control the experimental incubation system. The residual percentage, half-life, intrinsic clearance, amplified clearance, hepatic clearance and hepatic extraction ratio were determined. The entire experiment was performed in triplicate. All data calculations were performed by Microsoft Excel software. Peak areas were detected by extracting the ionic spectrum. The *in vitro* half-life ($t_{1/2}$) of the parent compound was detected by fitting the natural logarithm of the percentage elimination of the parent compound linearly to time.

The *in vitro* half-life ($t_{1/2}$) was calculated by slope: in vitro t1/2 = 0.693/k

The *in vitro* intrinsic clearance (in μL/min/mg protein) was calculated using the following equation: *in vitro* $CL_{int}$ = kV/N

The amplified clearance (in mL/min/kg) was calculated using the following equation:

$$\text{Amplified } CL_{int} = kV/N \times \text{the amplification factor}$$

The hepatic clearance (in mL/min/kg) was calculated using the following equation:

$$\text{The predicted hepatic } CL_H = (Q \times Fu/R_B \times \text{the amplified } CL_{int}) / (Q + Fu/R_B \times \text{the amplified } CL_{int})$$

V = the incubation volume per well (0.2 mL);
N = the total amount of protein (mg)
Q = the hepatic blood flow (mL/min/kg)
Fu = the plasma free rate (assumed to be 1)
$R_B$ = the whole blood partition coefficient (assumed to be 1)

The hepatic uptake rate was calculated using the following equation:

$$\text{The hepatic extraction ratio (ER)} = (\text{the predicted hepatic } CL_H)/Q$$

Statistical Analysis

For the statistical analysis, the p-values were calculated by t-test (when comparing only two groups) or one-way ANOVA (when comparing more than two groups) using the default parameters in Excel or GraphPad Prism 8. All results are expressed as mean ± sem, and n represents the number of mice. The p-values are as follows: *P < 0.05; **P < 0.01; *** P < 0.001; ****P < 0.0001.

Results and Discussion

In order to find more efficient and safer β-gal prodrugs for targeting the senescent cells, we have carried out the screening and optimization of the β-gal-modified candidate parent compound. The ideal parent compounds should have the following characteristics: 1) the compounds have the suitable structural sites for β-gal modification and the appropriate molecular weights; 2) the compounds have a certain safety basis; and 3) the compounds themselves have high killing activity against the senescent cells and preferably have a certain degree of selectivity to the non-senescent cells.

We first performed *in vitro* screening of a series of compounds on the induced senescent A549 cell (human non-small cell lung cancer cell) and the induced senescent HEF cell (human embryonic fibroblast) models. Our *in vitro* compound screening results revealed that BRD4 inhibitors and Bcl-xL inhibitors showed the best performance in terms of the senescent cell killing activity and selectivity. Although some of these BRD4 inhibitors, such as MZ1, dBET6, and

ARV825, have better *in vitro* killing activity (Figure 1A), all of them are PROTAC molecules with large molecular weights and relatively complex structures that are not suitable for modification as a parent compound. The screening results also revealed that the novel Bcl-xL inhibitor molecules, such as A1331852 and A1155463, have a good senescent cell killing activity and selectivity *in vitro* (Figure 1A). We found through the further experimental results that A1331852 and A1155462 have a selective killing effect on the senescent cells, and their killing activity on the senescent cells is higher than that of the classical small molecule inhibitor ABT-263 (Figures 1B and 1C). Combining the screening results on *in vitro* cell models and the analysis of the structural properties of the compounds, we finally established the compound A1331852 as the parent compound for a β-gal modification.

Based on the compound A1331852 (abbreviated as A13), we synthesized the β-gal prodrugs by the synthetic route shown in Figure 2A. We first synthesized an acetylated galactoside intermediate with a nitrobenzene linker (the product 4a), which was reacted with the parent compound A1331852 to obtain the A1331852 prodrug with an acetylated form of galactoside modification (the product 6a), followed by deacetylation to obtain another form of the galactoside-modified A1331852 prodrug (the product 7a). The product 7a was named A13bGa1, *i.e.*, one of the forms of the β-gal prodrugs.

Next, we performed the enzymatic hydrolysis experiments *in vitro* to verify the cleavage release of the parent compound A13 from A13bGa1 under the action of β-gal enzyme as schematically shown in Figure 2B. We observed the peak efflux times of the parent compound A13, as well as the prodrug A13bGa1, with and without the addition of β-gal enzyme by the high-performance liquid chromatography (HPLC) method. The experimental results showed that the peak efflux time of the parent compound A13 was at minute 19.0, and the peak efflux time of the prodrug A13bGa1 was at minute 18.1. After the reaction with the addition of β-gal enzyme, the peak efflux of the prodrug appeared at minute 19.0, which indicated that the prodrug A13bGa1 hydrolysed under the action of β-gal enzyme to release the parent compound A13 (Figure 2C).

We then evaluated and compared the senescent cell killing activity and selectivity of the parent compound A13 and the modified prodrug A13bGa1 on a variety of *in vitro* induced senescent cell models. The induced senescence cell models include the HEF cell (primary human embryonic skin fibroblast), HELF cell (primary human embryonic lung fibroblast), A549 cell (human non-small cell lung cancer cell line), MRC5 cell (human embryonic lung fibroblast cell line), BJ cell (human skin fibroblast cell line), and HUVEC cell (human umbilical vein endothelial cell line) models. We detected the expression of the senescence marker β-gal before and after the induction of senescence in each cell model, and the senescence-induced cells became larger in morphology and stained positive for β-gal (Figure 3A). Meanwhile, the results of the killing experiments on the above multiple cell models showed that the prodrug A13bGa1 was highly efficient and selective in killing the senescent cells originated from the human skin-derived fibroblasts (Figures 3B and 3D), the lung-derived cells (Figures 3C, 3E and 3G) or the vascular endothelial-derived cells (Figure 3F). Although the overall killing activity of the prodrug A13bGa1 on the senescent cells was somewhat reduced compared with that of the parent compound A13 (Figures 3B-3G), its toxicity to the normal cells was greatly reduced, especially for some cells of primary origins that are sensitive to the Bcl-xL inhibition (Figures 3A, 3C and 3E). The results of the above killing experiments indicate that the modified prodrug A13bGa1 can be able to efficiently and selectively kill the senescent cells of multiple types of origins *in vitro,* and has the potential to greatly reduce toxicity to the normal cells.

According to the published literatures, the Bcl-xL inhibitors have severe platelet toxicity when used *in vivo,* and this side effect greatly limits their clinical application. Considering that the β-gal modification enhanced the specificity of the parent compound A13, we speculated that the platelet toxicity of the prodrug A13bGa1 could be significantly ameliorated compared with the parent compound A13. We administered the different concentrations of both compounds separately in mice, and then monitored changes in the platelet counts in peripheral blood. The results of single intraperitoneal administration showed that A13bGa1 was significantly less toxic to the platelets than the parent compound A13 (Figure 4A), and had a strong platelet-destroying effect only at the higher dose of 20 mg/kg, but overall its effect on the magnitude of the platelet changes in peripheral blood was significantly less than that of A13. In addition, we conducted multiple rounds of administration tests according to the administration regimen of eliminating the senescent cells *in vivo* for 3 consecutive days for 1 week (Figure 4B). The results revealed that the parent compound A13 at the lowest dose of 2 mg/kg had very serious toxic effects on the platelets, and its three doses tested all had strong destructive effects on the platelets, leading to the dramatic fluctuations in the number of the peripheral blood platelets during the monitoring process (Figure 4C). In contrast, under this multi-round administration regimen, the toxic side effects of the prodrug A13bGa1 on the platelets were significantly reduced, and the changes in the number of the peripheral blood platelets were relatively stable (Figure 4C). In addition, we closely monitored the status and clinical manifestations of the mice in each group during the administration process, and found that after multiple rounds of administration for 3 consecutive weeks, the mice in the group administered with the parent compound A13 showed the abnormalities such as hair loss and depression at the doses of 5 mg/kg and 20 mg/kg (Figure 4D), which suggests that the consecutive multiple rounds of administration at these doses has a greater adverse effect on the normal body functions of the mice. Based on the above results, the prodrug A13bGa1 can significantly reduce the platelet toxicity and side effects *in vivo* and improve the safety of dosing *in vivo.*

We then tested the therapeutic effect of the prodrug A13bGa1 *in vivo* in a mouse model of Bleomycin-induced

pulmonary fibrosis. The Bleomycin modelling and the drug treatment protocol are shown in Figure 5A. The mice were modelled and treated after 4 consecutive weeks of drug administration, during which the mice were monitored for changes in body weigh (Figure 5B). Upon completion of the treatment, a lung sampling was performed to calculate the lung specific gravity index and the pathology was assessed. The experimental results showed that compared with the unmodelled control group, the lung index of the mice after modelling with bleomycin was significantly increased, and recovered to the normal level after the administration of A13bGa1, in contrast, no improvement in the lung index of the mice was observed after the administration of the parent compound A13 (Figure 5C). Correspondingly, the results of the pathological sections of the lungs reflected that the lungs of the mice after modelling showed the pathological manifestations, such as the scattered infiltration of the inflammatory cells, thickening of the pulmonary septum, and consolidation; and the inflammation and consolidation of the mouse lungs showed significant improvement after the A13bGa1 administration, whereas no improvement was observed in the A13 administration treatment group (Figure 5D and Table 1).

Table 1. The results of HE pathological changes in the lungs

| **Intergroup summary of the major pathological changes in lung HE** | | | | | | |
|---|---|---|---|---|---|---|
| **Group** | | | **Saline** | **Vehicle** | **A13** | **A13bGa1** |
| **Dose (mg/kg)** | | | 0 | 0 | 2 | 5 |
| **The number of animals** | | | 5 | 6 | 6 | 6 |
| **The infiltration of the inflammatory cells** | **negative** | - | 5 | 2 | 1 | 4 |
| | **slight** | + | 0 | 0 | | 1 |
| | **mild** | ++ | 0 | 0 | 2 | 0 |
| | **moderate** | +++ | 0 | 4 | 1 | 1 |
| **Bleeding, alveolar/interstitial** | **negative** | - | 2 | 2 | 2 | 3 |
| | **slight** | + | 2 | 4 | 2 | 3 |
| | **mild** | ++ | 1 | 0 | 2 | 0 |
| | **moderate** | +++ | 0 | 0 | 0 | 0 |
| **Thickening and consolidation of the pulmonary septum** | **negative** | - | 2 | 0 | 2 0 | 1 |
| | **slight** | + | 2 | 2 | 1 | 0 |
| | **mild** | ++ | 1 | 1 | 2 | 5 |
| | **moderate** | +++ | 0 | 2 | 1 | 0 |
| | **severe** | ++++ | 0 | 1 | 2 | 0 |
| **Exudative oedema, alveolar** | **negative** | - | 4 | 3 | 0 | 2 |
| | **slight** | + | 1 | 2 | 1 | 1 |
| | **mild** | ++ | 0 | 1 | 1 | 2 |
| | **moderate** | +++ | 0 | 0 | 1 | 1 |
| | **severe** | ++++ | 0 | 0 | 3 | 0 |

Meanwhile, the results of Masson staining further demonstrated that the pathological manifestations of the pulmonary fibrosis in mice were improved after the treatment by administering A13bGa1 and not improved in the A13 administration group, as shown in Figure 5D and Table 2.

Table 2. The masson staining results of lungs

| **Intergroup summary of the degree of fibrosis in the lungs using Masson staining** | | | | | |
|---|---|---|---|---|---|
| **Group** | | **Saline** | **Vehicle** | **A13** | **A13bGa1** |
| **Dose (mg/kg)** | | 0 | 0 | 2 | 5 |
| **The number of animals** | | 5 | 6 | 6 | 6 |
| **not positive** | - | 5 | 0 | 0 | 3 |
| **weakly positive** | + | 0 | 2 | | 2 |
| **moderately positive** | ++ | 0 | 2 | 2 1 | 1 |
| **Moderately strong positive** | +++ | 0 | 0 | 1 | 0 |
| **extremely strong positive** | ++++ | 0 | 2 | 2 | 0 |

The *in vivo* experimental results of the Bleomycin-induced pulmonary fibrosis model showed that the prodrug

A13bGa1 exerted the good anti-inflammatory and anti-fibrotic therapeutic effects *in vivo.*

We also tested the *in vivo* therapeutic effects of the prodrug A13bGa1 in a mouse model of oxygen-induced retinopathy. The establishment of the oxygen-induced retinopathy model and the drug treatment protocol are shown in Figure 6A. The mice were evaluated for retinopathy after 75% hyperoxia-induced modelling and the drug administration through the intravitreal injection. We used the green fluorescent-labelled phytohemagglutinin IB4 for the microangiopathy analysis of the retina, and the experimental results showed that the area of the ischemic lesion area of the retina center was significantly reduced in the model mice after treatment with the different doses of A13bGa1 compared with the single vehicle control drug administration group (Figures 6B and 6C). Meanwhile, we observed and compared the abnormal neovascular lesions in the retinas in each group of the model mice, and found that the abnormal neovascular lesions in the retinas of the model mice were improved and the area of the abnormal neovascular area was significantly reduced after the treatment by administering A13bGa1 (Figures 6D and 6E). The above experimental results indicate that the treatment of the prodrug A13bGa1 can effectively improve the retinal oxygen-induced microangiopathy of the mice, and it is expected to exert a good therapeutic effect on various retinopathy-associated diseases.

We also conducted a preliminary test to evaluate the stability and metabolism of the prodrug A13bGa1 *in vivo.* The results showed that A13bGa1 was highly stable in the plasma of several species of human monkey, canine, rat, and mouse, as shown in Table 3.

Table 3. Plasma metabolic stability of A13bGa1 prodrug in different species

| The peak number | Time (min) | The mass-to-charge ratio *m/z* | The mass deviation (ppm) | The mass shift | The biotransformation | The peak area after normalisation at minute 120 | | | | |
|---|---|---|---|---|---|---|---|---|---|---|
| | | | | | | Huamn | Monkey | Canine | Rat | Mouse |
| 1 | 8.72 | 493.6733 (z=2) | 0.4 | 13.9795 | oxidative dehydrogenation | 0.07% | 0.04% | 0.48% | 0.13% | 0.04% |
| 2 | 9.44 | 486.6835 (z=2) | 0.1 | 0 | Initial drug A13bGa1 | 99.93% | 99.96% | 99.52% | 99.87% | 99.96% |

A13bGa1 also showed a good moderate metabolic stability in the liver microsomal metabolic stability study (see Table 4).

Table 4. The liver microsomal metabolic stability of A13bGal prodrug in different species.

| **Name of the compound** | **Species** | **The half-life of metabolism $T_{1/2}$ (min)** | **The intrinsic clearance *in vitro* $Cl_{int}$ (μL/min/mg protein)** | **The amplified intrinsic clearance $Cl_{int}$ (mL/min/kg)** | **The predicted intrinsic hepatic clearance $Cl_{int}$ (mL/min/kg)** | **The hepatic extraction ratio (E)** |
|---|---|---|---|---|---|---|
| | Human | 10.37 | 133.64 | 167.61 | 18.42 | 0.89 |
| | Monkey | 3.99 | 347.37 | 521.05 | 40.23 | 0.92 |
| Verapamil | Canine | 14.46 | 95.82 | 238.86 | 27.36 | 0.89 |
| | Rat | 7.27 | 190.53 | 341.43 | 47.52 | 0.86 |
| | Mouse | 5.04 | 274.89 | 1209.52 | 83.77 | 0.93 |
| | Human | 2.25 | 616.94 | 773.75 | 20.16 | 0.97 |
| | Monkey | 1.72 | 805.59 | 1208.38 | 42.08 | 0.97 |
| 7-hydroxycoumarin | Canine | 1.68 | 823.77 | 2053.50 | 30.44 | 0.99 |
| | Rat | 2.47 | 561.58 | 1006.34 | 52.33 | 0.95 |
| | Mouse | 1.68 | 824.13 | 3626.16 | 87.82 | 0.98 |
| | Human | 13.12 | 105.64 | 132.49 | 17.90 | 0.86 |
| | Monkey | 21.21 | 65.35 | 9802 | 30.18 | 0.69 |
| A13bGal | Canine | 47.29 | 29.31 | 73.06 | 21.72 | 0.70 |
| | Rat | 87.68 | 15.81 | 28.33 | 18.72 | 0.34 |
| | Mouse | 100.52 | 13.79 | 60.67 | 36.24 | 0.40 |

The above preliminary pharmacological results suggest that the prodrug A13bGal has certain stability and good pharmacological properties *in vivo.*

The specific examples described above provide a further detailed description of the objects, technical solutions and beneficial effects of the present invention, and it should be understood that the above description is only a specific example of the present invention, and is not intended to limit the present invention. Any modifications, equivalent substitutions, improvements, and the like made within the spirit and principles of the present invention shall be included in the scope of the present invention.

## Claims

1. An active agent for selectively killing one or more senescent cells or alleviating one or more symptoms associated with an inflammatory disorder, wherein the active agent comprises:

   (i) a β-gal moiety as shown in Formula I,

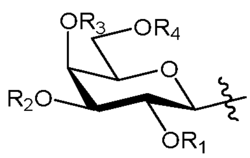

Formula I

   (ii) a cytotoxic moiety as shown in Formula II,

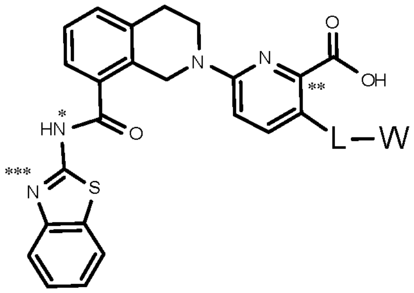

Formula II

(iii) a linker X as shown in any of Formulae (a)-(j):

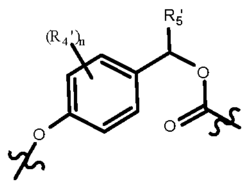
(a)

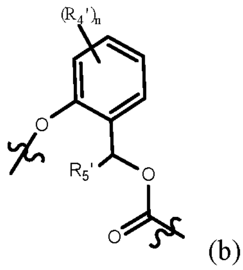
(b)

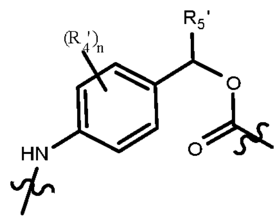
(c)

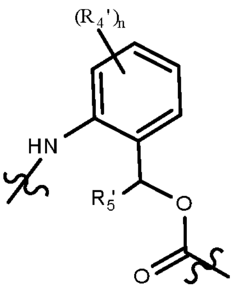
(d)

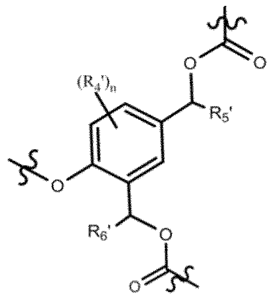
(e)

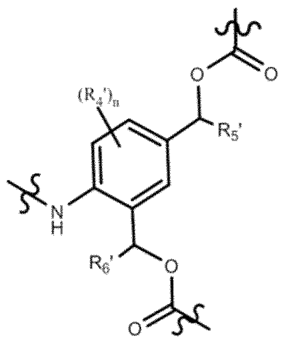
(f)

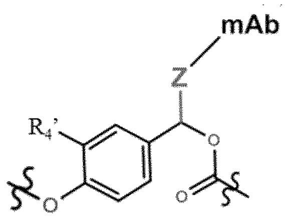
(g)

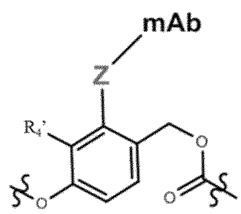
(h)

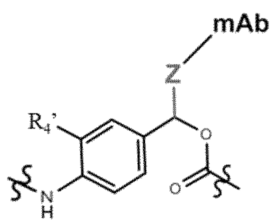
(i)

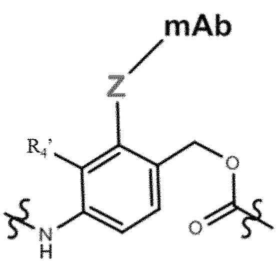
(j)

wherein, one or more of the hydrogen bonded to the N atom at position *, the carboxyl of the C atom at position ** and the hydrogen on W of Formula II are substituted with the linker X, so that the cytotoxic moiety is linked to

the β-gal moiety via the linker X; or the N atom(s) at position * and/or position *** is/are directly linked to the β-gal moiety;
wherein, the linker X is linked to the β-gal moiety at the

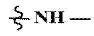

or

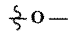

as a linking position, and to the cytotoxic moiety at the

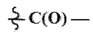

as a linking position,
wherein, in the linker X of Formulae (e) and (f), the cytotoxic moieties linked to the two

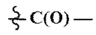

linking positions are the same or different;
wherein,
$R_1$, $R_2$, $R_3$, and $R_4$ are independently selected from H, -C(O)$R_5$, -C(O)O$R_6$, -C(O)N$R_7R_8$, and -$PO_3R_9$, wherein $R_5$, $R_6$, $R_7$, $R_8$, and $R_9$ are independently selected from hydrogen, substituted or unsubstituted alkyl, substituted or unsubstituted aryl, substituted or unsubstituted heteroaryl, substituted or unsubstituted cyclic group, and substituted or unsubstituted heterocyclic group;
Each of $R_4$', $R_5$' and $R_6$' are independently selected from hydrogen, nitro, cyano, isocyano, amino, hydroxy, thiol, halogen, substituted or unsubstituted alkyl, substituted or unsubstituted alkoxyl, alkylamino, dialkylamino, carboxy, substituted or unsubstituted carbonyl, acylamino, sulfonyl, sulfo, phosphoryl, and phosphono;
n is 0, 1, 2, 3 or 4;
mAb is a monoclonal antibody and Z is a linking group for the antibody conjugation;
L is a linking group selected from substituted or unsubstituted alkyl, substituted or unsubstituted alkoxyl, substituted or unsubstituted aryl, substituted or unsubstituted heteroaryl, a substituted or unsubstituted cyclic group and a substituted or unsubstituted heterocyclic group;
L is preferably a linking group selected from the following structures:

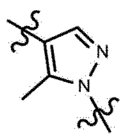 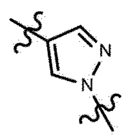 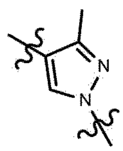

which is linked to the W group at the nitrogen atom in the ring; and

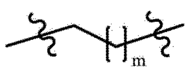 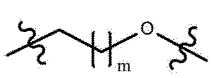 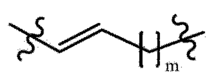 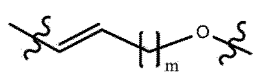

m is 0, 1, 2, or 3;
L is most preferably a linking group selected from the following structures:

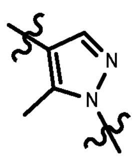 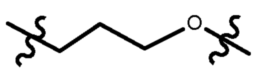 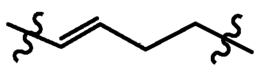

,

and
W is selected from a substituted or unsubstituted cyclic group, a substituted or unsubstituted heterocyclic group, substituted or unsubstituted aryl, substituted or unsubstituted heteroaryl, substituted or unsubstituted bridging cycloalkyl, substituted or unsubstituted hetero-bridging cycloalkyl, substituted or unsubstituted spirocycloalkyl, substituted or unsubstituted hetero-spirocycloalkyl, substituted or unsubstituted fused cycloaryl, and substituted or unsubstituted fused heterocycloaryl;
provided that the hydrogen on W is substituted with the linker X, W has an amino group, a sec-amino group, a cyclic sec-amino group or a hydroxy, and the hydrogen bonded to the N atom or O atom in the amino group, the sec-amino group, the cyclic sec-amino group or the hydroxy is substituted with the linker X.

**2.** The active agent according to claim 1, wherein $R_4$' is independently selected from hydrogen, nitro, cyano, isocyano, amino, hydroxy, thiol and halogen.

**3.** The active agent according to claim 2, wherein $R_4$' is independently selected from hydrogen or nitro.

**4.** The active agent according to claim 1, wherein $R_5$' and $R_6$' are independently hydrogen.

**5.** The active agent according to claim 1, wherein $R_5$' and $R_6$' are independently selected from lysosomal-directed units shown below:

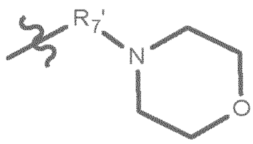

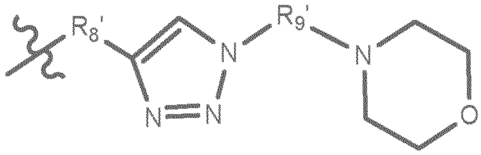

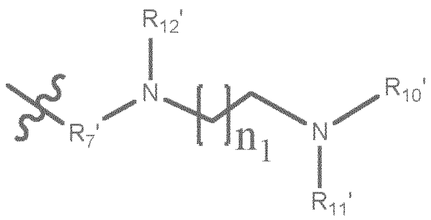

wherein, $R_7$', $R_8$' and $R_9$' are independently absent, alkylene, alkyleneoxyl, alkyleneamino or alkylenecarbonyl; wherein, $R_{10}$', $R_{11}$' and $R_{12}$' are independently selected from hydrogen, nitro, cyano, isocyano, amino, hydroxy, thiol, halogen, substituted or unsubstituted alkyl, substituted or unsubstituted alkoxyl, alkylamino, dialkylamino, carboxy, substituted or unsubstituted carbonyl, acylamino, sulfonyl, sulfo, phosphoryl, and phosphono;
$n_1$ is 0, 1, 2, 3 or 4.

**6.** The active agent according to claim 5, wherein $R_5$' and $R_6$' are independently selected from the following structures:

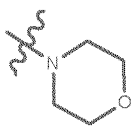 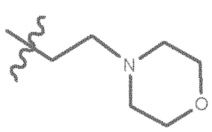 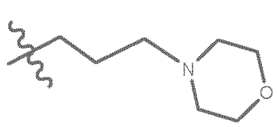 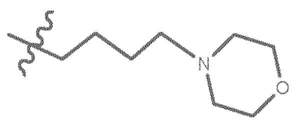

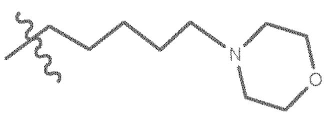 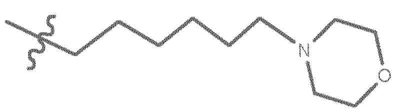

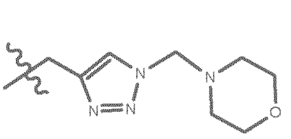 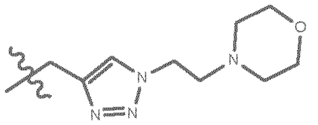 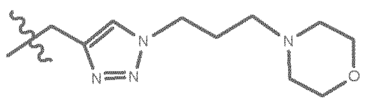

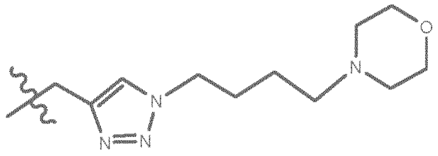

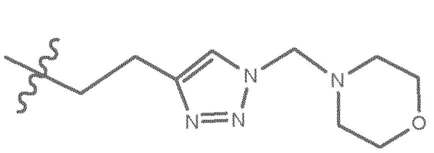 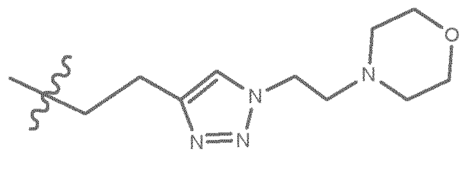

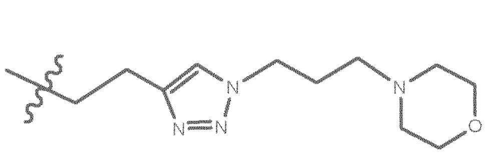 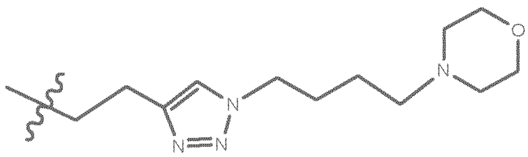

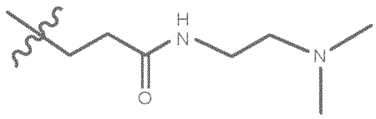 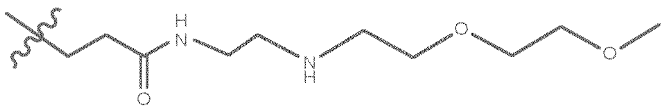

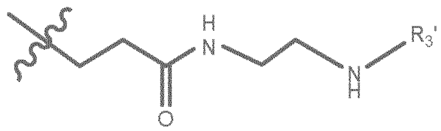 ,

wherein, $R_3$' is substituted or unsubstituted alkyl, substituted or unsubstituted alkylamino, or substituted or unsubstituted alkoxyl.

**7.** The active agent according to any one of claims 1-6, wherein the linker X is independently selected from the following structures:

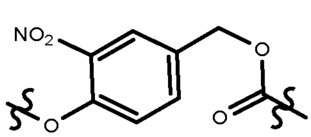 , 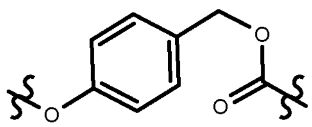 , 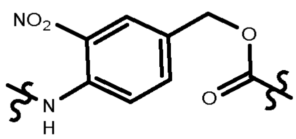 ,

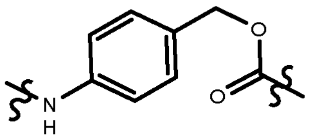 , 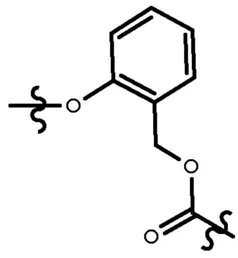 , 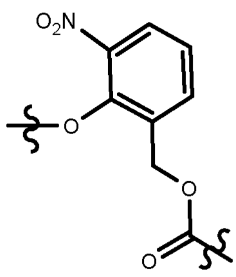 , 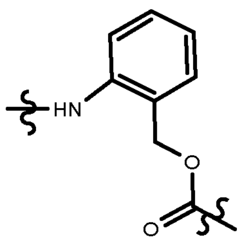 ,

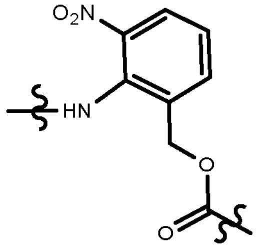 , 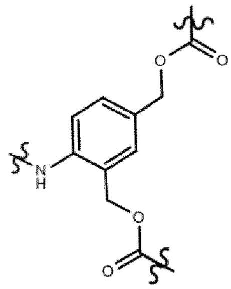 ;

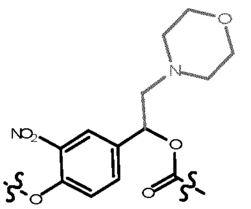 , 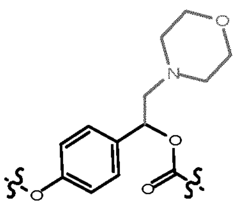 , 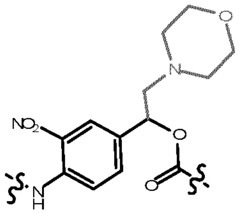 , 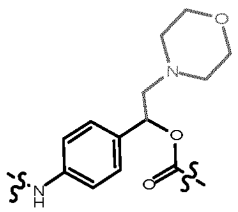 ,

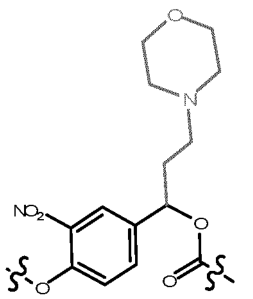 , 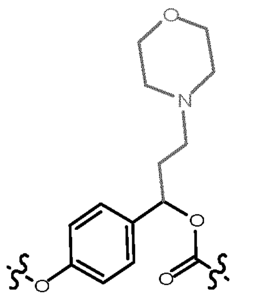 , 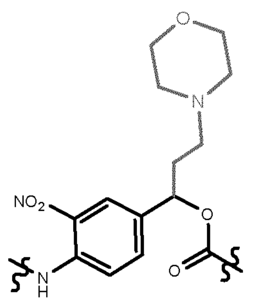 , 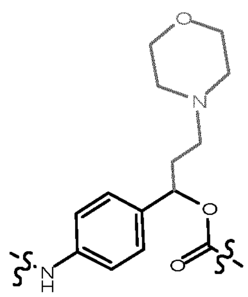 ,

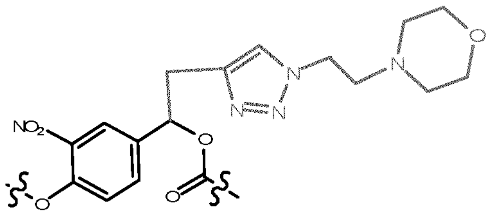 , 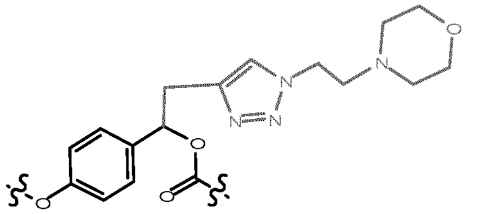 ,

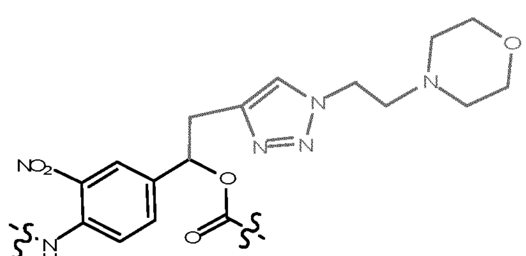 , 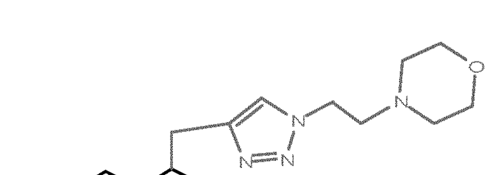 ,

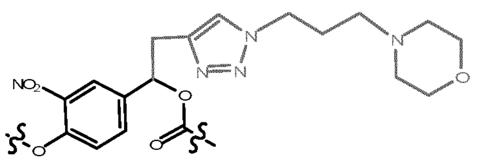 , 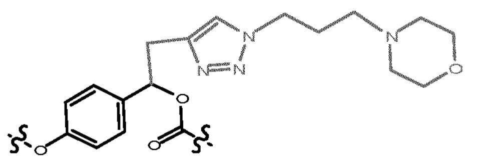 ,

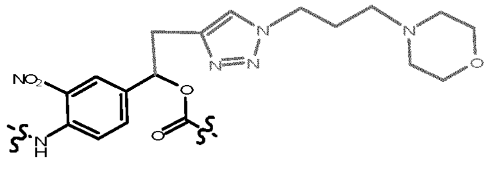

, and

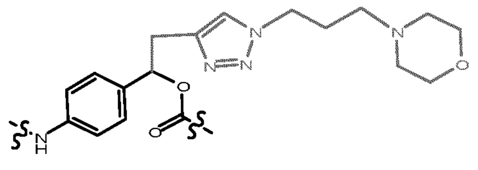

.

8. The active agent according to claim 7, wherein the linker X is independently selected from the following structures:

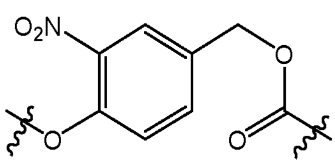

or

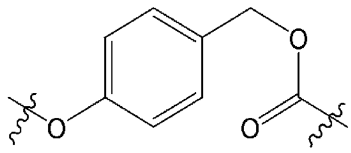

.

9. The active agent according to claim 1, wherein the Z group is selected from a group consisting of:

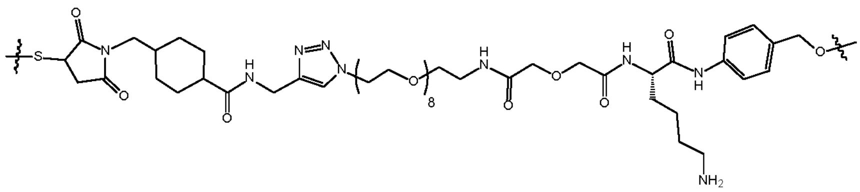

CL2A,

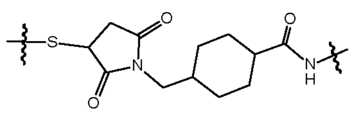

MCC,

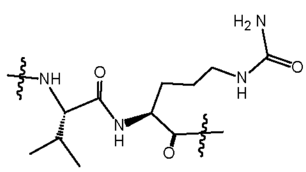

VC,

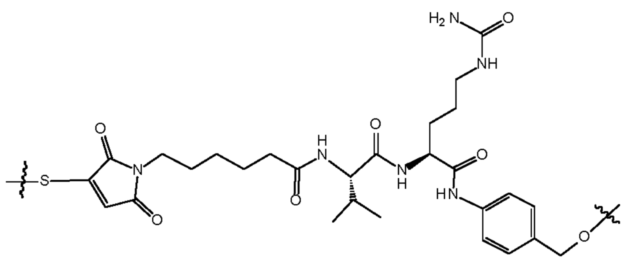

MC-VC-PAB,

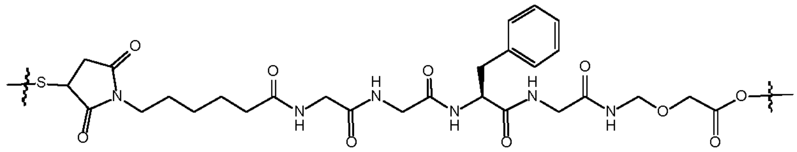

MC-GGFG,

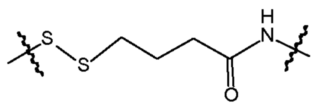

SPDB,

and

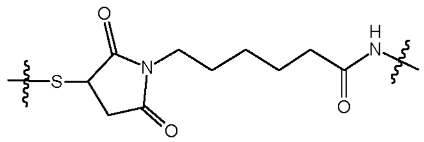

MHH.

**10.** The active agent according to claim 1, wherein $R_1$, $R_2$, $R_3$ and $R_4$ are independently H or -C(O)Rs.

**11.** The active agent according to claim 10, wherein $R_5$ is independently substituted or unsubstituted alkyl.

**12.** The active agent according to claim 1, wherein the β-gal moiety is independently selected from the following structures:

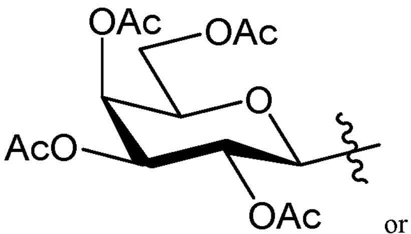 or 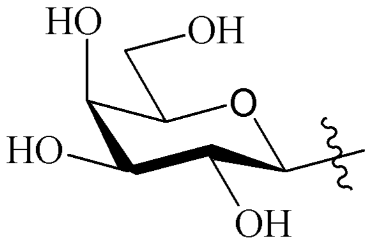 .

**13.** The active agent according to claim 1, wherein W is selected from the following groups:

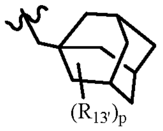 , 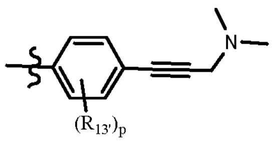 , 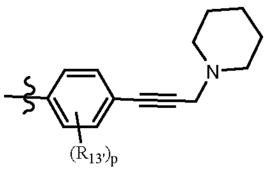 , 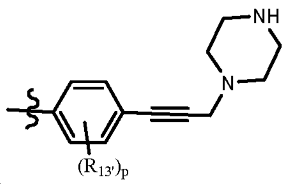 ,

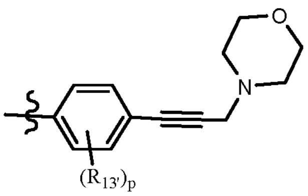 , 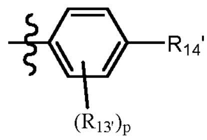 , 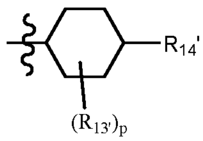 , 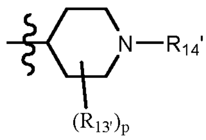 ,

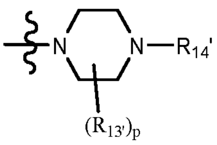 , 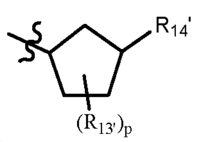 , 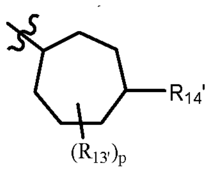 , 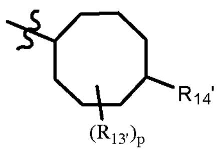

wherein, $R_{13'}$ is hydrogen, deuterium, halogen, amino, hydroxy, nitro, thiol, cyano, isocyano, substituted or unsubstituted alkyl, substituted or unsubstituted alkoxyl, alkylamino, dialkylamino, carboxyl, substituted or unsubstituted carbonyl, acylamino, sulfonyl, sulfo, phosphoryl and phosphono; wherein, $R_{14}$' is hydrogen, deuterium, substituted or unsubstituted alkyl, substituted or unsubstituted aryl, substituted or unsubstituted heteroaryl, a substituted or

unsubstituted cyclic group, and a substituted or unsubstituted heterocyclic group; wherein, p is 0, 1, 2, 3, or 4.

**14.** The active agent according to claim 1, wherein the active agent has a structure selected from:

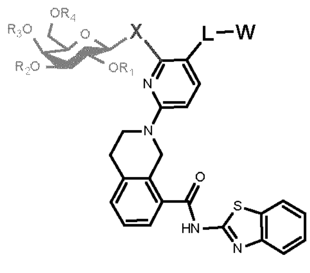
,
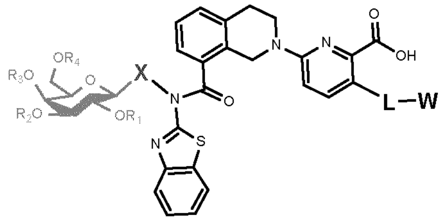
,

a b

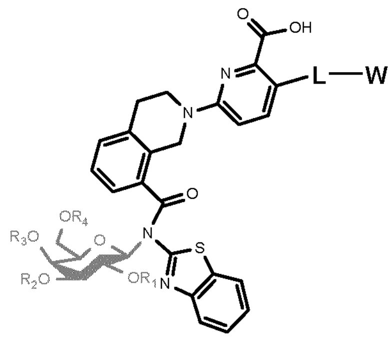
,
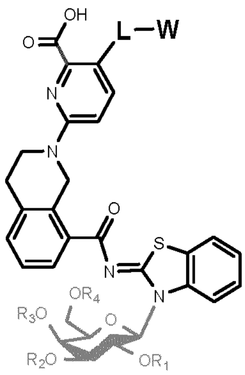
,

c d

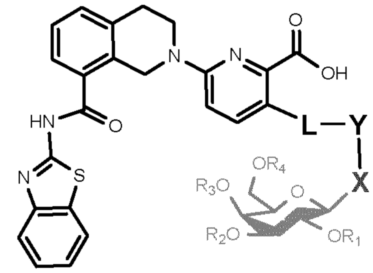
,
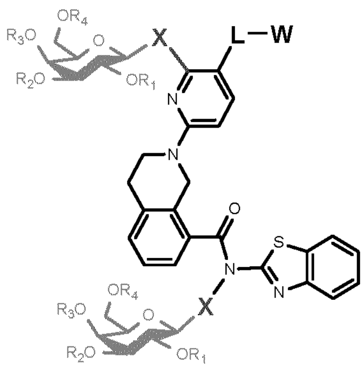
,

e f

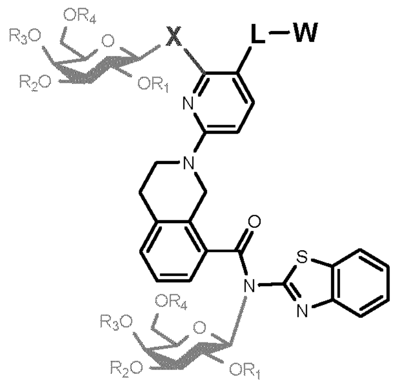

,

g

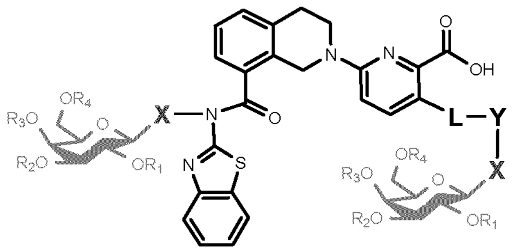

,

h

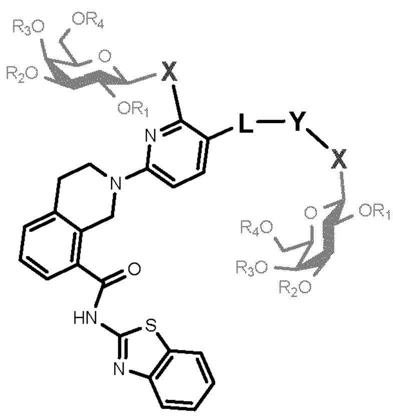

,

i

or

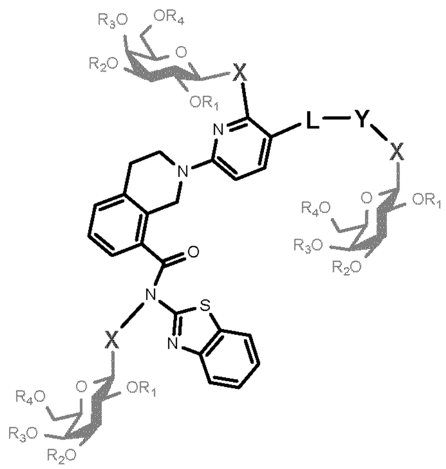

,

j

wherein, Y is W and has an amino group, a sec-amino group, a cyclic sec-amino group, or a hydroxy, and wherein, the hydrogen bonded to the N atom or O atom in the amino group, the sec-amino group, the cyclic sec-amino group or the hydroxy is substituted with the linker X.

**15.** The active agent according to claim 1, the active agent has a structure selected from:

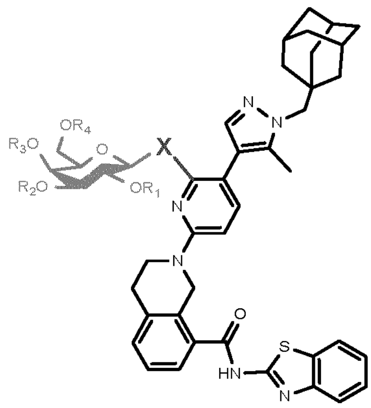

a1

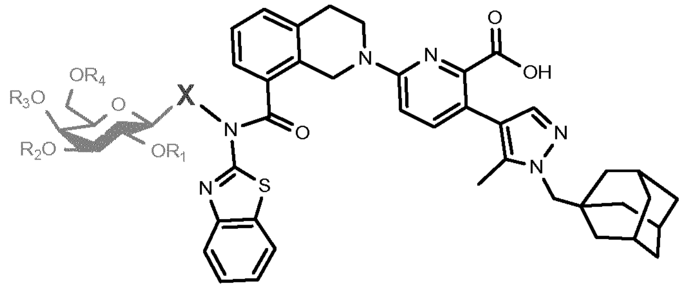

b1

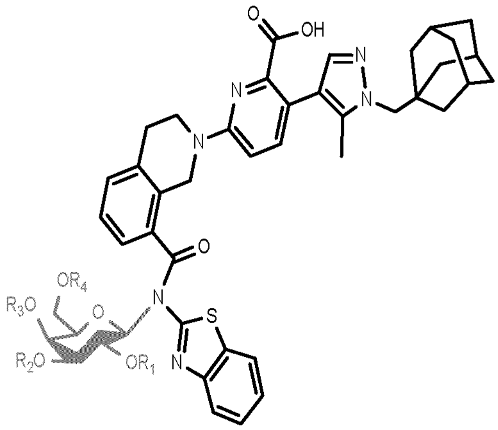

c1

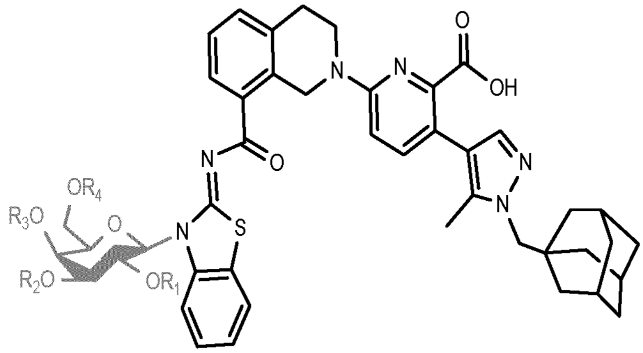

d1

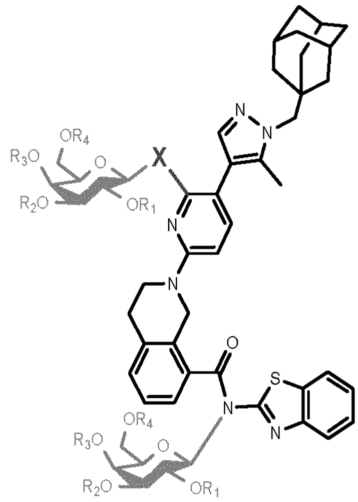

e1

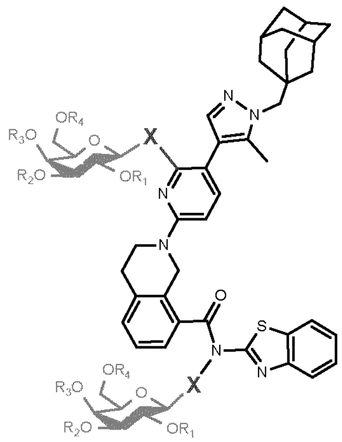

f1.

**16.** The active agent according to claim 14, wherein Y is selected from the following structures:

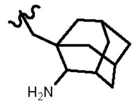, 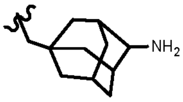, 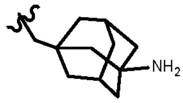, 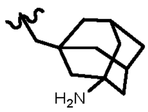, 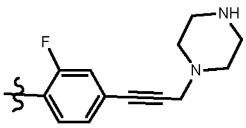,

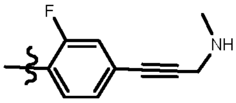, 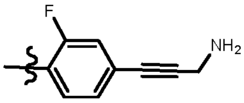, 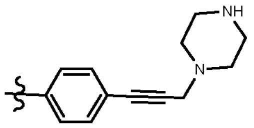, 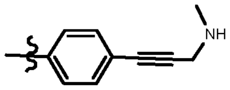,

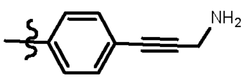, 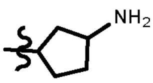, 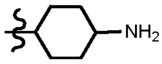, 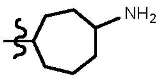, 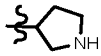, 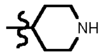,

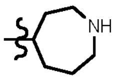, 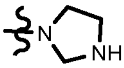, 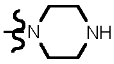, 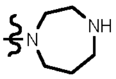, 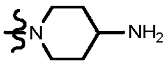, 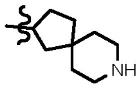, 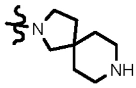.

**17.** The active agent according to claim 1, wherein the active agent is selected from a group consisting of:

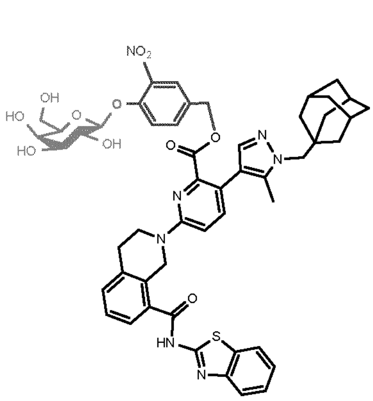, 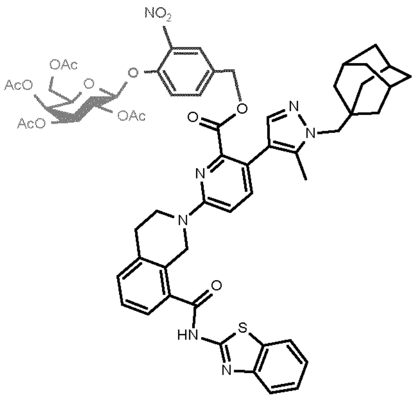,

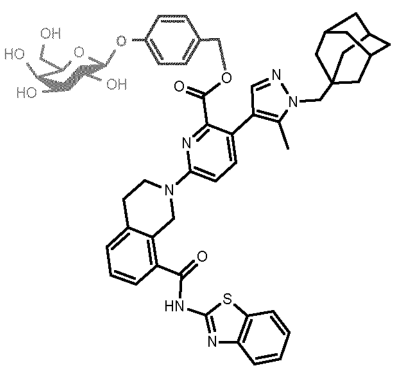, 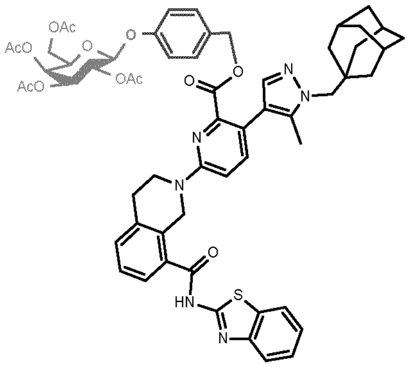,

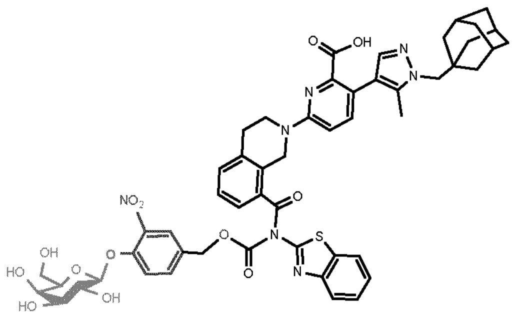
,
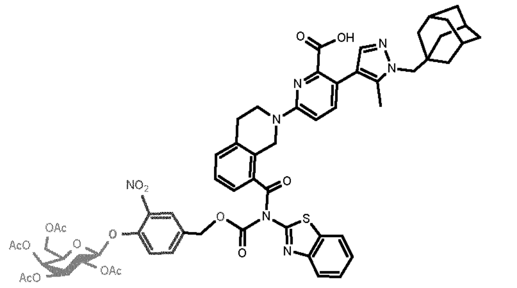
,
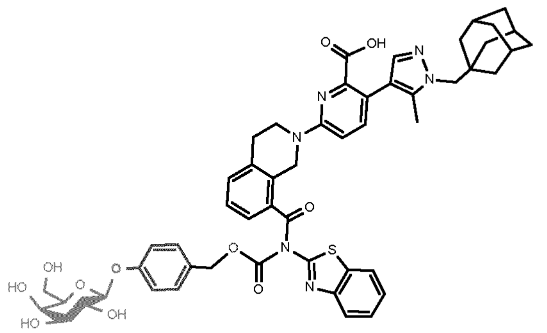
,
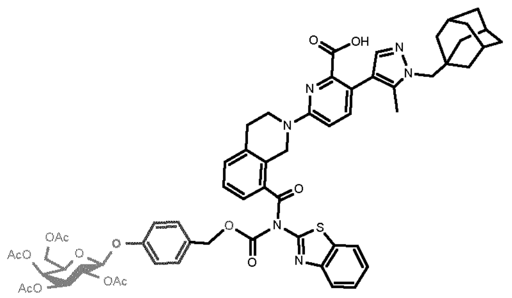
,
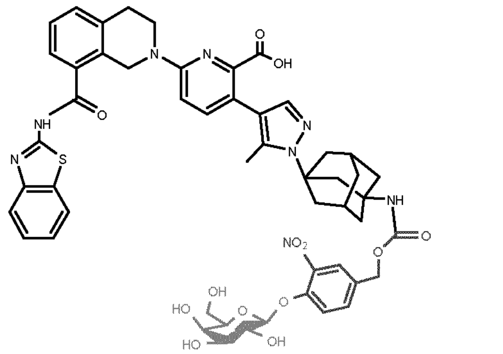
,
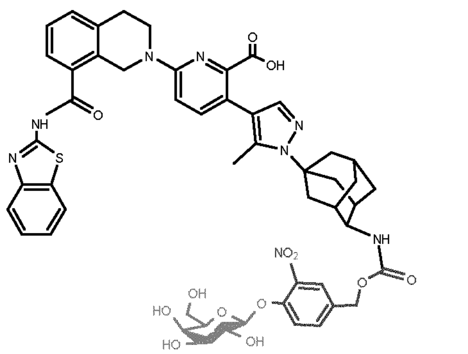
,
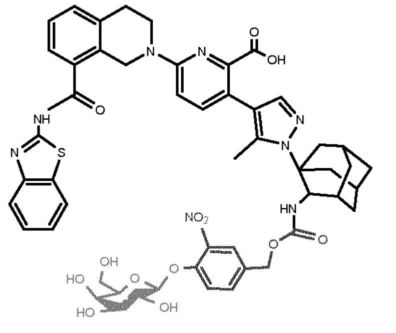
,
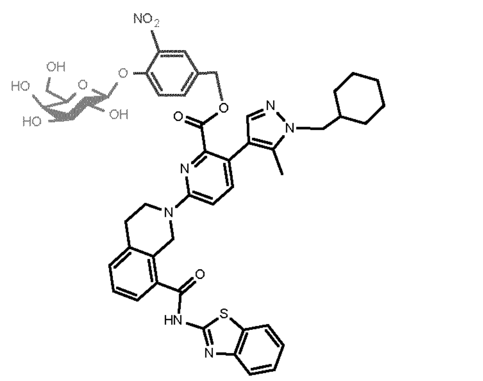
,

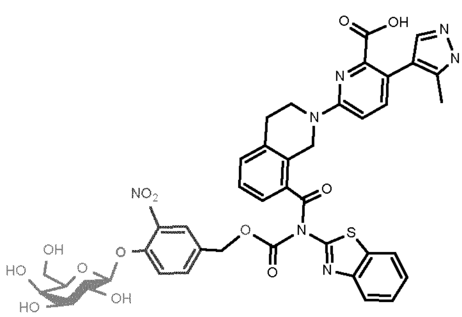

,

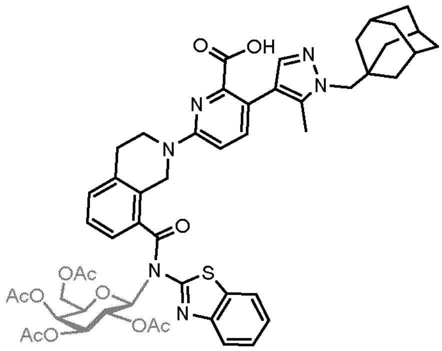

,

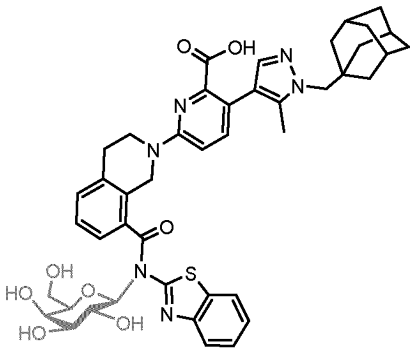

,

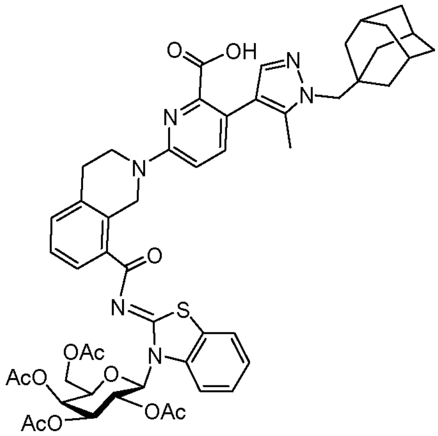

,

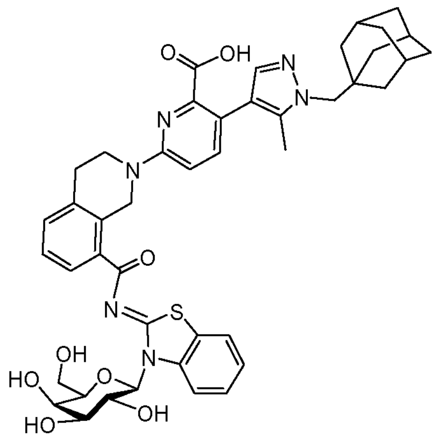

,

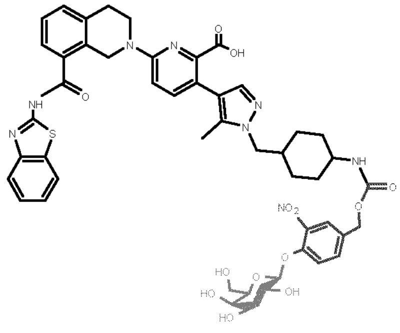

,

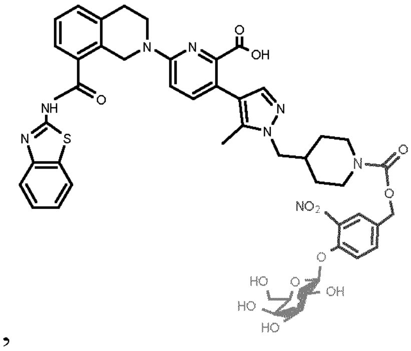

,

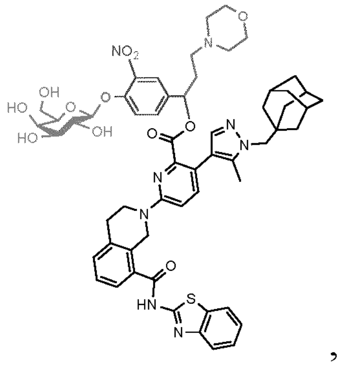

, ,

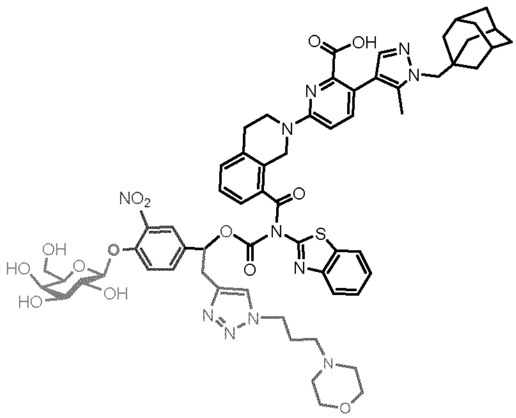

,

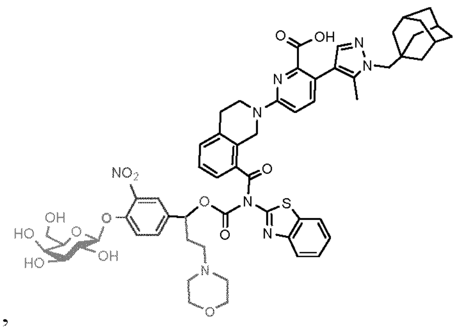

.

**18.** The active agent according to claim 1, wherein, when W comprises a phenyl group substituted with an amino group or a hydroxy, the hydrogen bonded to the N atom or O atom in the amino group or hydroxy is substituted with the β-gal moiety such that the cytotoxic moiety is directly linked to the β-gal moiety without via the linker X.

**19.** The active agent according to claim 18, wherein the active agent is:

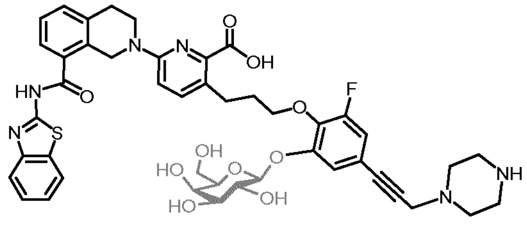

,

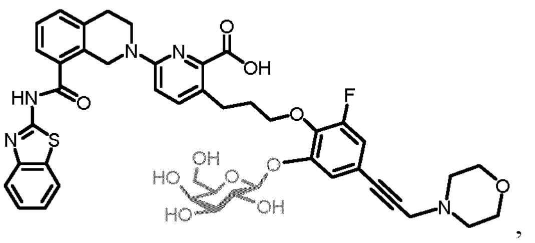

,

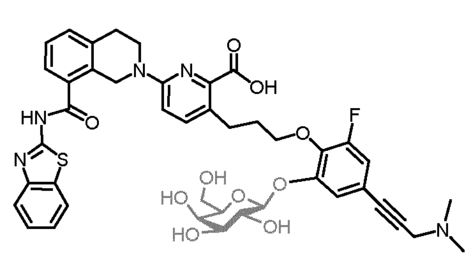,

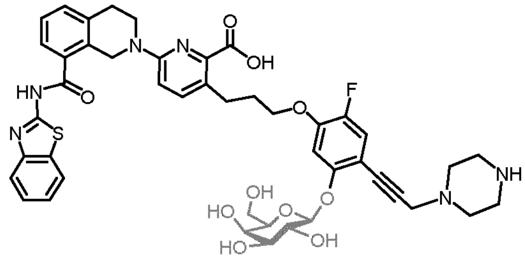,

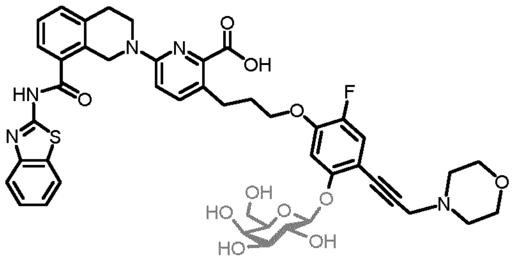,

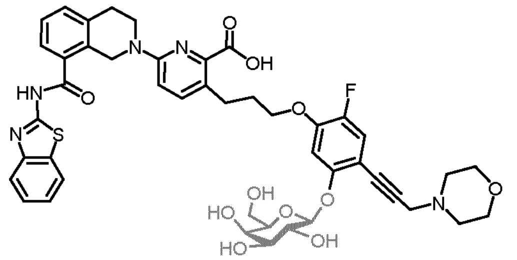,

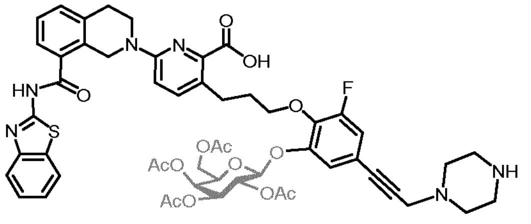,

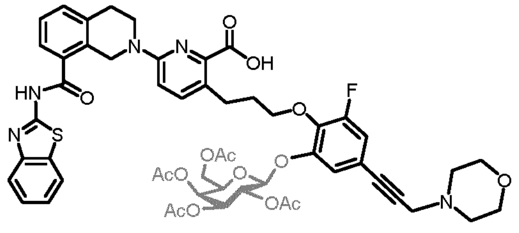,

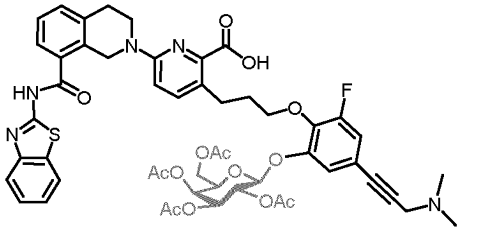,

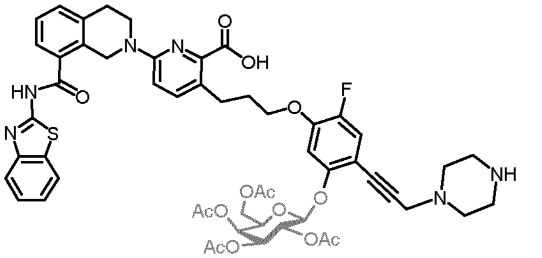,

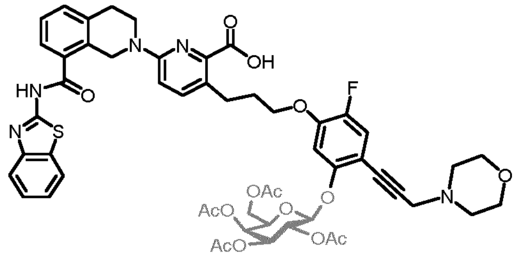,

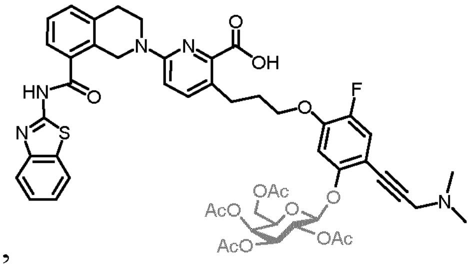,

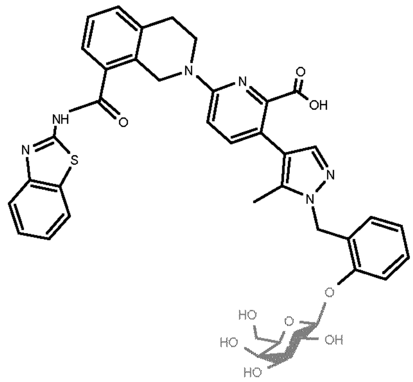
,
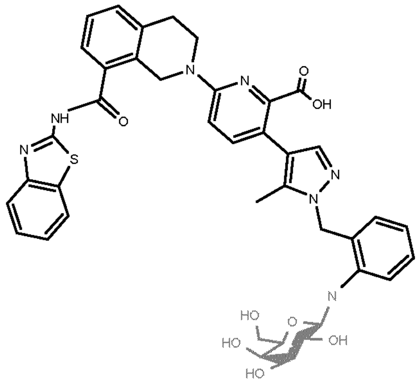
.

**20.** A composition comprising an effective amount of the active agent according to any one of claims 1-19 for killing one or more senescent cells or alleviating one or more symptoms associated with an inflammatory disorder.

**21.** The composition according to claim 20, further comprising a pharmaceutically acceptable carrier.

**22.** A method of selectively killing one or more senescent cells or alleviating one or more symptoms associated with an inflammatory disorder in a subject, the method comprising administering to the subject a therapeutically effective amount of the composition according to claim 20 or 21.

**23.** The method according to claim 22, wherein the subject suffers from an aging-related disease or disorder, or an inflammatory disease or disorder.

**24.** The method according to claim 22 or 23, wherein the aging-related disease or disorder is a metabolic disease, an inflammatory disease or disorder, a pulmonary disease or disorder, a neurological disease or disorder, a proliferative disorder, a renal disease or disorder, an ocular disease or disorder, or a dermatologic disorder or disease.

**25.** The method according to any one of claims 22-24, wherein the aging-related disease or disorder is selected from a group consisting of:

(i) an inflammatory or autoimmune disease or disorder selected from oral mucositis, inflammatory bowel disease, kyphosis, and intervertebral disc herniation;
(ii) a neurological disease or disorder selected from Alzheimer's disease, Parkinson's disease, Huntington's disease, dementia, mild cognitive impairment, macular degeneration, and motor neuron dysfunction;
(iii) a metabolic disease selected from diabetic ulcer, metabolic syndrome and obesity;
(iv) a pulmonary disease selected from pulmonary fibrosis, chronic obstructive pulmonary disease, asthma, cystic fibrosis, emphysema, bronchiectasis and age-related loss of lung function;
(v) an ocular disease or disorder selected from macular degeneration, glaucoma, cataract, presbyopia and loss of vision;
(vi) an age-related disorder selected from renal failure, weakness, hearing loss, muscle fatigue, skin condition, skin wound healing, hepatic fibrosis, pancreatic fibrosis, oral submucous fibrosis, and sarcopenia; and
(vii) a skin disease or disorder selected from eczema, psoriasis, hyperpigmentation, nevus, rash, atopic dermatitis, urticaria, a disease and disorder associated with photosensitivity or photoaging, wrinkle, pruritus, dysesthesia, eczematous rash, eosinophilic dermatosis, reactive neutrophilic dermatosis, pemphigus, pemphigoid, immune bullous dermatosis, cutaneous fibrohistiocytic hyperplasia, cutaneous lymphomas, and cutaneous lupus.

**26.** The method according to claim 25, wherein the subject is diagnosed with cancer and optionally is undergoing a cancer treatment selected from a chemotherapy and a radiotherapy.

**27.** The method according to claim 26, wherein the active agent

(i) reduces the cells that have been determined to be senescent or cancer cells;
(ii) reduces one or more side effects produced by senescent cells, wherein the side effects include an inflammation, promotion of cancer growth and metastasis;

(iii) reduces one or more side effects of a chemotherapy; or
(iv) reduces one or more side effects of a radiotherapy.

**28.** The method according to any one of claims 22-27, wherein the cytotoxic moiety is cleaved after *in vivo* delivery.

**29.** The method according to any one of claims 22-28, wherein the composition produces the following structure in an effective amount of killing one or more senescent cells after *in vivo* administration:

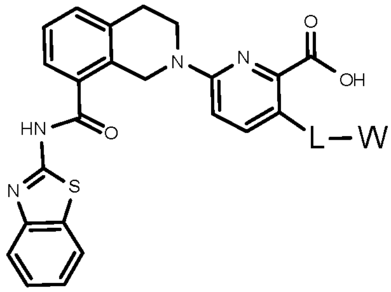

wherein, the linking group L is selected from substituted or unsubstituted alkyl, substituted or unsubstituted alkoxyl, substituted or unsubstituted aryl, substituted or unsubstituted heteroaryl, a substituted or unsubstituted cyclic group, and a substituted or unsubstituted heterocyclic group;
L is preferably a linking group selected from the following structures:

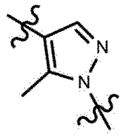 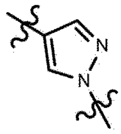 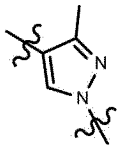

which is linked to the W group at the nitrogen atom in the ring; and

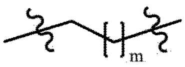 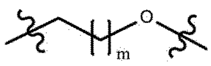 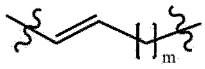 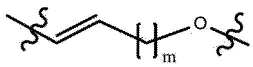

m is 0, 1, 2, or 3;
L is most preferably a linking group selected from the following structures:

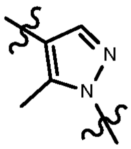 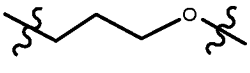 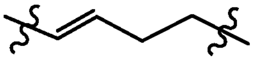

W is selected from a substituted or unsubstituted cyclic group, a substituted or unsubstituted heterocyclic group, substituted or unsubstituted aryl, substituted or unsubstituted heteroaryl, substituted or unsubstituted bridging cycloalkyl, substituted or unsubstituted hetero-bridging cycloalkyl, substituted or unsubstituted spirocycloalkyl, substituted or unsubstituted hetero-spirocycloalkyl, substituted or unsubstituted fused ring aryl, and substituted or unsubstituted hetero fused ring aryl.

**30.** The method according to claim 22 or 23, wherein the subject has a viral infection, optionally, wherein the viral infection causes an overproduction of activated macrophages in the subject.

**31.** The method according to claim 30, wherein the viral infection is a coronavirus infection selected from a group consisting of a coronaviruse, Severe Acute Respiratory Syndrome (SARS) coronavirus (CoV), SARS-CoV-2 (which causes COVID-19 (coronavirus disease) 2019)) and Middle East Respiratory Syndrome (MERS)-CoV.

**32.** The method according to claim 31, wherein the subject has a SARS-CoV-2 infection and has been diagnosed with

coronavirus disease 2019.

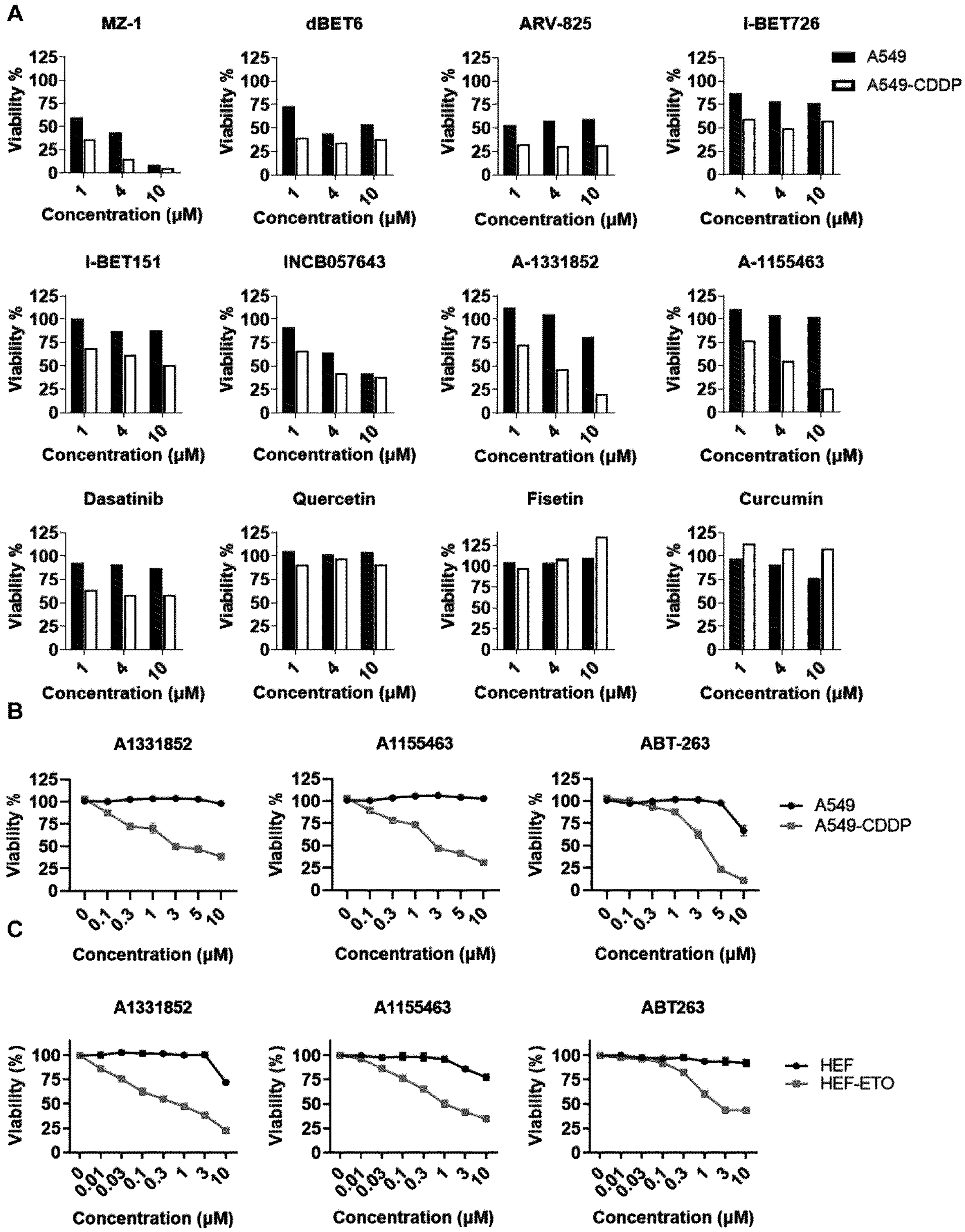
A
MZ-1
dBET6
ARV-825
I-BET726
A549
A549-CDDP
I-BET151
INCB057643
A-1331852
A-1155463
Dasatinib
Quercetin
Fisetin
Curcumin
Viability %
Concentration (µM)
B
A1331852
A1155463
ABT-263
A549
A549-CDDP
C
A1331852
A1155463
ABT263
Viability (%)
HEF
HEF-ETO

Fig. 1

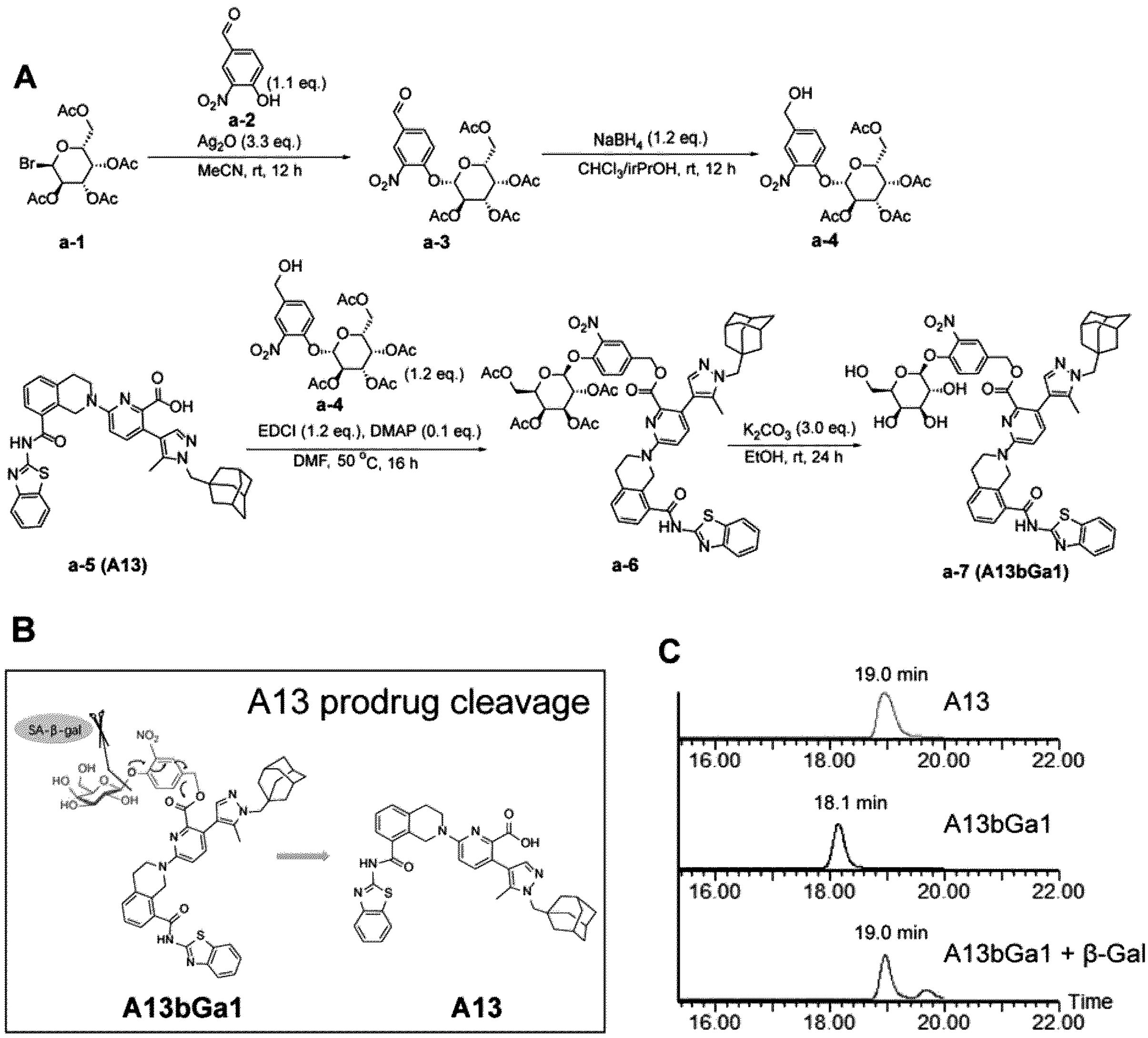
A
a-2 (1.1 eq.)
Ag2O (3.3 eq.)
MeCN, rt, 12 h
NaBH4 (1.2 eq.)
CHCl3/irPrOH, rt, 12 h
a-1
a-3
a-4
a-4 (1.2 eq.)
EDCI (1.2 eq.), DMAP (0.1 eq.)
DMF, 50 °C, 16 h
K2CO3 (3.0 eq.)
EtOH, rt, 24 h
a-5 (A13)
a-6
a-7 (A13bGa1)
B
A13 prodrug cleavage
SA-β-gal
A13bGa1
A13
C
19.0 min
A13
18.1 min
A13bGa1
19.0 min
A13bGa1 + β-Gal
Time
16.00
18.00
20.00
22.00

Fig. 2

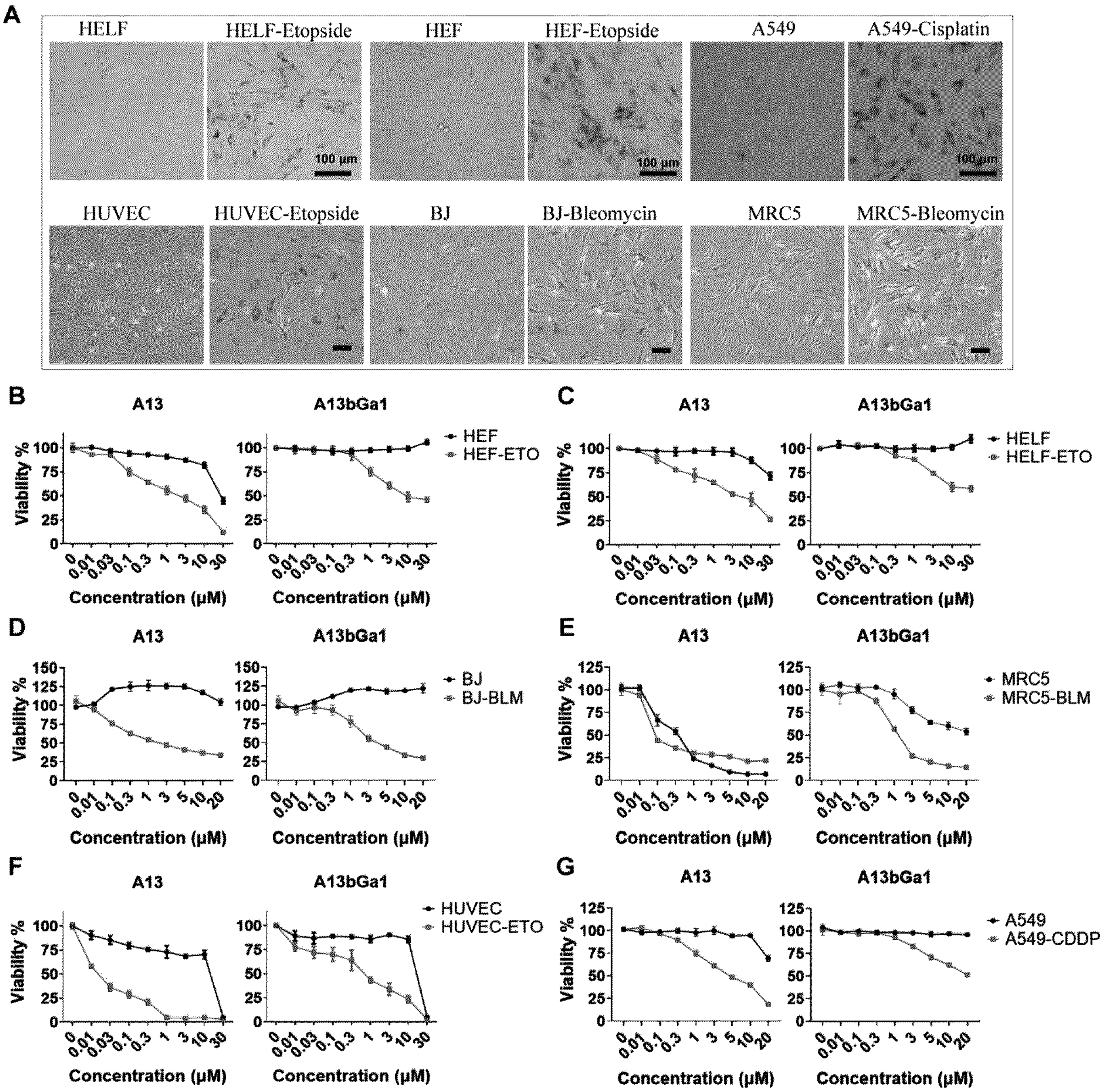
A
HELF
HELF-Etopside
HEF
HEF-Etopside
A549
A549-Cisplatin
100 μm
HUVEC
HUVEC-Etopside
BJ
BJ-Bleomycin
MRC5
MRC5-Bleomycin
B
A13
A13bGa1
HEF
HEF-ETO
C
HELF
HELF-ETO
D
BJ
BJ-BLM
E
MRC5
MRC5-BLM
F
HUVEC
HUVEC-ETO
G
A549
A549-CDDP
Viability %
Concentration (μM)

Fig. 3

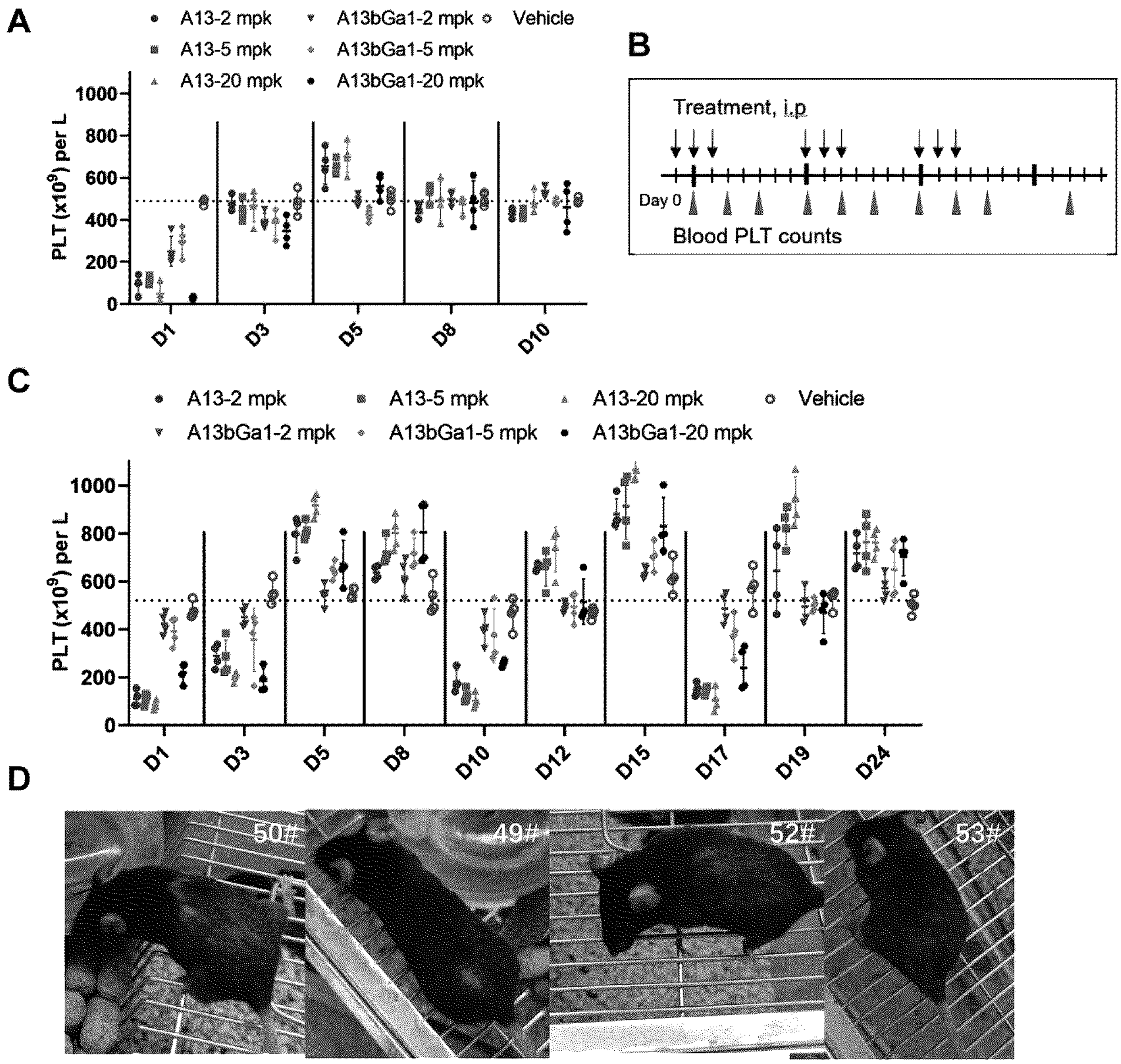
A
A13-2 mpk
A13-5 mpk
A13-20 mpk
A13bGa1-2 mpk
A13bGa1-5 mpk
A13bGa1-20 mpk
Vehicle
PLT (x10⁹) per L
D1
D3
D5
D8
D10
B
Treatment, i.p
Day 0
Blood PLT counts
C
D12
D15
D17
D19
D24
D
50#
49#
52#
53#

Fig. 4

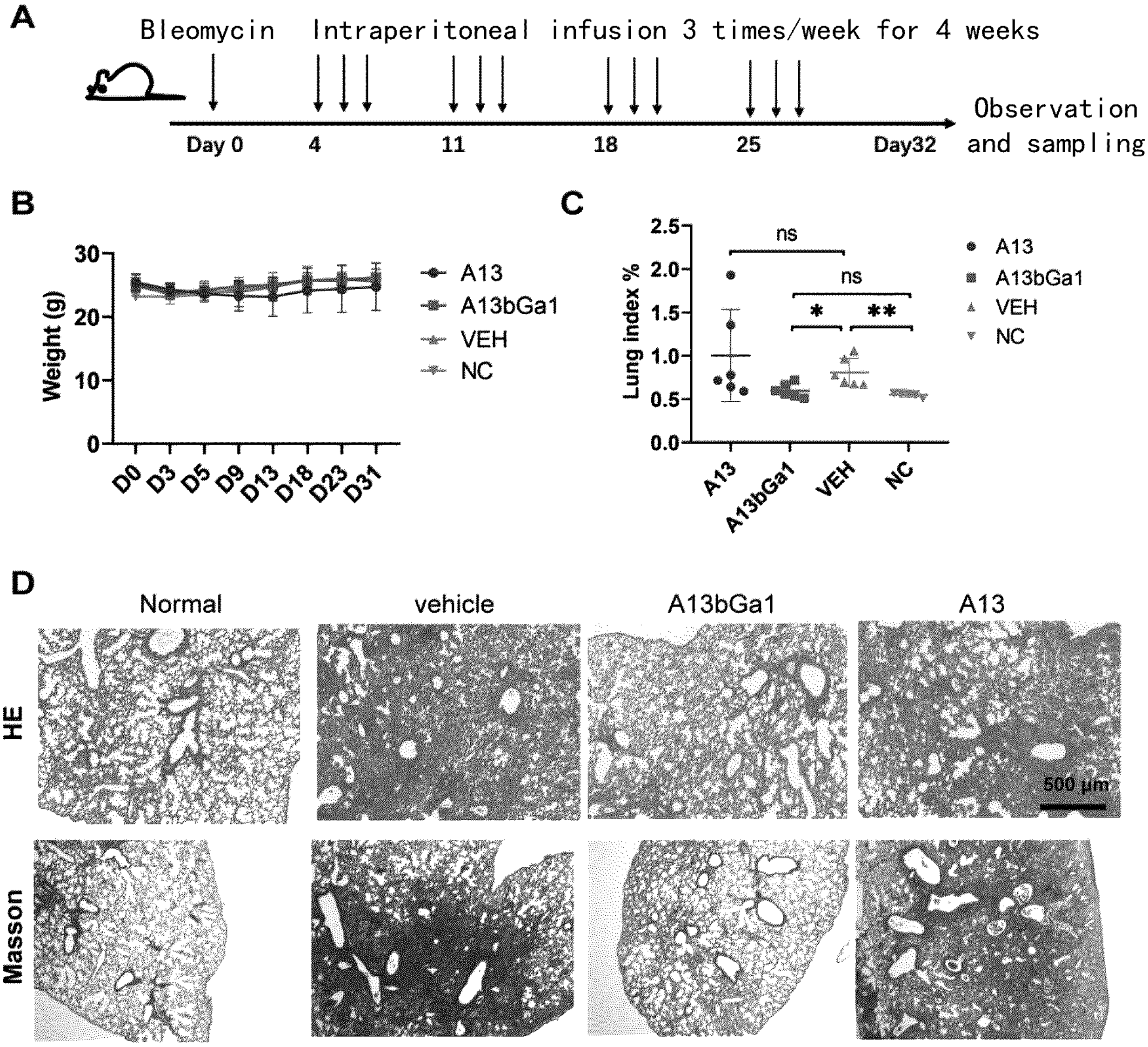
A
Bleomycin
Intraperitoneal infusion 3 times/week for 4 weeks
Day 0
4
11
18
25
Day32
Observation and sampling
B
Weight (g)
A13
A13bGa1
VEH
NC
D0
D3
D5
D9
D13
D18
D23
D31
C
Lung index %
ns
ns
*
**
A13
A13bGa1
VEH
NC
D
Normal
vehicle
A13bGa1
A13
HE
Masson
500 μm

Fig. 5

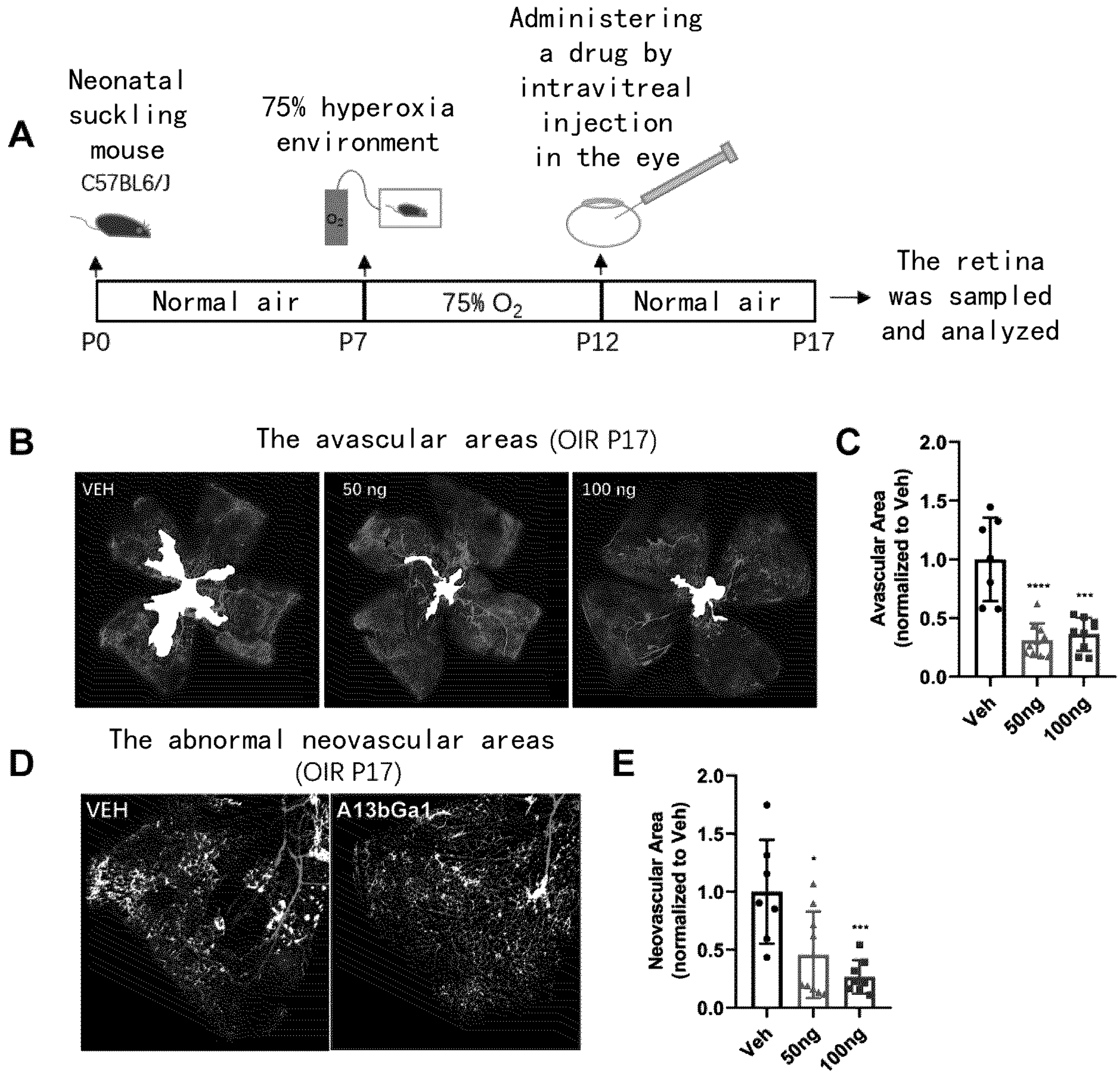
A
Neonatal suckling mouse
C57BL6/J
75% hyperoxia environment
Administering a drug by intravitreal injection in the eye
Normal air
75% O2
Normal air
P0
P7
P12
P17
The retina was sampled and analyzed
B
The avascular areas (OIR P17)
VEH
50 ng
100 ng
C
Avascular Area (normalized to Veh)
2.0
1.5
1.0
0.5
0.0
****
***
Veh
50ng
100ng
D
The abnormal neovascular areas (OIR P17)
VEH
A13bGa1
E
Neovascular Area (normalized to Veh)
2.0
1.5
1.0
0.5
0.0
*
***
Veh
50ng
100ng

Fig. 6

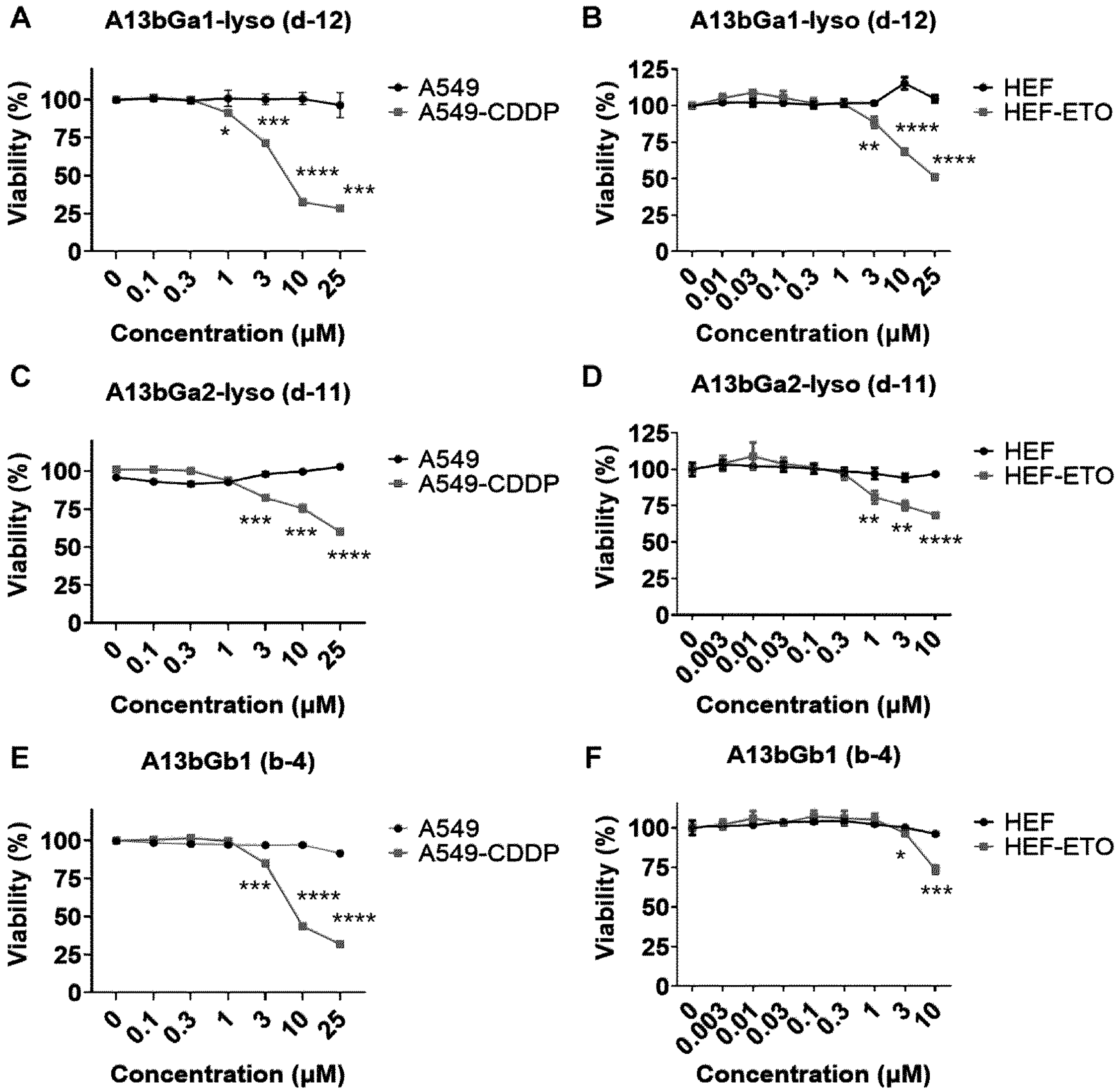
A
A13bGa1-lyso (d-12)
Viability (%)
Concentration (μM)
A549
A549-CDDP
B
A13bGa1-lyso (d-12)
HEF
HEF-ETO
C
A13bGa2-lyso (d-11)
A549
A549-CDDP
D
A13bGa2-lyso (d-11)
HEF
HEF-ETO
E
A13bGb1 (b-4)
A549
A549-CDDP
F
A13bGb1 (b-4)
HEF
HEF-ETO

Fig. 7

## INTERNATIONAL SEARCH REPORT

International application No.
**PCT/CN2023/083593**

### A. CLASSIFICATION OF SUBJECT MATTER

A61K47/68(2017.01)i； A61K31/4725(2006.01)i； A61P43/00(2006.01)i； A61P11/00(2006.01)i； A61P35/00(2006.01)i

According to International Patent Classification (IPC) or to both national classification and IPC

### B. FIELDS SEARCHED

Minimum documentation searched (classification system followed by classification symbols)

IPC： A61K A61PC07K C07D

Documentation searched other than minimum documentation to the extent that such documents are included in the fields searched

Electronic data base consulted during the international search (name of data base and, where practicable, search terms used)

Databases: DWPI; CNABS; CNTXT; ENTXTC; WPABSC; VEN; CNKI; PUBMED; STN; 读秀, DUXIU Keywords: 结构检索, structure search, A1331852, A1155463, Bcl-2, Bcl-XL, 半乳糖, 前药, ADC, 衰老细胞, 炎性, 癌症, 肺纤维化, 视网膜, b-gal, galactose, prodrug, senescent cell, inflammatory, cancer, lung Fibrosis, Retina

### C. DOCUMENTS CONSIDERED TO BE RELEVANT

| Category* | Citation of document, with indication, where appropriate, of the relevant passages | Relevant to claim No. |
|---|---|---|
| A | CN 107223123 A (ABBVIE INC.) 29 September 2017 (2017-09-29)<br>claims 1-82 | 1-32 |
| A | CN 109963870 A (ABBVIE INC.) 02 July 2019 (2019-07-02)<br>claims 49-118, description, page 195 paragraph [1241] | 1-32 |
| A | CN 109562170 A (ABBVIE INC.) 02 April 2019 (2019-04-02)<br>claims 42-115 | 1-32 |
| A | CN 102316733 A (GENENTECH, INC.; WALTER AND ELIZA HALL INSTITUTE OF MEDICAL RESEARCH; ABBOTT LABORATORIES) 11 January 2012 (2012-01-11)<br>abstract, and description, paragraphs [0150]-[0151] | 1-32 |
| A | CN 112469697 A (RUBEDO LIFE SCIENCES, INC.) 09 March 2021 (2021-03-09)<br>description, paragraph [0005] | 1-32 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25.6月2023(25.06.2023)** | **06 July 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/083593**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **22-32**
because they relate to subject matter not required to be searched by this Authority, namely:

Claims 22-32 relate to a method for selectively killing one or more senescent cells or alleviating symptoms associated with inflammatory conditions in a subject, the method comprising administering to a subject a composition, which method falls within methods for treatment of diseases, and falls within the cases set out in PCT Rule 39.1(iv) for which a search is not required. The following opinion is provided with regard to the use of the composition corresponding to claims 22-32 for preparing a corresponding drug.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/083593**

| Patent document cited in search report | | | Publication date (day/month/year) | Patent family member(s) | | | Publication date (day/month/year) |
|---|---|---|---|---|---|---|---|
| CN | 107223123 | A | 29 September 2017 | US | 2020246460 | A1 | 06 August 2020 |
| | | | | SG | 10201911585 | XA | 30 January 2020 |
| | | | | AU | 2020210218 | A1 | 20 August 2020 |
| | | | | AU | 2015360613 | A1 | 29 June 2017 |
| | | | | IL | 252799 | A0 | 31 August 2017 |
| | | | | WO | 2016094509 | A1 | 16 June 2016 |
| | | | | US | 2016339117 | A1 | 24 November 2016 |
| | | | | SG | 11201704710 | PA | 28 July 2017 |
| | | | | RU | 2020123953 | A | 18 September 2020 |
| | | | | KR | 20170093943 | A | 16 August 2017 |
| | | | | RU | 2017123942 | A | 11 January 2019 |
| | | | | RU | 2017123942 | A3 | 20 June 2019 |
| | | | | JP | 2020143062 | A | 10 September 2020 |
| | | | | MX | 2017007629 | A | 17 May 2018 |
| | | | | IL | 268712 | A | 31 October 2019 |
| | | | | US | 2019142941 | A1 | 16 May 2019 |
| | | | | BR | 112017012351 | A2 | 27 February 2018 |
| | | | | EP | 3230282 | A1 | 18 October 2017 |
| | | | | JP | 2018508463 | A | 29 March 2018 |
| | | | | CA | 2970155 | A1 | 16 June 2016 |
| | | | | IL | 282594 | A | 30 June 2021 |
| CN | 109963870 | A | 02 July 2019 | JP | 2021006527 | A | 21 January 2021 |
| | | | | PE | 20190177 | A1 | 01 February 2019 |
| | | | | DK | 3458479 | T3 | 08 February 2021 |
| | | | | BR | 112018075626 | A2 | 19 March 2019 |
| | | | | UA | 124198 | C2 | 04 August 2021 |
| | | | | RS | 61828 | B1 | 30 June 2021 |
| | | | | CL | 2018003520 | A1 | 15 March 2019 |
| | | | | PL | 3458479 | T4 | 26 July 2021 |
| | | | | AU | 2017279550 | A1 | 03 January 2019 |
| | | | | KR | 20190015755 | A | 14 February 2019 |
| | | | | KR | 102434626 | B1 | 24 August 2022 |
| | | | | DOP | 2018000276 | A | 31 December 2018 |
| | | | | SG | 10201914119 | TA | 27 February 2020 |
| | | | | MX | 2018015271 | A | 12 August 2019 |
| | | | | CR | 20180603 | A | 29 July 2019 |
| | | | | SG | 11201811193 | TA | 30 January 2019 |
| | | | | CL | 2019002250 | A1 | 25 October 2019 |
| | | | | IL | 263600 | A | 31 January 2019 |
| | | | | US | 2017355769 | A1 | 14 December 2017 |
| | | | | US | 10640563 | B2 | 05 May 2020 |
| | | | | WO | 2017214335 | A1 | 14 December 2017 |
| | | | | WO | 2017214335 | A4 | 08 March 2018 |
| | | | | LT | 3458479 | T | 25 February 2021 |
| | | | | PT | 3458479 | T | 01 March 2021 |
| | | | | ZA | 201900059 | B | 30 November 2022 |
| | | | | JP | 2019528240 | A | 10 October 2019 |
| | | | | JP | 6751165 | B2 | 02 September 2020 |
| | | | | CA | 3027045 | A1 | 14 December 2017 |
| | | | | PH | 12018502601 | A1 | 30 September 2019 |

Form PCT/ISA/210 (patent family annex) (July 2022)

**INTERNATIONAL SEARCH REPORT**
**Information on patent family members**

International application No.
**PCT/CN2023/083593**

| Patent document cited in search report | Publication date (day/month/year) | Patent family member(s) | Publication date (day/month/year) |
|---|---|---|---|
| | | ECSP 19000282 A | 31 January 2019 |
| | | HUE 053356 T2 | 28 June 2021 |
| | | US 2021171637 A1 | 10 June 2021 |
| | | SI 3458479 T1 | 31 August 2021 |
| | | HRP 20210170 T1 | 19 March 2021 |
| | | UY 37278 A | 31 January 2018 |
| | | ES 2861499 T3 | 06 October 2021 |
| | | TW 201809003 A | 16 March 2018 |
| | | TWI 762487 B | 01 May 2022 |
| | | CO 2018013471 A2 | 28 December 2018 |
| | | EP 3458479 A1 | 27 March 2019 |
| | | EP 3458479 B1 | 04 November 2020 |
| | | EP 3835322 A2 | 16 June 2021 |
| | | EP 3835322 A3 | 06 October 2021 |
| | | RU 2018146948 A | 14 July 2020 |
| | | RU 2018146948 A3 | 17 June 2021 |
| | | RU 2764651 C2 | 19 January 2022 |
| CN 109562170 A | 02 April 2019 | AU 2017277920 A1 | 03 January 2019 |
| | | US 2023135723 A1 | 04 May 2023 |
| | | BR 112018075651 A2 | 09 April 2019 |
| | | WO 2017214462 A2 | 14 December 2017 |
| | | WO 2017214462 A3 | 18 January 2018 |
| | | WO 2017214462 A4 | 05 April 2018 |
| | | JP 2019524649 A | 05 September 2019 |
| | | EP 3468596 A2 | 17 April 2019 |
| | | CA 3027047 A1 | 14 December 2017 |
| | | MX 2018015268 A | 12 August 2019 |
| | | US 2020121803 A1 | 23 April 2020 |
| CN 102316733 A | 11 January 2012 | EP 2373163 A1 | 12 October 2011 |
| | | EP 2373163 A4 | 24 October 2012 |
| | | EP 2373163 B1 | 10 June 2015 |
| | | ES 2544452 T3 | 31 August 2015 |
| | | CL 2011001485 A1 | 24 February 2012 |
| | | RU 2011129776 A | 27 January 2013 |
| | | RU 2525116 C2 | 10 August 2014 |
| | | AU 2009335843 A1 | 21 July 2011 |
| | | ECSP 11011213 A | 30 September 2011 |
| | | KR 20110102473 A | 16 September 2011 |
| | | KR 101685718 B1 | 12 December 2016 |
| | | PE 20120334 A1 | 16 April 2012 |
| | | WO 2010080503 A1 | 15 July 2010 |
| | | DOP 2011000199 A | 15 October 2011 |
| | | US 2010210622 A1 | 19 August 2010 |
| | | US 8232273 B2 | 31 July 2012 |
| | | BRPI 0918360 A2 | 10 October 2017 |
| | | BRPI 0918360 A8 | 05 December 2017 |
| | | CA 2747170 A1 | 15 July 2010 |
| | | CA 2747170 C | 18 July 2017 |
| | | MX 2011006509 A | 19 October 2011 |
| | | CO 6390075 A2 | 29 February 2012 |

Form PCT/ISA/210 (patent family annex) (July 2022)

**INTERNATIONAL SEARCH REPORT**
**Information on patent family members**

International application No.
**PCT/CN2023/083593**

| Patent document cited in search report | | | Publication date (day/month/year) | Patent family member(s) | | | Publication date (day/month/year) |
|---|---|---|---|---|---|---|---|
| | | | | IL | 213596 | A0 | 31 July 2011 |
| | | | | ZA | 201104467 | B | 31 October 2012 |
| | | | | NZ | 593537 | A | 26 July 2013 |
| | | | | CR | 20110388 | A | 09 September 2011 |
| | | | | JP | 2012512891 | A | 07 June 2012 |
| | | | | JP | 5770102 | B2 | 26 August 2015 |
| | | | | SG | 172259 | A1 | 28 July 2011 |
| CN | 112469697 | A | 09 March 2021 | US | 2021299154 | A1 | 30 September 2021 |
| | | | | MX | 2021000395 | A | 12 May 2021 |
| | | | | CA | 3105982 | A1 | 16 January 2020 |
| | | | | SG | 11202012099 | VA | 28 January 2021 |
| | | | | EP | 3790861 | A1 | 17 March 2021 |
| | | | | EP | 3790861 | A4 | 30 March 2022 |
| | | | | WO | 2020014409 | A1 | 16 January 2020 |
| | | | | KR | 20210030905 | A | 18 March 2021 |
| | | | | US | 2020016185 | A1 | 16 January 2020 |
| | | | | US | 11026963 | B2 | 08 June 2021 |
| | | | | JP | 2021531249 | A | 18 November 2021 |
| | | | | IL | 279755 | A | 01 March 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **LOPEZ-OTIN et al.** *Cell*, 2013, vol. 153, 1194-1217
- **VAN DEURSEN et al.** *Nature*, 2014, vol. 509, 439-446
- **MCHUGH et al.** *J Cell Biol*, 2018, vol. 217, 65-77
- **COPPE et al.** *PLoS Biol*, 2008, vol. 6, 2853-2868
- **COPPE et al.** *Annu Rev Pathol*, 2010, vol. 5, 99-118
- **XU et al.** *Nat Med*, 2018, vol. 24, 1246-1256
- **BAKER et al.** *Nature*, 2011, vol. 479, 232-236
- **BAKER et al.** *Nature*, 2016, vol. 530, 184-189
- **KIRKLAND et al.** *J Am Geriatr Soc*, 2017, vol. 65, 2297-2301
- **LOZANO-TORRES et al.** *Nature Reviews Chemistry*, 2019, vol. 3, 426-441
- **ZHU et al.** *Aging Cell*, 2015, vol. 14, 644-658
- **CHANG et al.** *Nat Med*, 2016, vol. 22, 78-83
- **ZHU et al.** *Aging Cell*, 2016, vol. 15, 428-435
- **FUHRMANN-STROISSNIGG et al.** *Nat Commun*, 2017, vol. 8, 422
- **BAAR et al.** *Cell*, 2017, vol. 169, 132-147, e116
- **HE et al.** *Nat Commun*, 2020, vol. 11, 1996
- **CZABOTAR, P. E et al.** *Nat Rev Mol Cell Biol*, 2014, vol. 15, 49-63
- **CANG et al.** *Journal of Hematology & Oncology*, 2015, vol. 8, 129
- **HERNANDEZ-SEGURA et al.** *Trends in Cell Biology*, 2018, vol. 28, 436-453
- **ANA GUERRERO et al.** *Aging Cell*, 2020, vol. 19, e13133
- **DIMRI et al.** *PNAS*, 1995, vol. 92, 9363-9367
- **LEE et al.** *Aging Cell*, 2006, vol. 5, 187-195
- **LOZANO-TORRES et al.** *J Am Chem Soc*, 2017, vol. 139, 8808-8811
- **RAGHU G et al.** *Am J Respir Crit Care Med*, 2011, vol. 183, 788-824
- **WOLTERS PJ et al.** *Annu Rev Pathol*, 2014, vol. 9, 157-179
- **LEY B et al.** *Am J Respir Crit Care Med*, 2011, vol. 183, 431-440
- **HUTCHINSON JP et al.** *Annals of the American Thoracic Society*, 2014, vol. 11 (8), 1176-1185
- **RICHELDI L et al.** *N Engl J Med*, 2014, vol. 370, 2071-2082
- **KING TE et al.** *N Engl J Med*, 2014, vol. 370, 2083-2092
- **NOBLE PW et al.** *Lancet*, 2011, vol. 377, 1760-1769
- **FANER R et al.** *Am J Respir Crit Care Med*, 2012, vol. 186, 306-313
- **MARISSA J et al.** *Nat Commun*, 2017, vol. 8, 14532
- **KUWANO K et al.** *Am J Respir Crit Care Med*, 1996, vol. 154, 477-483
- **LOMAS N. J et al.** *Int. J. Clin. Exp. Pathol.*, 2012, vol. 5, 58-71
- **YANAI H et al.** *Aging*, 2015, vol. 7, 664-672
- **JIN PAN et al.** *Int J Radiat Oncol Biol Phys*, 2017, vol. 99 (2), 353-361
- **JAMIE N et al.** *EBioMedicine*, 2019, vol. 40, 554-563
- **IZBICKI G et al.** *Int. J. Exp. Pathol*, 2002, vol. 83, 111-119
- **KNUDTSON ; KLEIN et al.** *Arch Ophthalmol*, 2009, vol. 124 (2), 243-249
- **KNUDTSON ; KLEIN et al.** *Arch Gerontol Geriatr*, 2006, vol. 49 (1), 22-26
- **LITTLE et al.** *Ophthalmology*, 1985, vol. 92, 279-283
- **FINTAK et al.** *Retina*, 2008, vol. 28, 1395-1399
- **GRUNWALD et al.** *Ophthalmology*, 2014, vol. 121, 150-161
- **HOLZ FG et al.** *JAMA Ophthalmol.*, 2018, vol. 136 (6), 666-77
- **MARAZITA MC et al.** *Redox Biol.*, 2016, vol. 7, 78-87
- **SREEKUMAR PG et al.** *Investigative Ophthalmology & Visual Science*, 2016, vol. 57 (3), 1238-53
- **SUPANJI et al.** *Exp Eye Res.*, 2013, vol. 112, 79-92
- **CHAE JB et al.** *Geroscience*, 2021, vol. 43 (6), 2809-2833
- **CRESPO- GARCIA S et al.** *Cell Metab.*, 2021, vol. 33, 818-832
- **ROCHA LR et al.** *Aging Cell*, 2020, vol. 19 (2), e13089
- **SMITH, LE et al.** *Invest Ophthalmol Vis Sci*, 1994, vol. 35, 101-111
- **CONNOR, KM et al.** *Nat Protoc*, 2009, vol. 4, 1565-1573
- **GRISANTI, S et al.** *Prog Retin Eye Res*, 2008, vol. 27, 372-390

- **SIMO, R et al.** *Curr Diabetes Rev*, 2006, vol. 2, 71-98
- **STAHL, A et al.** *Invest Ophthalmol Vis Sci*, 2010, vol. 51, 2813-2826
- **OUBAHA M et al.** *Sci Transl Med.*, 2016, vol. 8 (362), 362ra144
- Handbook of Pharmaceutical Excipients. Pharmaceutical Press, 2003
- **CHILDS et al.** *Nat Med*, 2015, vol. 21 (12), 1424-1435
- **HERNANDEZ-SEGURA et al.** *Trends Cell Biol*, 2018, vol. 28 (6), 436-453
- **SHARPLESS & SHERR**. *Nat Rev Cancer*, 2015, vol. 15 (7), 397-408
- **BURSUKER** ; **RHODES** ; **GOLDMAN**. *J Cell Physiol*, 1982, vol. 112, 385-390
- **FRESCAS et al.** *Cell Cycle*, 2017, vol. 16, 1526-1533
- **HALL et al.** *Aging*, 2016, vol. 8, 1294-1315
- **OISHI** ; **MANABE**. *NPJ Aging Mech Dis*, 2016, vol. 2, 16018
- **MCHUGH et al.** *J. Cell Biol.*, 2018, vol. 217 (1), 65-77
- **DIMRI et al.** *Proc. Natl. Acad. Sci. USA*, 1995, vol. 92, 9363-9367
- **HEBERT et al.** *Arch. Neurol.*, 2003, vol. 60, 119-1122
- **PEPEU**. *Dialogues in Clinical Neuroscience*, 2004, vol. 6, 369-377
- **TCHKONIA et al.** *Aging Cell*, 2010, vol. 9, 667-684
- **MINAMINO et al.** *Nat. Med.*, 2009, vol. 15, 1082-087
- **XIE, B. et al.** *Cell Res.*, 2019, vol. 29, 696-710
- **BIVAS-BENITA et al.** *Eur J Pharm Biopharm*, 2005, vol. 61, 214-218